# EUROPEAN PATENT SPECIFICATION

(11) **EP 1 625 154 B1**
(45) Date of publication and mention of the grant of the patent: **08.01.2014**
(21) Application number: 04731118.8
(22) Date of filing: 05.05.2004
(51) Int. Cl.: C07K 14/18, C12N 9/12

(54) **HEPATITIS C VIRUS NS5B POLYMERASE INHIBITOR BINDING POCKET**
BINDUNGSSTELLE FÜR INHIBITOREN DER NS5B POLYMERASE DES HEPATITIS C VIRUS
POCHE DE LIAISON DE L'INHIBITEUR DE LA POLYMERASE NS5B DU VIRUS DE L'HEPATITE C

(30) Priority: 09.05.2003 US 469604 P
(43) Date of publication of application: 15.02.2006
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: COULOMBE, René, Laval, Quebec H7S 2G5 (CA); BEAULIEU, Pierre, Louis, Laval, Quebec H7S 2G5 (CA); JOLICOEUR, Eric, Laval, Quebec H7S 2G5 (CA); KUKOLJ, George, Laval, Quebec H7S 2G5 (CA); LAPLANTE, Steven, Laval, Quebec H7S 2G5 (CA); POUPART, Marc-André, Laval, Quebec H7S 2G5 (CA)
(74) Representative: Simon, Elke Anna Maria
(86) International application number: PCT/CA2004/000676
(87) International publication number: WO 2004/099241

(56) References cited:
- EP-A- 1 256 628
- WO-A-03/007945
- LABONTE PATRICK ET AL: "Modulation of hepatitis C virus RNA-dependent RNA polymerase activity by structure-based site-directed mutagenesis." JOURNAL OF BIOLOGICAL CHEMISTRY, vol. 277, no. 41, 11 October 2002 (2002-10-11), pages 38838-38846, XP002291702 ISSN: 0021-9258 cited in the application
- BRESSANELLI S ET AL: "STRUCTURAL ANALYSIS OF THE HEPATITIS C VIRUS RNA POLYMERASE IN COMPLEX WITH RIBONUCLEOTIDES" JOURNAL OF VIROLOGY, THE AMERICAN SOCIETY FOR MICROBIOLOGY, US, vol. 76, no. 7, April 2002 (2002-04), pages 3482-3492, XP001088855 ISSN: 0022-538X
- AGO H ET AL: "Crystal structure of the RNA-dependent RNA polymerase of hepatitis C virus" STRUCTURE, CURRENT BIOLOGY LTD., PHILADELPHIA, PA, US, vol. 7, 1 November 1999 (1999-11-01), pages 1417-1426, XP002253342 ISSN: 0969-2126 cited in the application
- BRESSANELLI S ET AL: "CRYSTAL STRUCTURE OF THE RNA-DEPENDENT RNA POLYMERASE OF HEPATITIS C VIRUS" PROCEEDINGS OF THE NATIONAL ACADEMY OF SCIENCES OF USA, NATIONAL ACADEMY OF SCIENCE. WASHINGTON, US, vol. 96, no. 23, 9 November 1999 (1999-11-09), pages 13034-13039, XP002931586 ISSN: 0027-8424 cited in the application
- LESBURG C A ET AL: "CRYSTAL STRUCTURE OF THE RNA-DEPENDENT RNA POLYMERASE FROM HEPATITIS C VIRUS REVEALS A FULLY ENCIRCLED ACTIVE SITE" NATURE STRUCTURAL BIOLOGY, NEW YORK, NY, US, vol. 6, no. 10, October 1999 (1999-10), pages 937-943, XP000957743 ISSN: 1072-8368 cited in the application

## Description

### FIELD OF THE INVENTION

The present invention relates to Hepatitis C Virus NS5B polymerase (HCV NS5B), and in particular, to a novel HCV NS5B inhibitor binding pocket. The crystal structure of HCV NS5B is provided including the coordinates that define the novel inhibitor-binding pocket and 3-dimensional models for use in inhibitor screening. Methods of designing and screening NS5B inhibitors are also provided utilizing the crystal structure and inhibitor-binding information.

### BACKGROUND OF THE INVENTION

Hepatitis C virus (HCV) is the major etiological agent of post-transfusion and community-acquired non-A non-B hepatitis worldwide. It is estimated that over 200 million people worldwide are infected by the virus. A high percentage of carriers become chronically infected and many progress to chronic liver disease, so called chronic hepatitis C. This group is in turn at high risk for serious liver disease such as liver cirrhosis, hepatocellular carcinoma and terminal liver disease leading to death.

The mechanism by which HCV establishes viral persistence and causes a high rate of chronic liver disease has not been thoroughly elucidated. It is not known how HCV interacts with and evades the host immune system. In addition, the roles of cellular and humoral immune responses in protecting against HCV infection and disease have yet to be established.

HCV is an enveloped positive strand RNA virus in the *Flaviviridae* family. The single strand HCV RNA genome is of positive polarity and comprises one open reading frame (ORF) of approximately 9600 nucleotides in length, which encodes a linear polyprotein of approx. 3010 amino acids. In infected cells, this polyprotein is cleaved at multiple sites by cellular and viral proteases to produce structural and non-structural (NS) proteins. The structural proteins (C, E1, E2 and E2-p7) comprise polypeptides that constitute the virus particle. The non-structural proteins (NS2, NS3, NS4A, NS4B, NS5A, NS5B) encode for enzymes or accessory factors that catalyze and regulate the replication of the HCV RNA genome. Processing of the structural proteins is catalyzed by host cell proteases. The generation of the mature non-structural proteins is catalyzed by two virally encoded proteases. The first is the NS2/3 zinc-dependent metalloprotease which auto-catalyzes the release of the NS3 protein from the polyprotein. The released NS3 protein contains an N-terminal serine protease domain and catalyzes the remaining cleavages from the polyprotein. The released NS4A protein has at least two roles. The first role is forming a stable complex with NS3 protein and assisting in the membrane localization of the NS3/NS4A complex; the second is acting as a cofactor for NS3 protease activity. This membrane-associated complex, in turn catalyzes the cleavage of the remaining sites on the polyprotein, thus effecting the release of NS4B, NS5A and NS5B. The C-terminal segment of the NS3 protein also harbors nucleoside triphosphatase and RNA helicase activity. The function of the protein NS4B is unknown. NS5A is a highly phosphorylated protein that appears to be responsible for the interferon resistance of various HCV genotypes. NS5B is an RNA-dependent RNA polymerase (RdRp) that is involved in the replication of HCV.

The open reading frame of the HCV RNA genome is flanked on its 5' end by a non-translated region (NTR) of approx. 340 nucleotides that functions as the internal ribosome entry site (IRES), and on its 3' end by an NTR of approximately 230 nucleotides. Both the 5' and 3' NTRs are important for RNA genome replication. The genomic sequence variance is not evenly distributed over the genome and the 5'NTR and parts of the 3'NTR are the most highly conserved portions.

The cloned and characterized partial and complete sequences of the HCV genome have been analyzed with regard to appropriate targets for a prospective antiviral therapy. The following four viral enzyme activities provide possible targets: (1) the NS2/3 protease; (2) the NS3/4A protease complex, (3) the NS3 helicase and (4) the NS5B RNA-dependent RNA polymerase (NS5B RdRp). The NS5B RNA dependent RNA polymerase has also been crystallized to reveal a structure reminiscent of other nucleic acid polymerases (Ago *et al.* **1999;** Bressanelli *et al.* **1999;** Lesburg *et al.* **1999**) with an enclosed active site.

Virus-specific functions essential for replication are the most attractive targets for drug development. The absence of RNA dependent RNA polymerases in mammals, and the fact that this enzyme appears to be essential to viral replication, would suggest that the HCV NS5B polymerase is an ideal target for anti-HCV therapeutics. It has recently been demonstrated that mutations destroying NS5B activity abolish infectivity of HCV RNA in a chimp model (Kolykhalov, A.A. *et al.* **2000**). The initial step of viral RNA replication is recognition of the 3'-end of RNA template by NS5B (RdRp), which may occur directly or indirectly with the help of cellular proteins (Lai, **1998;** Strauss *et al.*, **1999**). HCV polymerase then proceeds to elongate this template and form a complementary RNA product.

Several groups have described the crystal structure of HCV NS5B polymerase (Ago *et al.* **1999** *supra;* Bressanelli *et al.* **1999** *supra;* Lesburg *et al.* **1999** *supra*)*.* It resembles a flattened sphere with the approximate dimensions 70Å x 60Å x 40Å. The polypeptide chain encircles the active site, forming a cavity at the center of the molecule, and resulting in an appearance that is very different from other U-shaped polymerases. The domain organization of NS5B is consistent with other polymerases in that it is subdivided into finger, palm and thumb domains in which the palm domain, i.e. residues 188-225 and 287-370, is conserved. In contrast to other polymerases, extensive thumb and finger domain contacts result in a globular-shaped HCV polymerase. These contacts are mediated, in part, by loops that extend from the finger to the thumb domain. Knowledge of the crystal structure of NS5B is useful for structure-based drug design and, indeed, structures of NS5B polymerase/inhibitor complexes have been reported recently (Wang *et al.* **2003;** Love et al, **2003;** EP 1 256 628). Non-nucleoside analogue inhibitors were found to bind in a wedge-like fashion to a hydrophobic binding pocket located near the C-terminal region of the polymerase thumb domain. In this study, the enzyme was determined to undergo only minor conformational changes in the enzyme/inhibitor complex. At least two NTP binding sites have been characterized on NS5B, one in the active site palm and a second potential allosteric site on the thumb (O'Farrell *et al.* **2003;** Bressanelli *et al.* **2002**).

Interestingly, Labonté *et al.* **2002** have reported that a mutation of Leu30 in the N-terminal finger loop of the NS5B affects its polymerase activity and speculate that a local alteration in the structure of the Leu30 mutant is responsible for this decrease in activity. However, the authors are silent on the presence of a binding pocket that is "masked" by the finger loop in its native state and becomes exposed by a mutation or displacement of the Leu30 residue. The discovery of this peculiar binding pocket is the subject-matter of the present invention. Accordingly, the effort to develop effective treatments to HCV infection can be facilitated by increased knowledge of the structure of enzymes critical to HCV replication, most notably, the NS5B polymerase. An increased knowledge of enzyme structure, particularly when complexed with specific inhibitors, will lead to a means of identifying binding sites in the enzyme, as well as the conformation of enzyme/inhibitor complexes and susceptible residues in the enzyme, knowledge of each of which is critical to the process of drug design and optimization.

### SUMMARY OF THE INVENTION

It has now been found that the HCV polymerase, when complexed with certain inhibitors, adopts a conformation in which the finger loop region defined by amino acid residues 18 to 35 is displaced to expose a binding pocket defined generally by amino acid residues 392, 393, 395, 396, 399, 424, 425, 428, 429, 492, 493, 494, 495, 500 and 503. This is in contrast to NS5B crystal structures disclosed in the prior art in which the finger loop defined by amino acid residues 18 to 35 conceals this binding pocket in its native state. This newly "exposed" binding pocket defines a novel target in the search of further chemical entities which are capable of binding to HCV NS5B and modulating, or preferably inhibiting, the polymerase activity of HCV NS5B.

The present invention provides the method of claim 1.

Aspects and embodiments of the present invention are described in more detail herein by reference to the accompanying drawings in which:

### BRIEF DESCRIPTION OF THE DRAWINGS

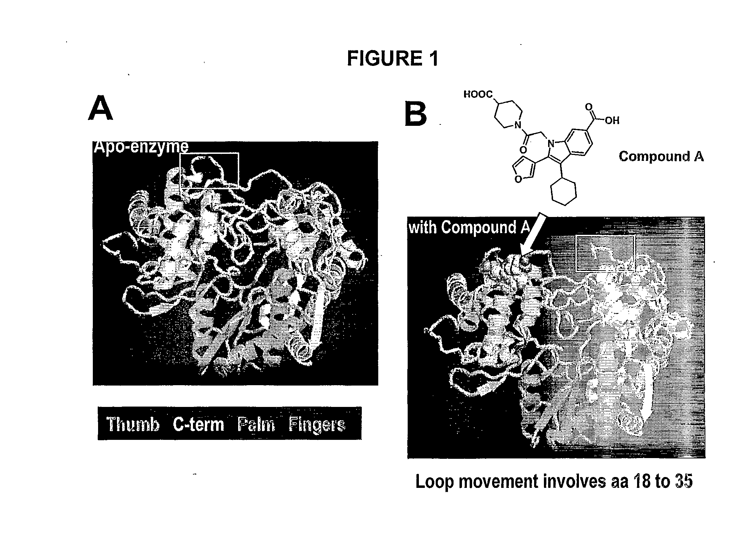

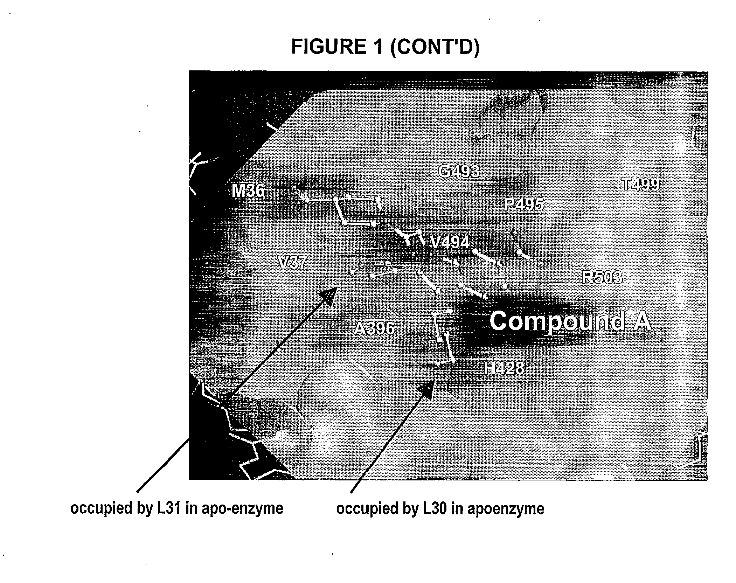

Figure **1A** depicts the NS5B apo-enzyme structure without compound;
Figure **1** **B** depicts compound **A** bound to the NS5B where amino acids 18-35 are displaced and not seen in the structure;
Figure **1C** depicts compound **A** bound within a solvent accessible surface representation of the binding pocket of the present invention;

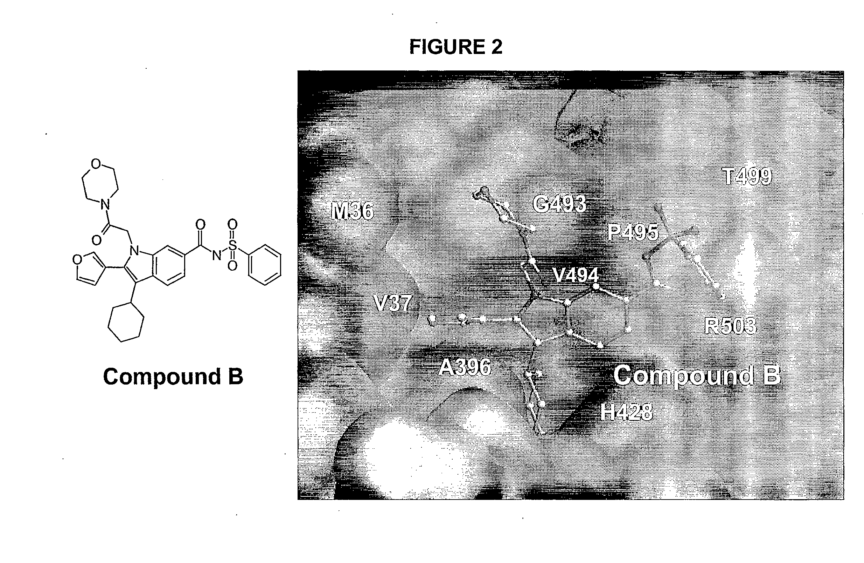

Figure **2** depicts compound **B** bound within a solvent accessible surface representation of the binding pocket of the present invention;

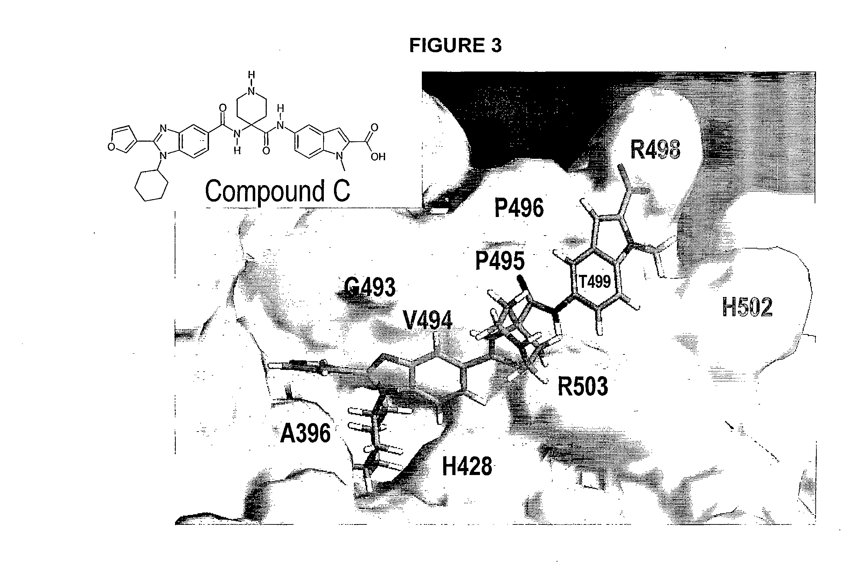

Figure **3** depicts a docked model of the NMR derived bound conformation of compound **C** bound within the accessible representation of the binding pocket of the present invention;
Figure 4 shows the atomic structure coordinates of the HCV NS5B of SEQ ID No. 1 complexed with the compound **A ;**
Figure **5** shows the atomic structure coordinates of the HCV NS5B of SEQ ID No.1 complexed with the compound **B**
Figure **6** shows the atomic structure coordinates of the HCV NS5B of SEQ ID No.1 complexed with the compound **C**

### DETAILED DESCRIPTION OF THE INVENTION

### Definitions

Unless defined otherwise, the scientific and technological terms and nomenclature used herein have the same meaning as commonly understood by a person of ordinary skill to which this invention pertains. Generally, the procedures for cell culture, infection, molecular biology methods and the like are well-known to those of skill in the art. Such standard techniques can be found in reference manuals such as for example Sambrook *et al.* (1989) and Ausubel *et al.* (1994).

The term "HCV NS5B" refers to the RNA-dependent RNA polymerase (RdRp) of a Hepatitis C virus (HCV) that is involved in the replication of HCV. As one of skill in the art is well aware, the term HCV encompasses a viral family including many different strains, isolates and subtypes. Moreover, the NS5B polymerase of each member of this family, although functionally equivalent and highly homologous structurally, will vary somewhat in amino acid sequence. The amino acid sequence of one HCV NS5B polymerase, NS5B from HCV genotype 1b, is shown in SEQ ID No: 1. The sequences of other isolated HCV NS5B polymerases may be accessed in publicly available sequence databases.

The term "binding pocket", as used herein, refers to a region of a molecule or molecular complex, that, as a result of its configuration, favorably associates with or is occupied by an entity or region of the same molecule or molecular complex, or an entity or region of a different molecule, molecular complex, chemical compound or other compound. In accordance with the present invention, the NS5B binding pocket defined herein becomes exposed by displacement of the finger loop domain of residues 18-35 thereby allowing binding of a compound that is capable of affecting NS5B activity, for example, inhibiting NS5B activity. Typically, a binding pocket, or at least a portion thereof, comprises a cavity which is the site of interaction with an entity of the same or different molecule. As will be appreciated by those of skill in the art, the nature of the cavity within a binding pocket will vary from molecule to molecule.

The term "isolated and purified" as it is used with respect to polypeptides according to the present invention refers to a polypeptide that is substantially free from other components.

The term "native HCV NS5B", as it is used herein with respect to the amino acid sequence of the binding pocket of the present invention, refers to the natural amino acid sequence of the binding pocket in a given HCV NS5B.

The term "native functional configuration" as it is used with respect to the binding pocket of the present invention refers to the natural arrangement, including spatial arrangement, of amino acids that form a pocket that can associate with or be occupied by certain compounds/entities.

As used herein the term "complex" refers to the combination of two or more entities, at least one of which is a protein or enzyme. In particular, complexes in accordance with the present invention are formed between an NS5B protein, including analogs thereof which may include amino acid substitutions, truncations or insertions, and another compound. The combination or "complexing" of a compound or chemical entity with a protein refers to the nature of the association/binding between the compound or chemical entity and the protein. The association between the components of the complex is the condition of proximity therebetween and may be non-covalent in nature, wherein the juxtaposition is energetically favored by hydrogen bonding, van der Waals forces or electrostatic interactions, or it may be covalent.

The term "analog" as used herein denotes, with respect to a molecular compound, a sequence of amino acids modified from the native or natural sequence of amino acids that retains the biological activity (either functional or structural) of the native sequence of amino acids. This analog may be from the same or different species and may be a natural analog or may be prepared synthetically. Such analogs include amino acid sequences having substitutions, deletions, or additions of one or more amino acids, provided that the biological activity of the protein is conserved. In particular, the term "conservative analog" denotes an analog having amino acid modifications that retain biological activity. Analogs including amino acid substitutions may include substitutions having either strong or weak similarity (see, for example, Dayhoff, M.O., (1978), Atlas of Protein Sequence and Structure, 5, suppl. 3, National Biomedical Research Foundation, Washington, D.C.) as defined according to the following "Table of Amino Acid Similarity":

| **Amino acid** | **Strong** | **Weak** |
|---|---|---|
| A | G, S | C, T, V |
| C | | A, S |
| D | E | G, H, K, N, Q, R, S |
| E | D | H, K, N, Q, R, S |
| F | W, Y | H, I, L, M |
| G | A | D, N, S |
| H | Y | D, E, F, K, N, Q, R |
| I | L, M, V | F |
| K | R | D, E, H, N, Q, S, T |
| L | I, M, V | F |
| M | I, L, V | F |
| N | Q | D, E, G, H, K, R, S, T |
| P | | S, T |
| Q | N | D, E, H, K, R, S |
| R | K | D, E, H, N, Q |
| S | A, T | C, D, E, G, K, N, P, Q |
| T | S | A, K, N, P, V |
| V | I, L, M | A, T |
| W | F, Y | |
| Y | F, H, W | |

The meaning of the term "functionally equivalent analog" as it is used herein with respect to the binding pocket polypeptide of the invention refers to substitutions, deletions or insertions of one or more of the amino acids of the binding pocket. Substitutions may be made as set out above, for example, with an appropriate conservative amino acid or conservative synthetic amino acid analog. In essence, the term "analog" corresponds with the foregoing definition. The phrase "functionally equivalent" indicates that the analog retains the biological activity of the native molecule.

The term "side chain" with reference to an amino acid or amino acid residue means a group attached to the α-carbon atom of the α-amino acid. For example, the R-group side chain for glycine is hydrogen, for alanine it is methyl, for valine it is isopropyl. For the specific R-groups or side chains of the α-amino acids reference is made to A.L. Lehninger's text on Biochemistry (see chapter 4).

The term "truncation" refers to any shortened or abbreviated segment of a molecule which, for the purposes of the present invention, retains its biological activity. Truncation may refer to the shortening of a native protein molecule, or to an analog thereof.

The term "root mean square deviation" or "rms deviation" or "rmsd" means the square root of the arithmetic mean of the square of the deviations from the mean. In the context of atomic objects, the numbers are given in angstroms (Å). It is a way to express the deviation or variation from a trend or object.

The following abbreviations are used herein:
DLB: differential line broadening;
DMSO: dimethyl sulfoxide;
DTT: dithiothreitol;
EDTA: ethylenediaminetetraacetic acid;
FID: free induction decay
IPTG: isopropyl-beta-D-thiogalactopyranoside
HEPES: 4-(2-hydroxyethyl)-1-piperazineethanesulfonic acid
MES: 2-(N-morpholino)ethanesulfonic acid,
MPD: 2-methyl-2,4-pentanediol
NMR: nuclear magnetic resonance;
NOESY: Nuclear Overhauser Effect Spectroscopy
PEG: polyethylene glycol;
PEG5k mme: monomethyl ether polyethylene glycol 5000;
Tris: tris(hydroxymethyl)aminomethane;
TSP: sodium 3-trimethylsilyltetradeuteriopropionate.

The present invention provides a method of identifying compounds that can bind to HCV NS5B, comprising the steps of:
a) applying a 3-dimensional molecular modeling algorithm to the structural coordinates of an HCV NS5B binding pocket defined by at least amino acid residues 392, 393, 395, 396, 399, 424, 425, 428, 429, 492, 493, 494, 495, 500 and 503 (and optionally one of: amino acid residues 37 and 496) of a native HCV NS5B (SEQ ID NO. 1) to determine the spatial coordinates of the binding pocket of HCV NS5B; and
b) electronically screening stored spatial coordinates of candidate compounds against the spatial coordinates of the HCV NS5B binding pocket to identify compounds that can bind within the HCV NS5B binding pocket.

In preferred embodiments of this aspect of the invention, the binding pocket may be defined additionally by one or more of amino acid residues 36, 426, 498 and 499, or by one or more of the amino acid clusters defined by amino acid residues 36-37, 392-399, 424-429 and 492-503.

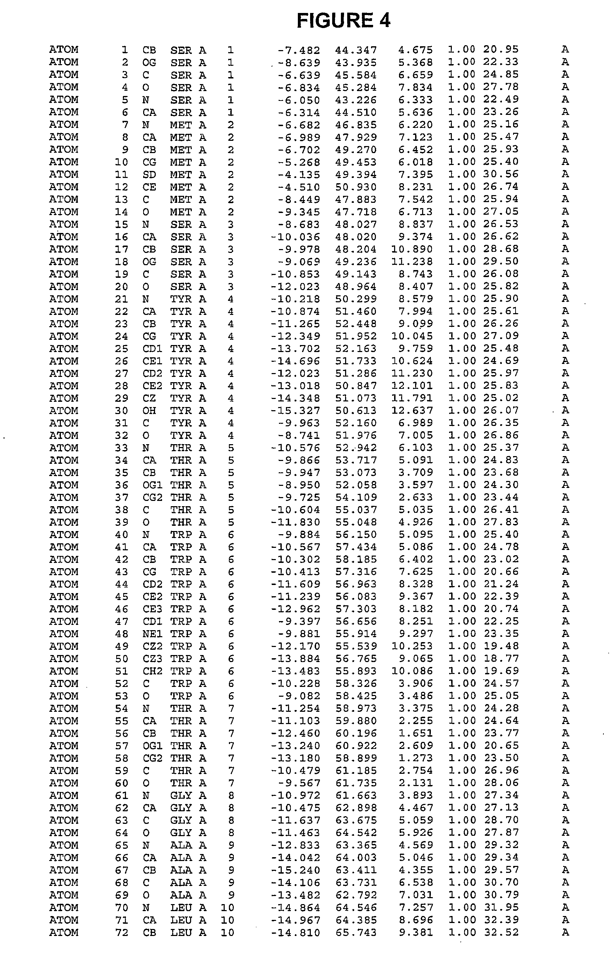

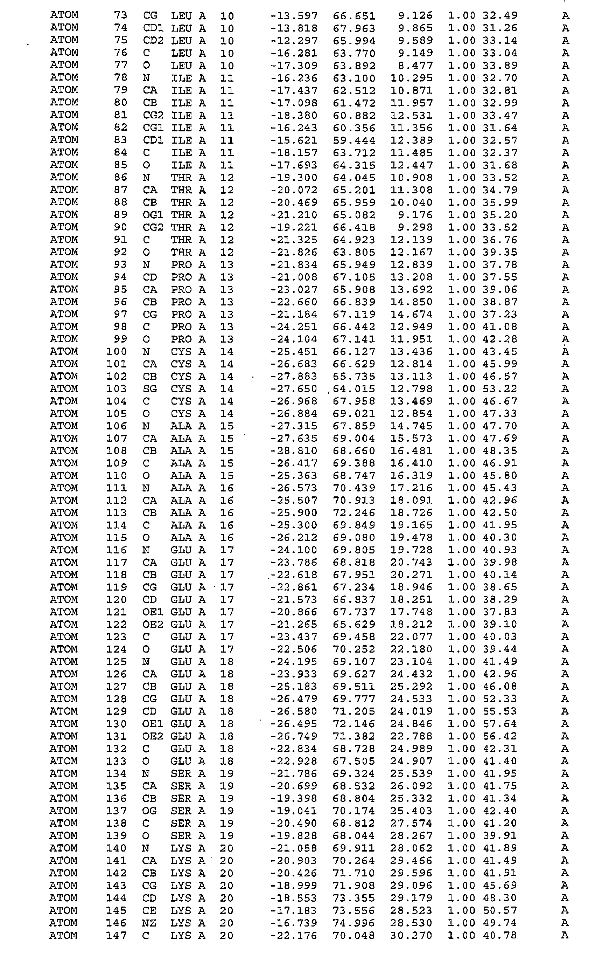

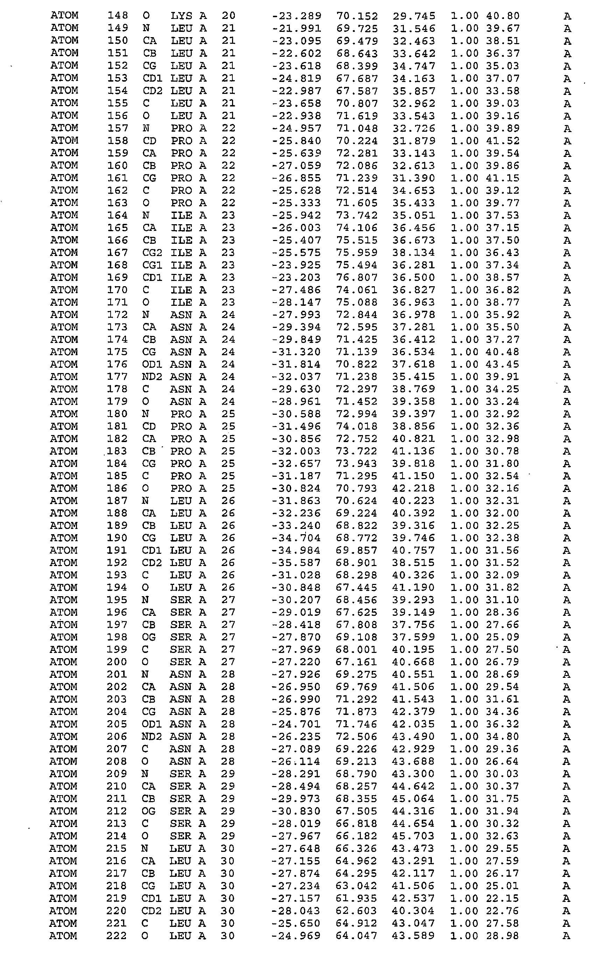

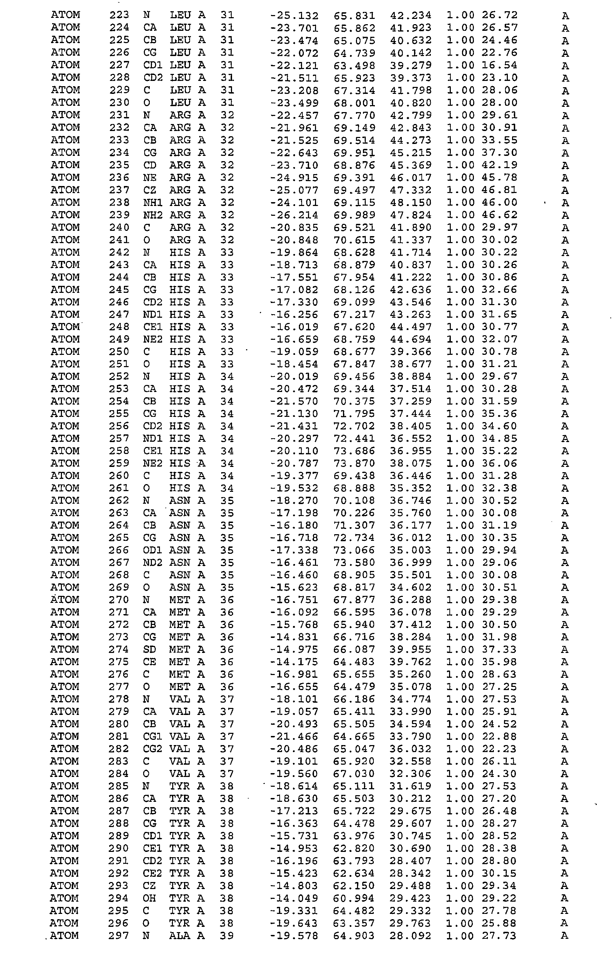

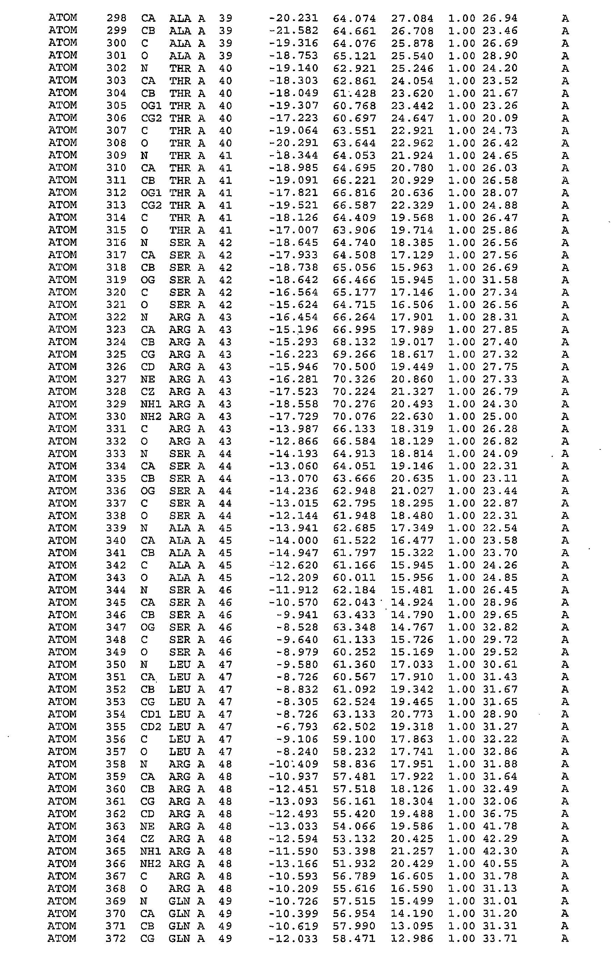

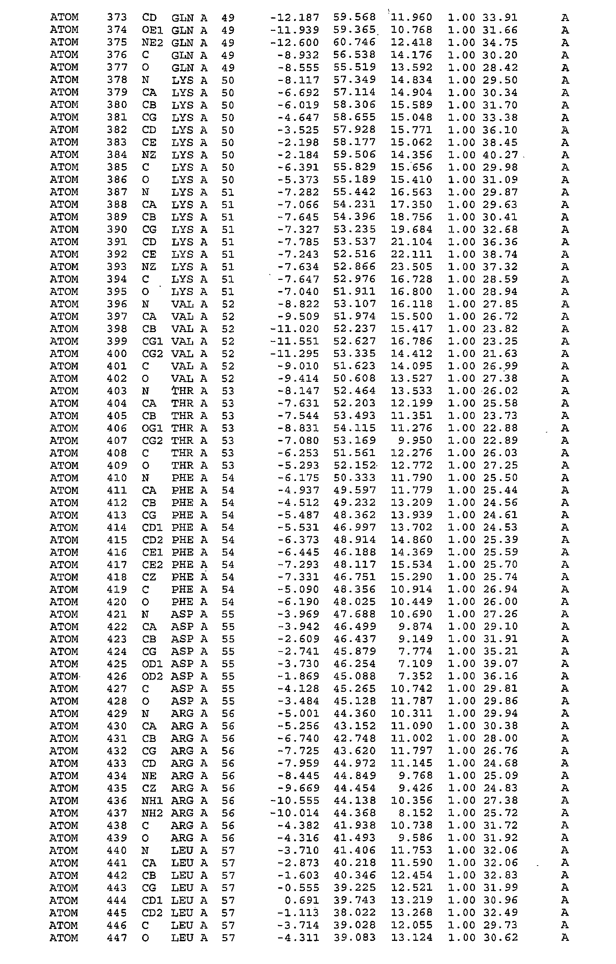

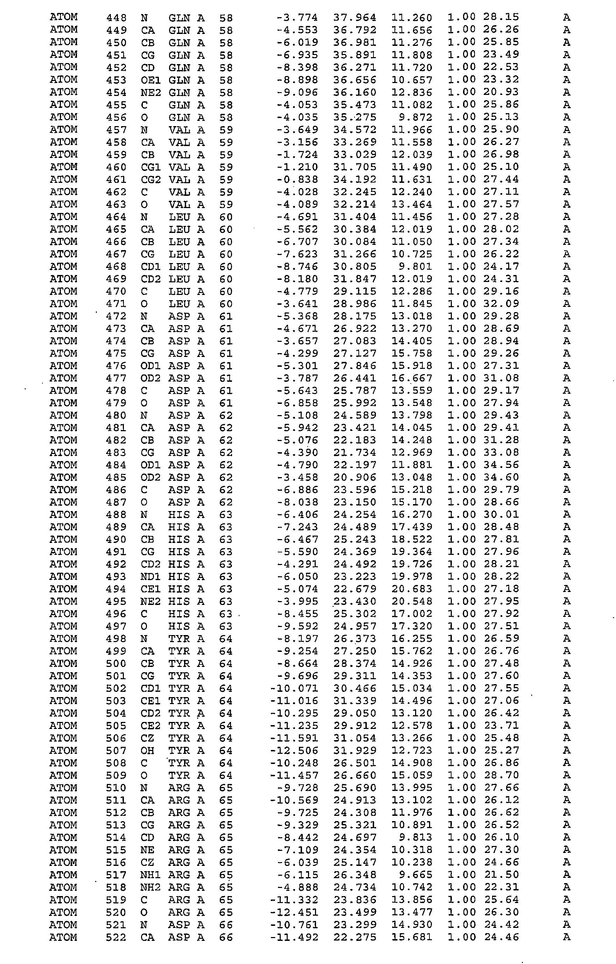

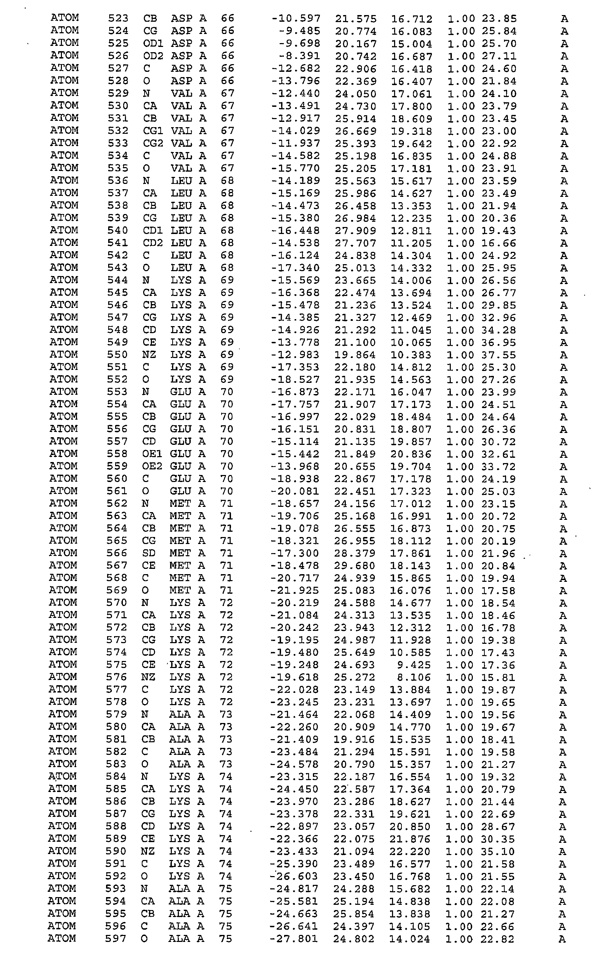

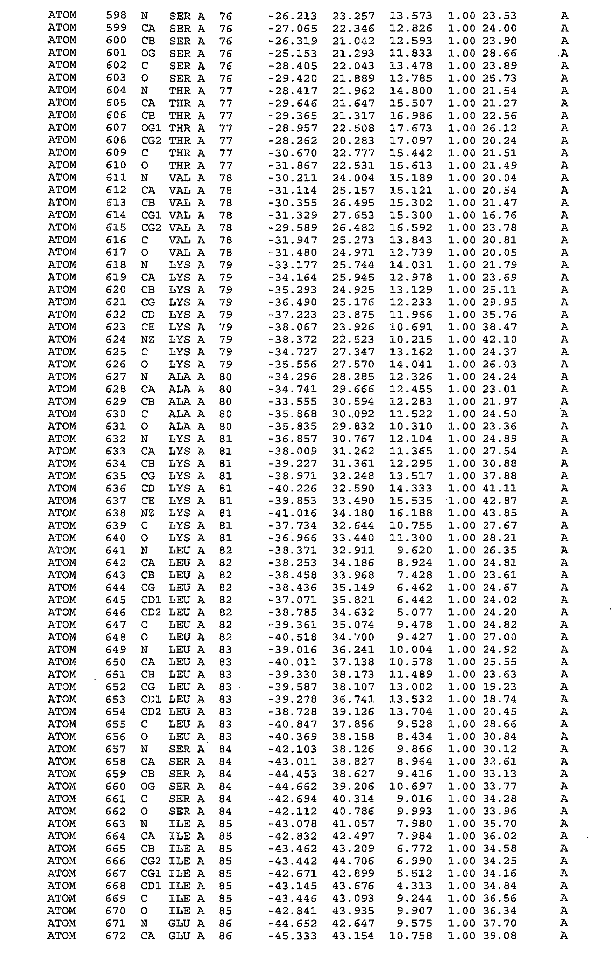

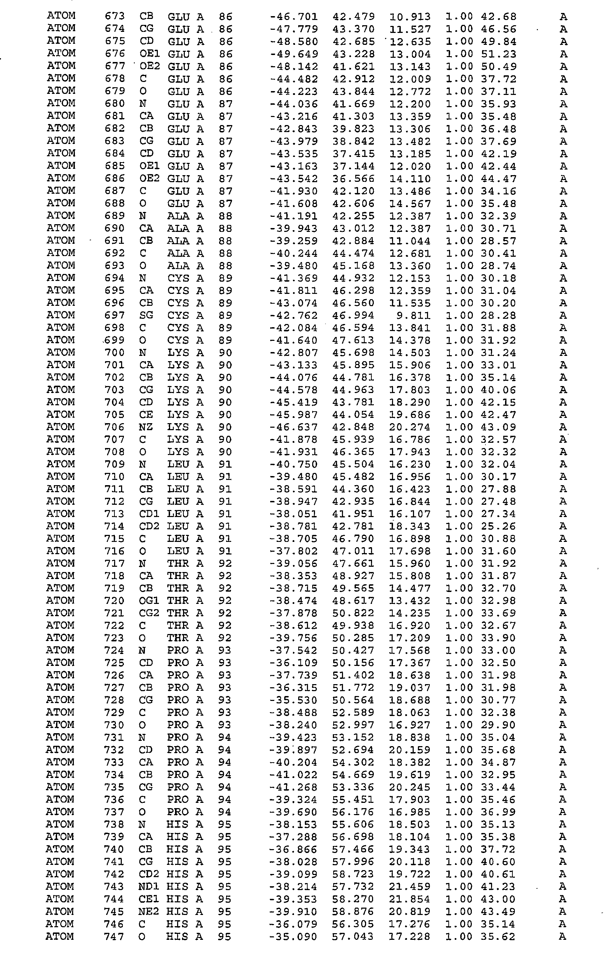

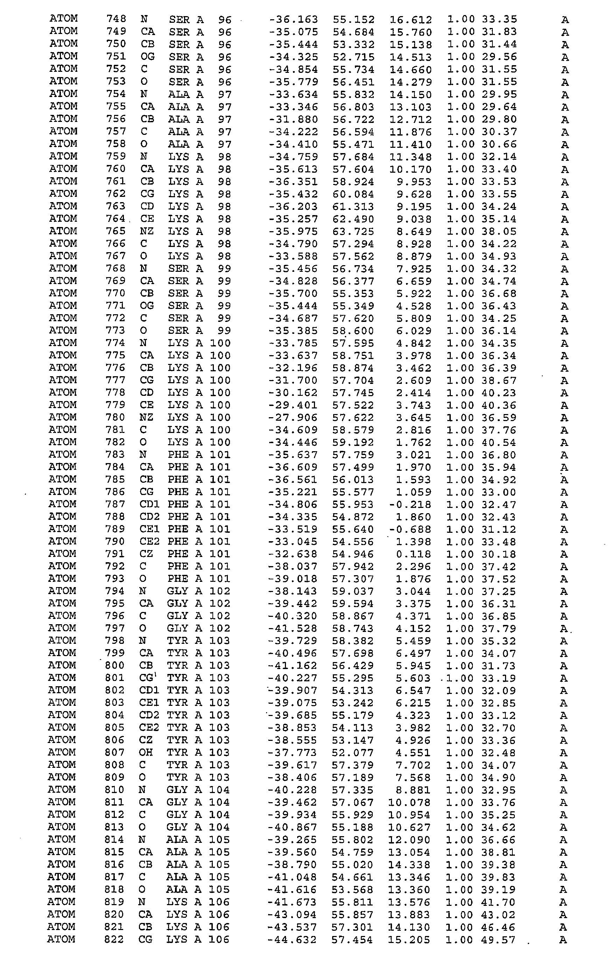

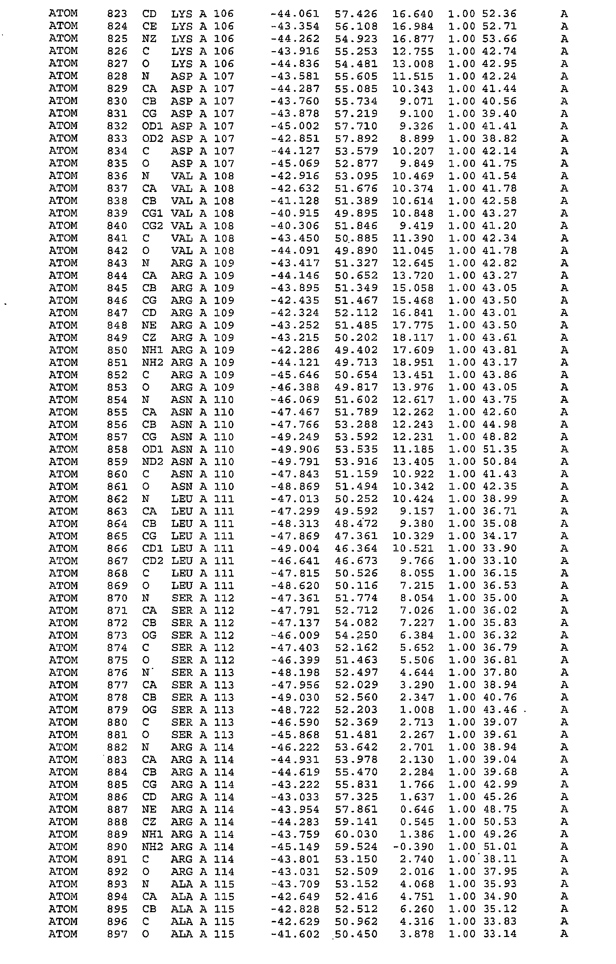

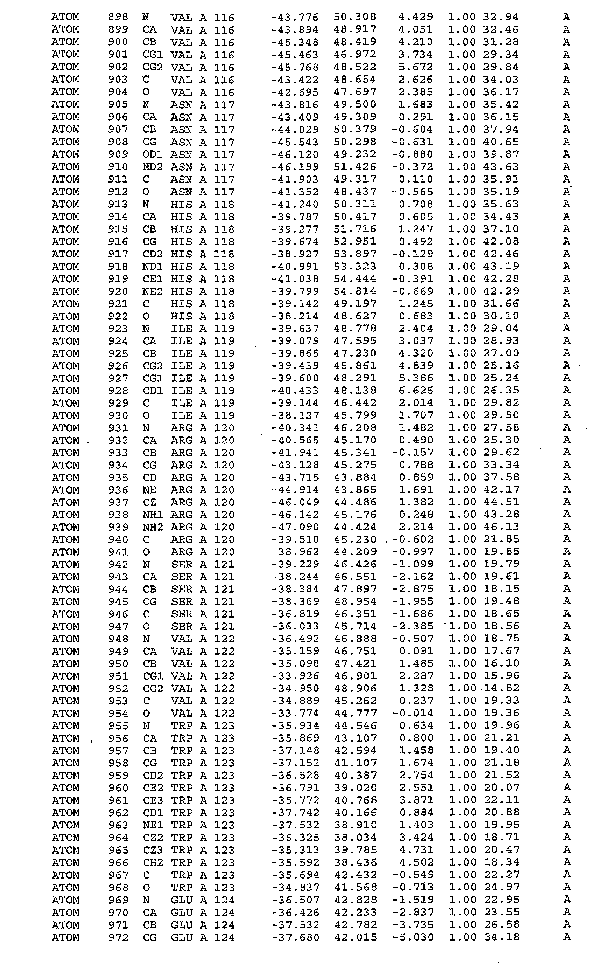

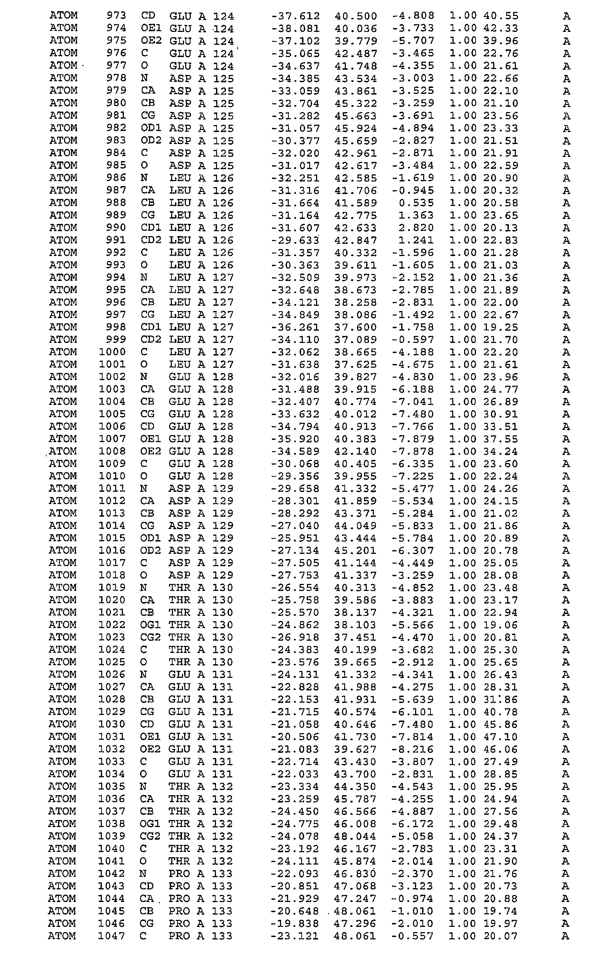

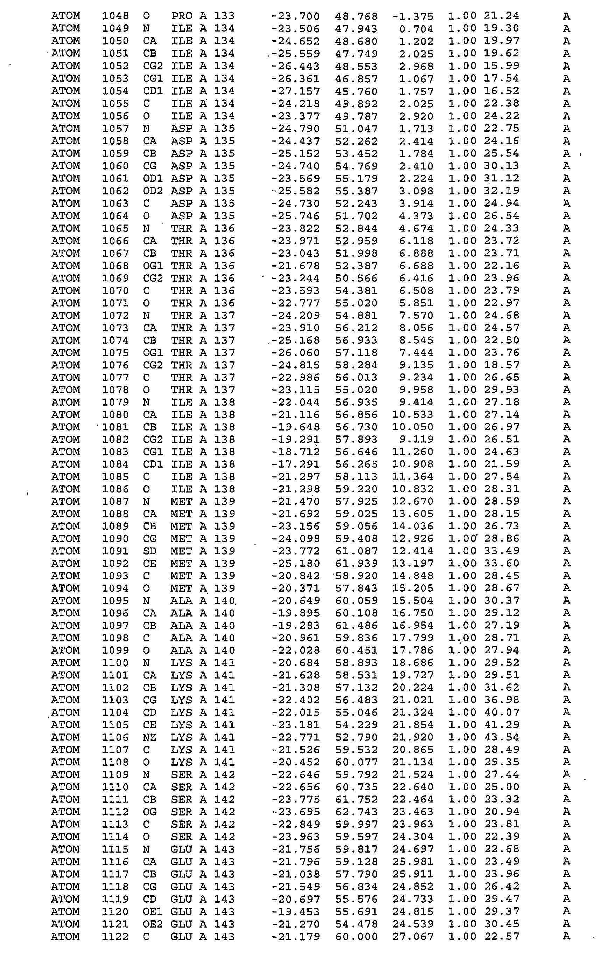

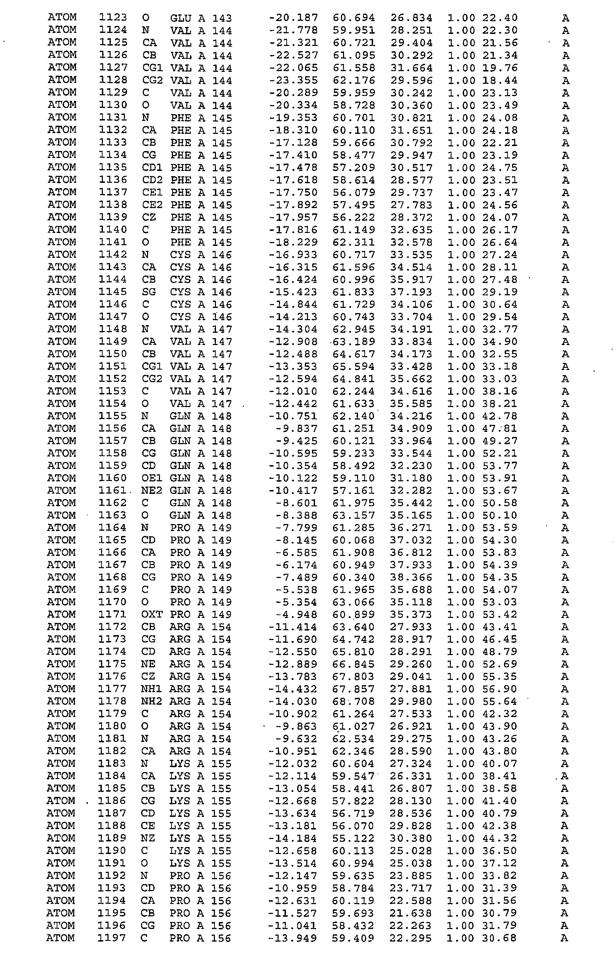

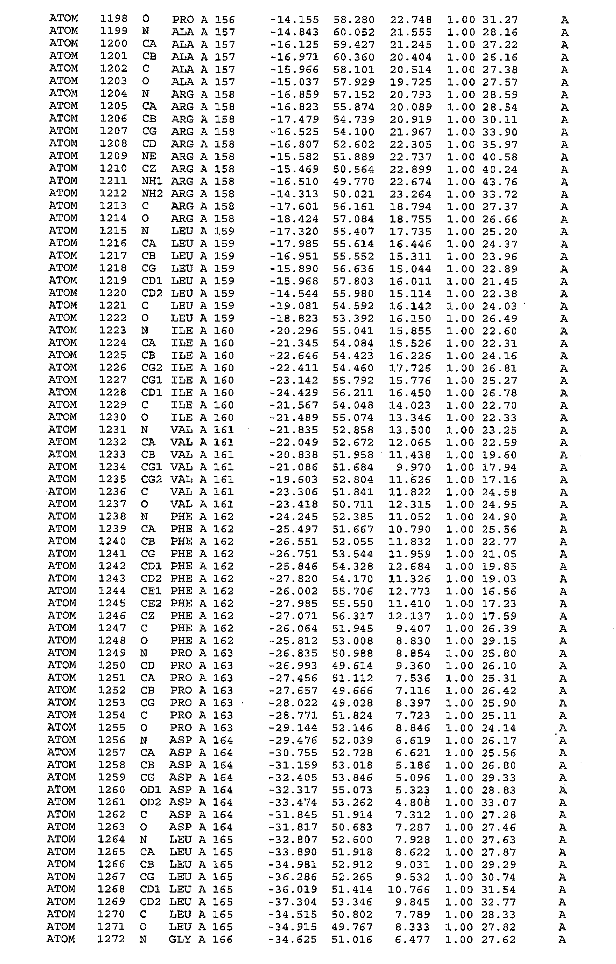

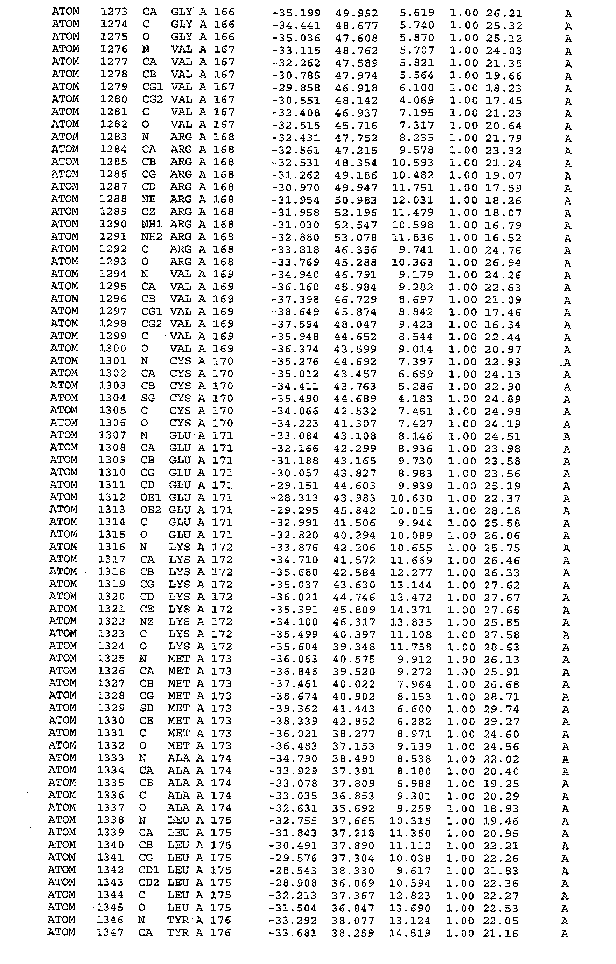

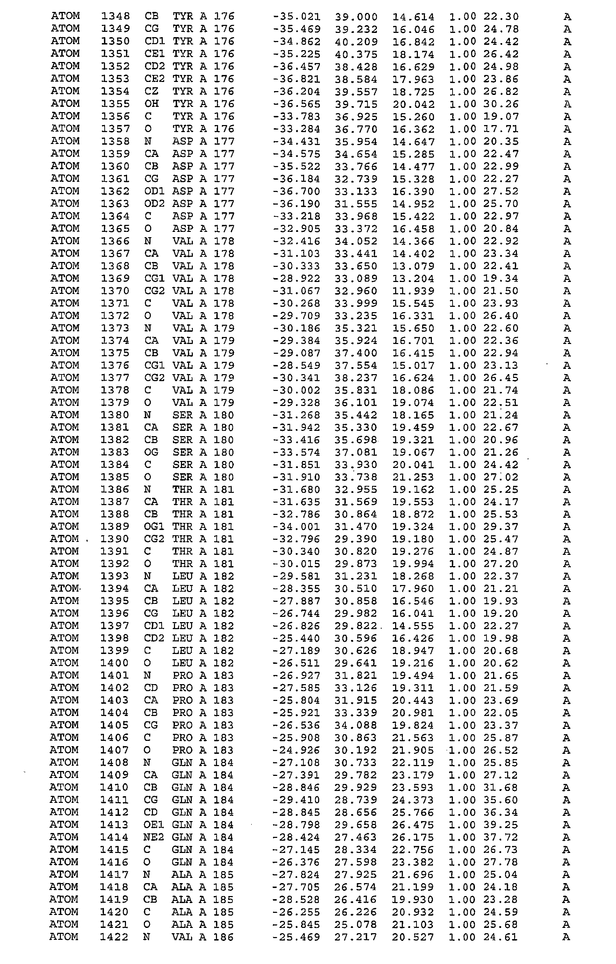

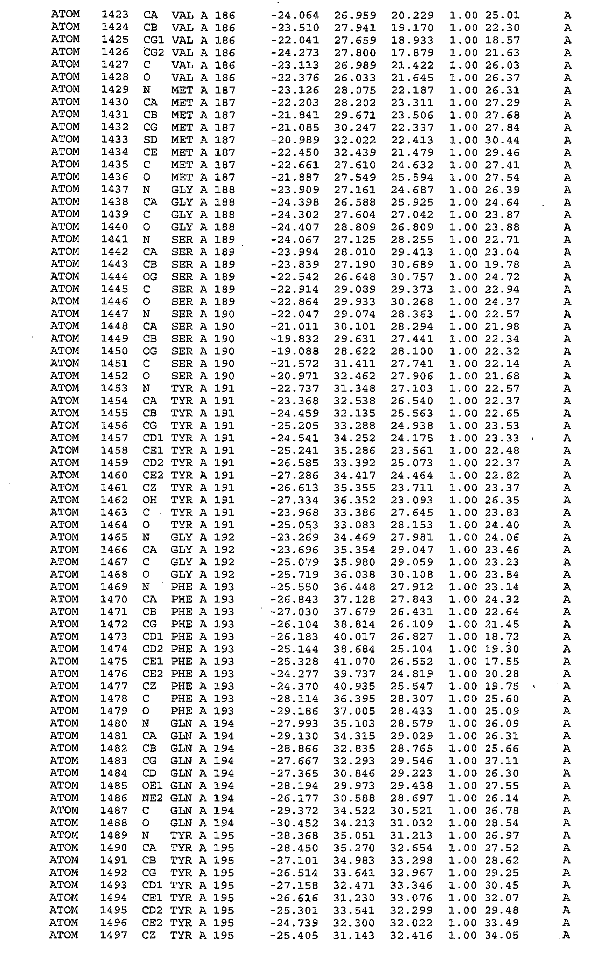

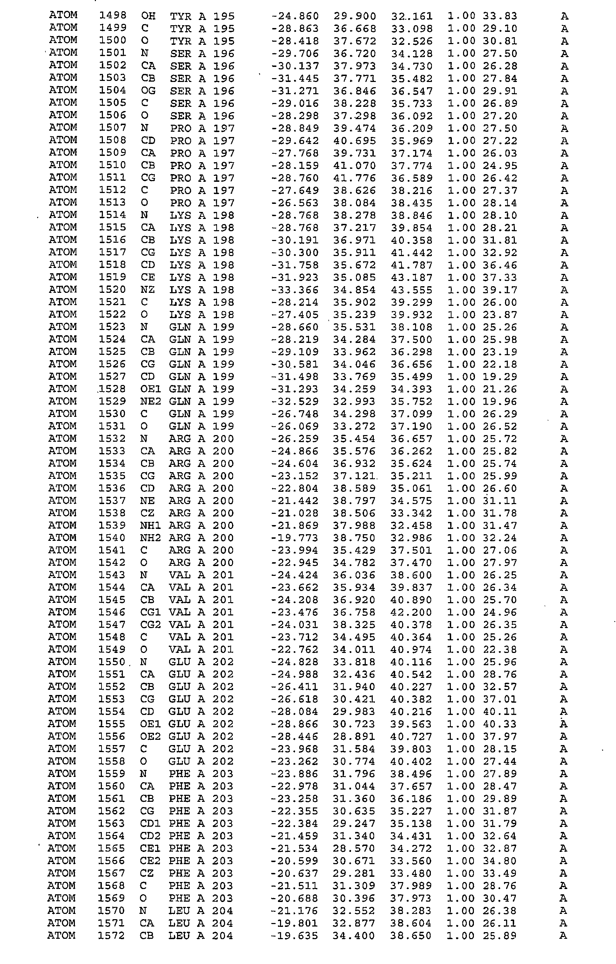

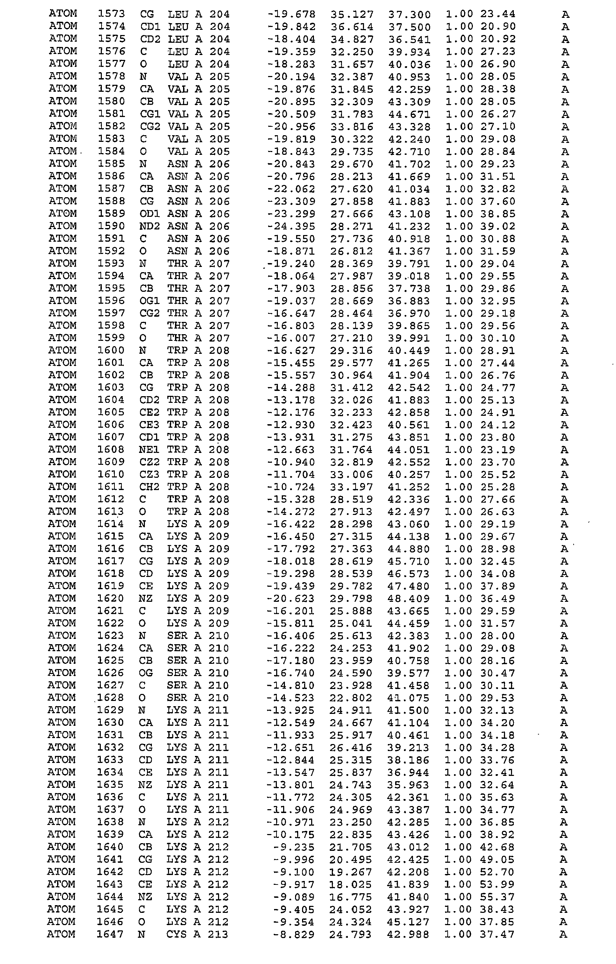

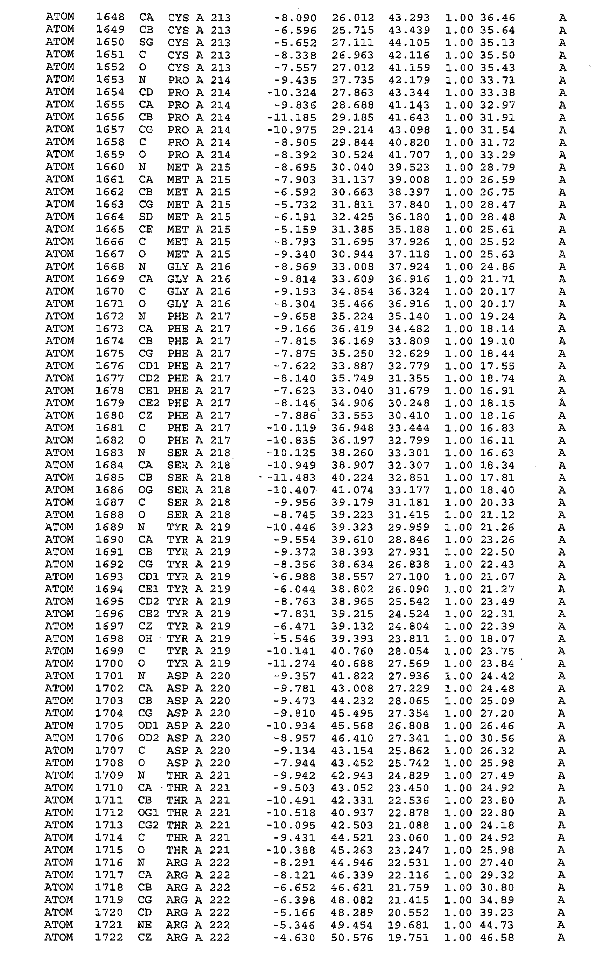

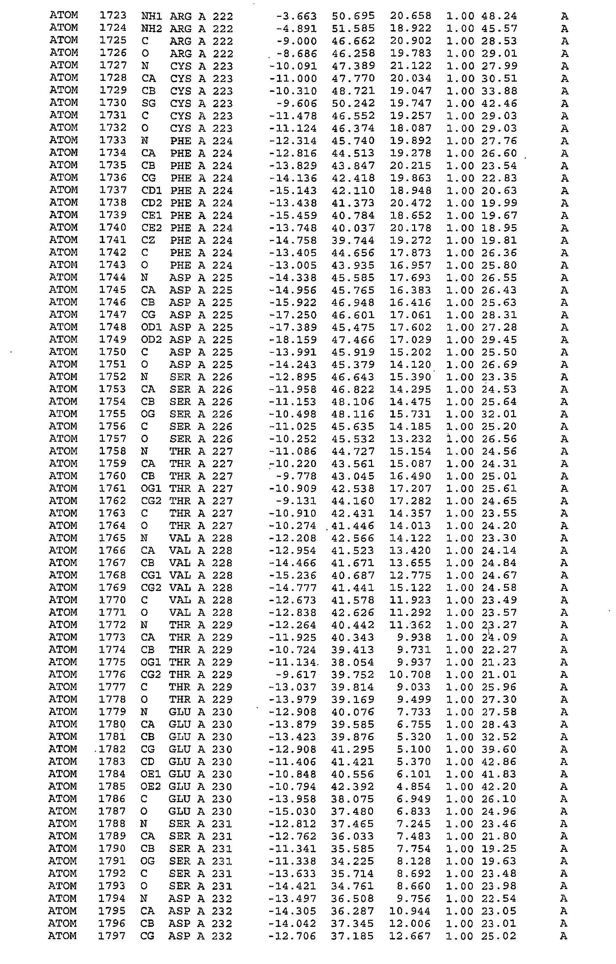

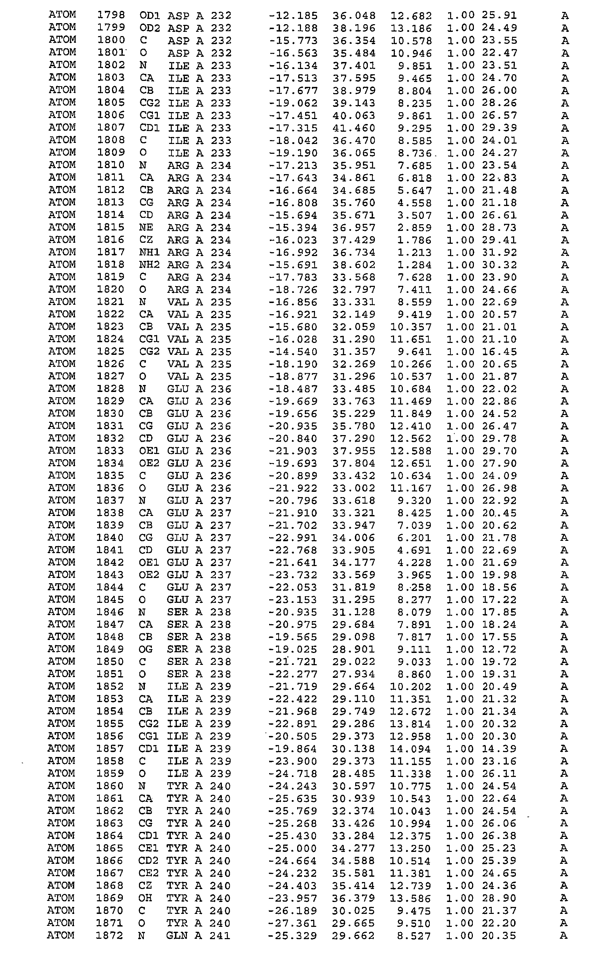

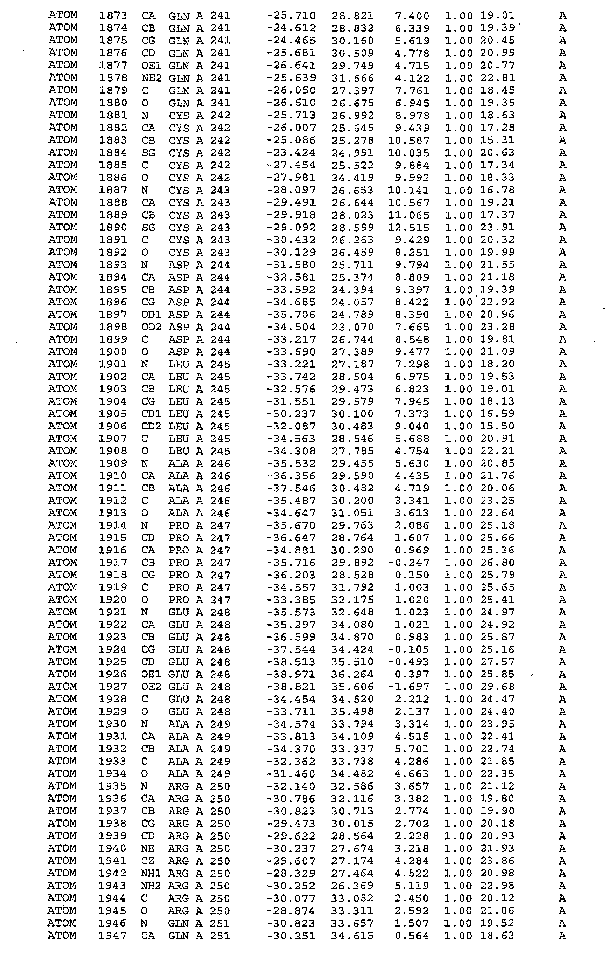

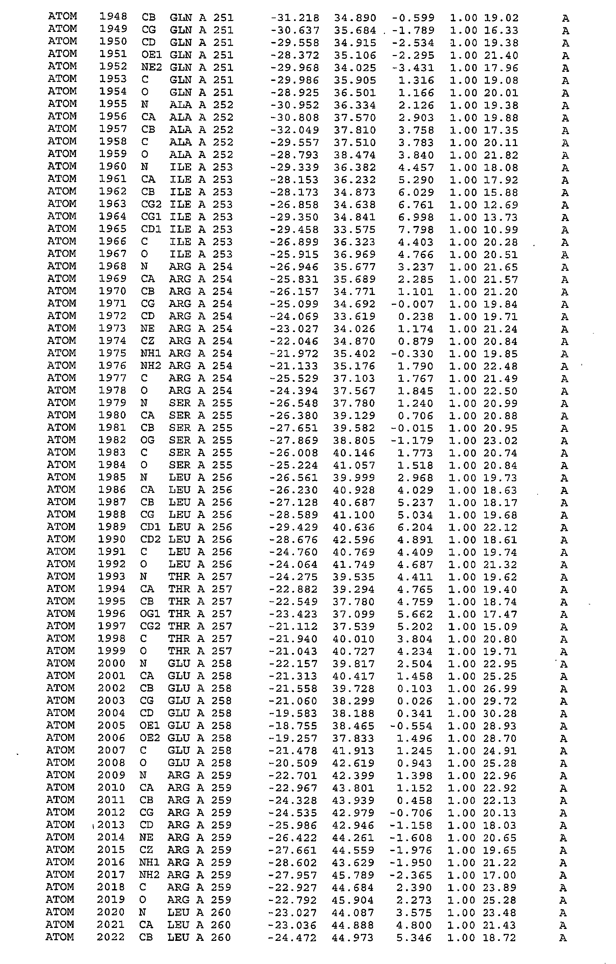

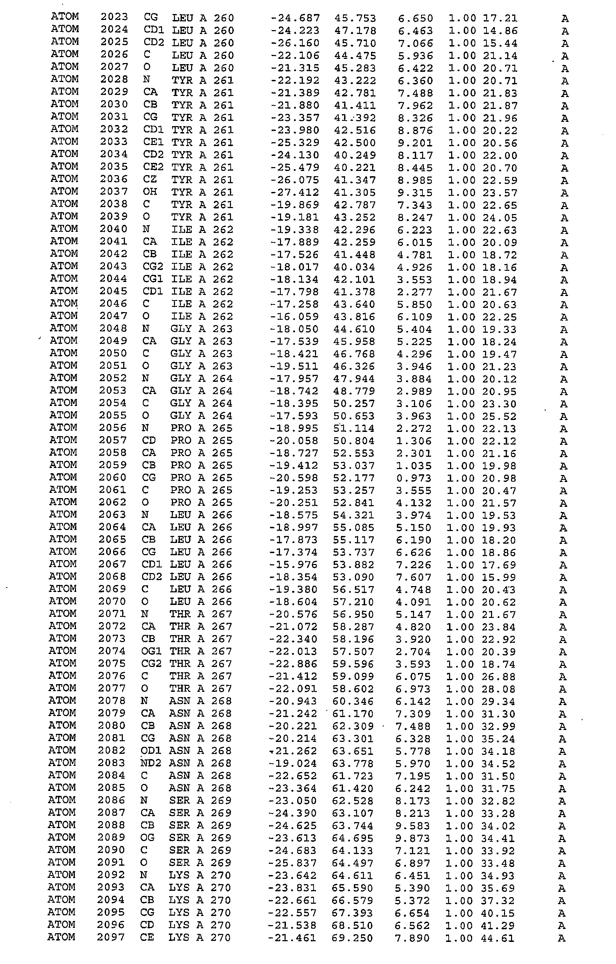

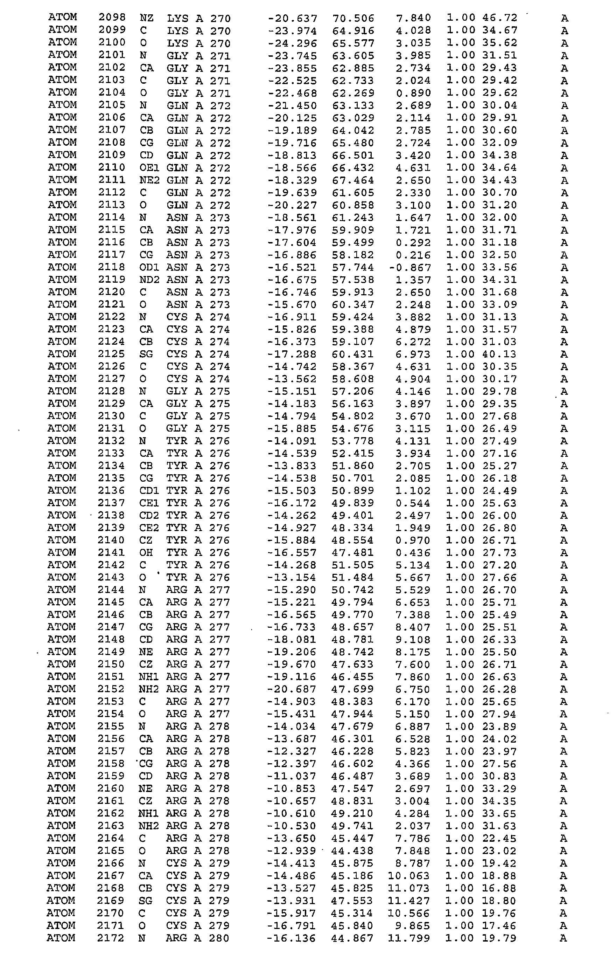

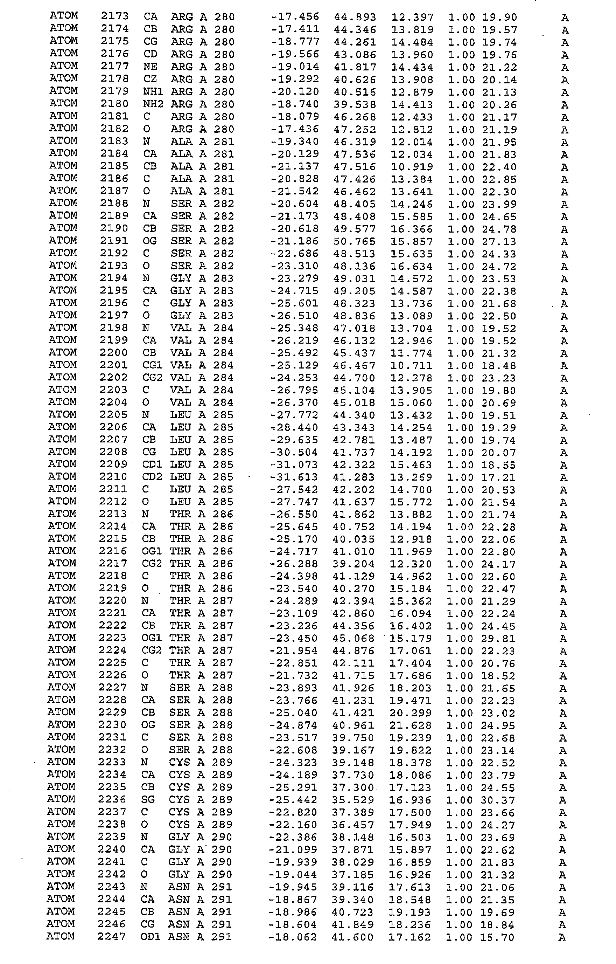

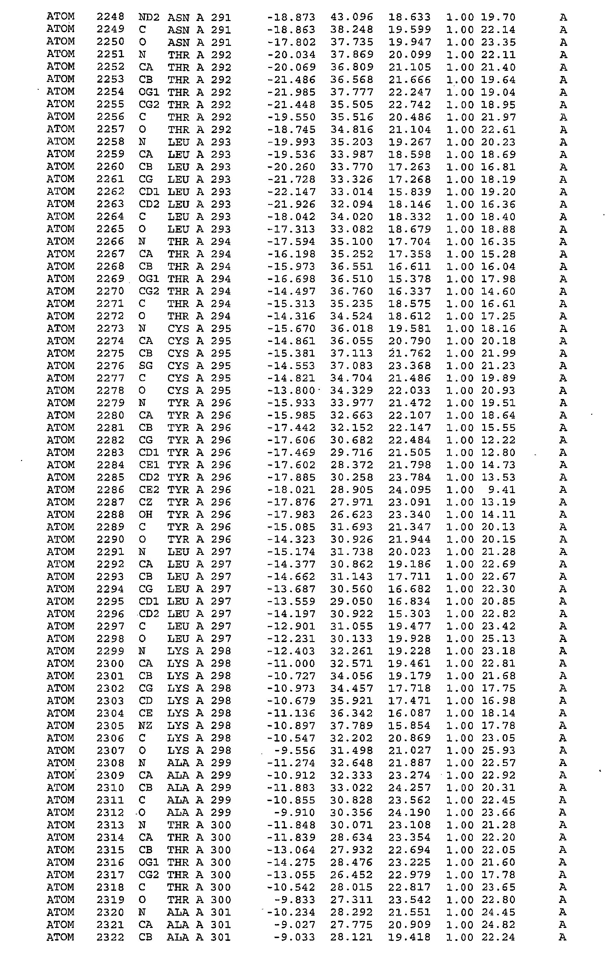

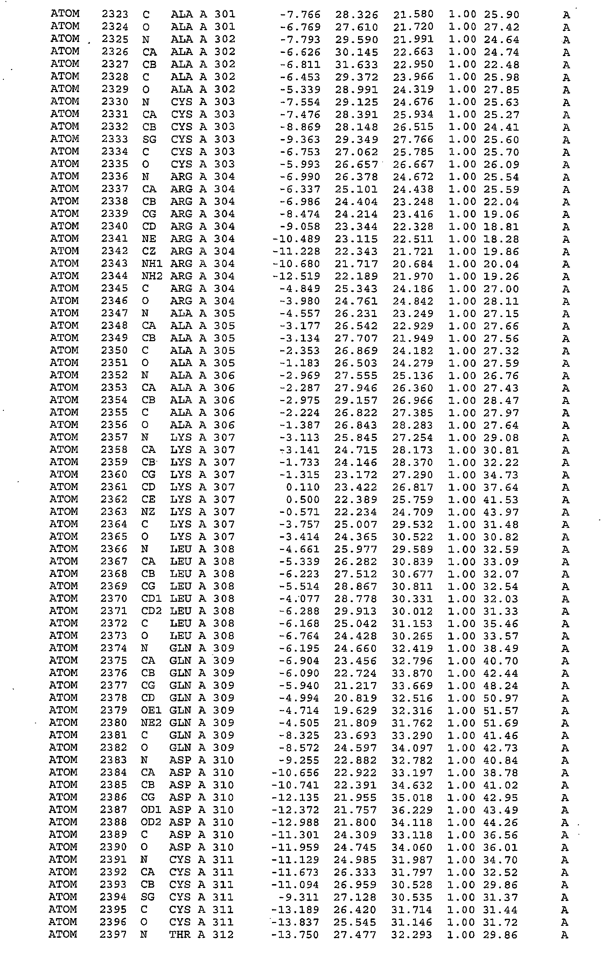

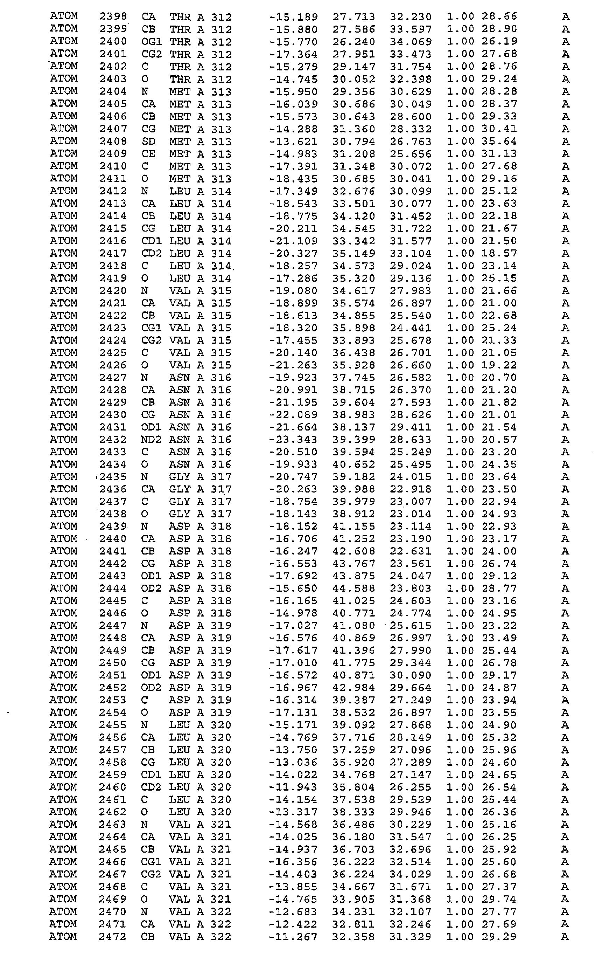

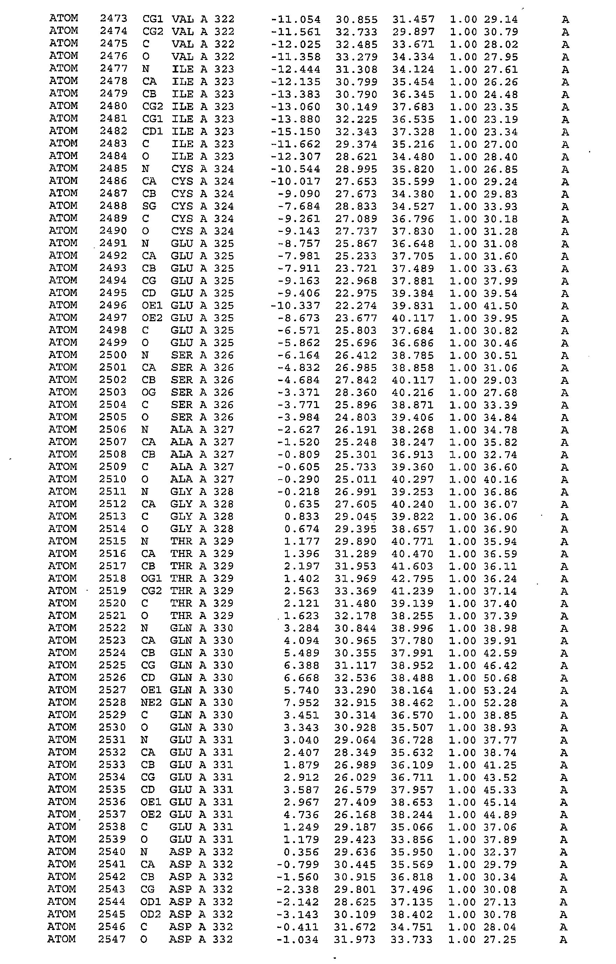

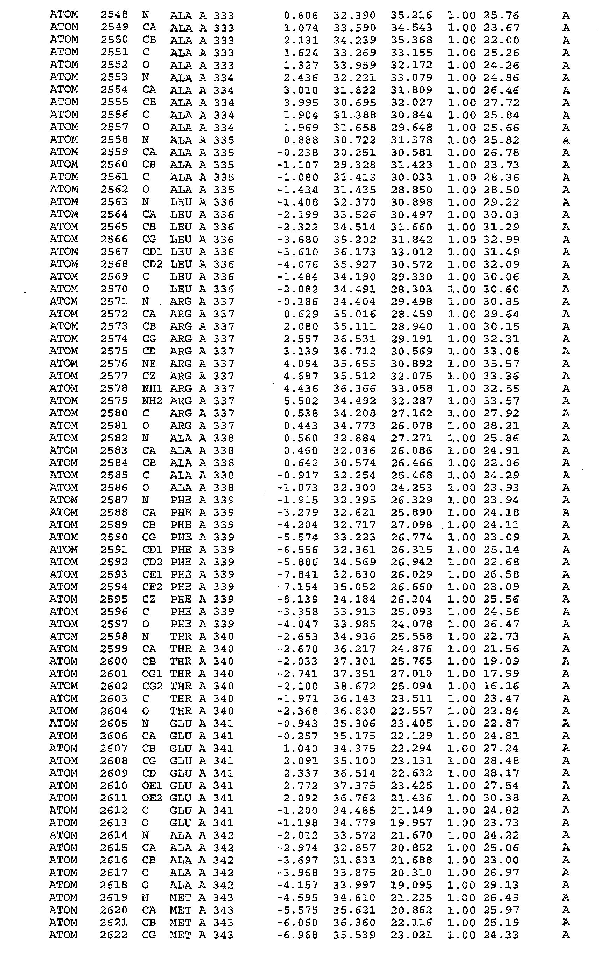

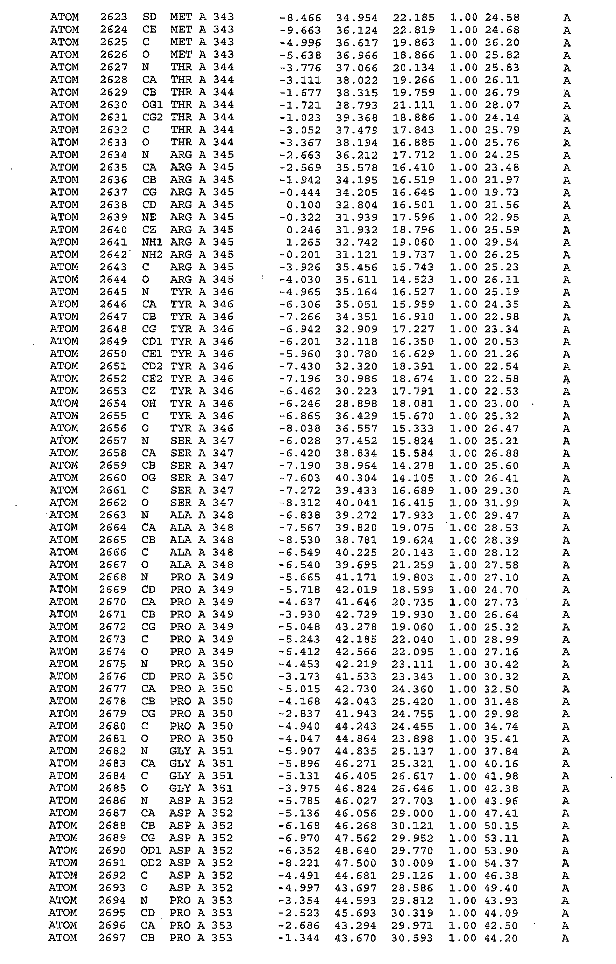

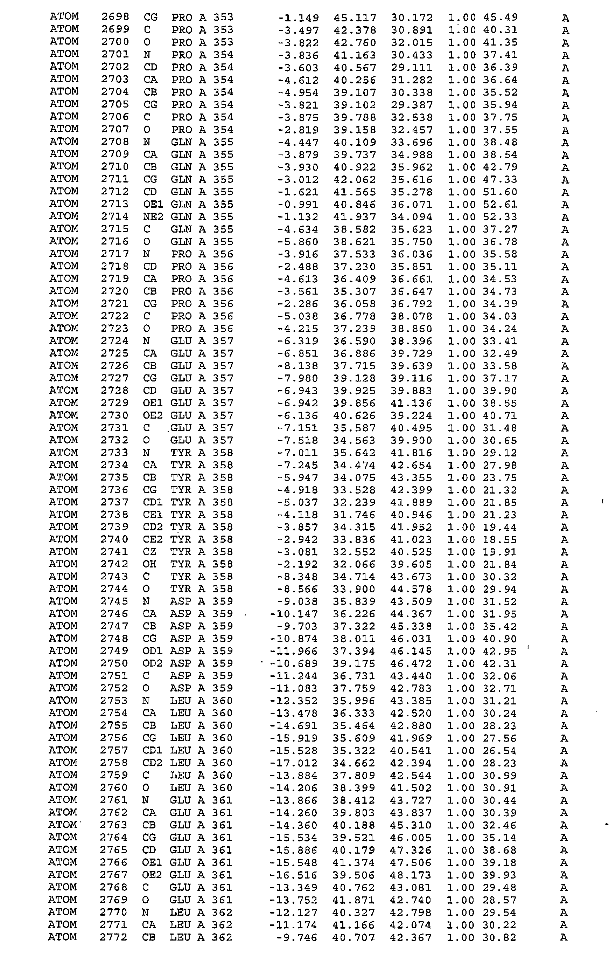

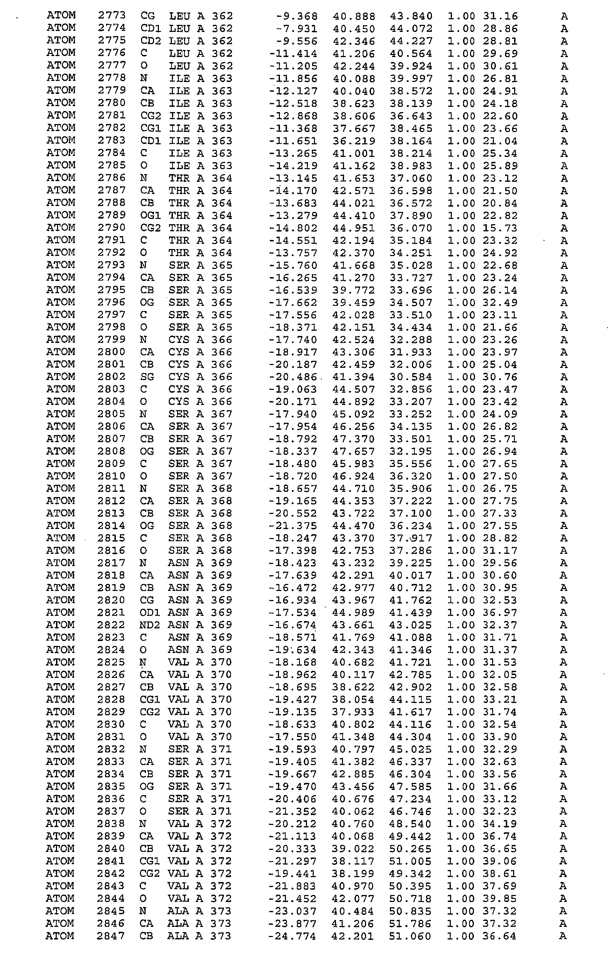

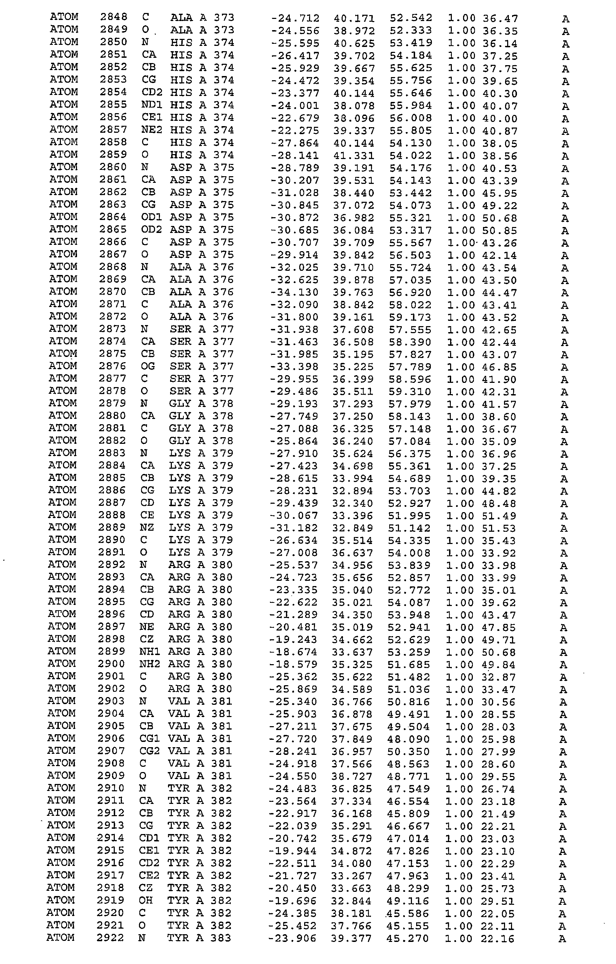

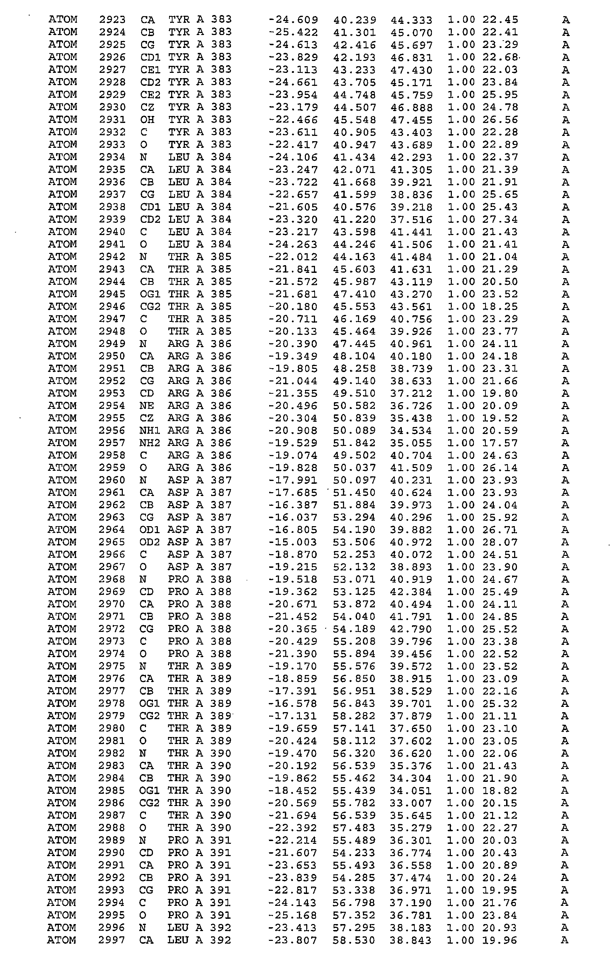

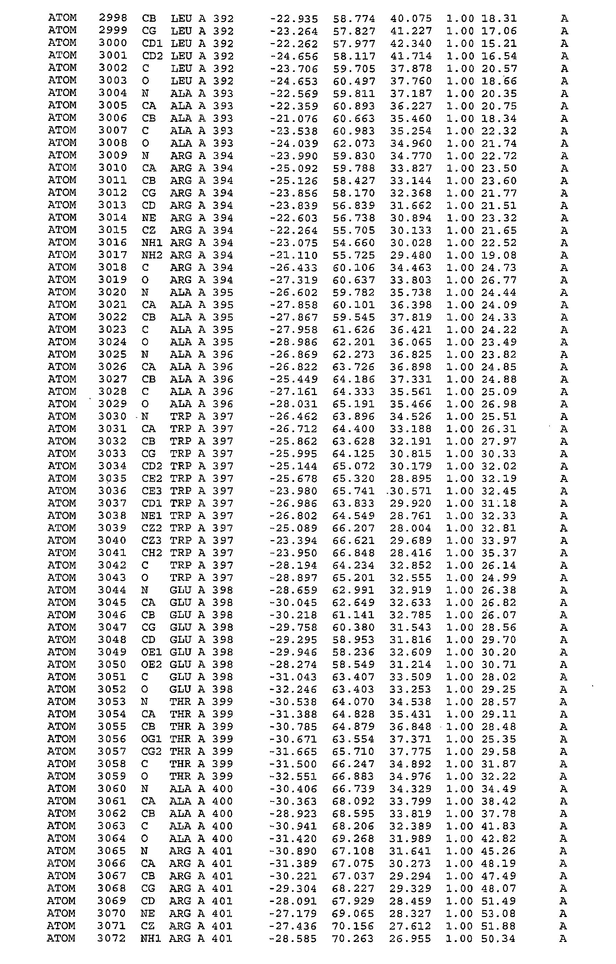

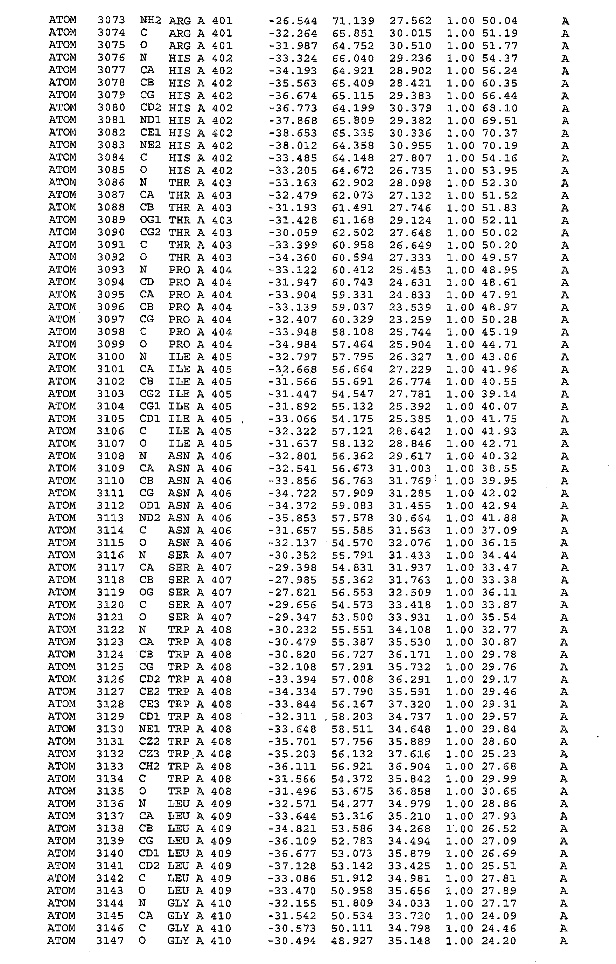

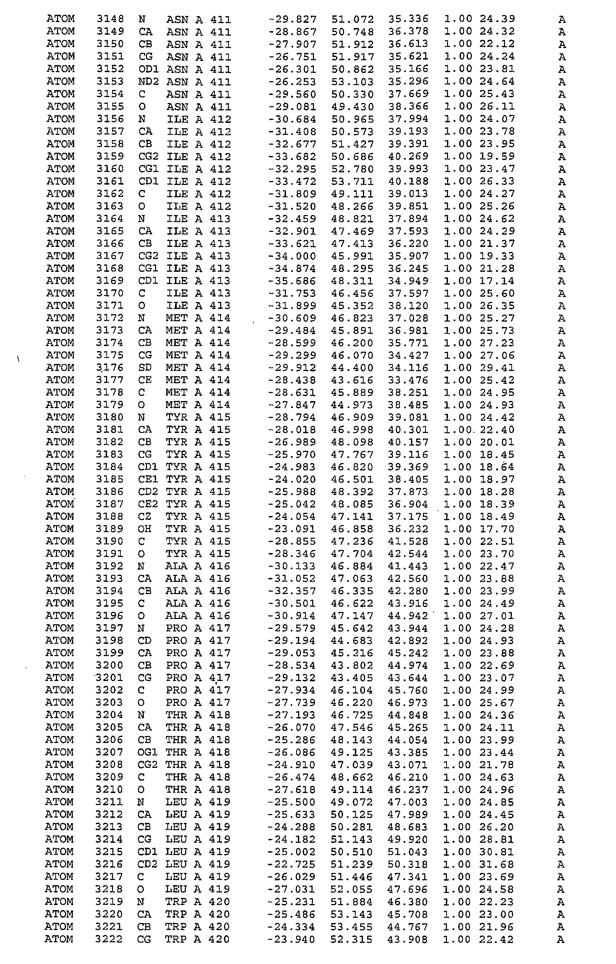

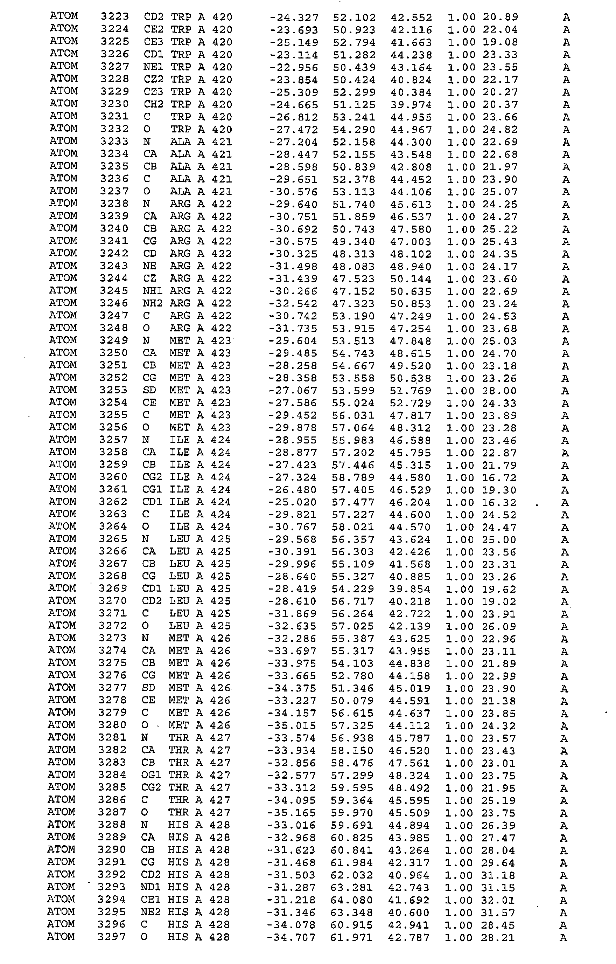

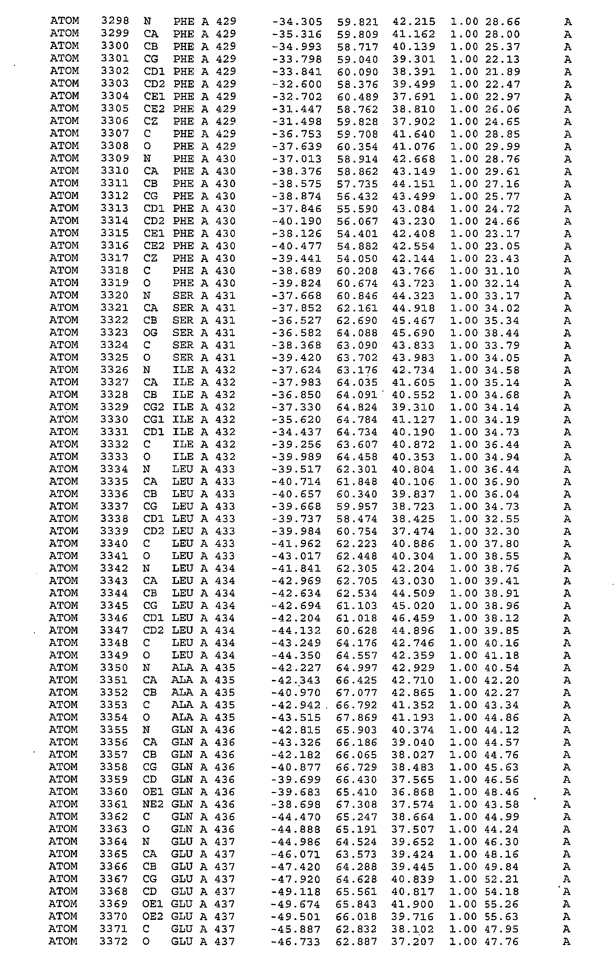

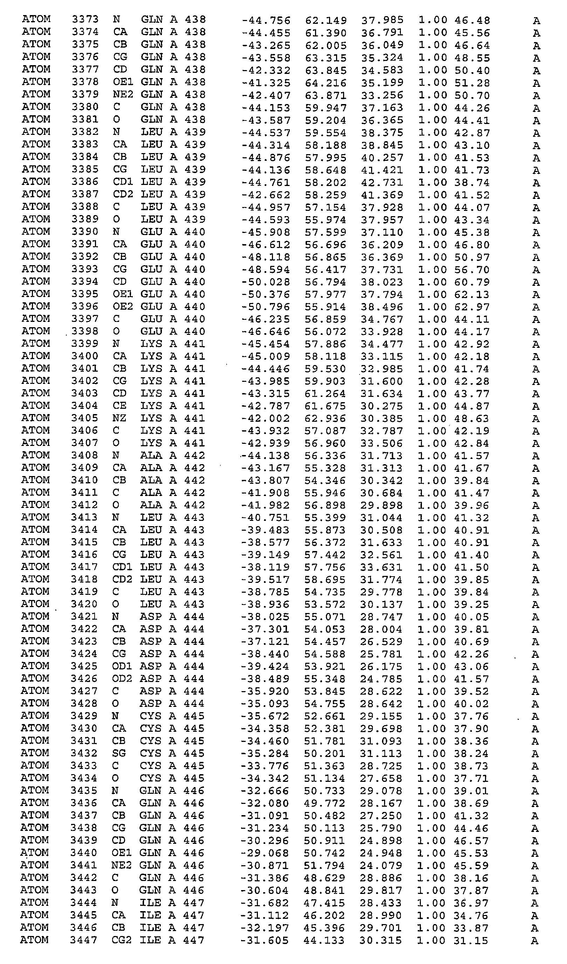

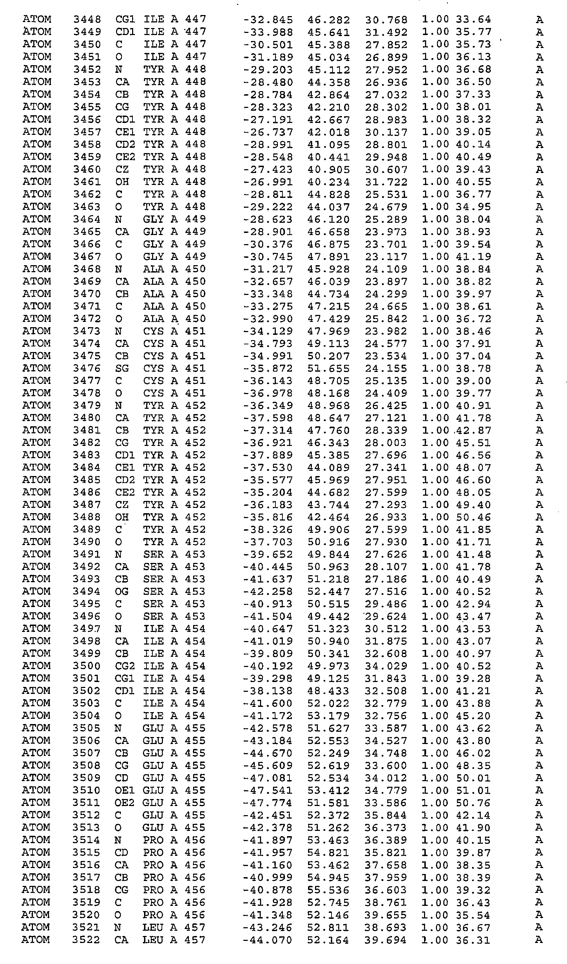

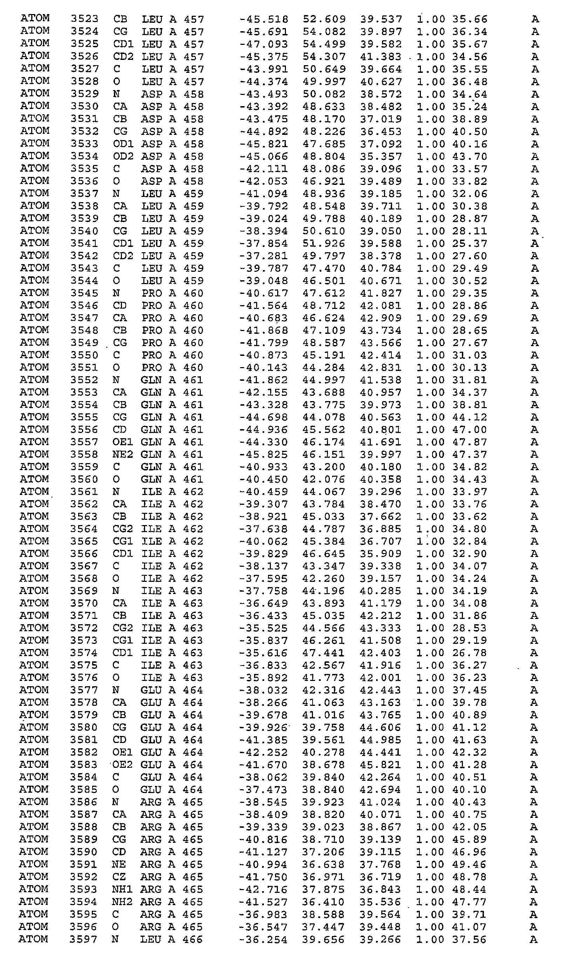

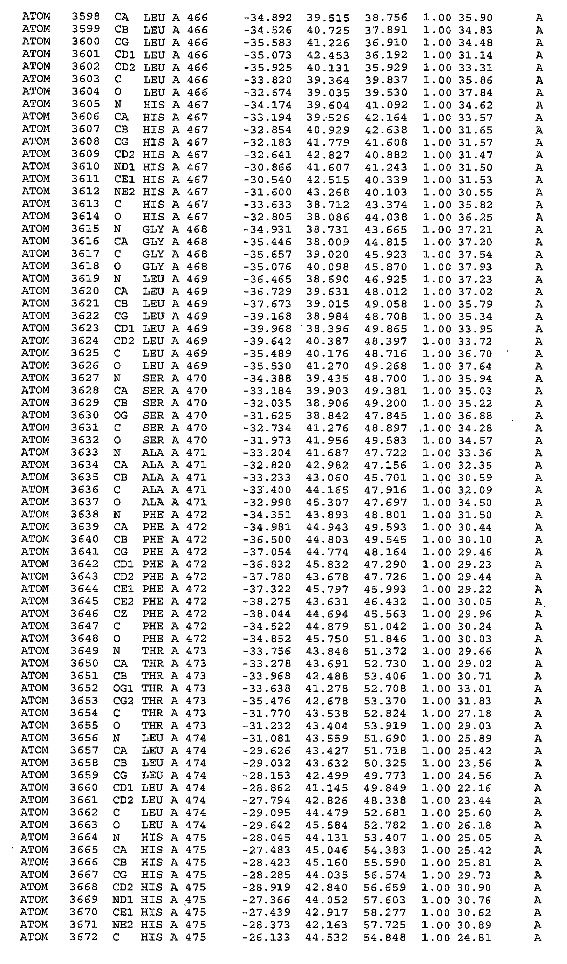

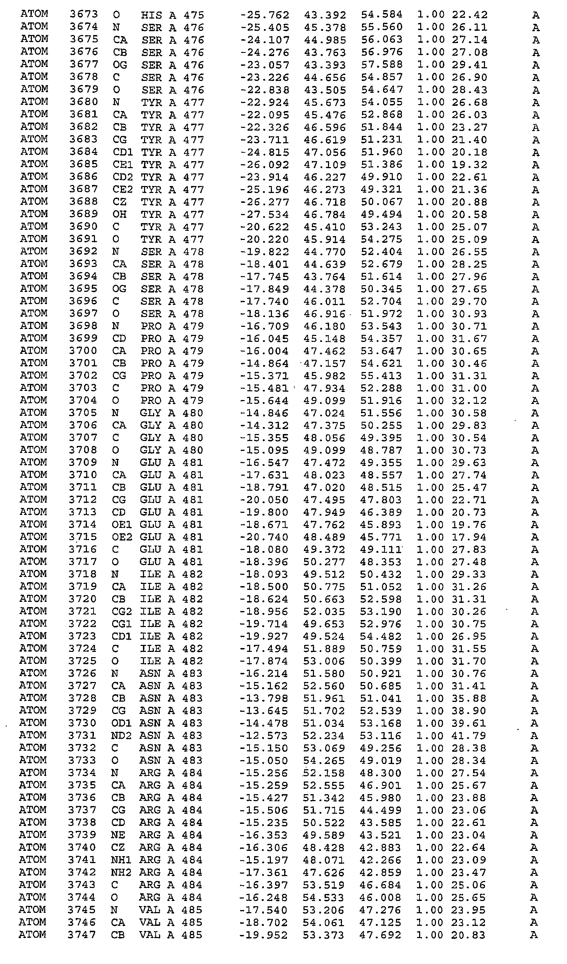

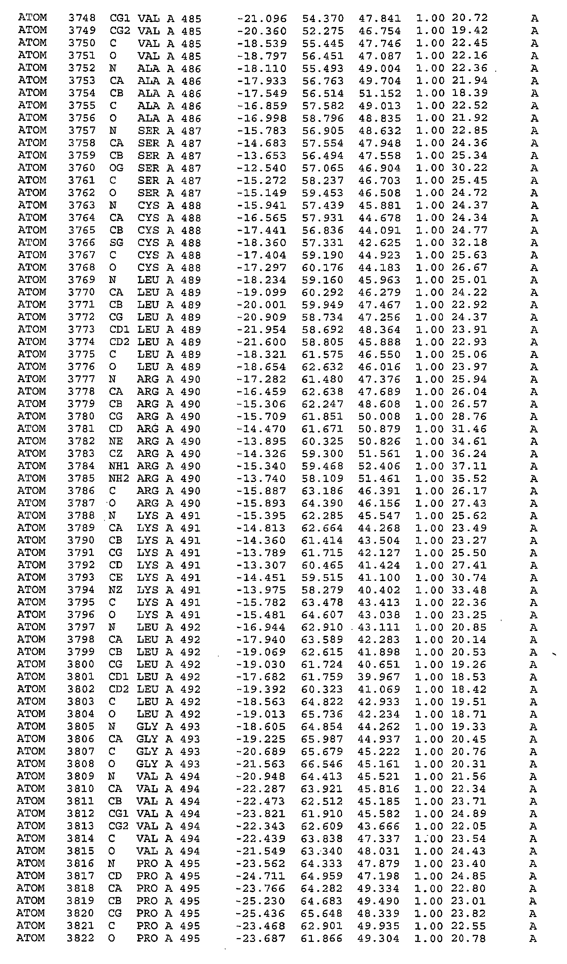

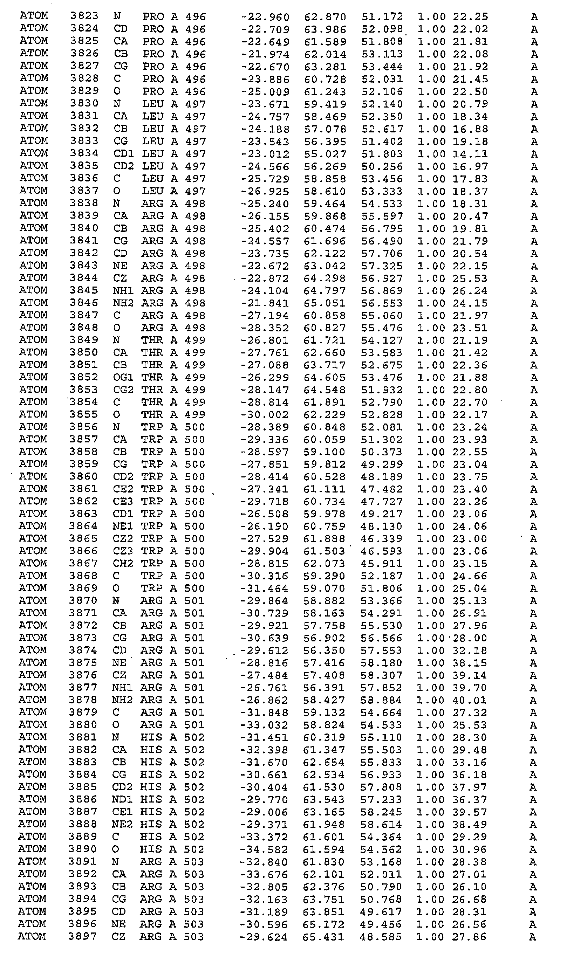

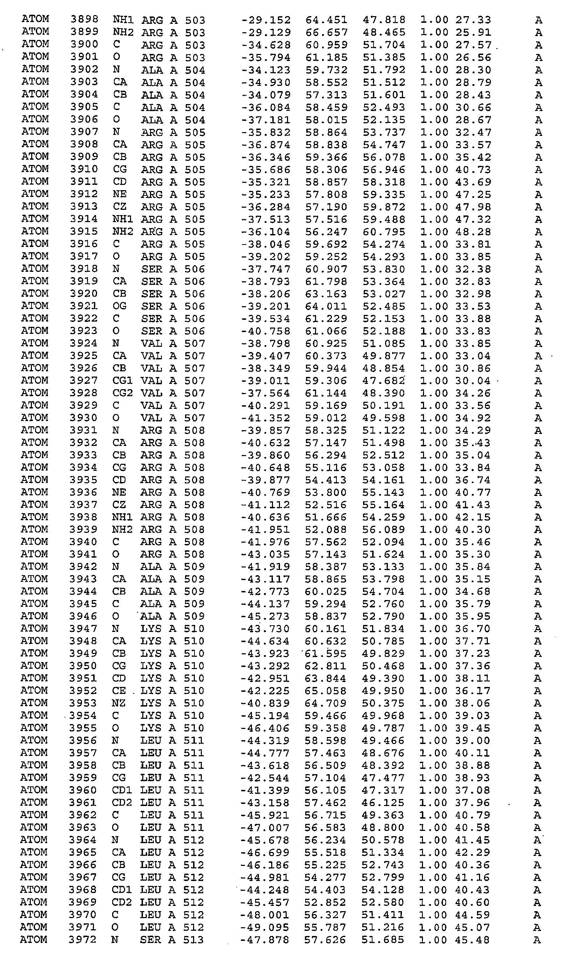

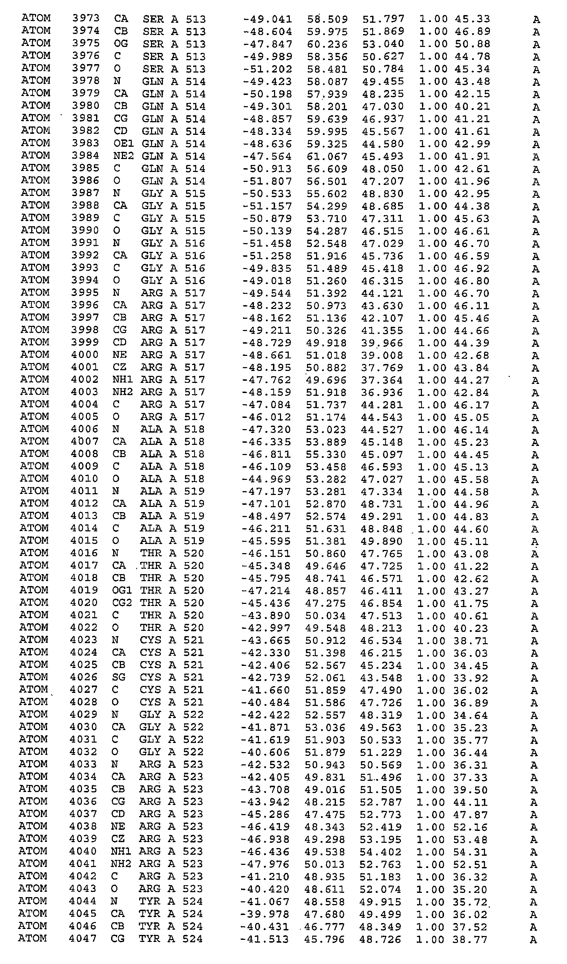

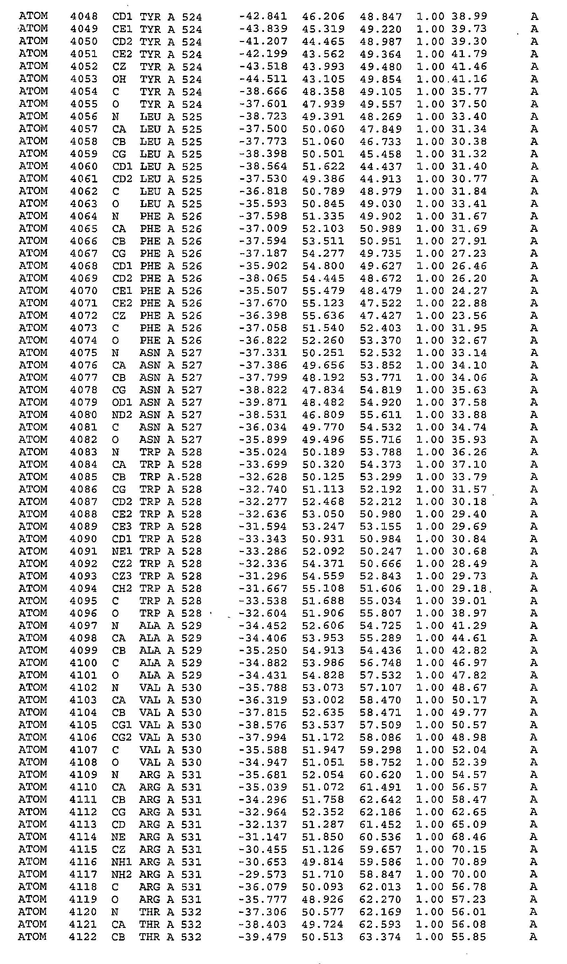

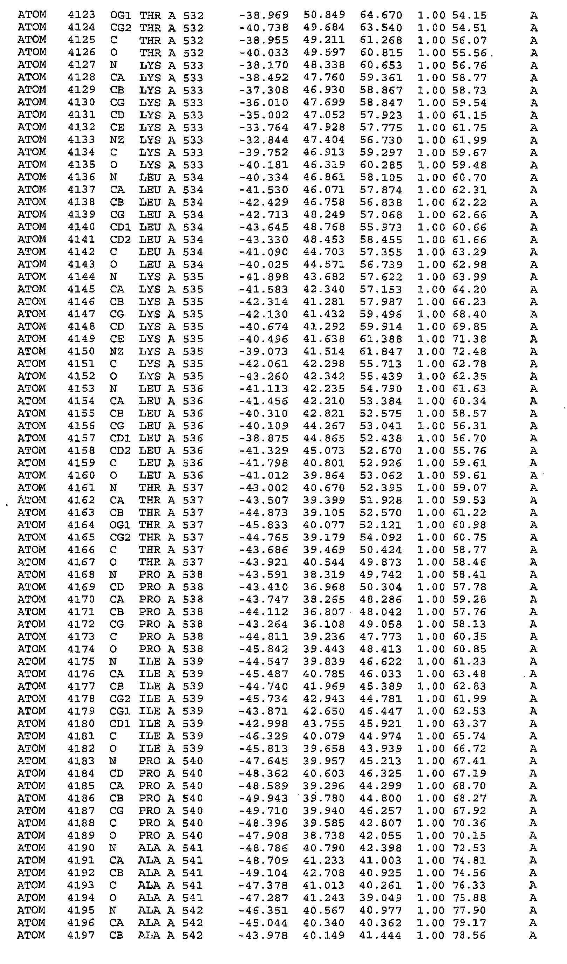

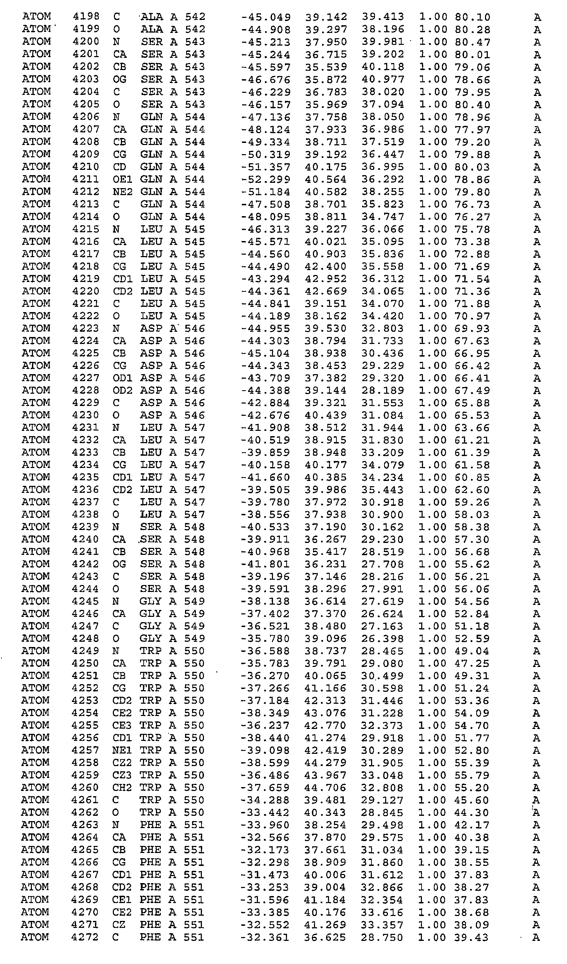

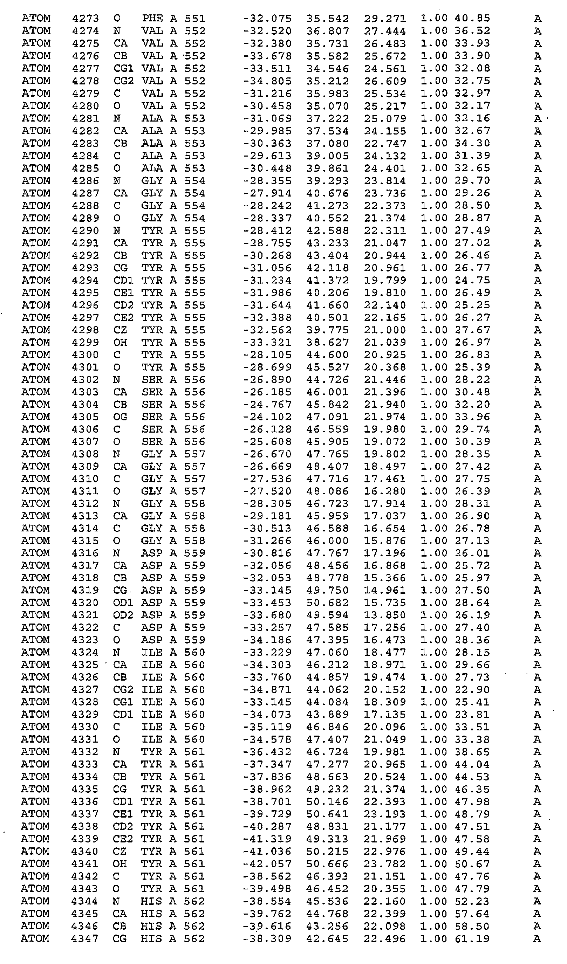

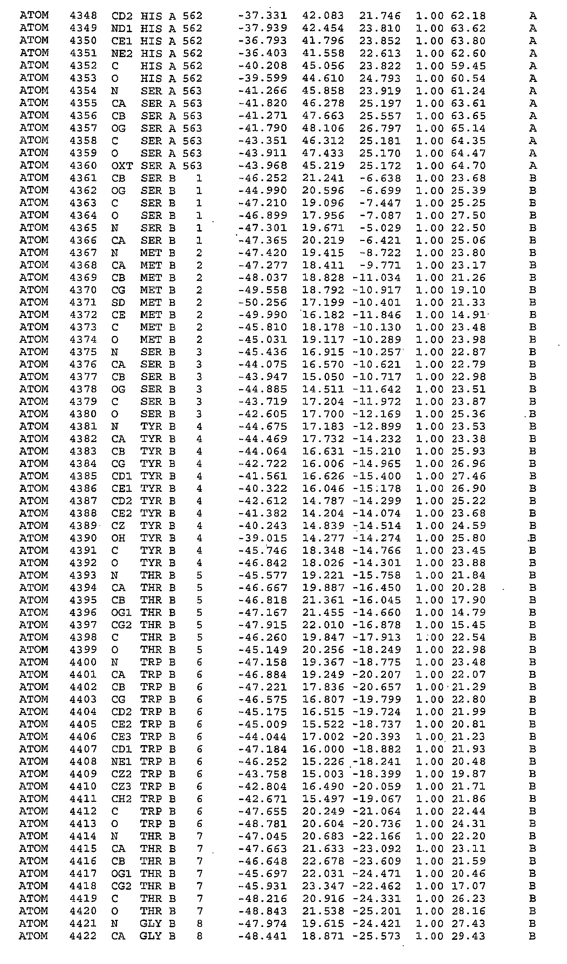

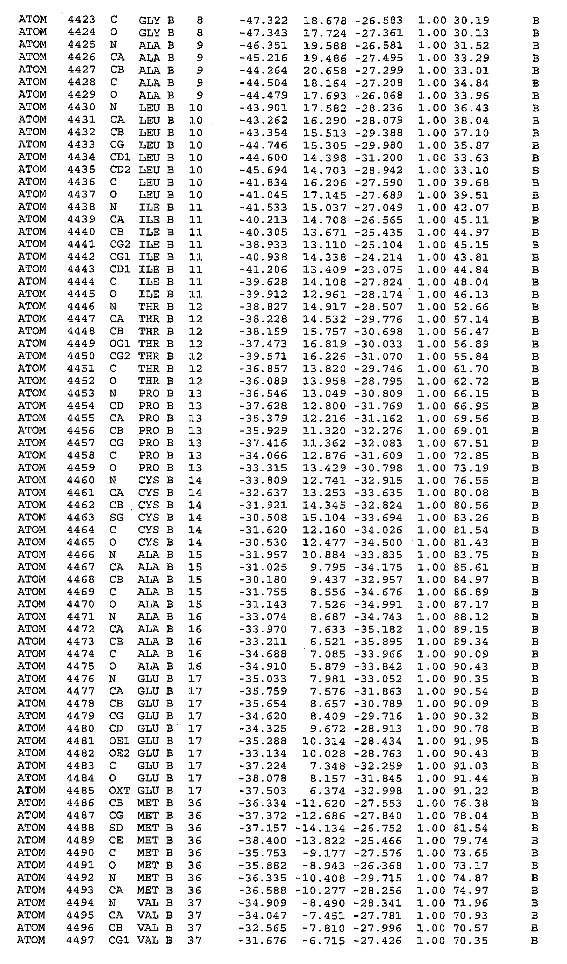

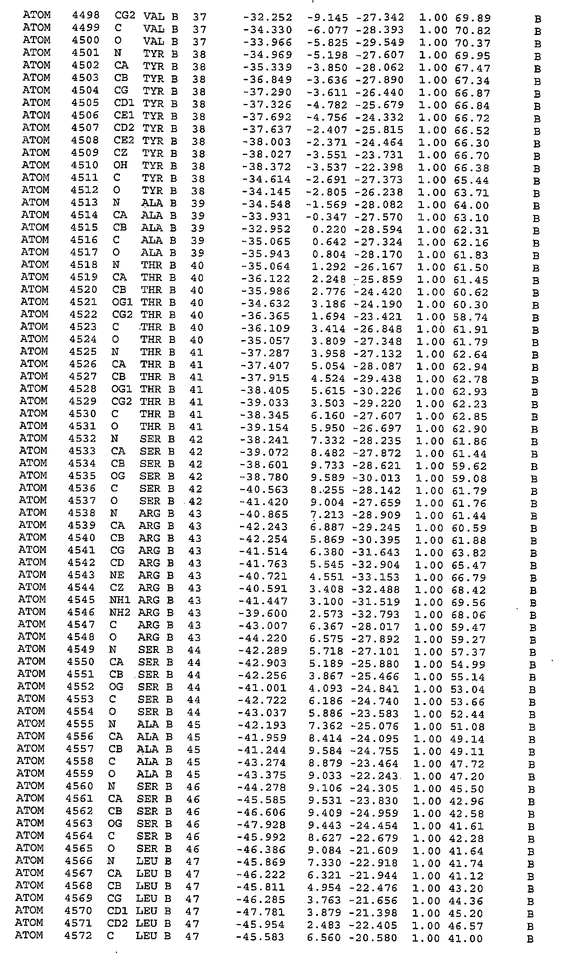

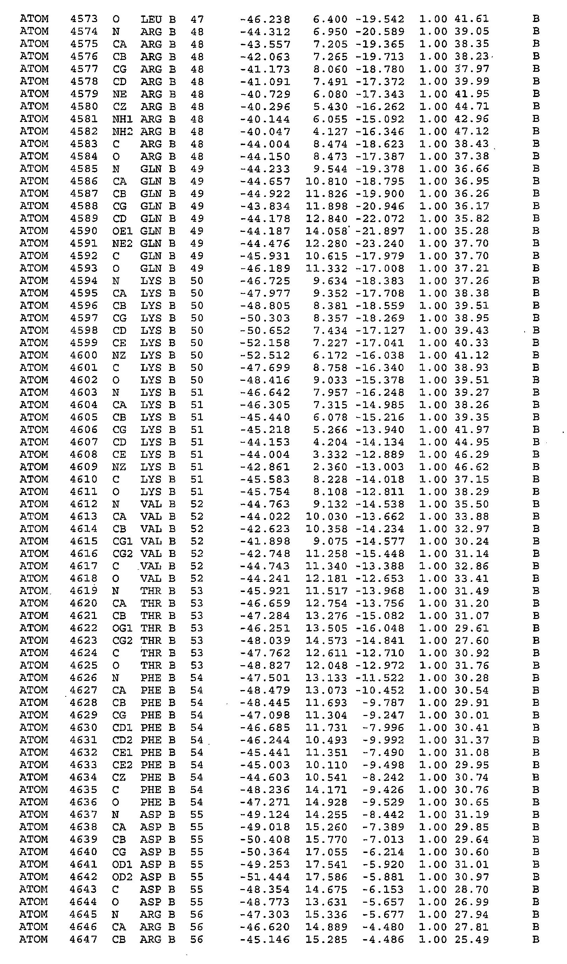

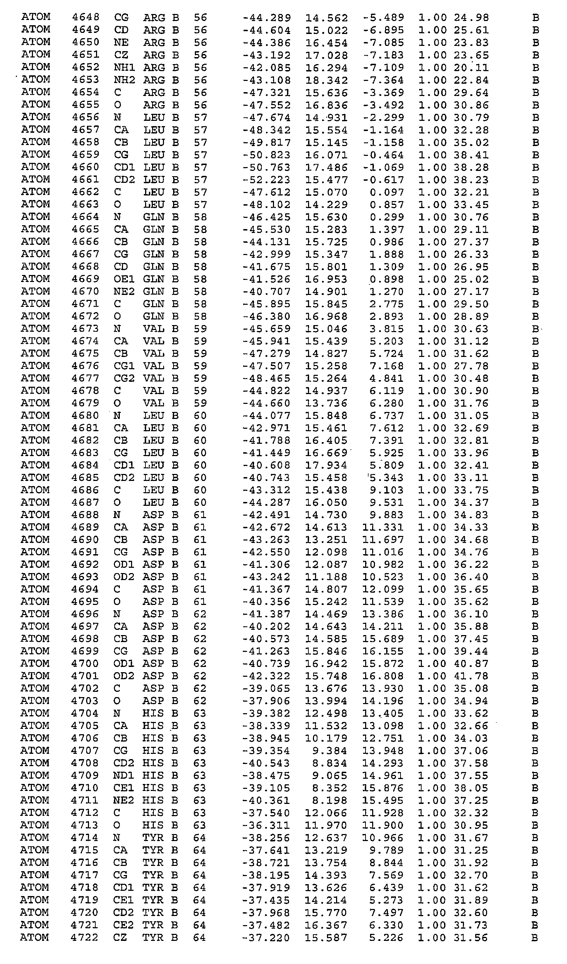

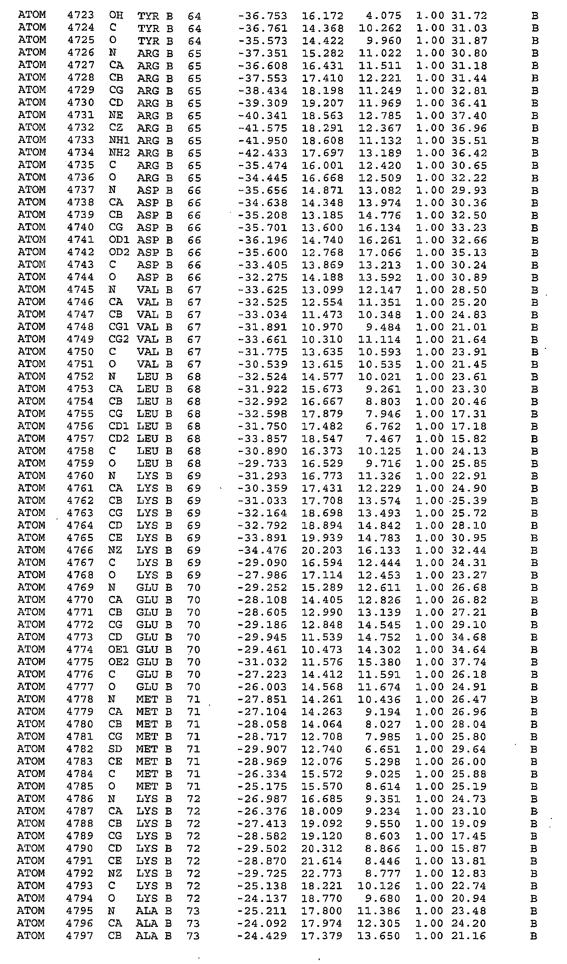

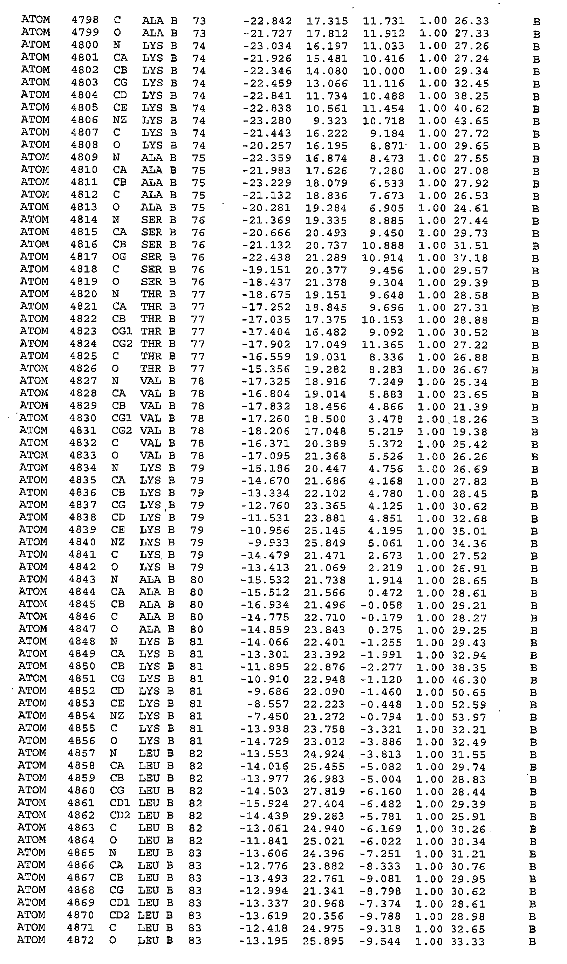

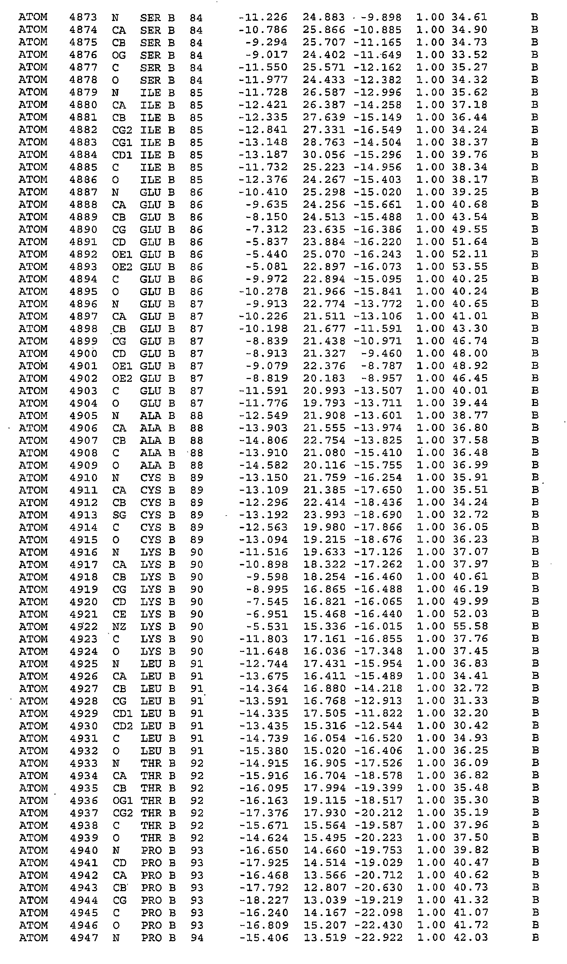

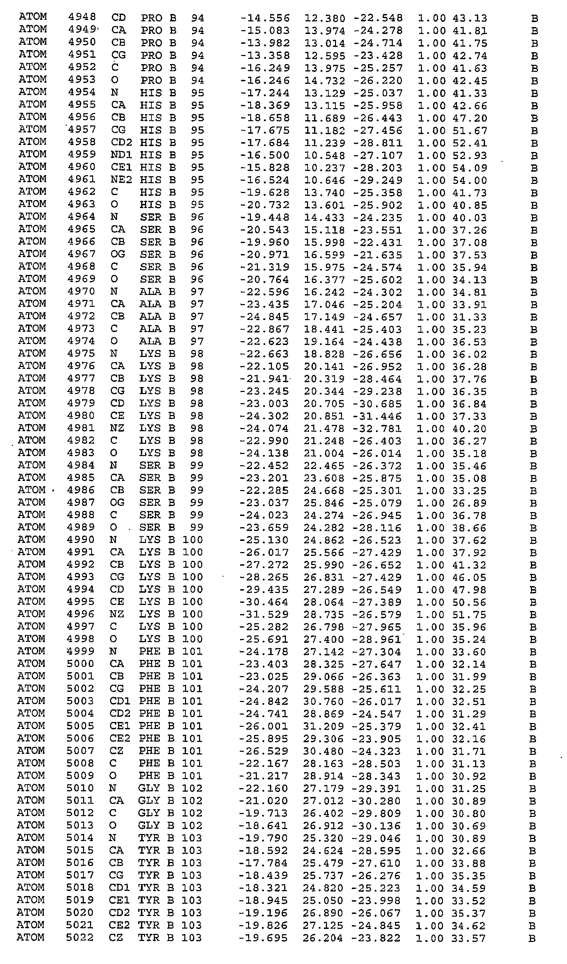

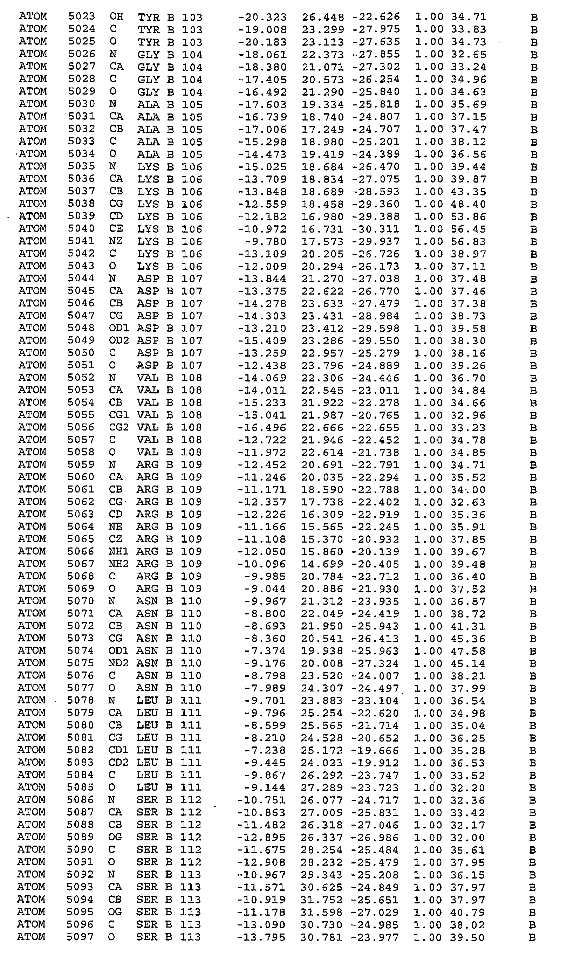

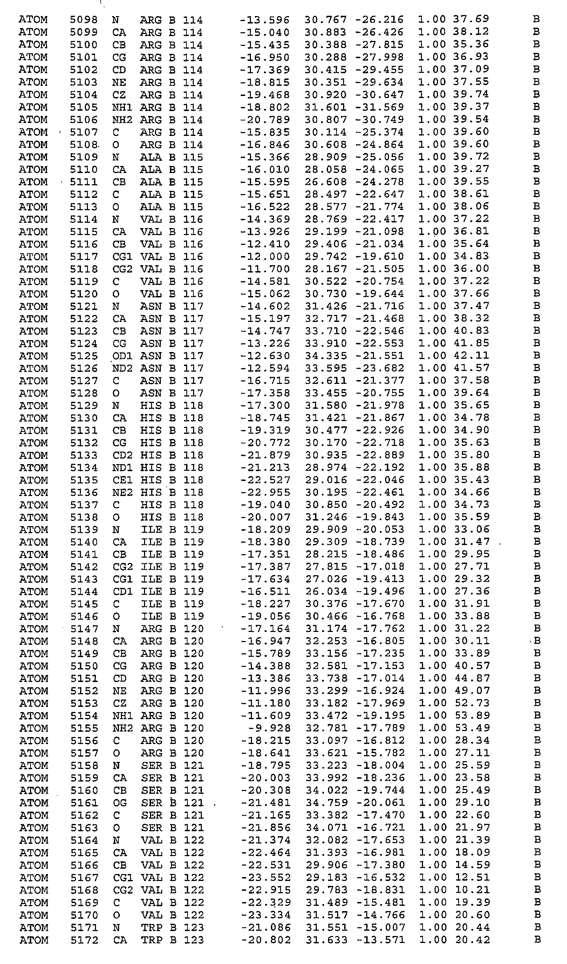

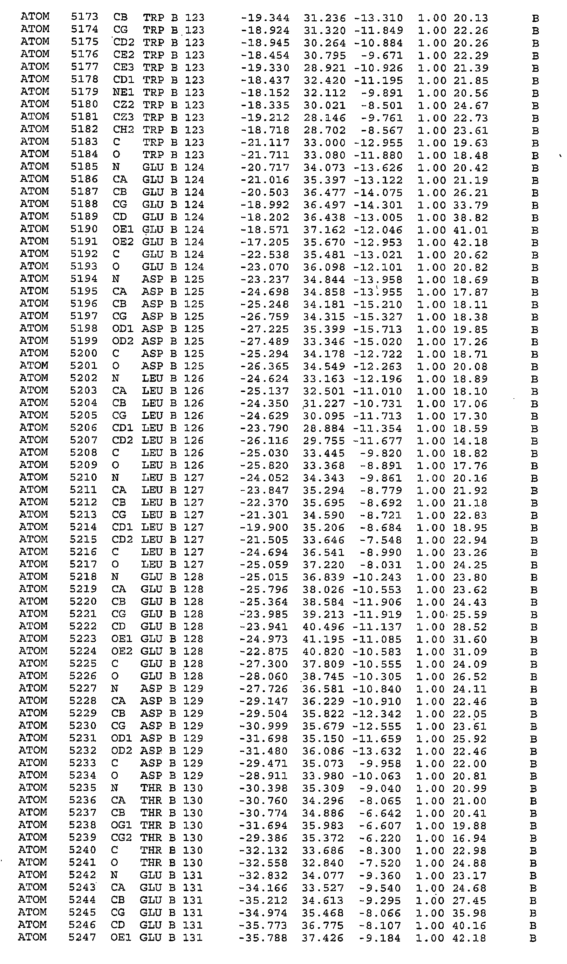

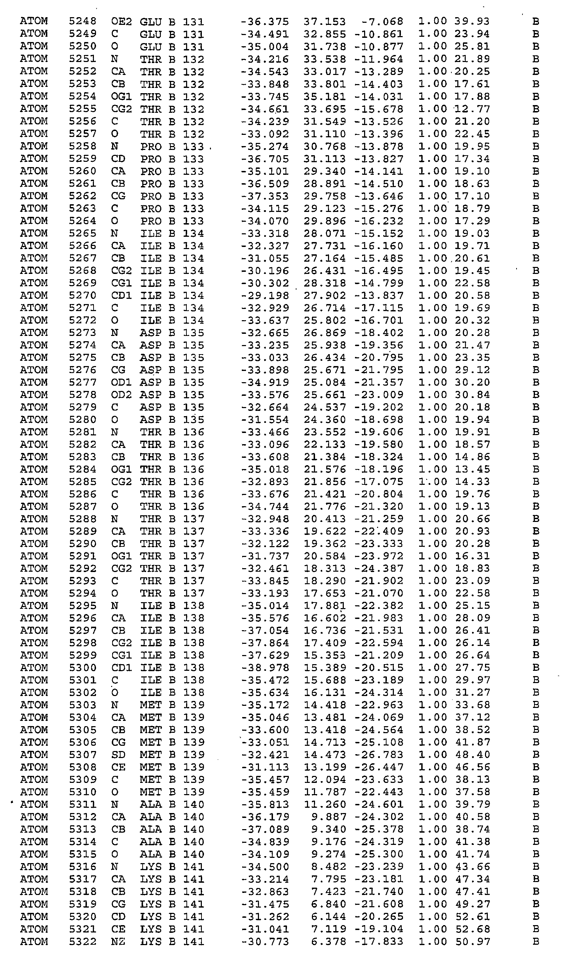

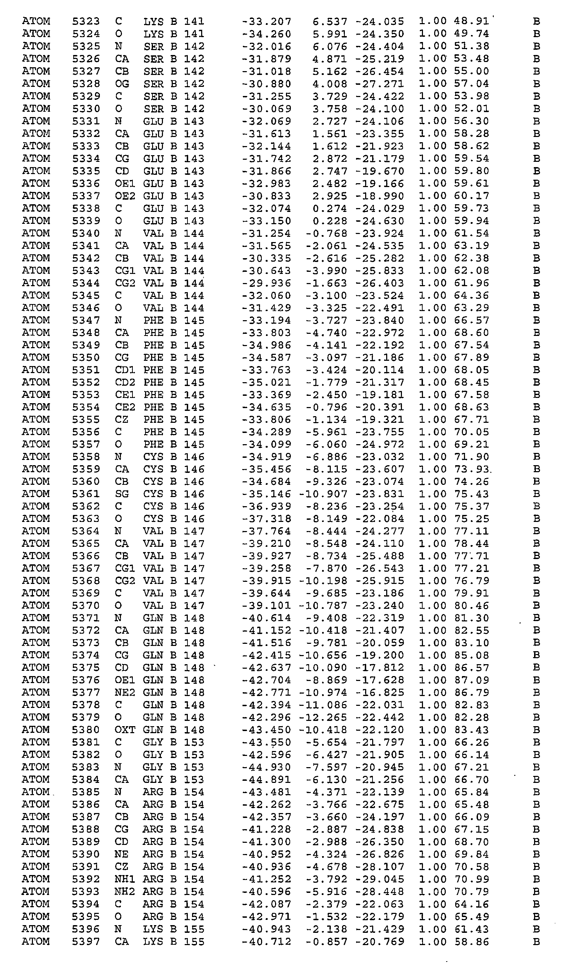

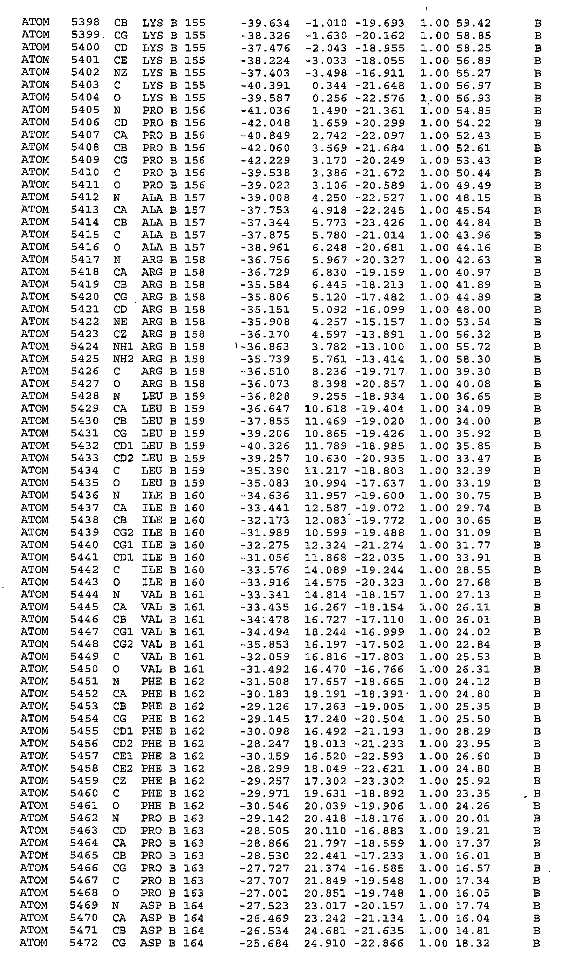

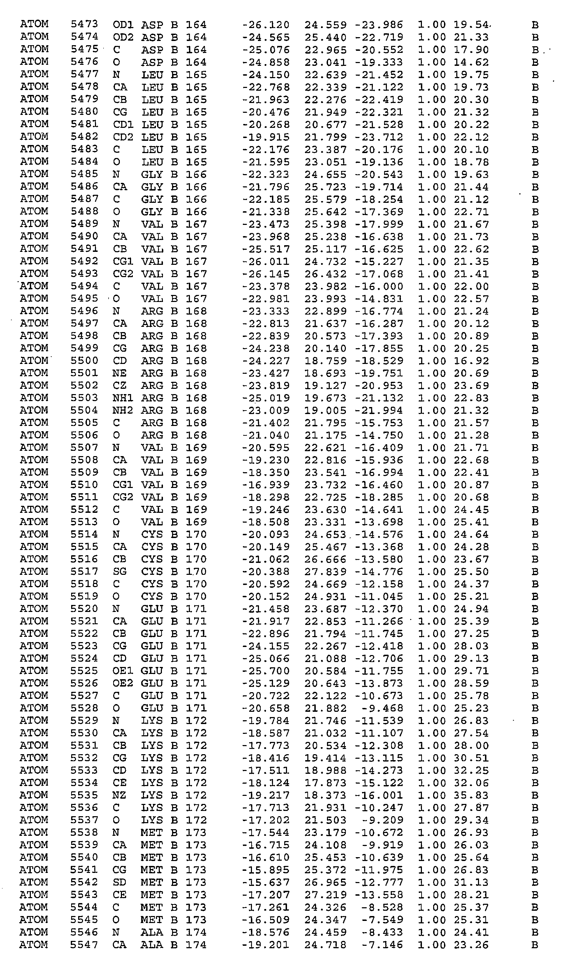

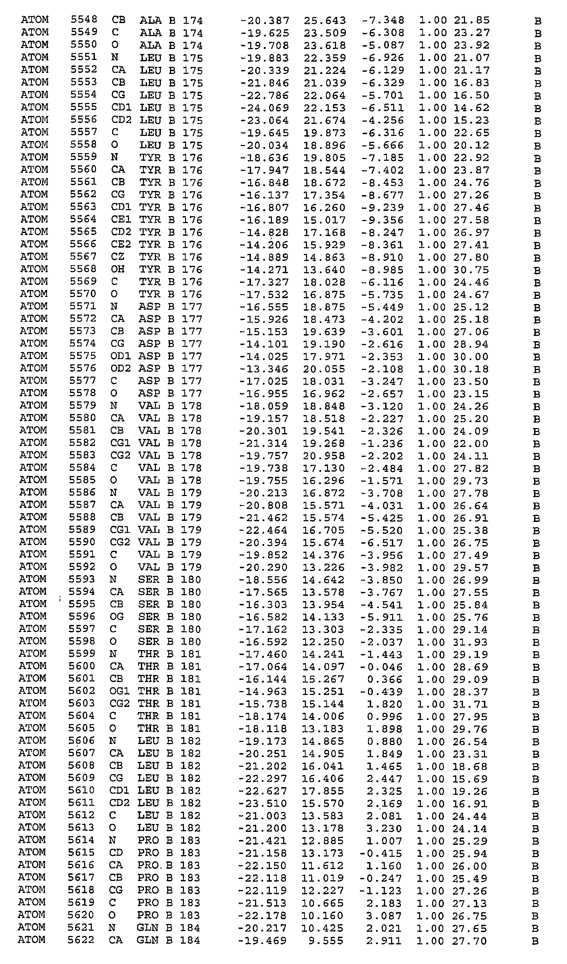

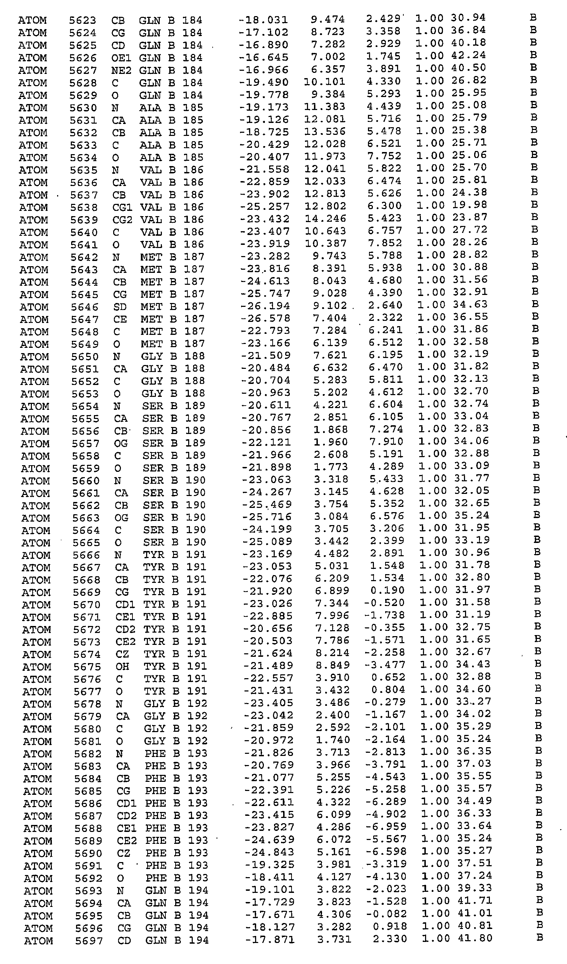

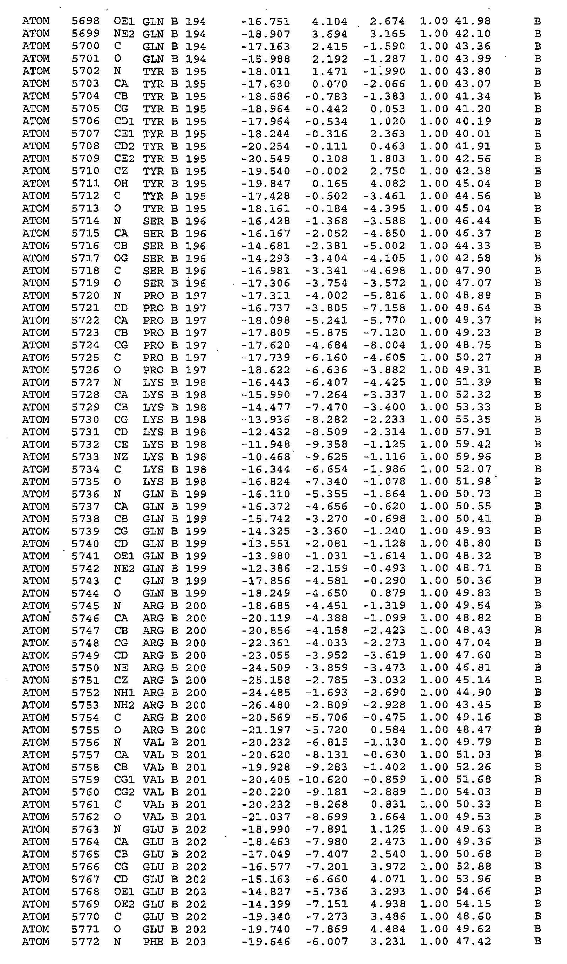

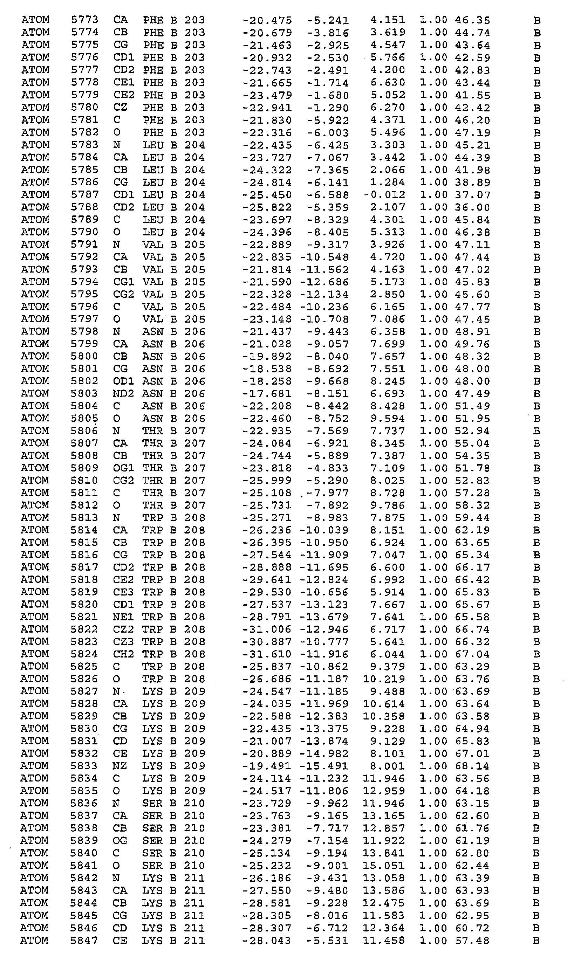

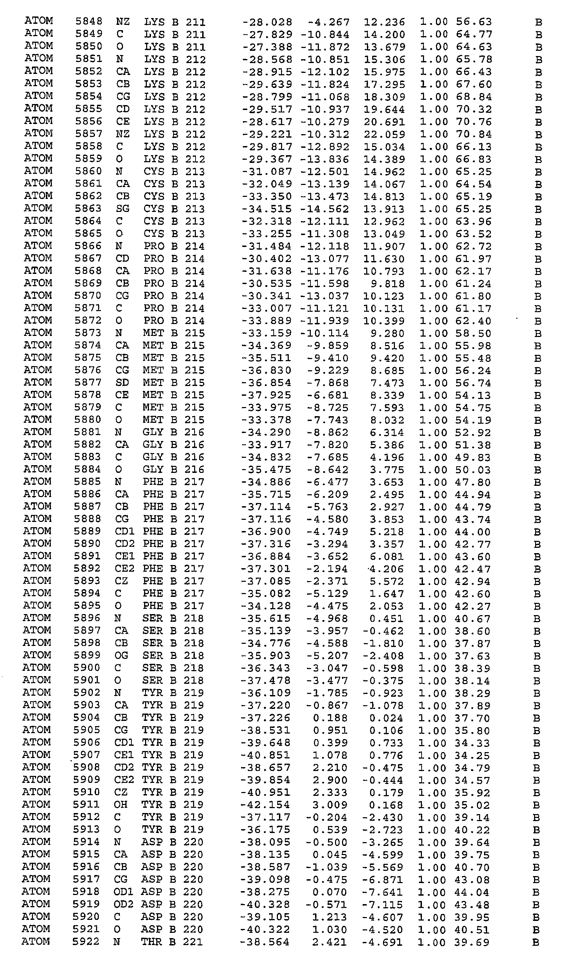

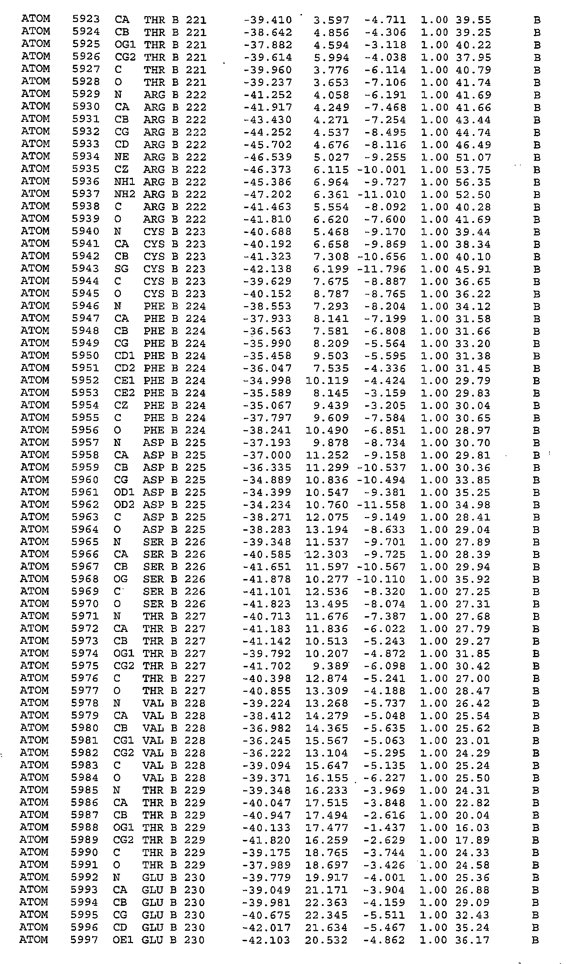

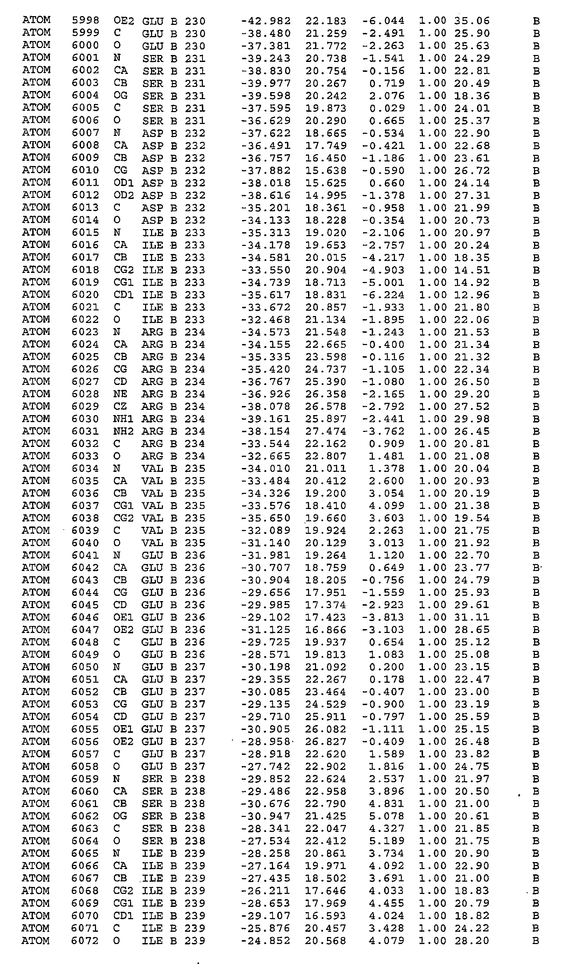

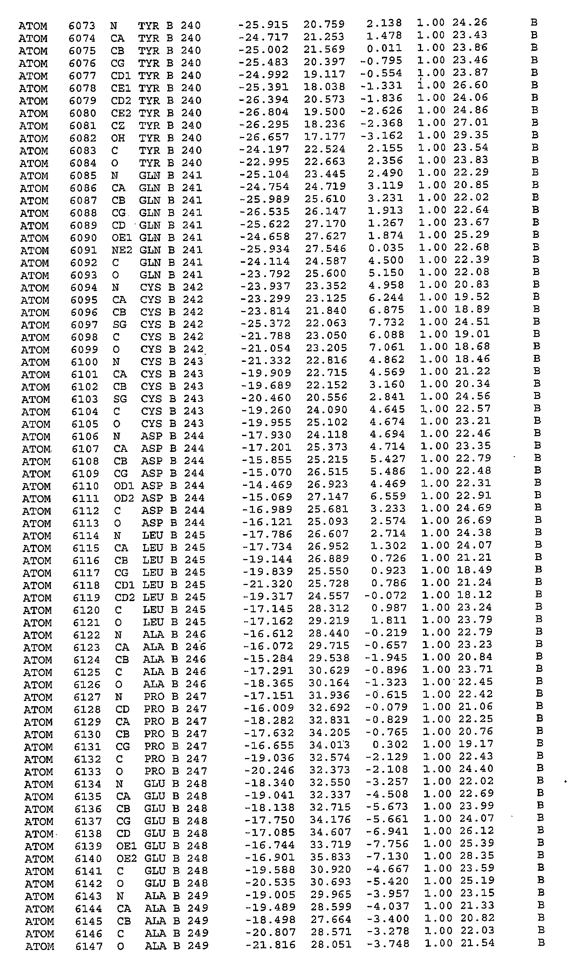

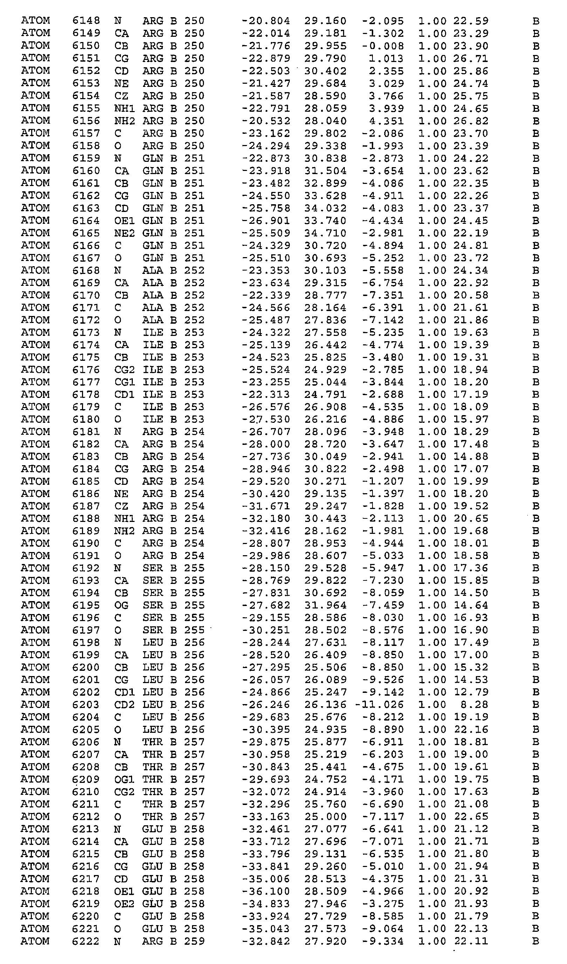

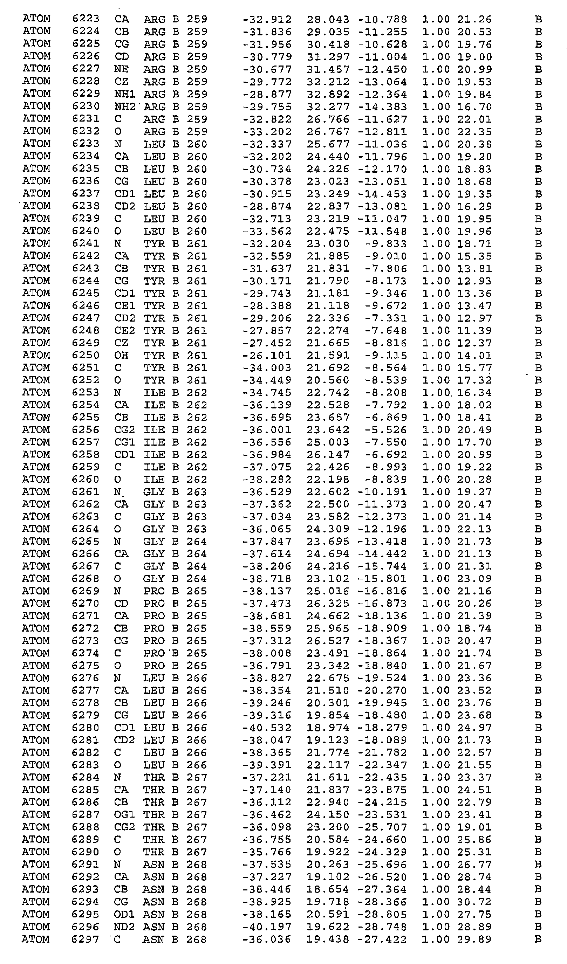

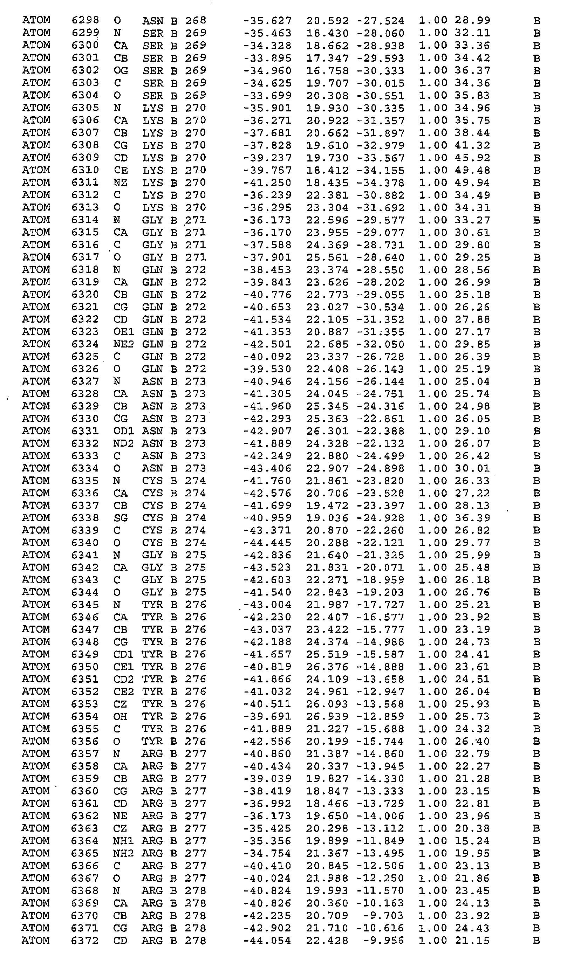

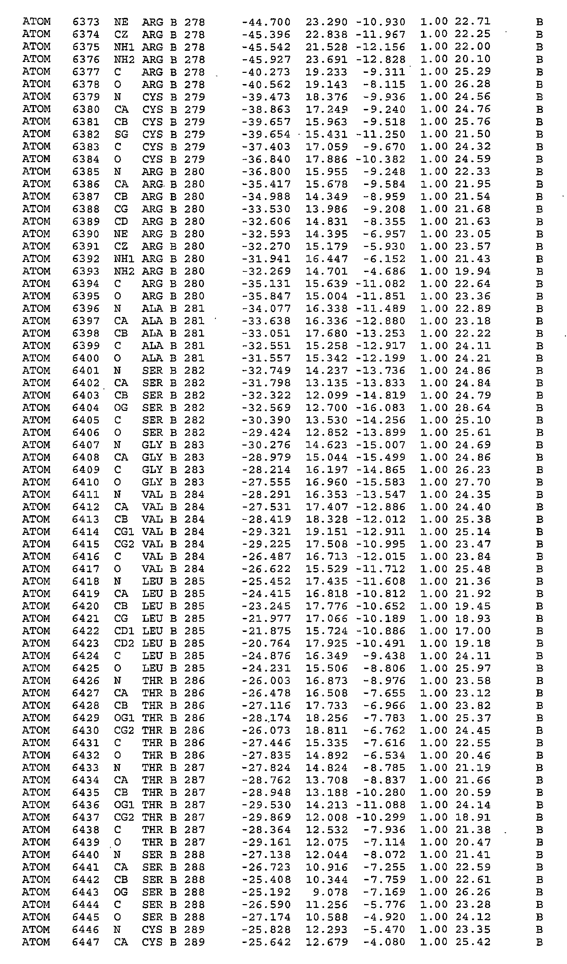

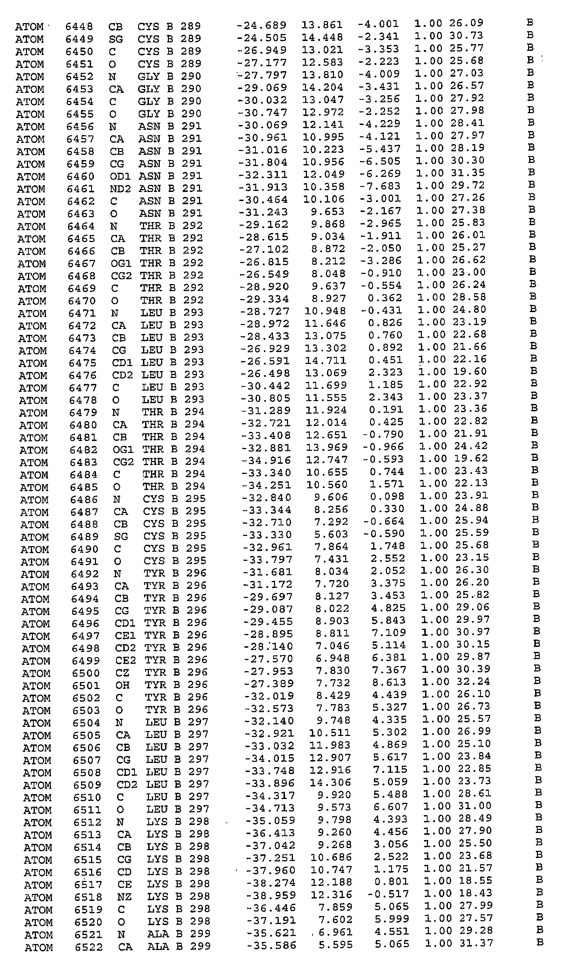

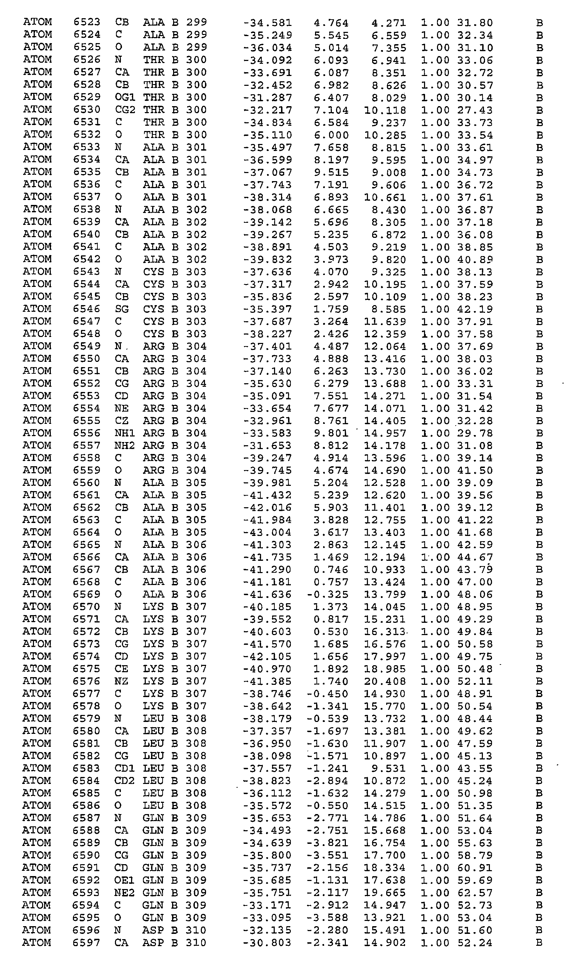

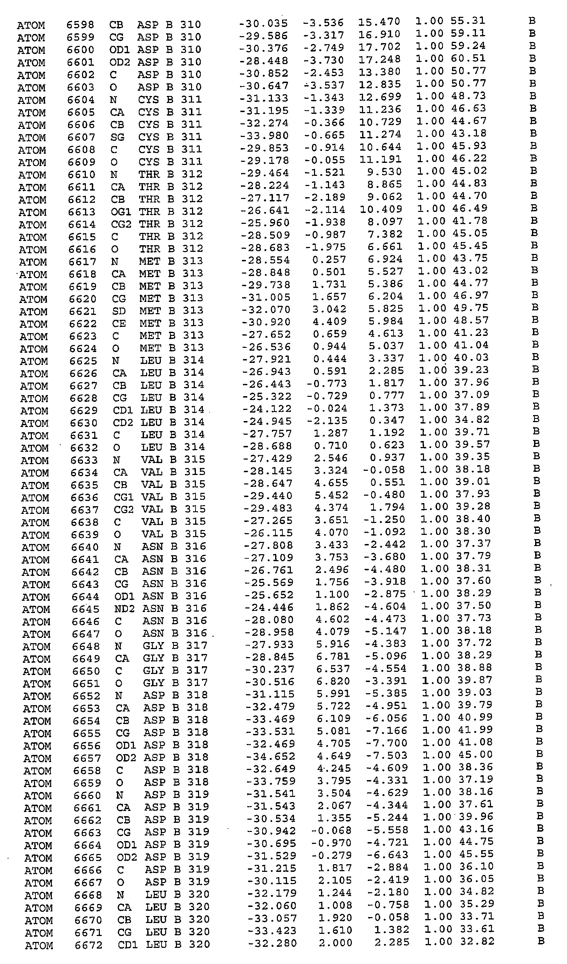

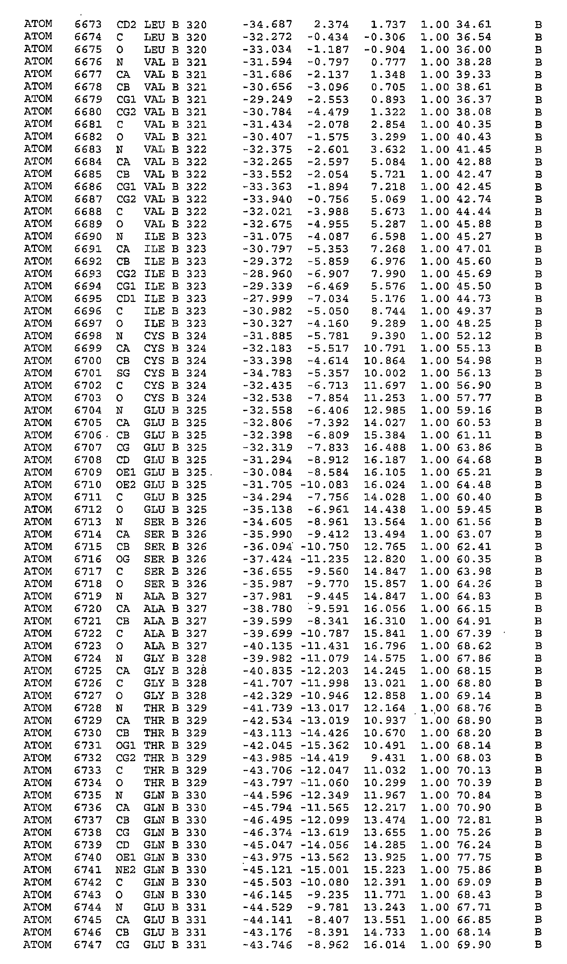

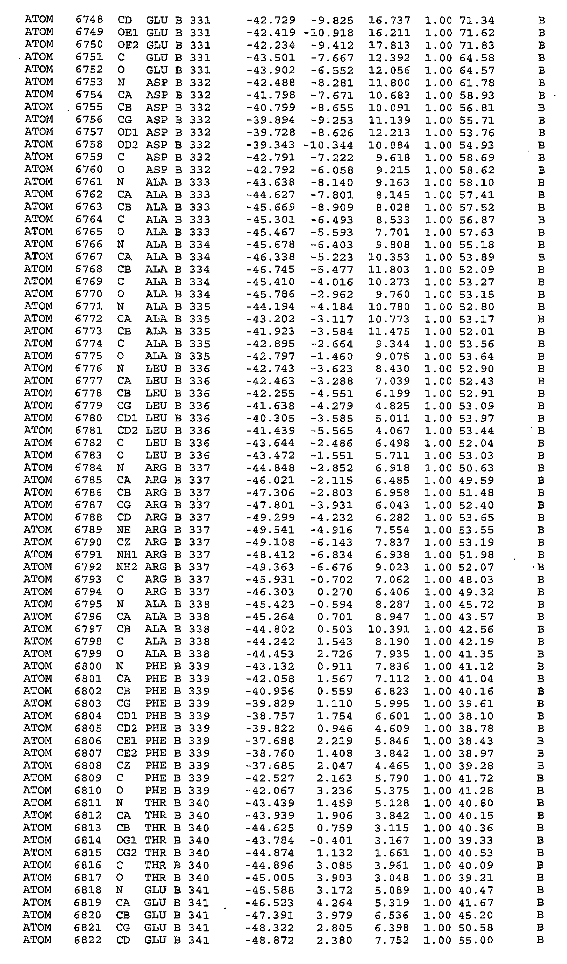

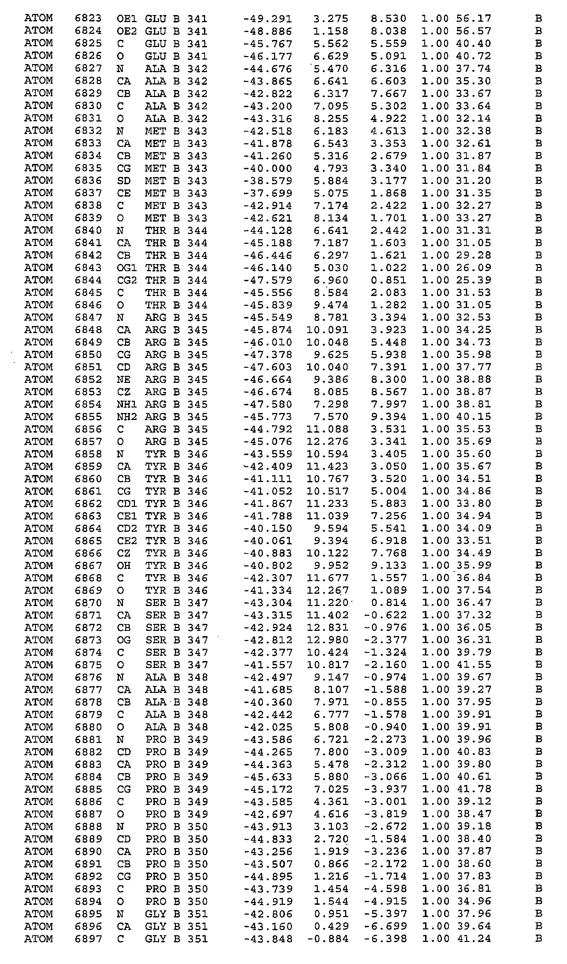

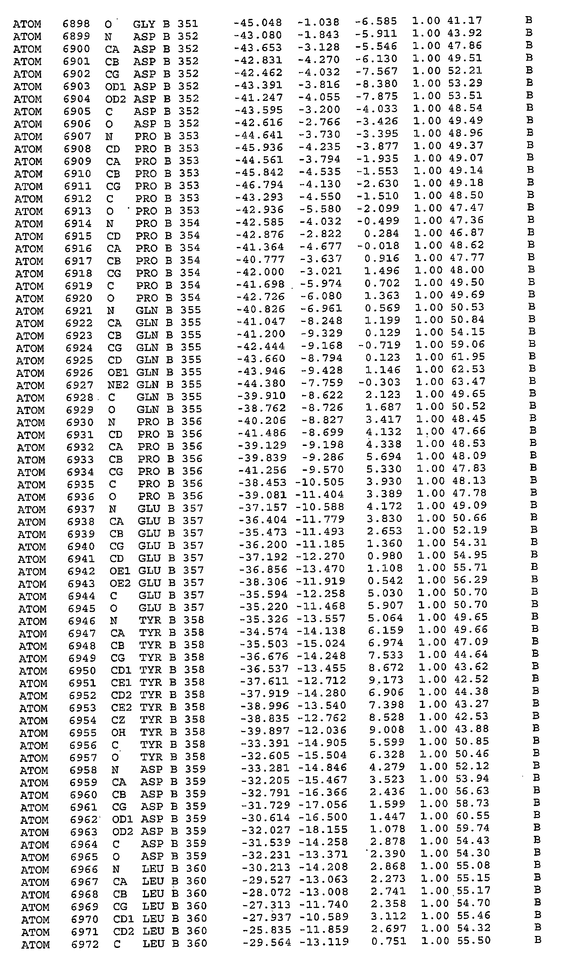

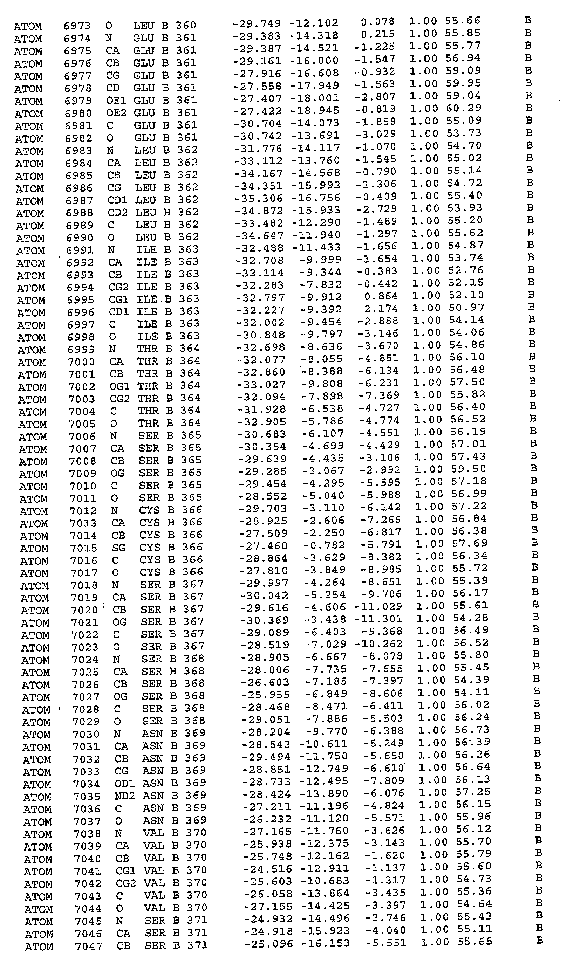

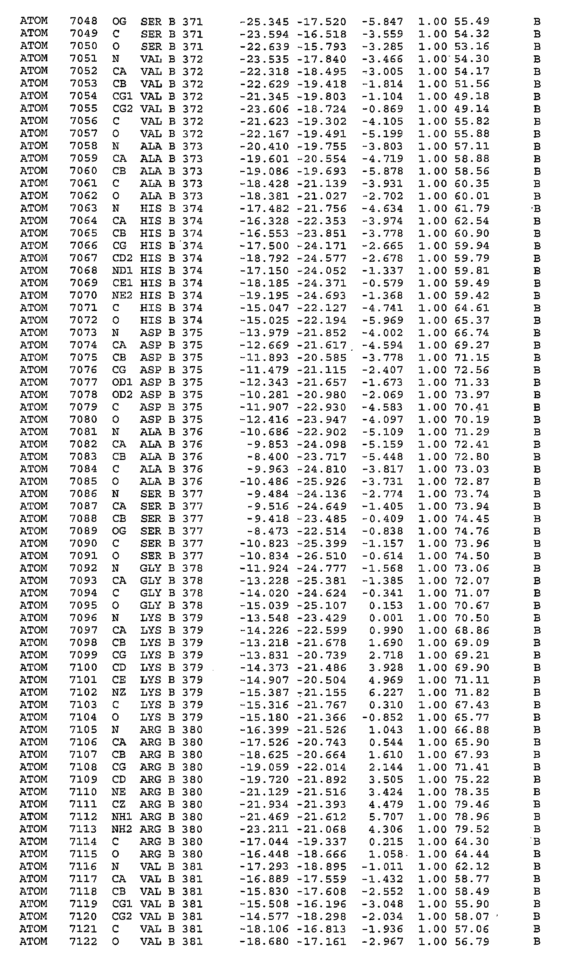

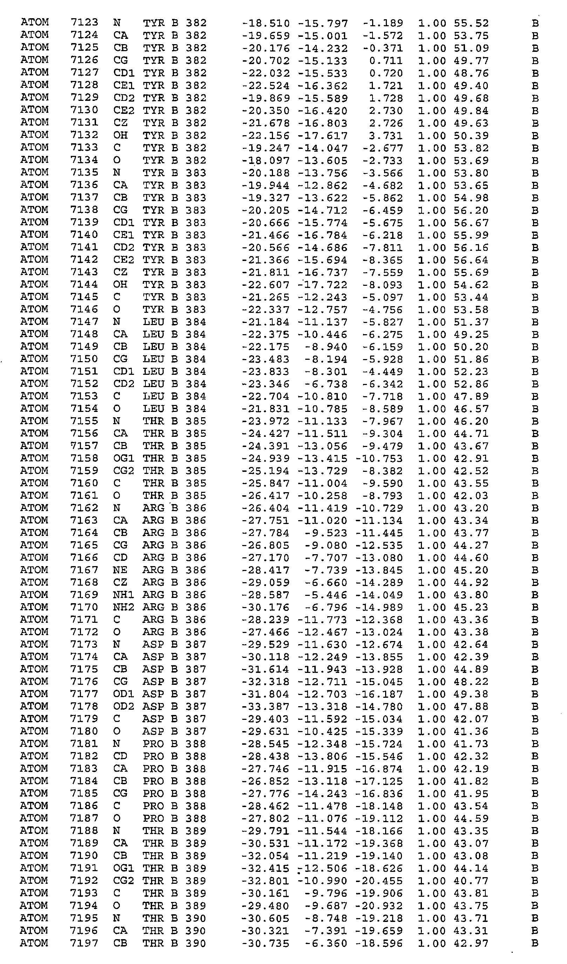

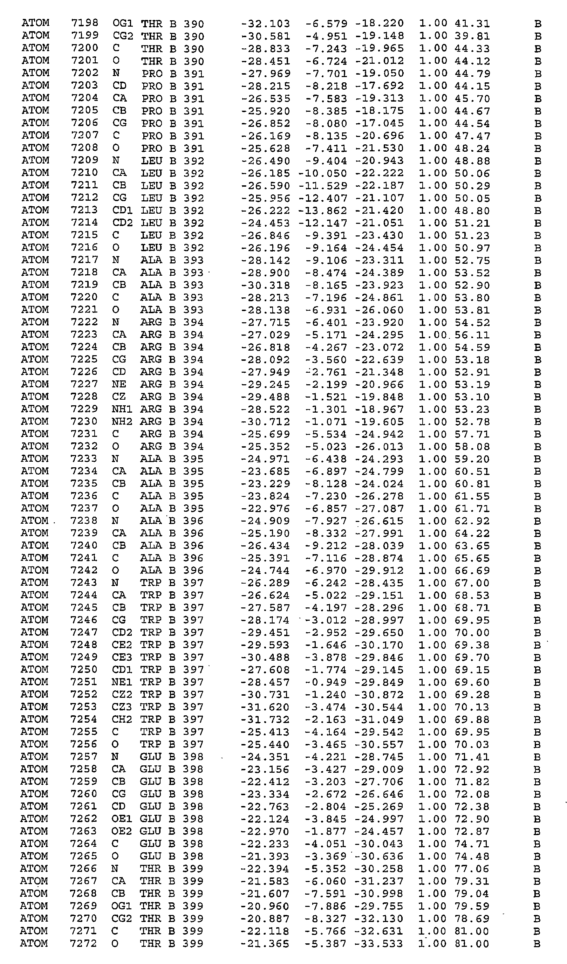

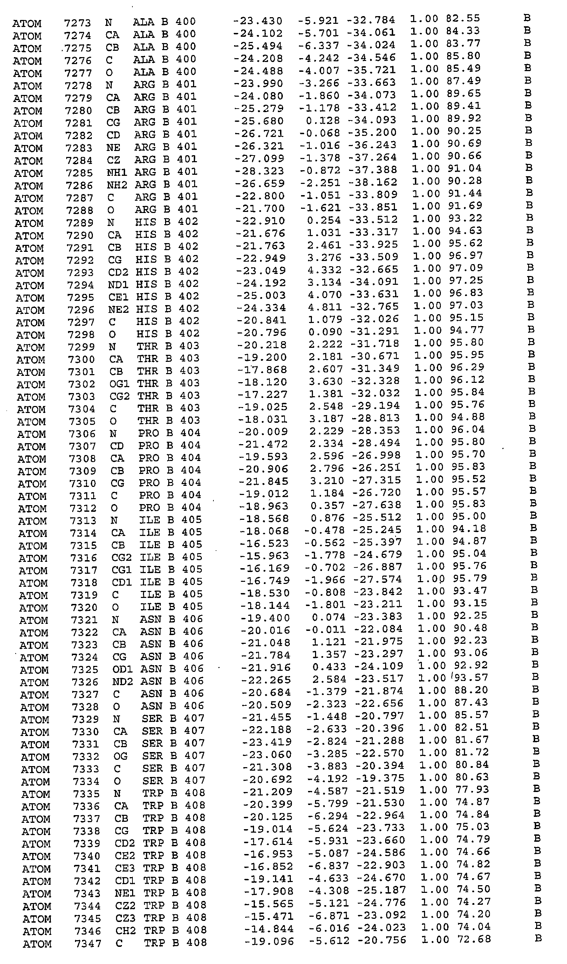

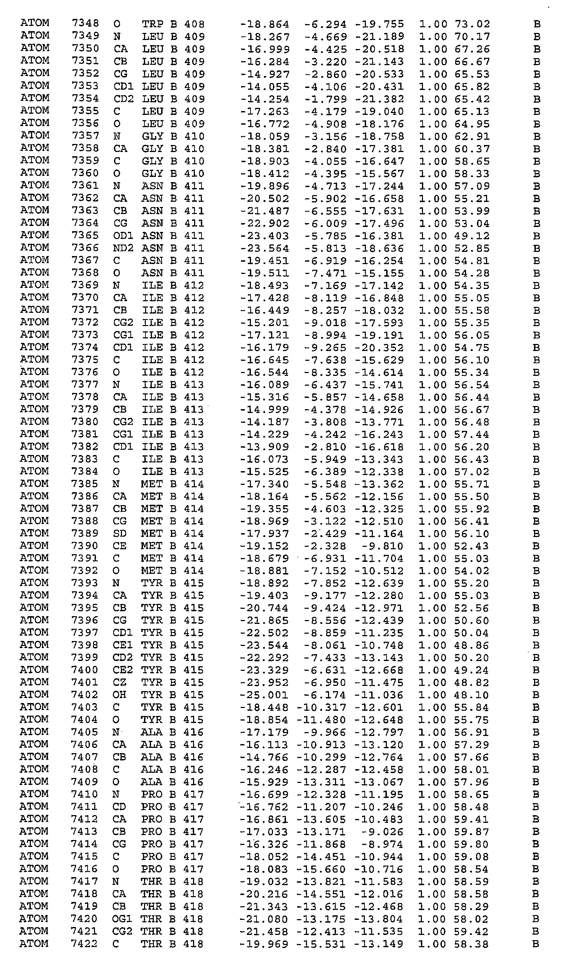

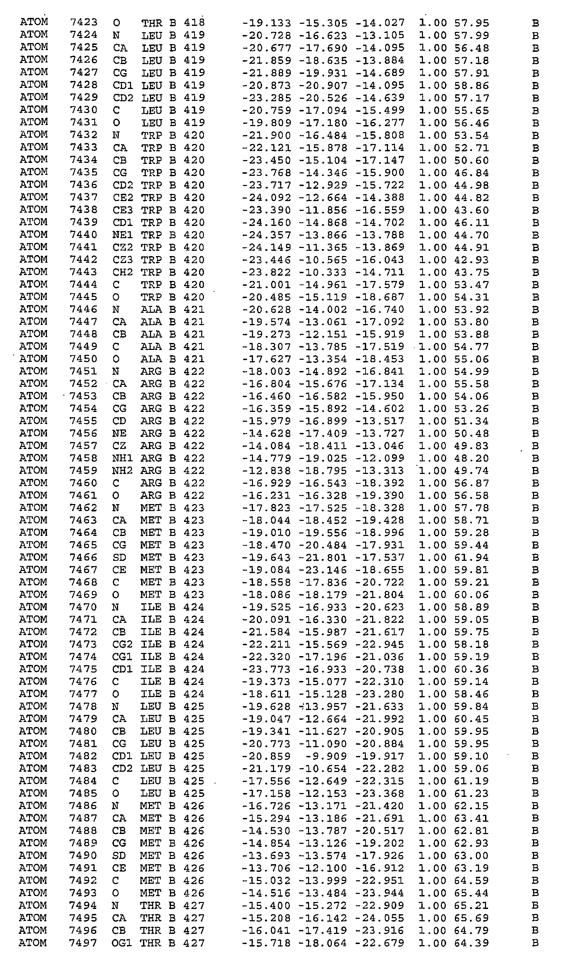

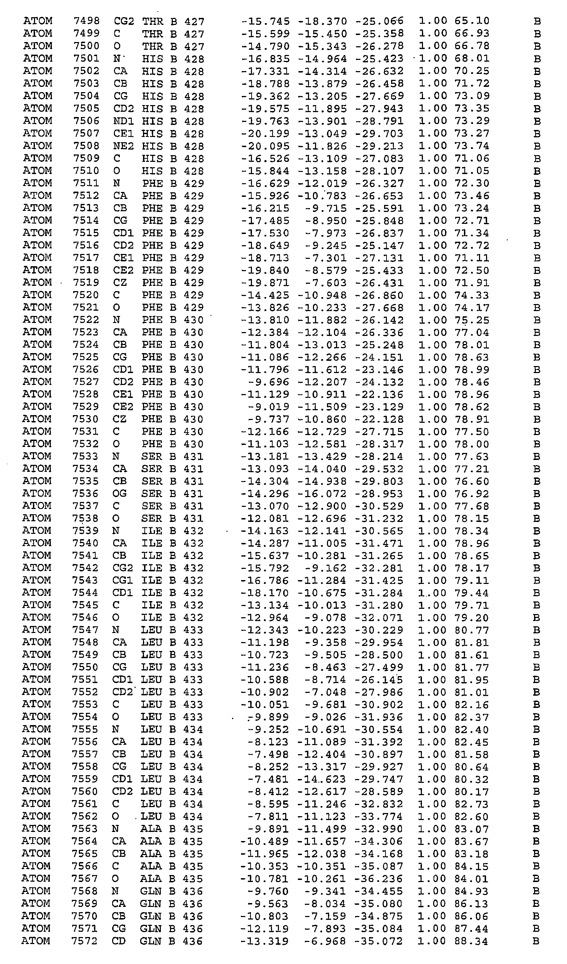

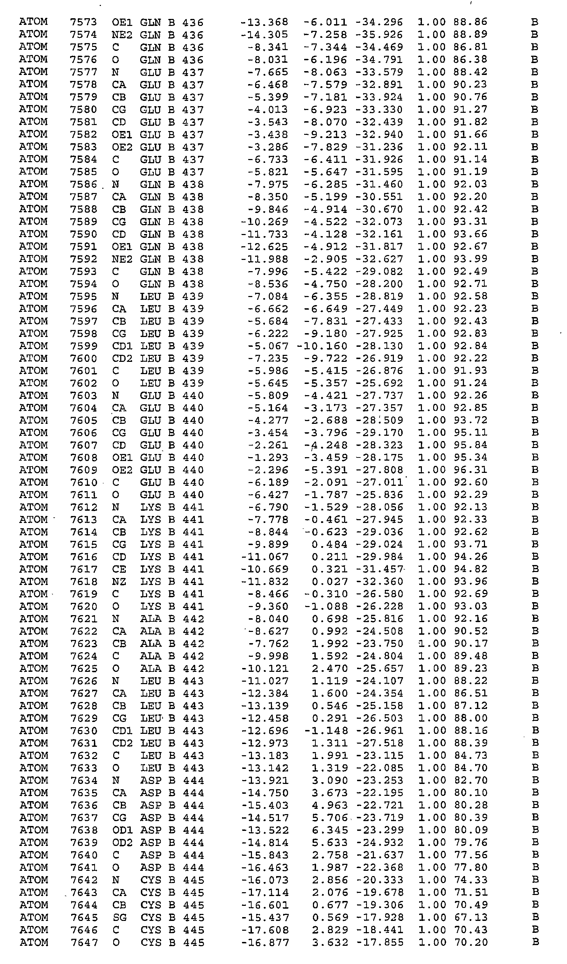

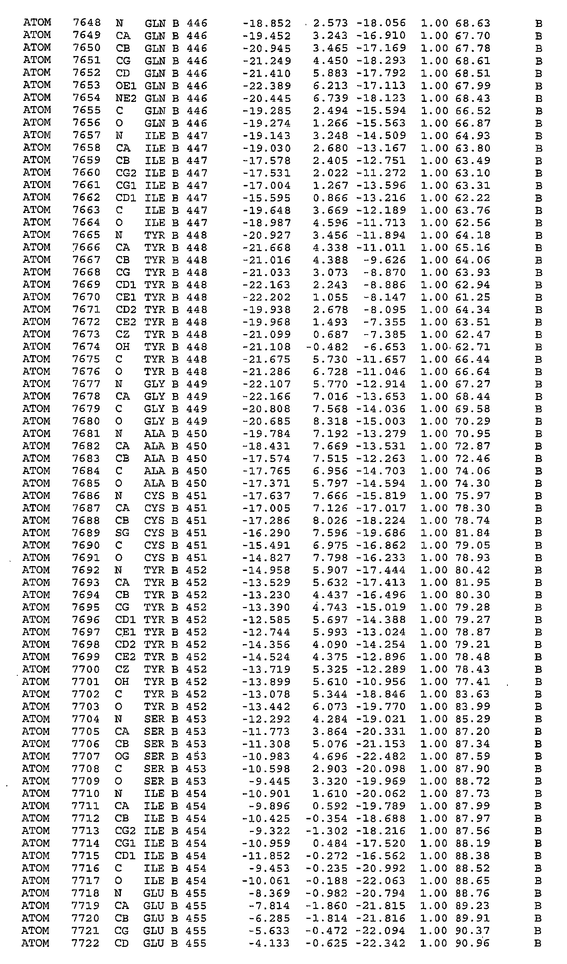

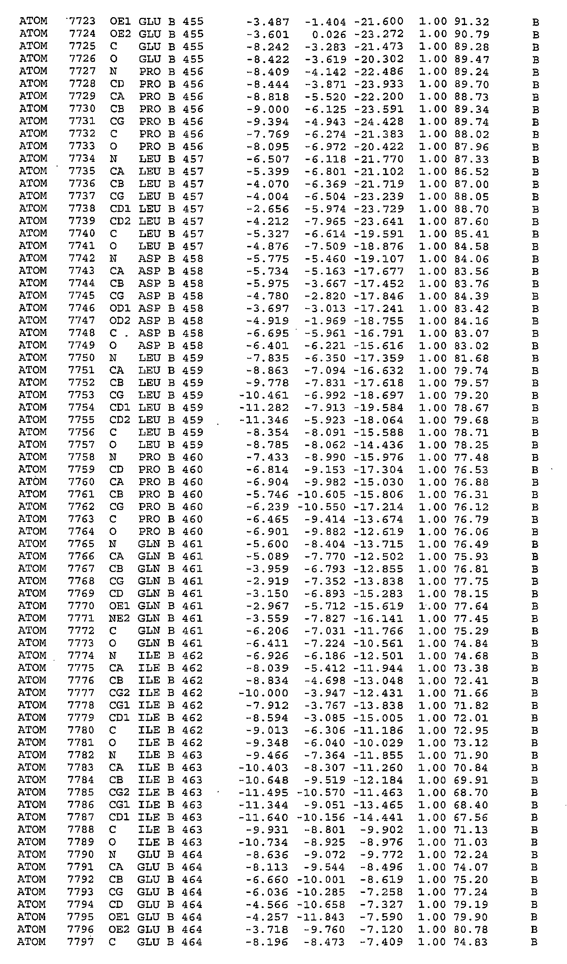

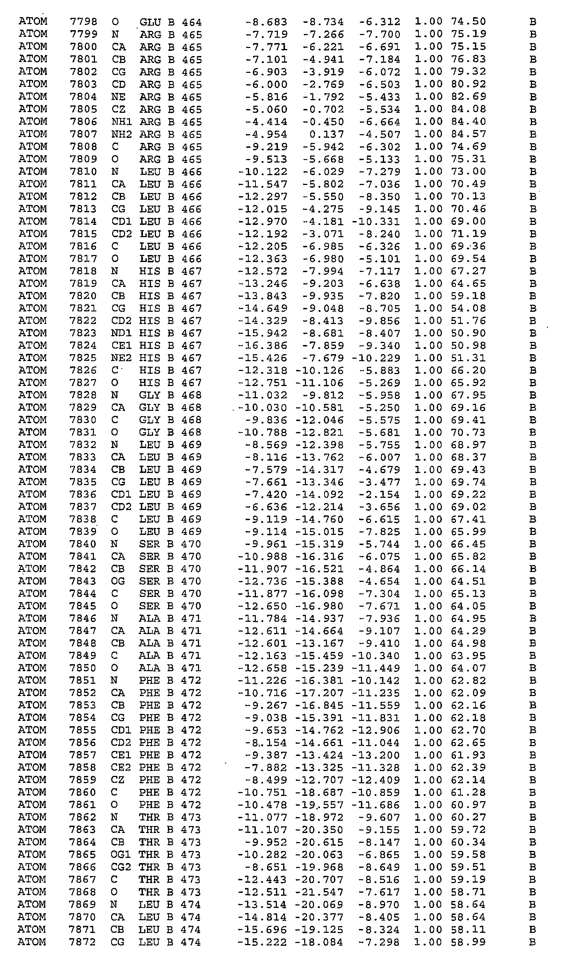

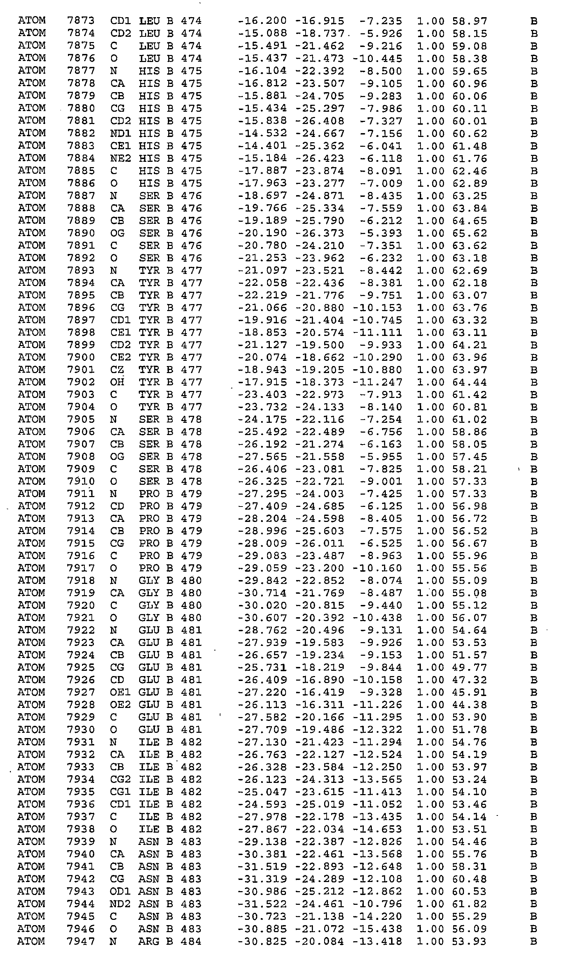

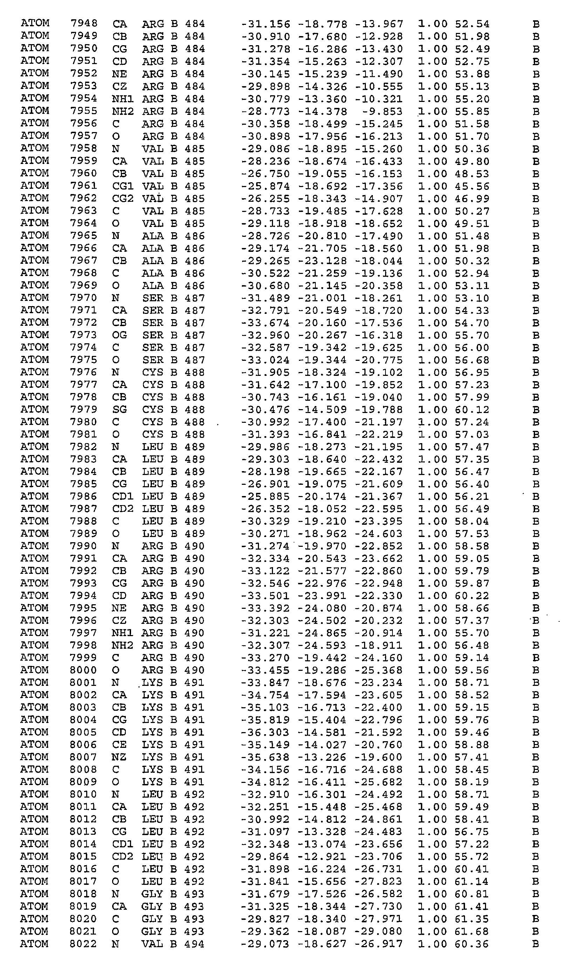

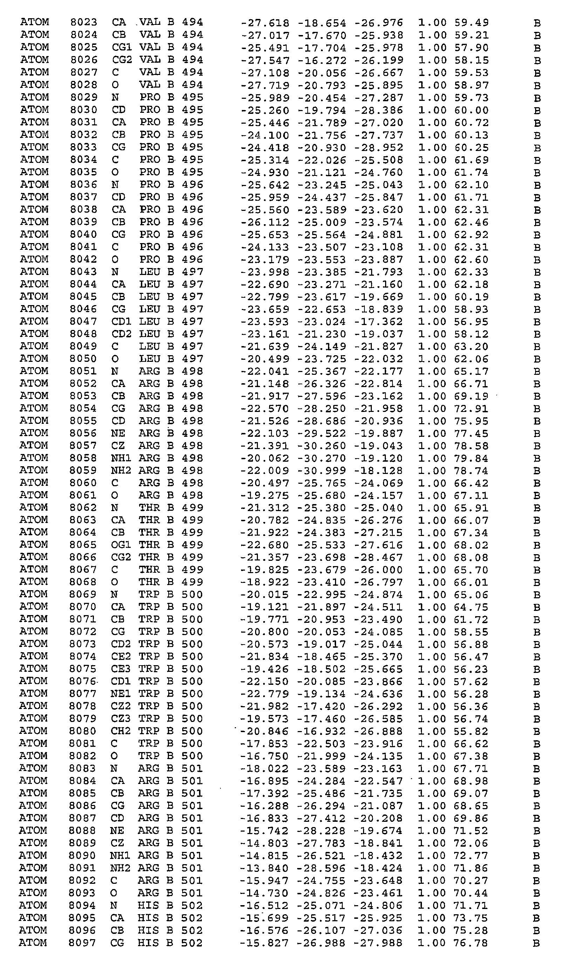

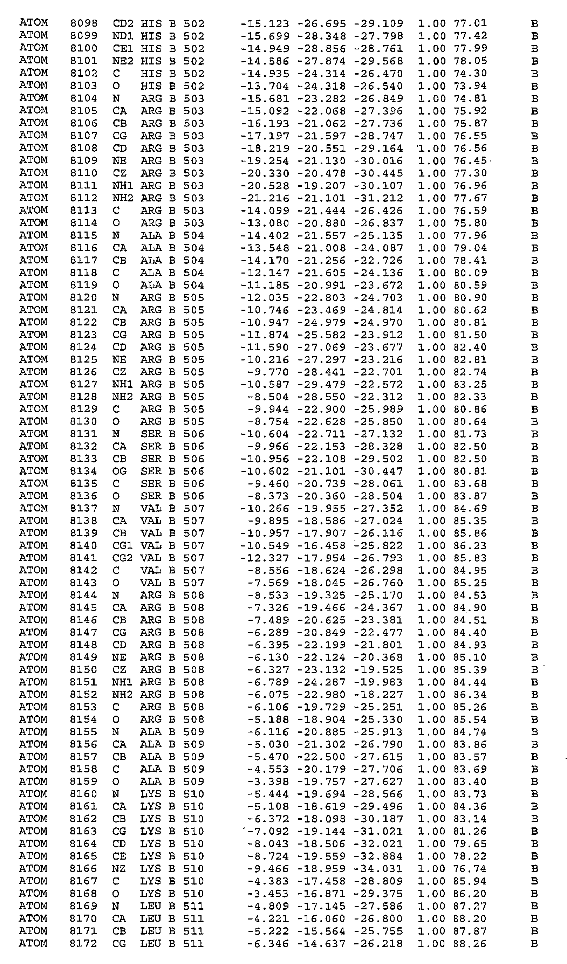

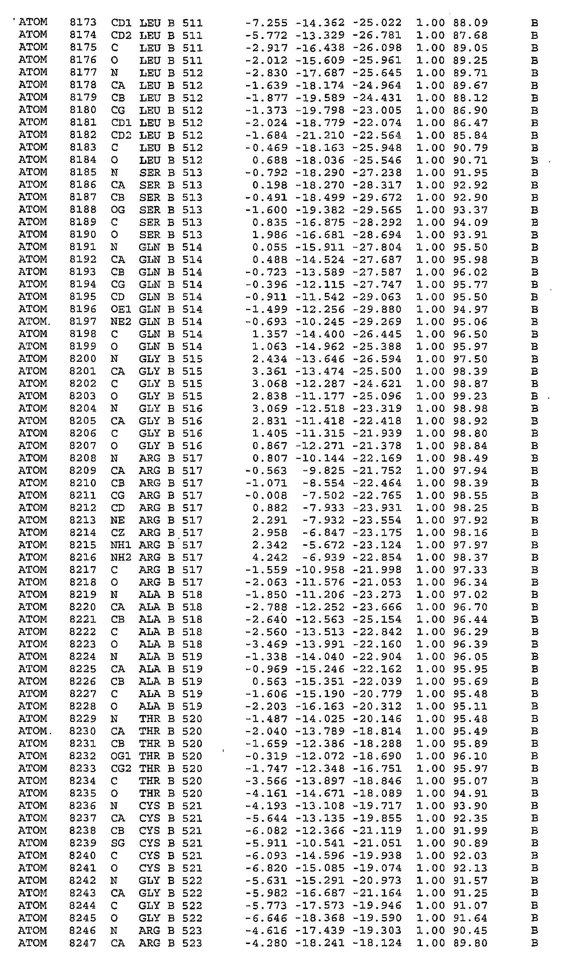

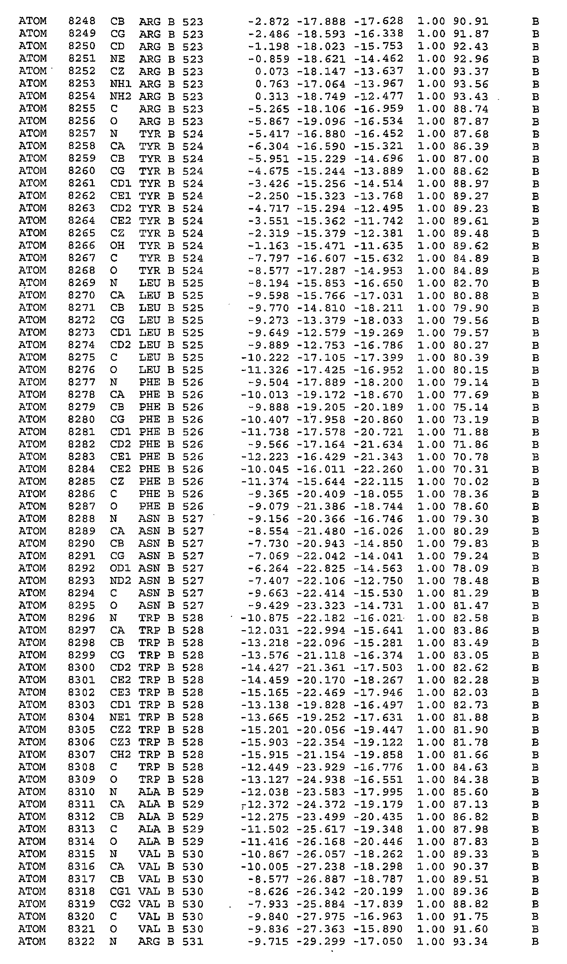

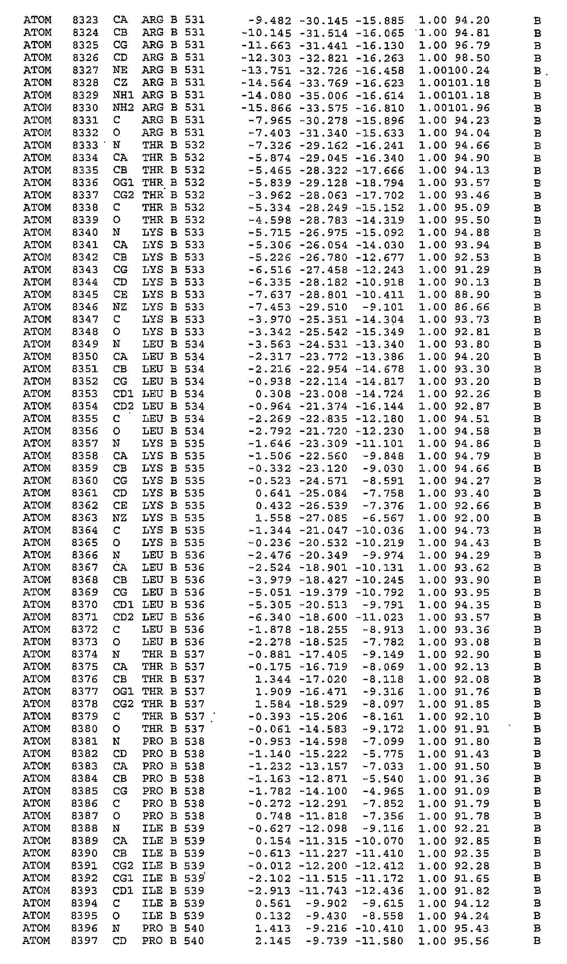

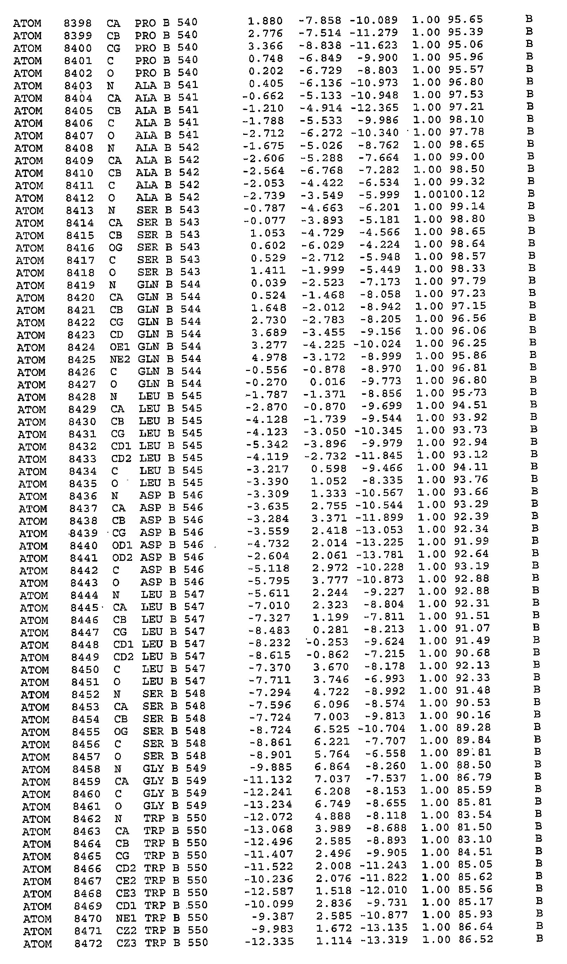

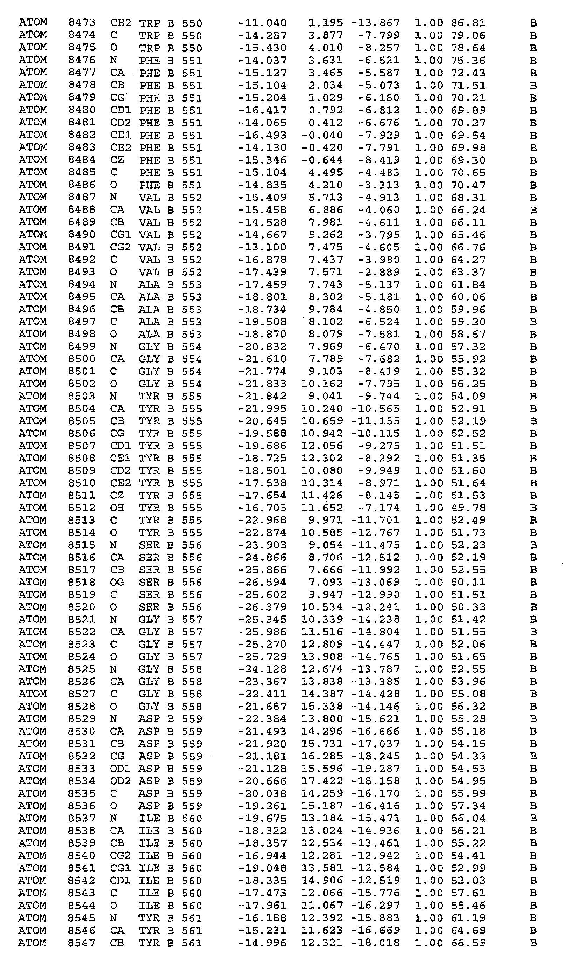

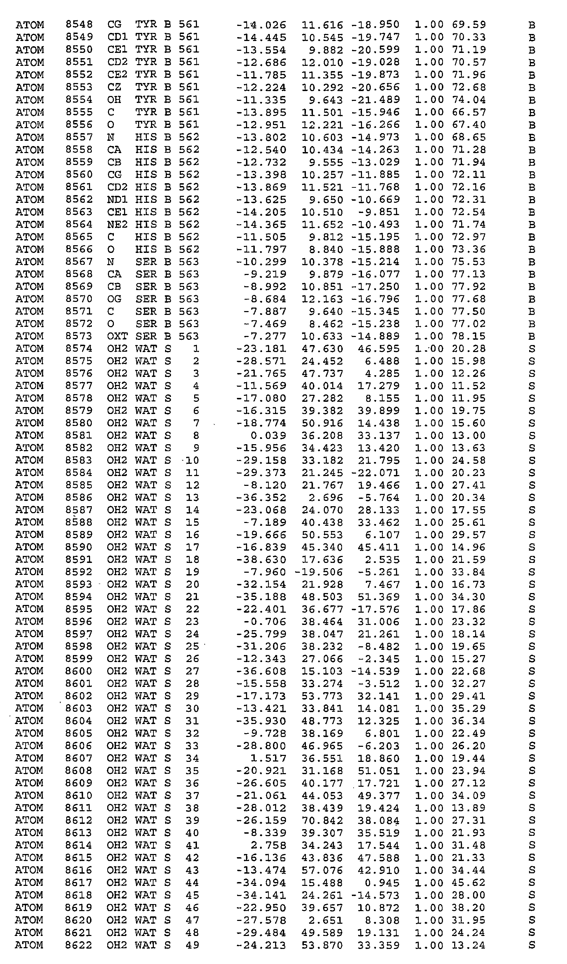

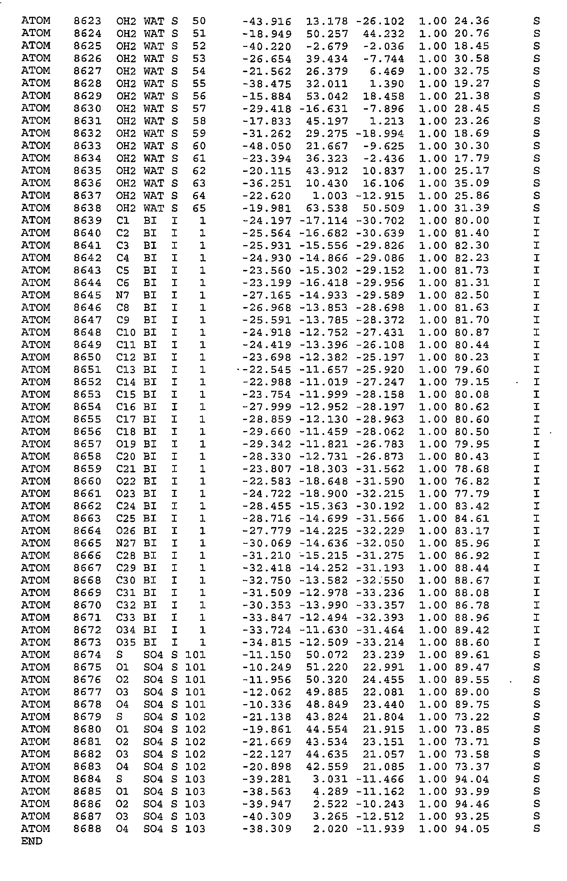

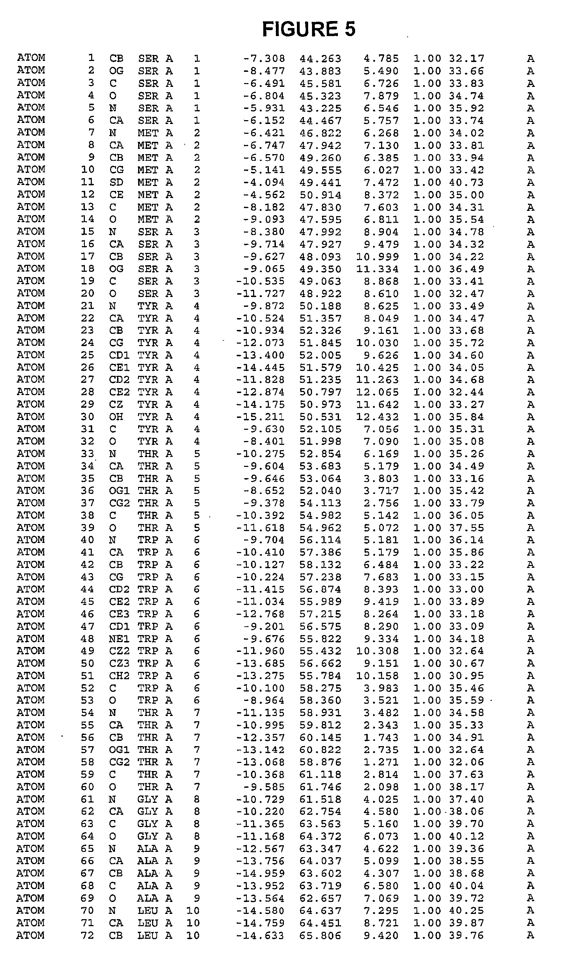

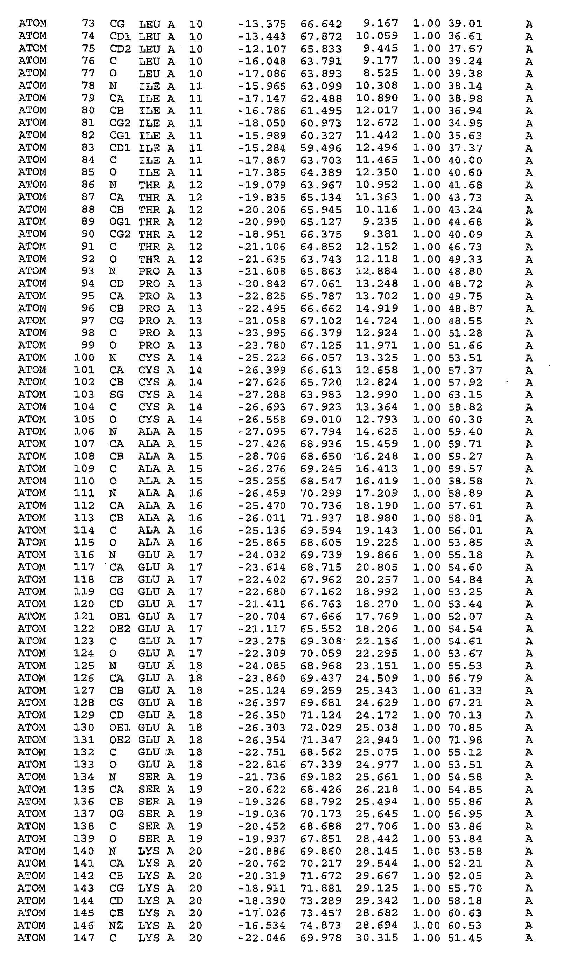

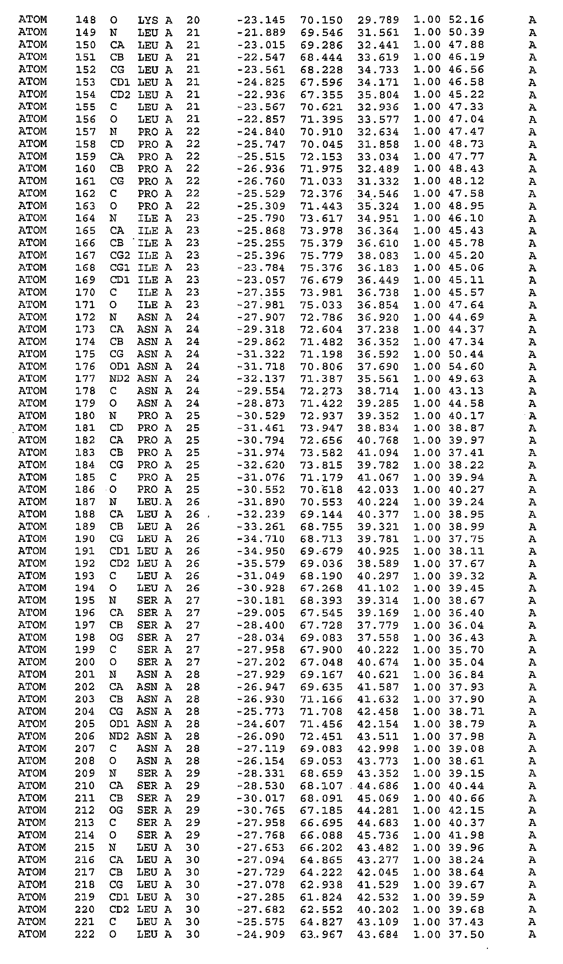

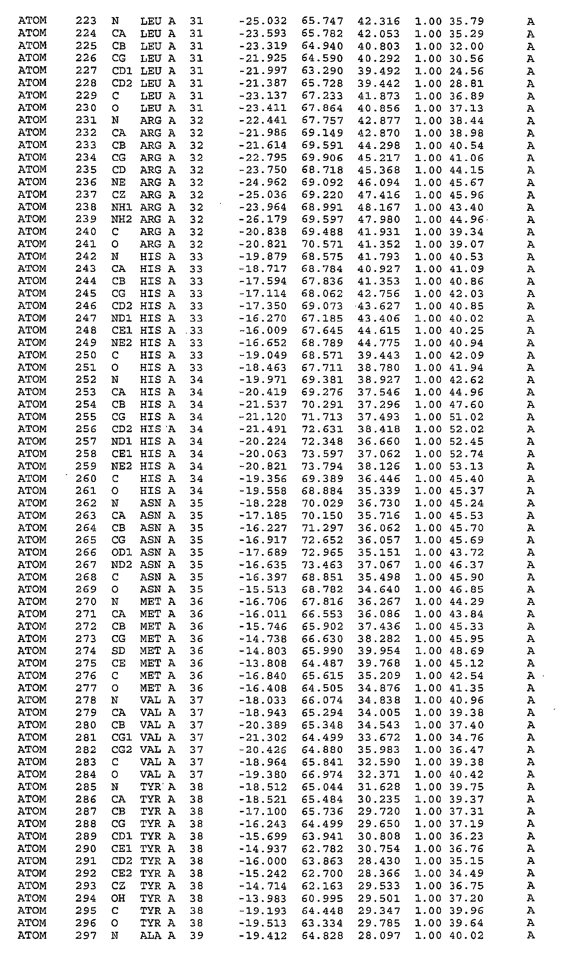

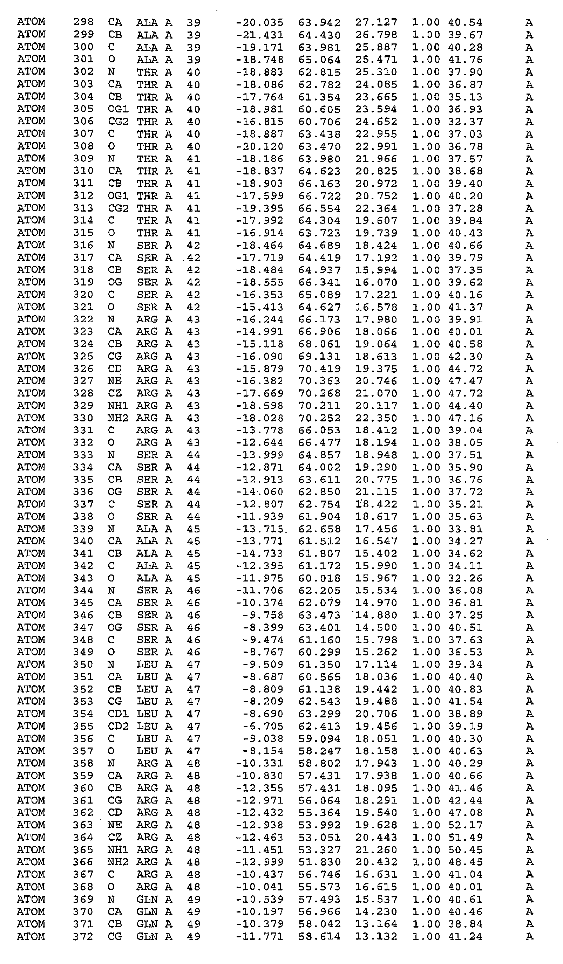

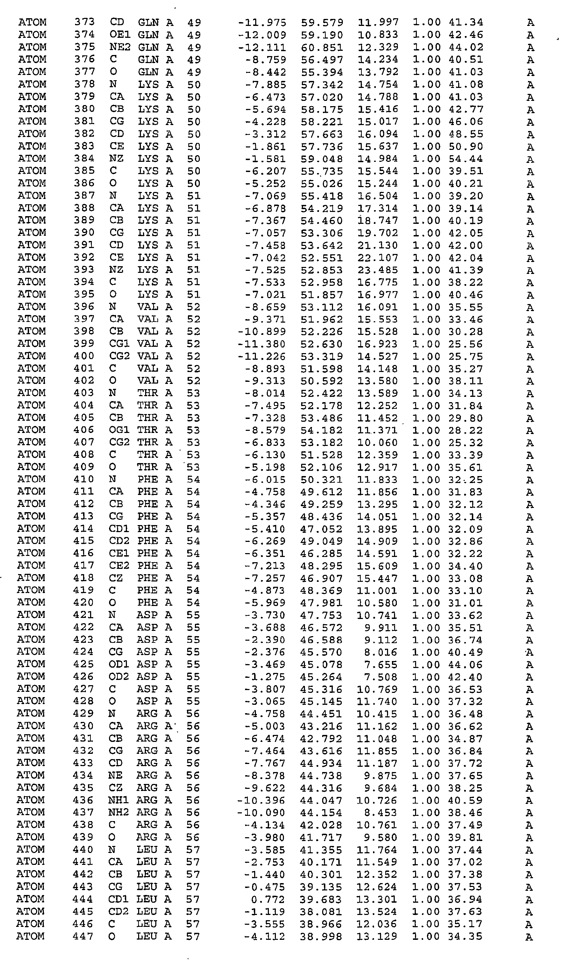

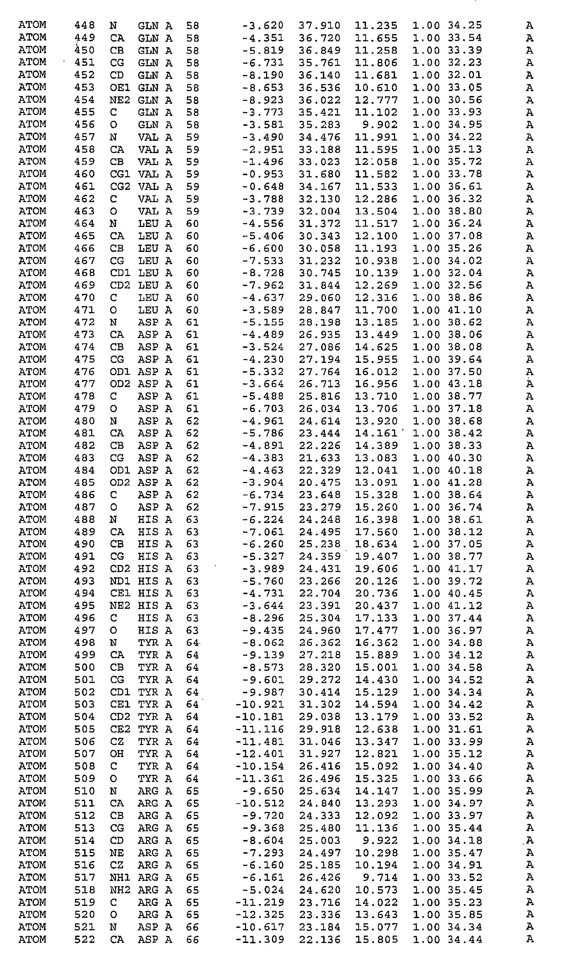

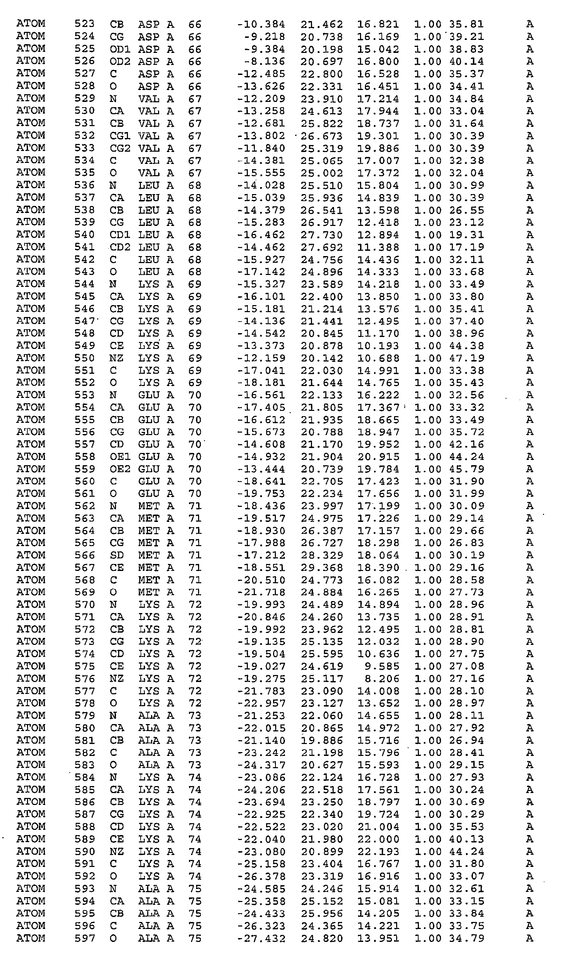

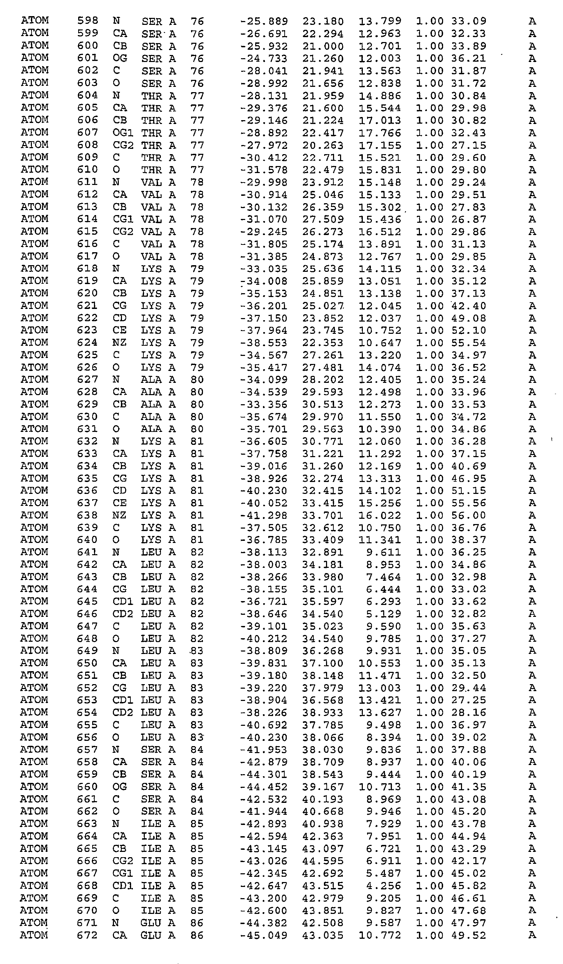

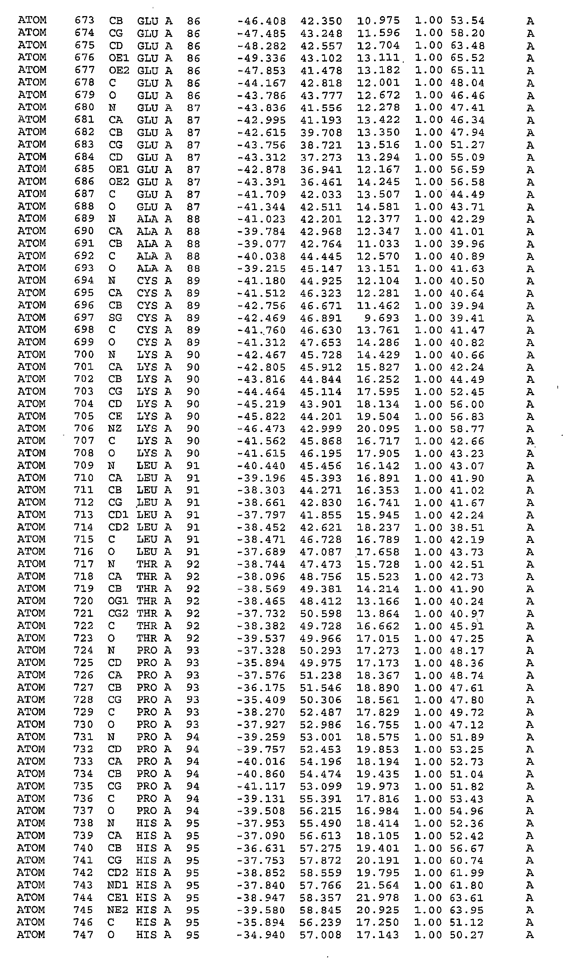

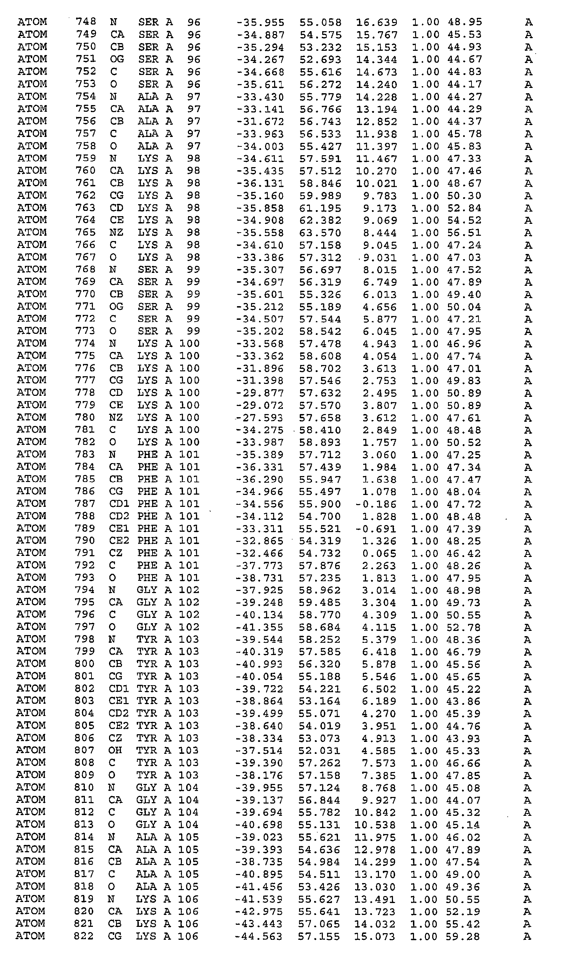

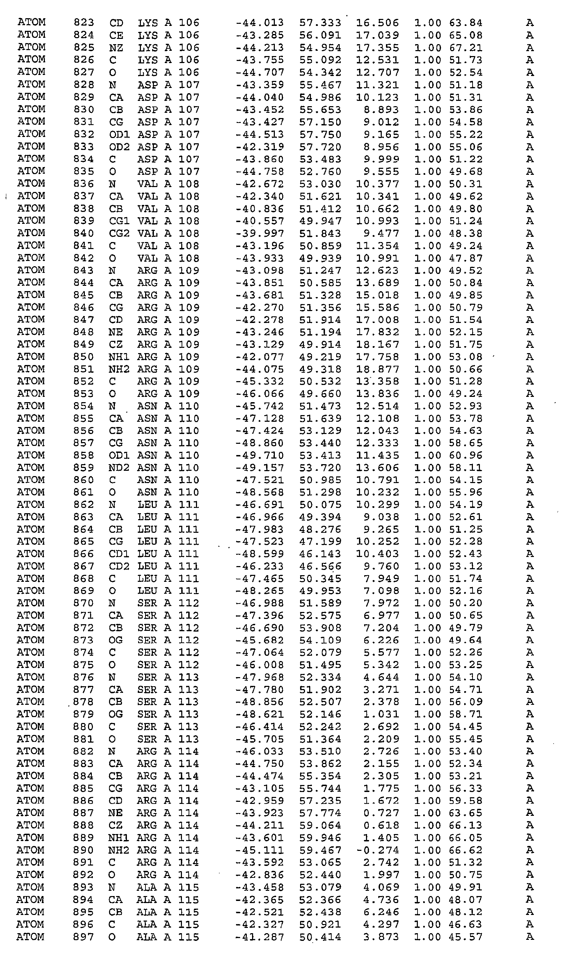

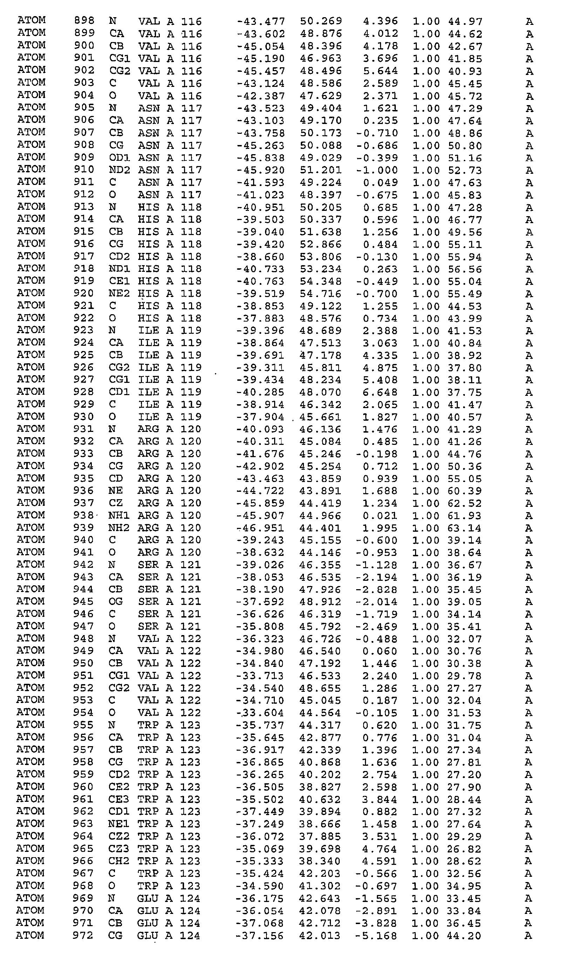

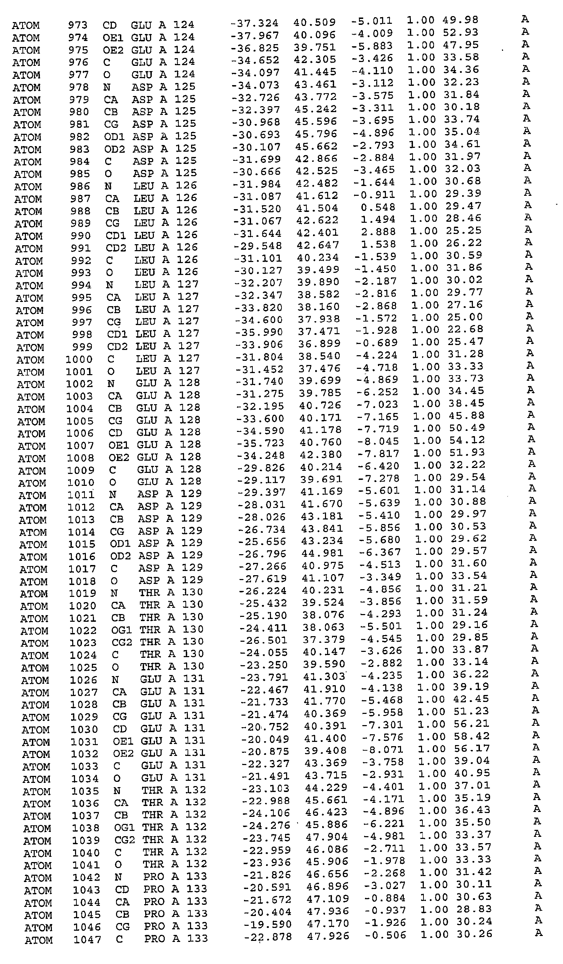

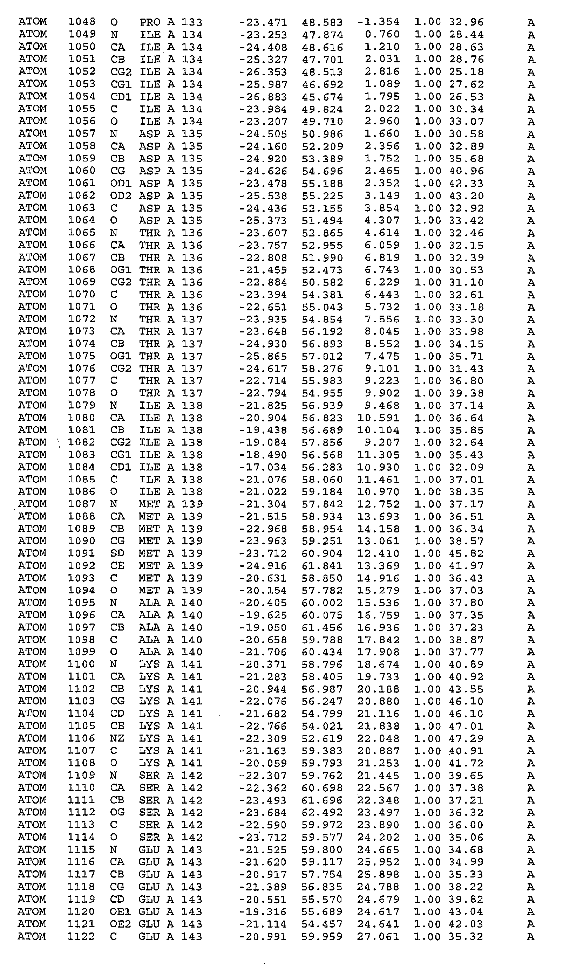

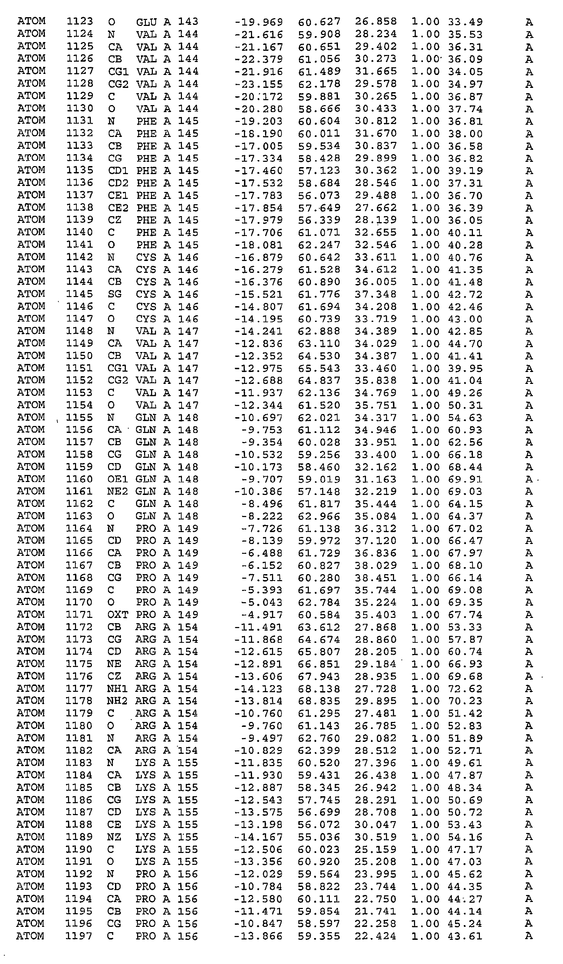

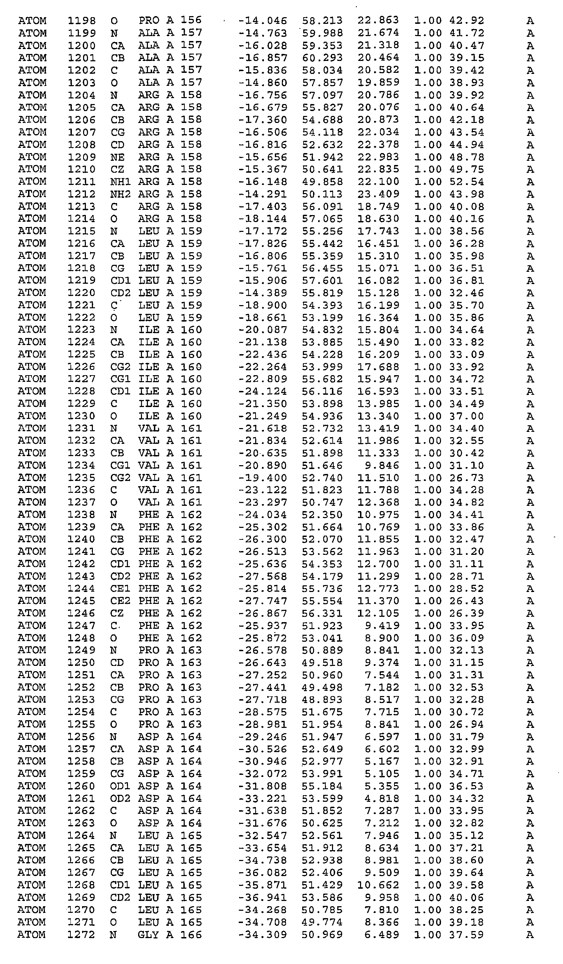

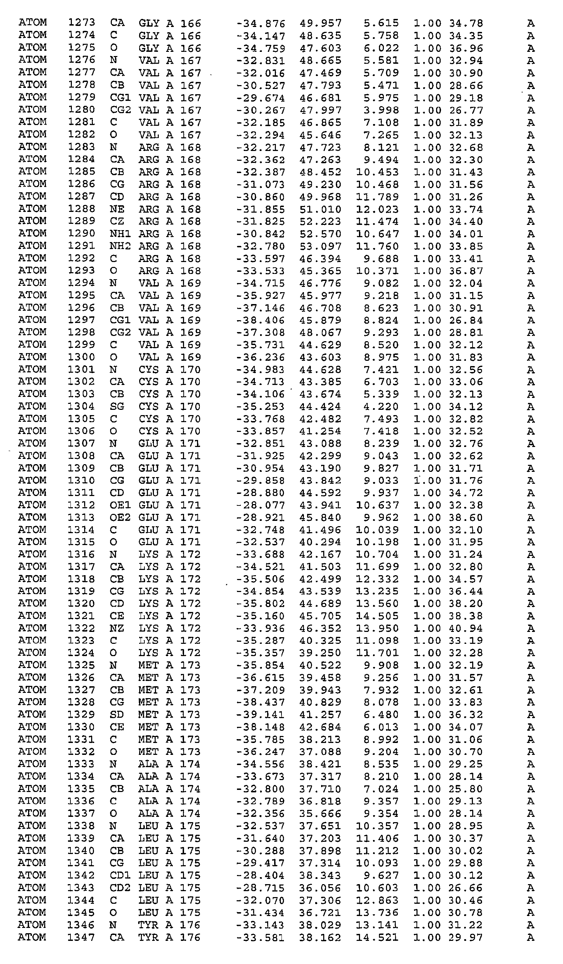

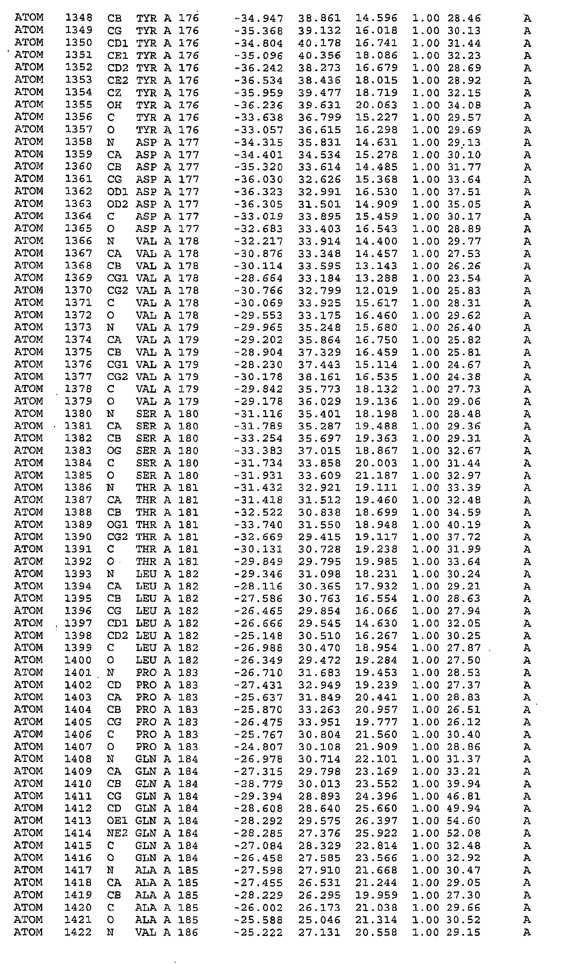

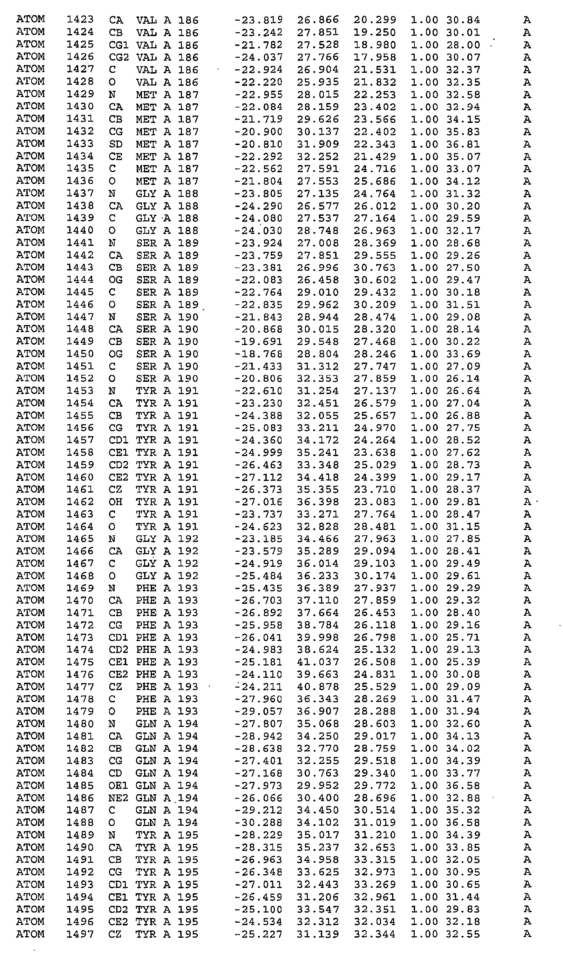

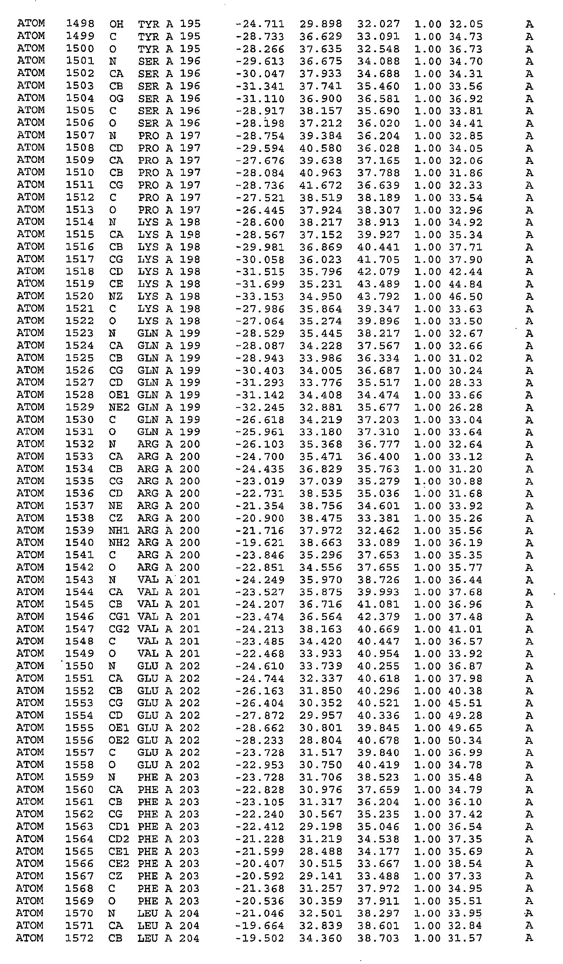

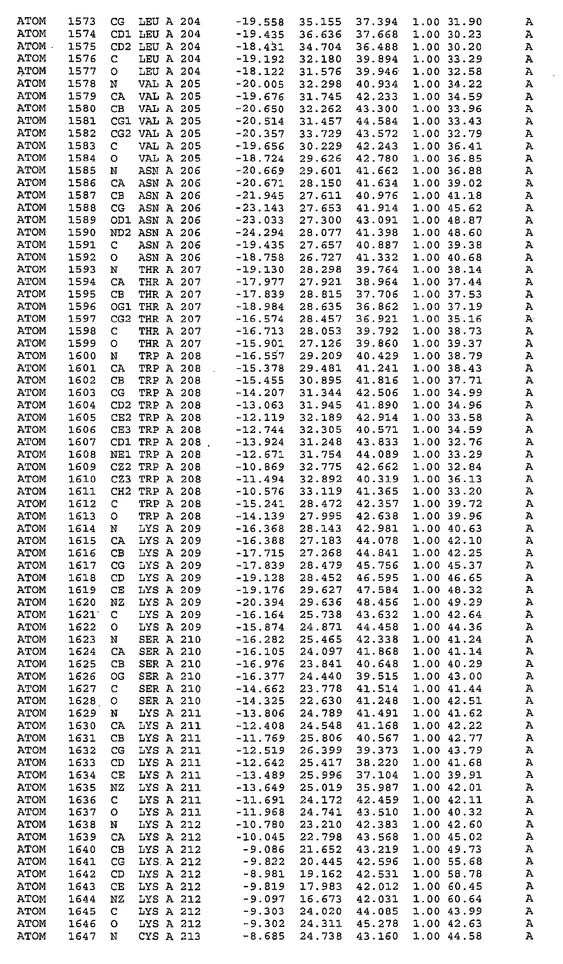

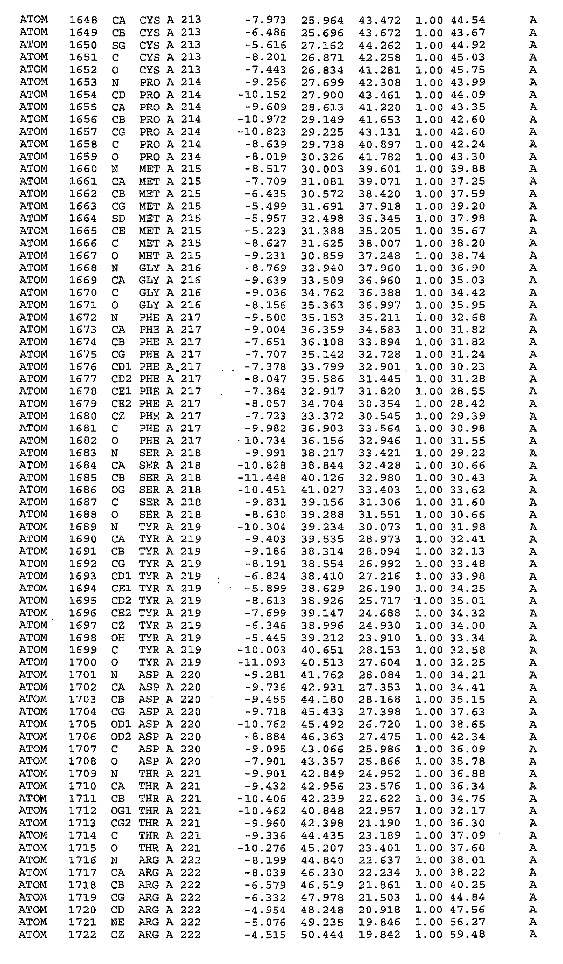

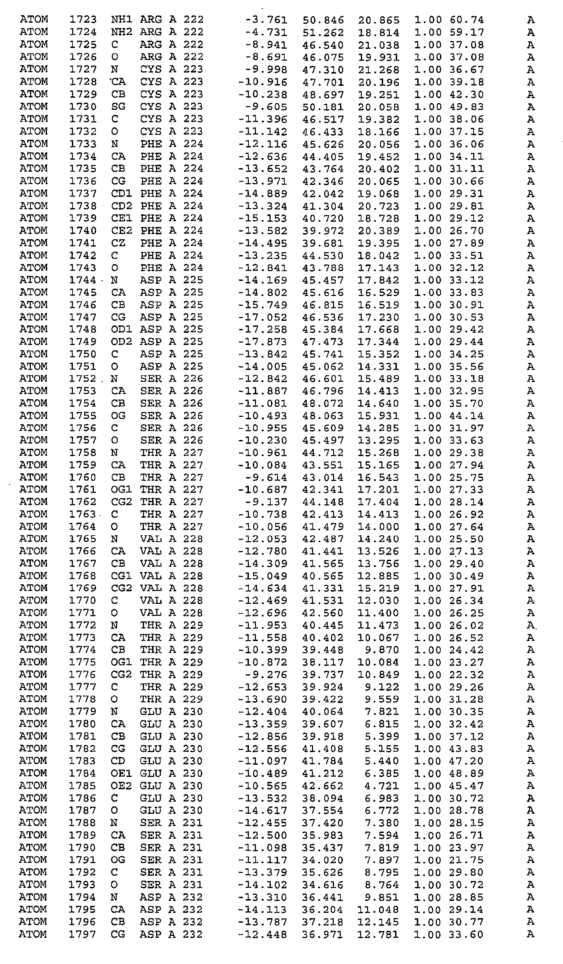

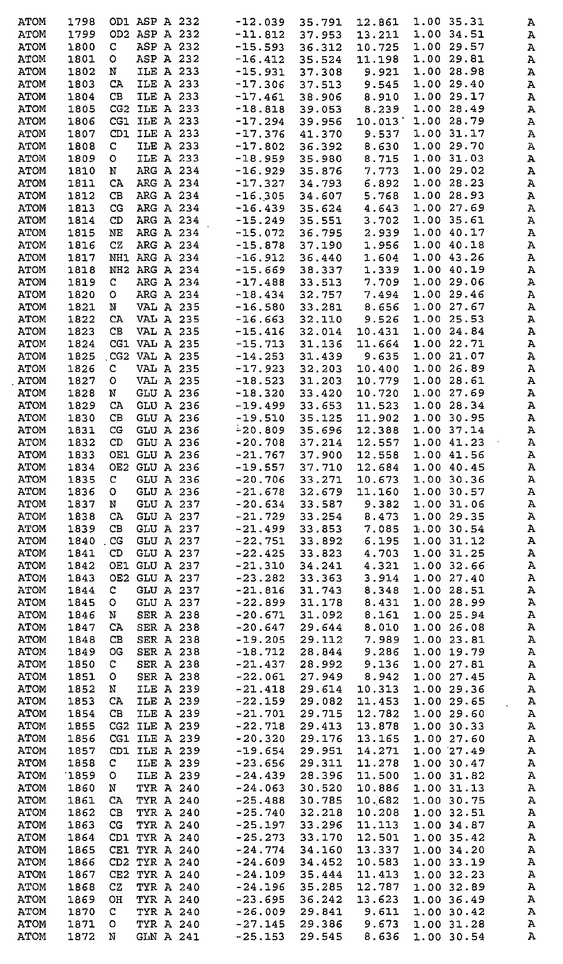

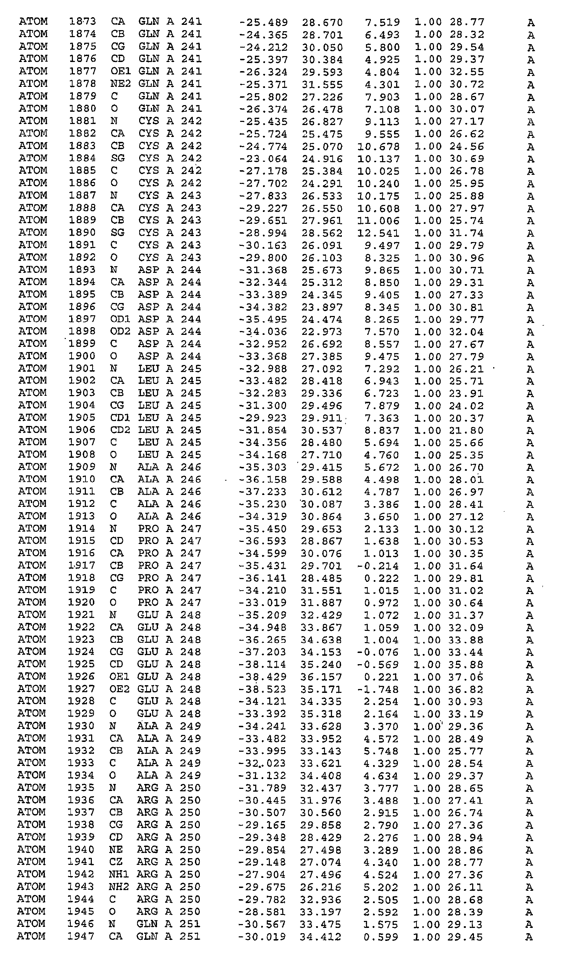

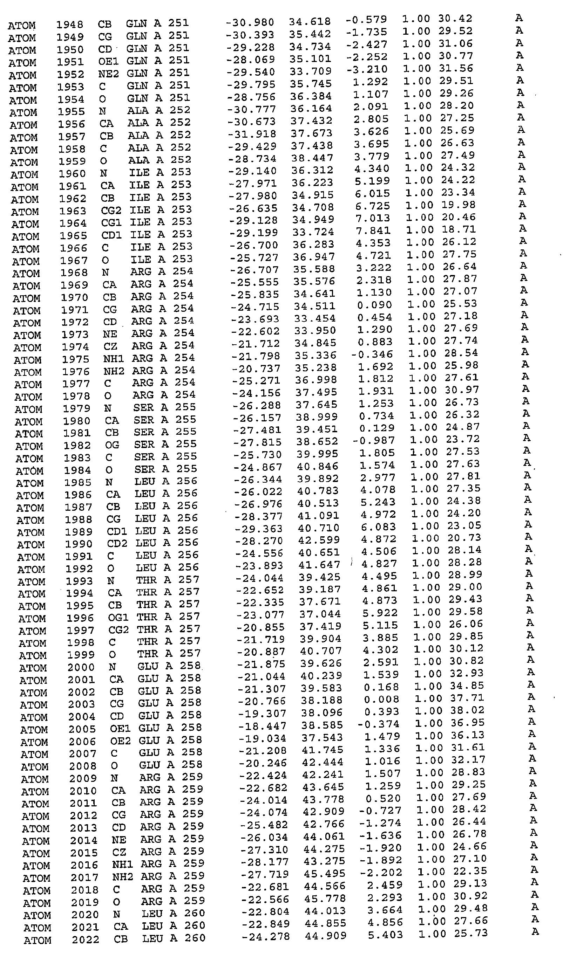

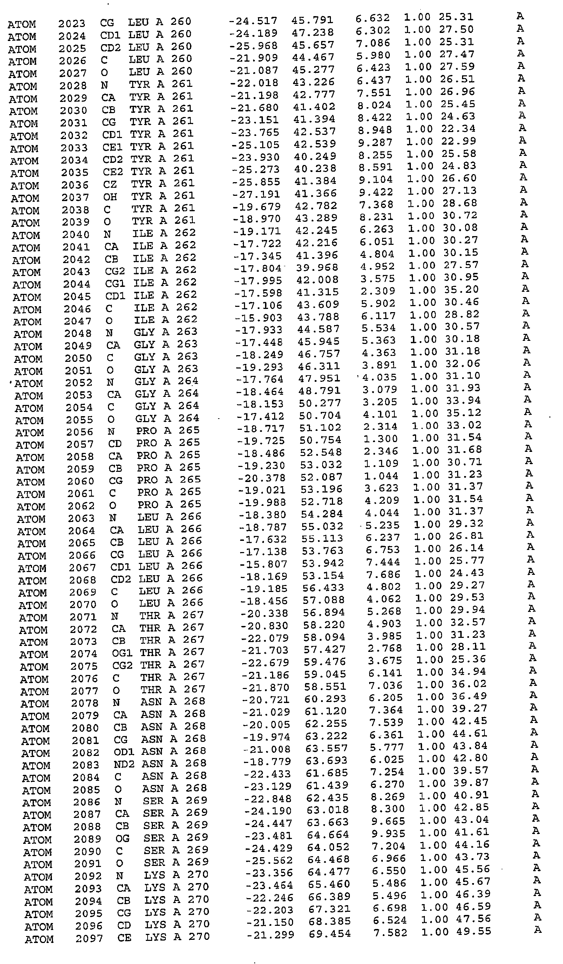

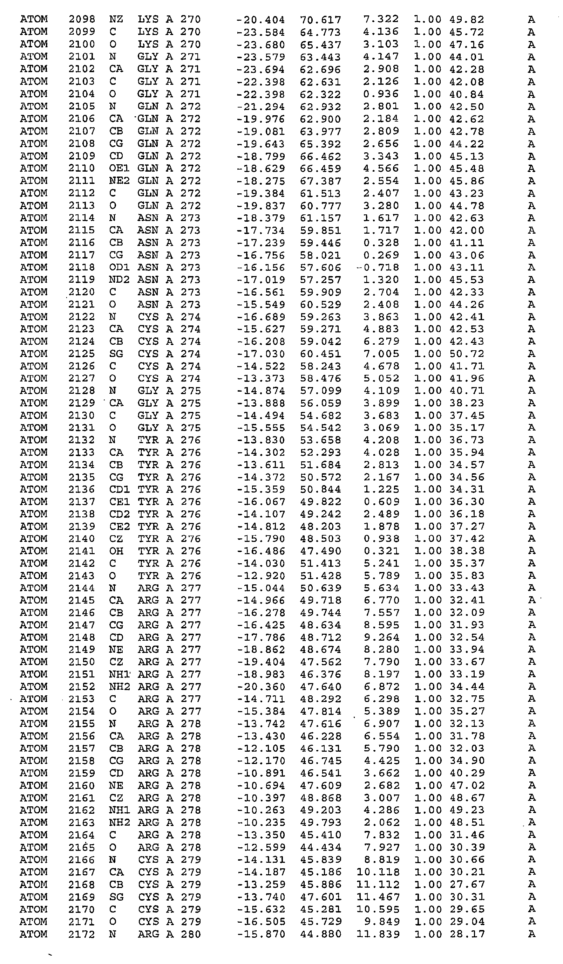

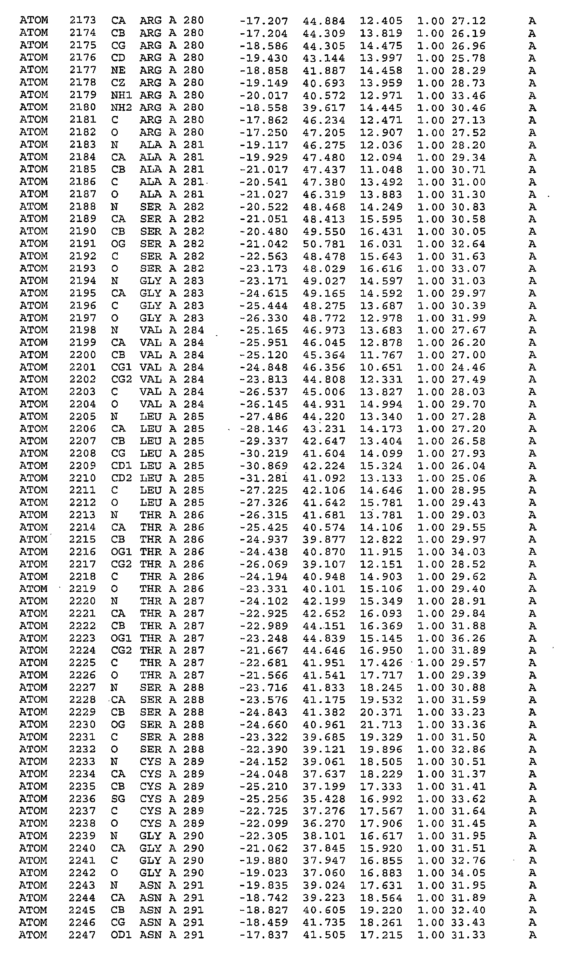

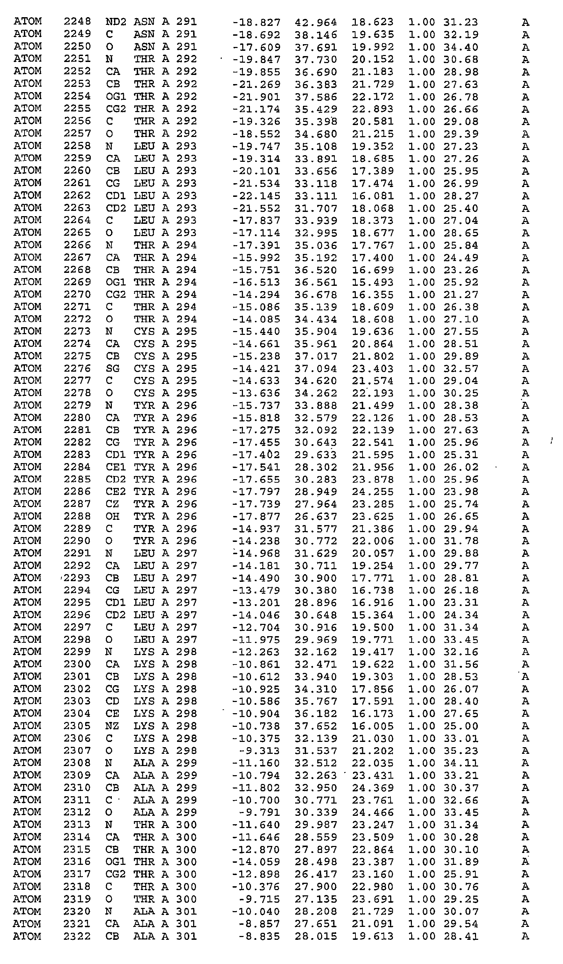

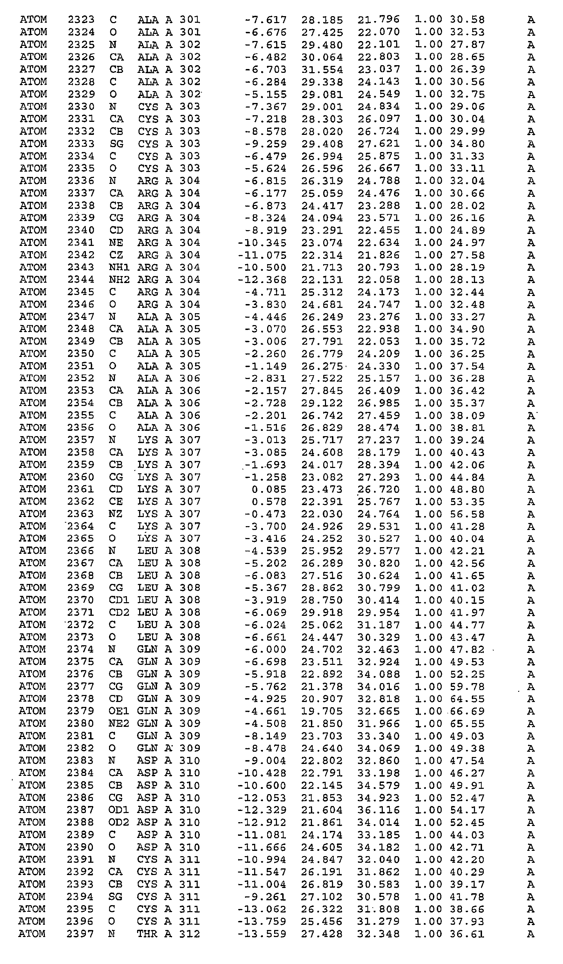

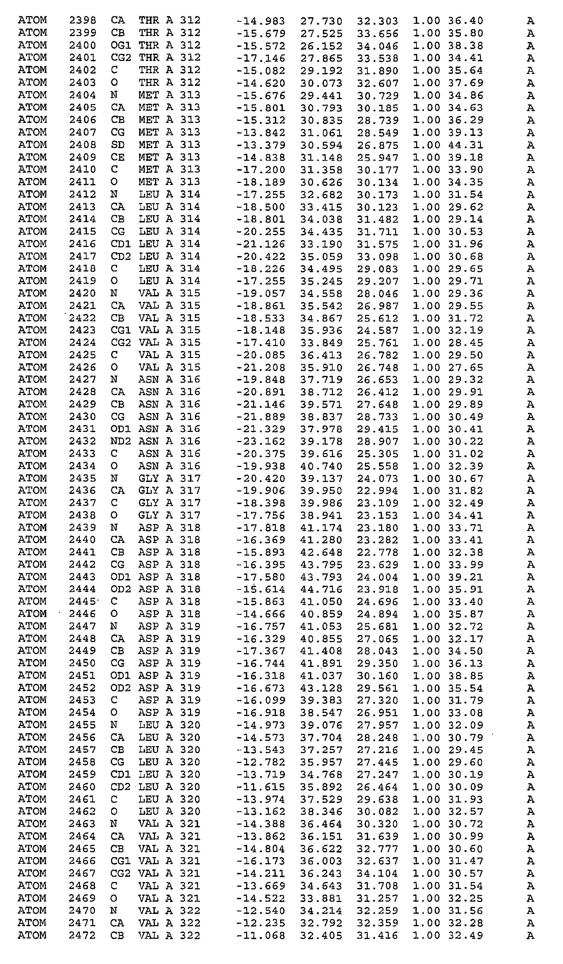

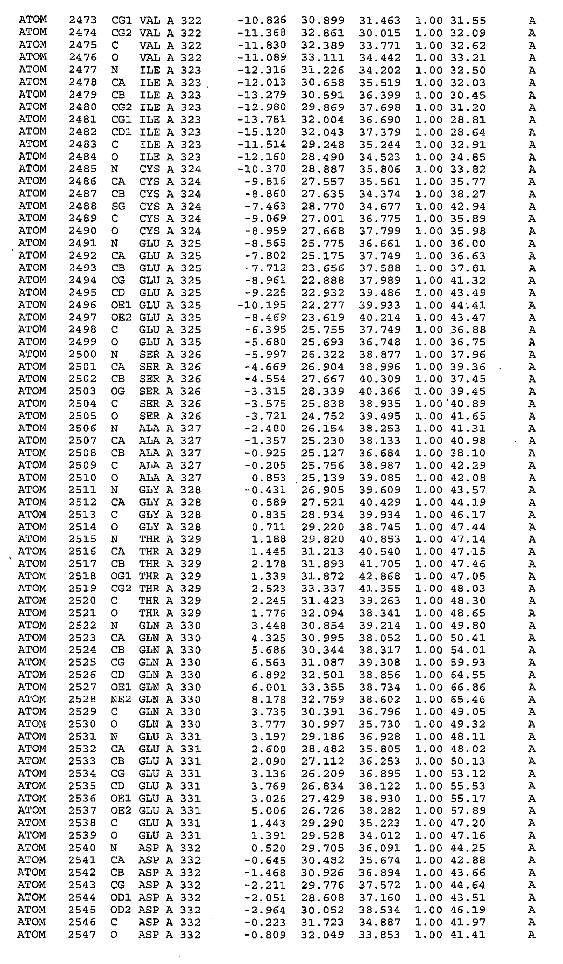

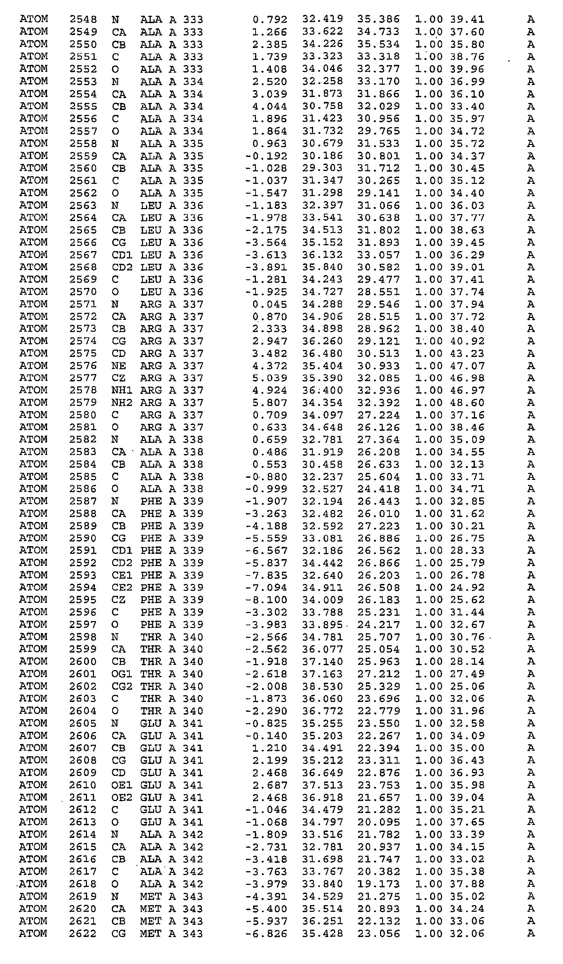

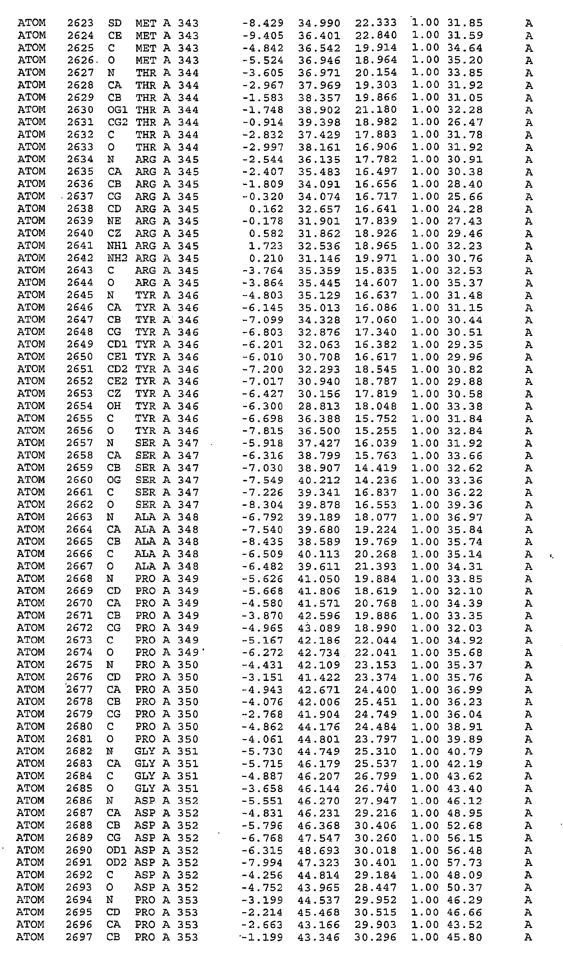

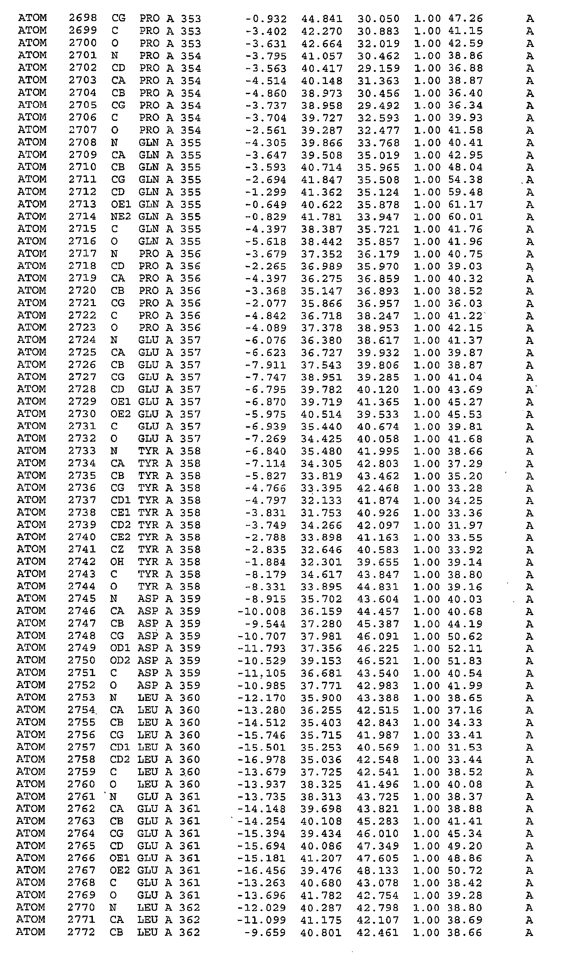

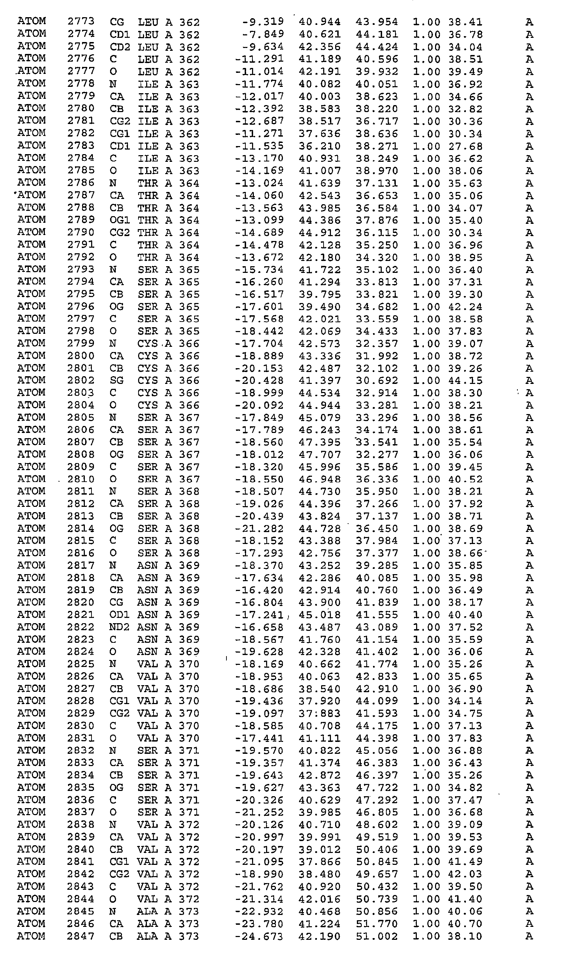

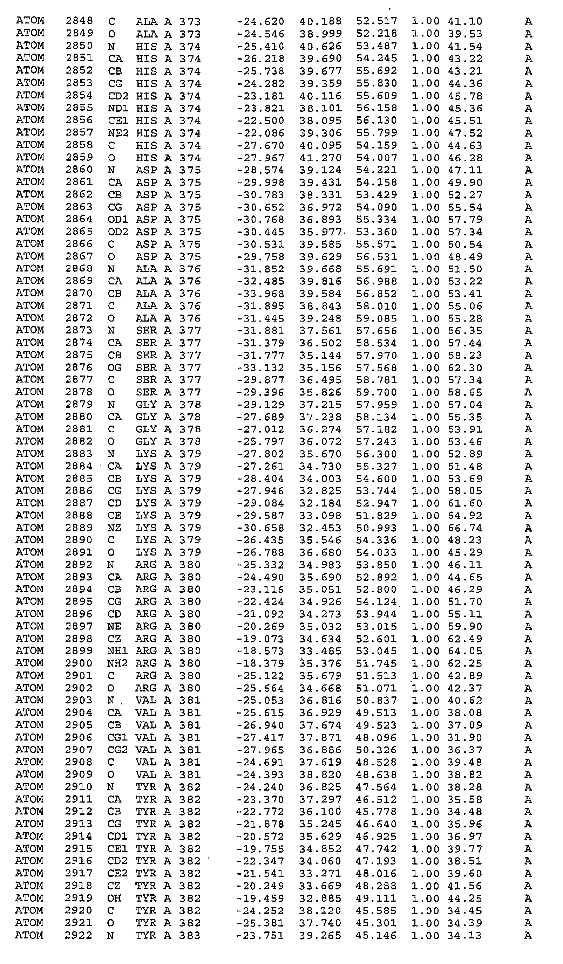

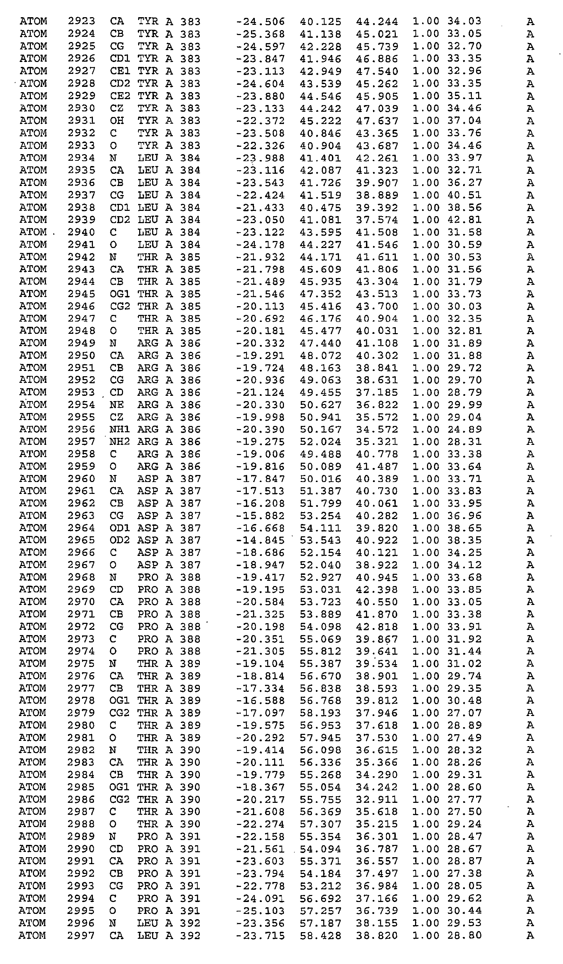

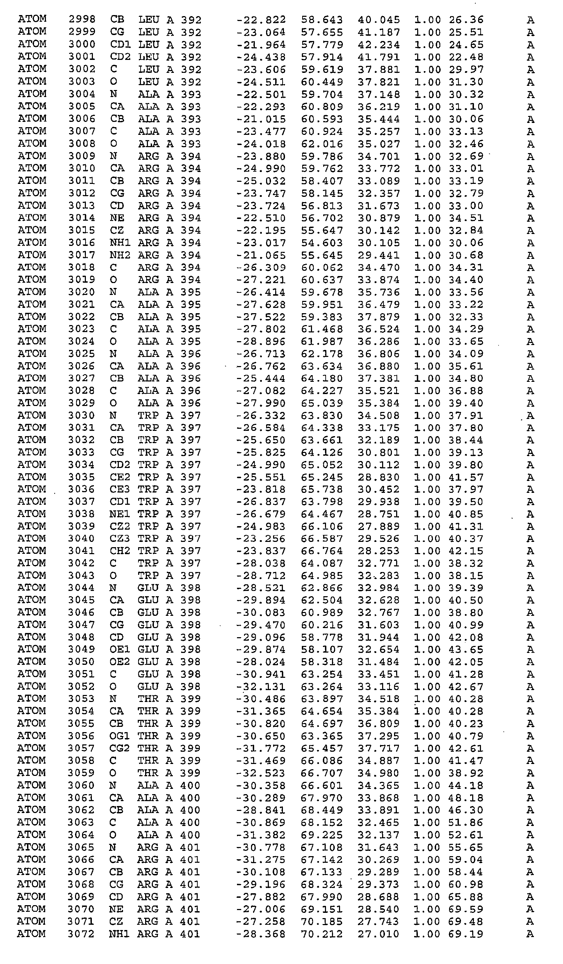

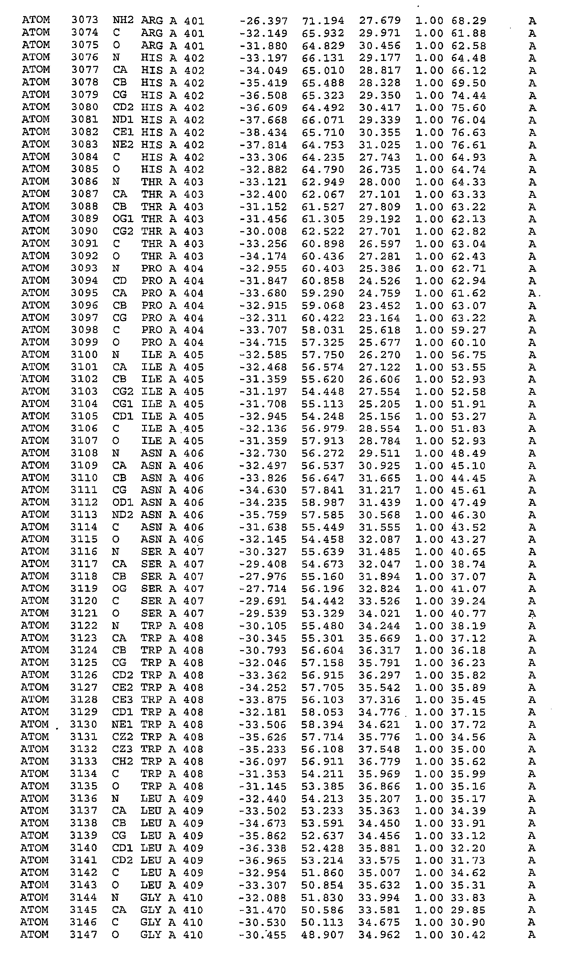

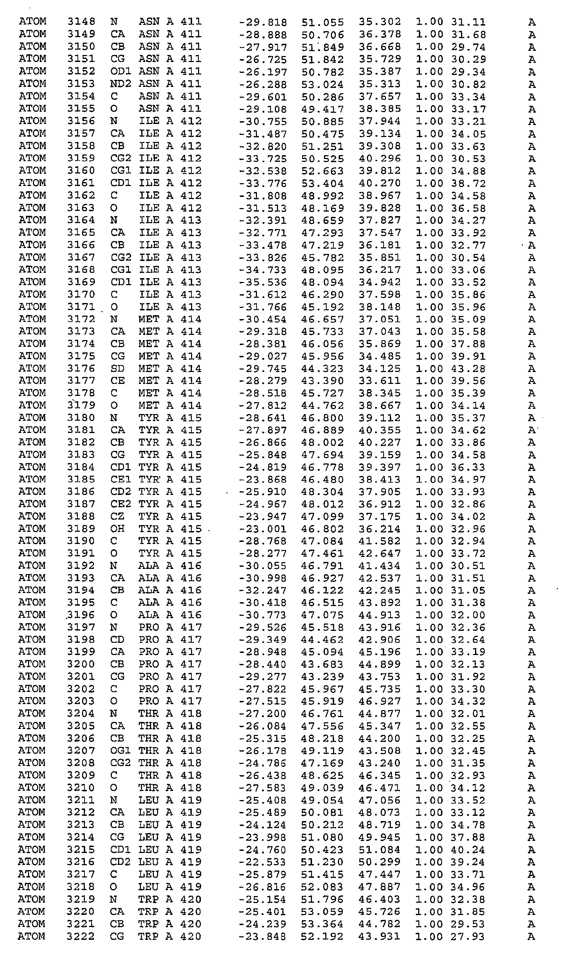

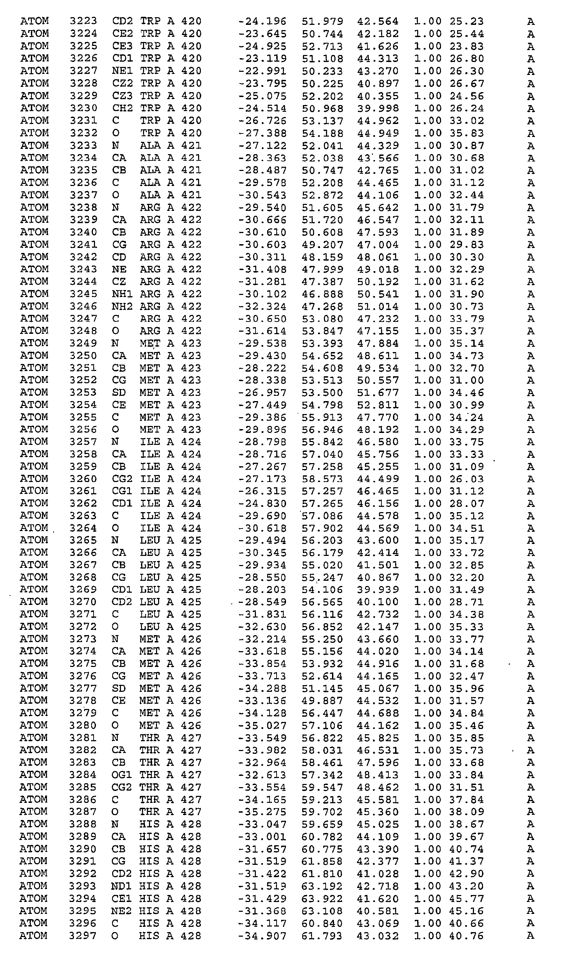

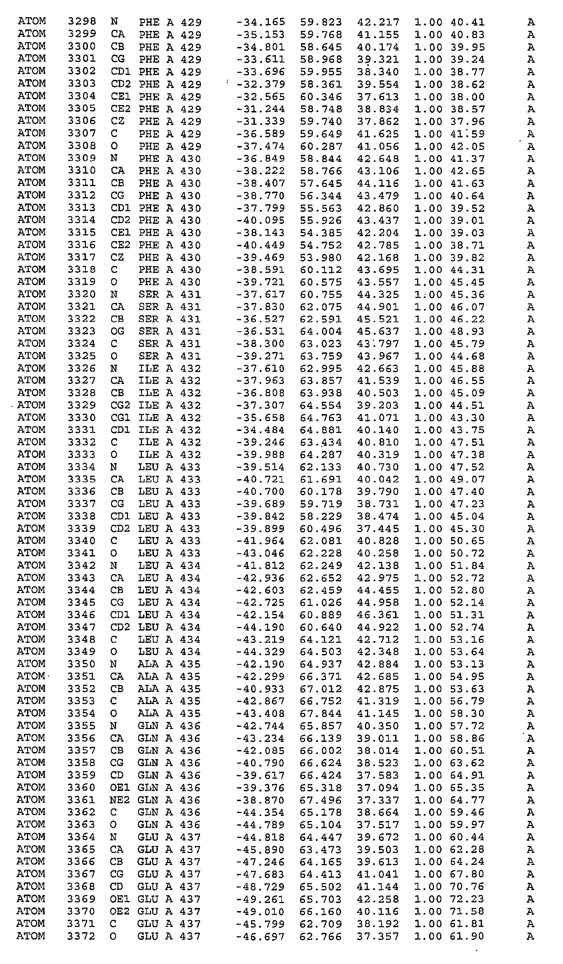

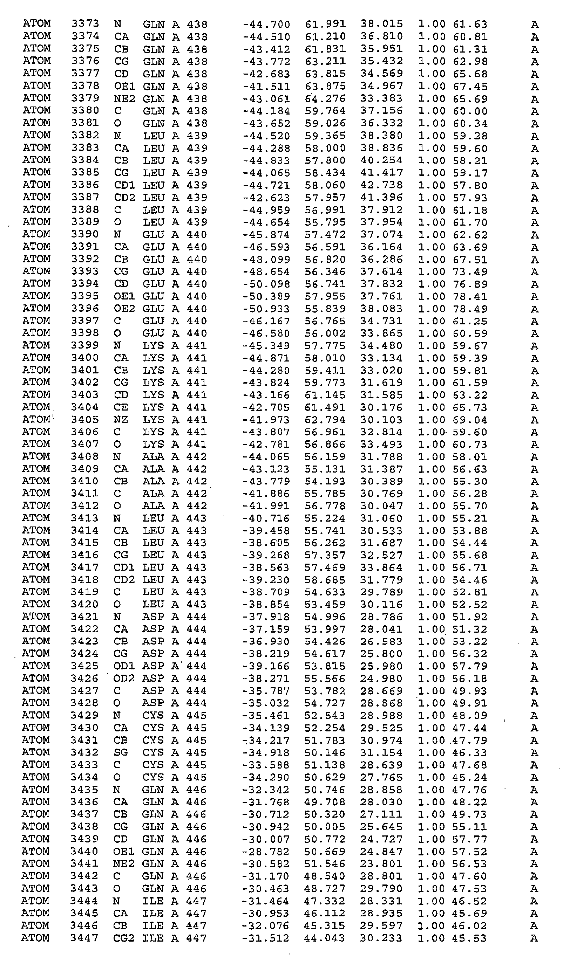

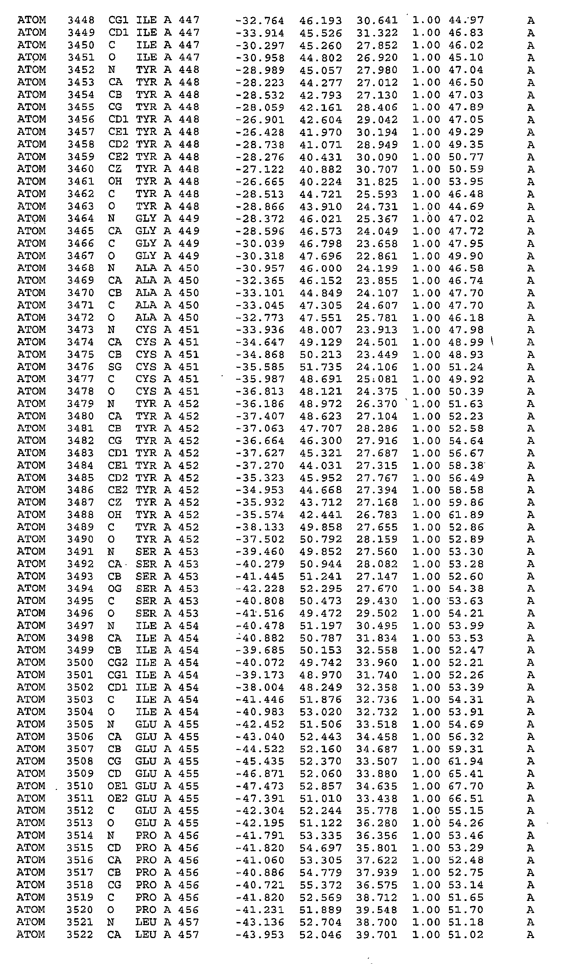

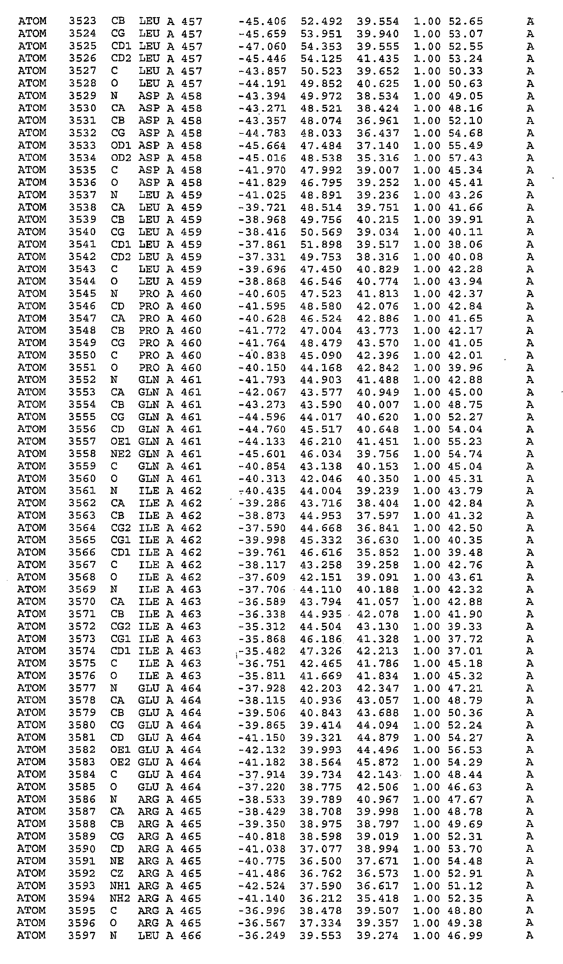

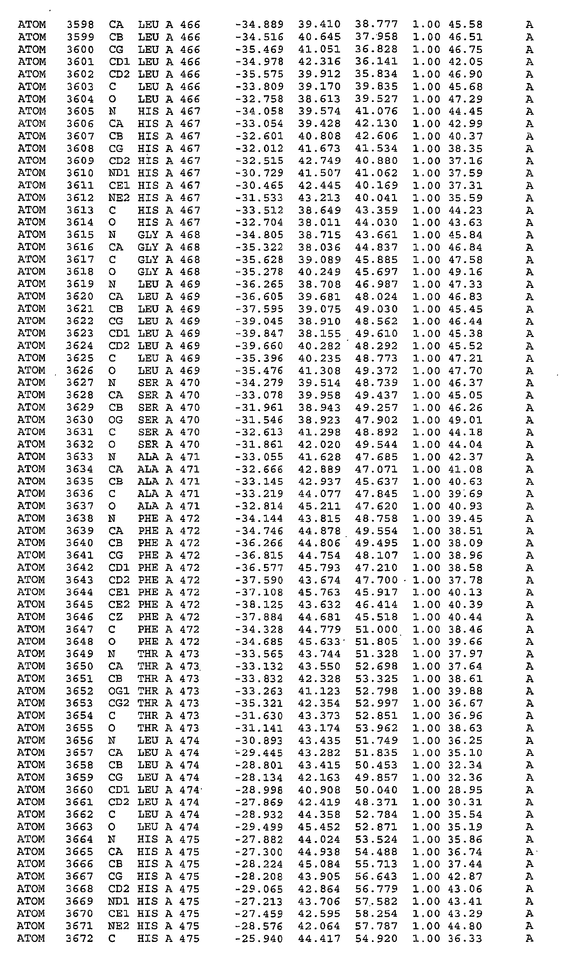

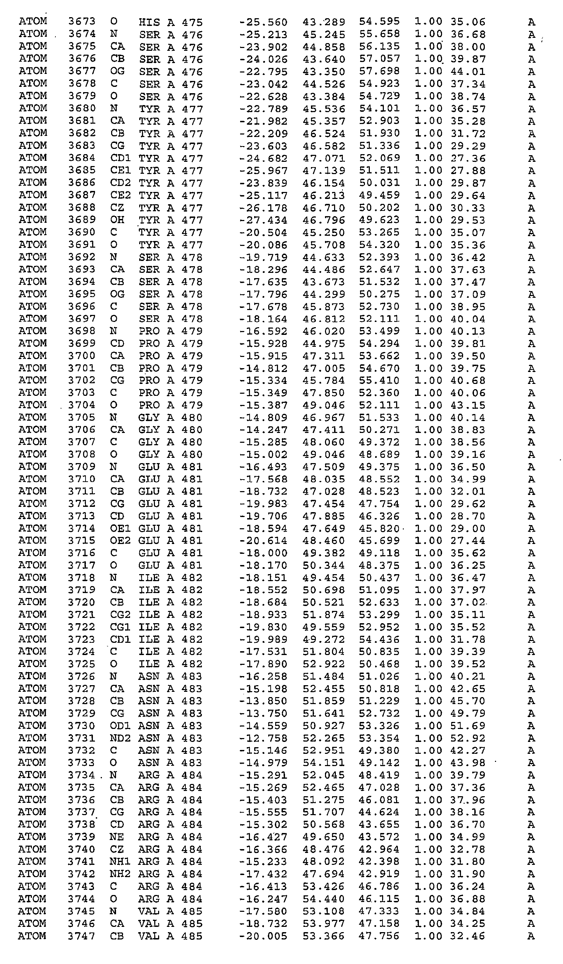

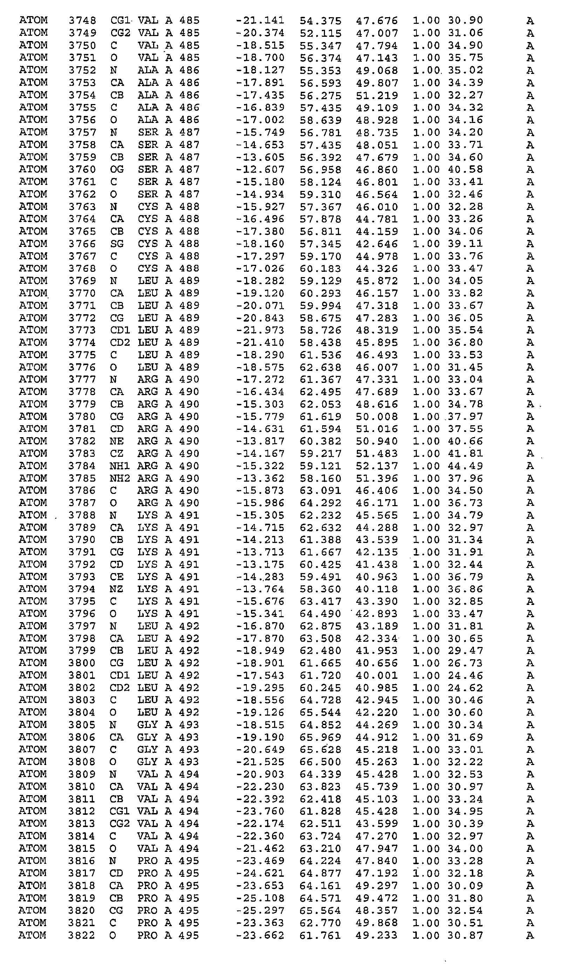

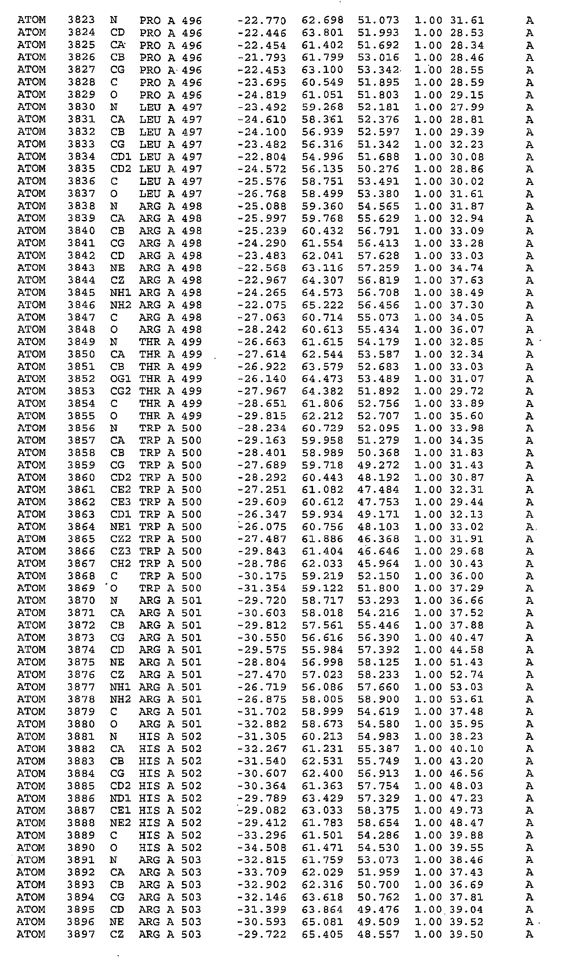

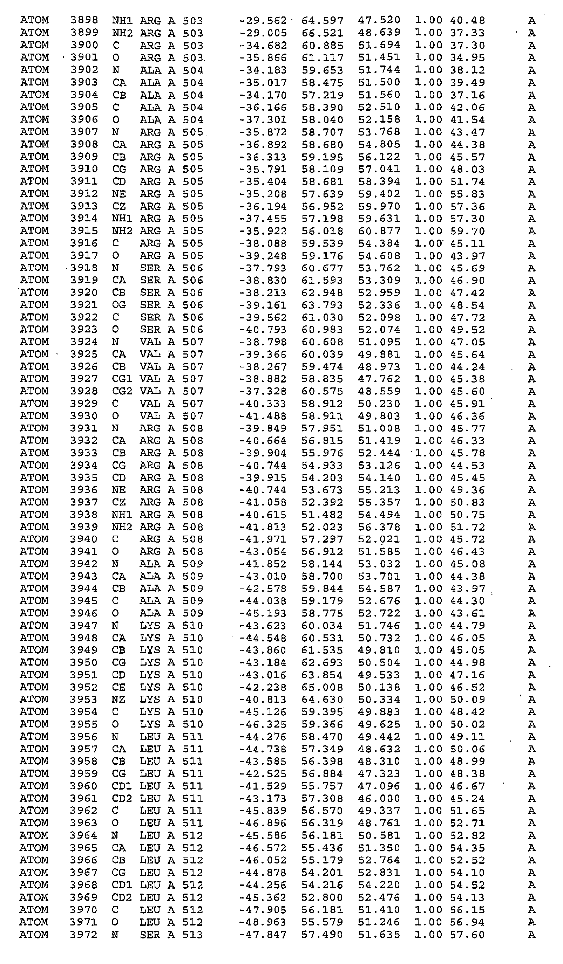

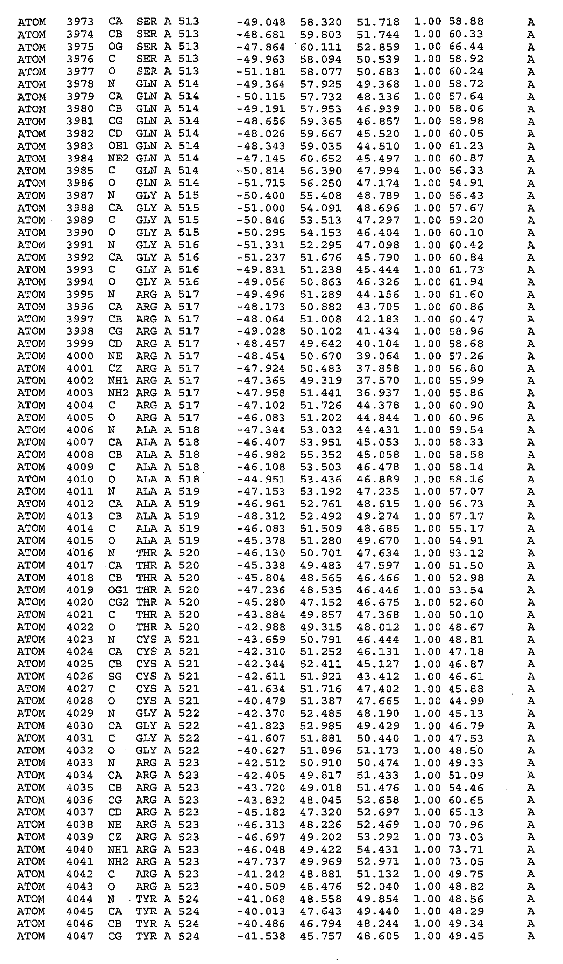

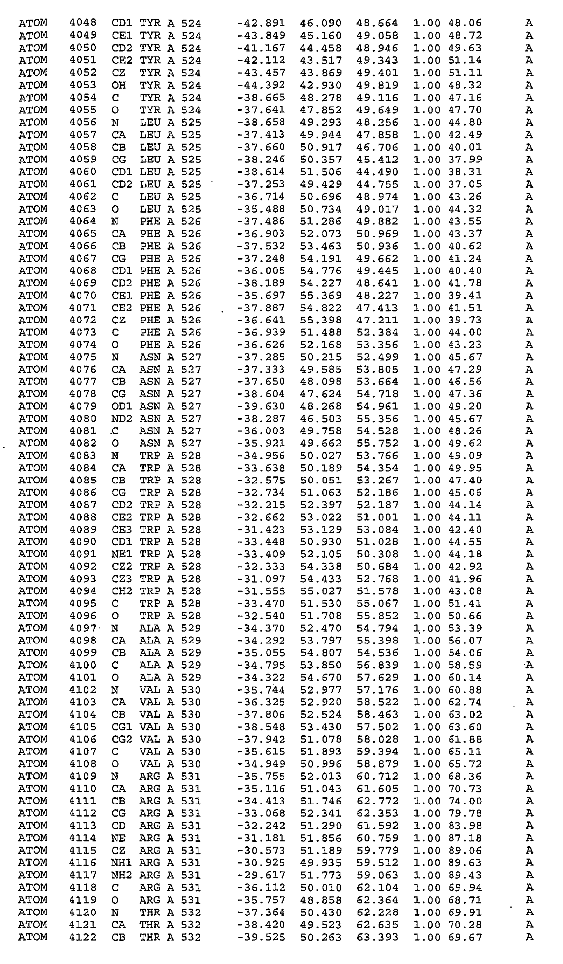

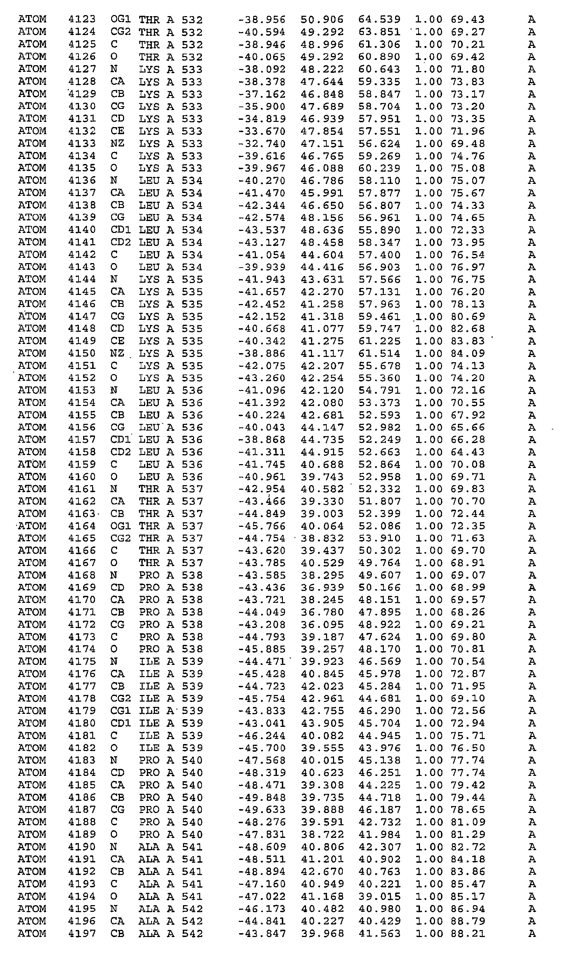

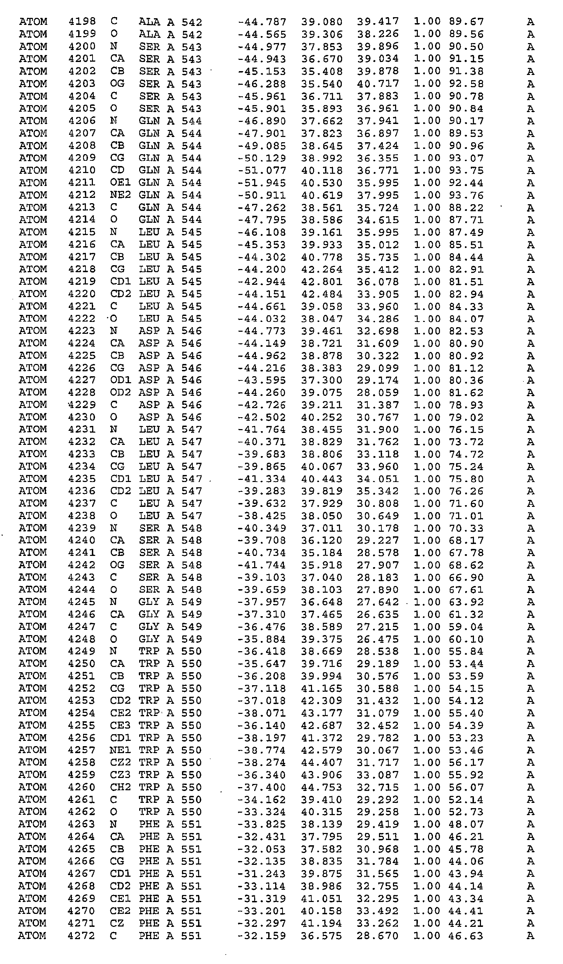

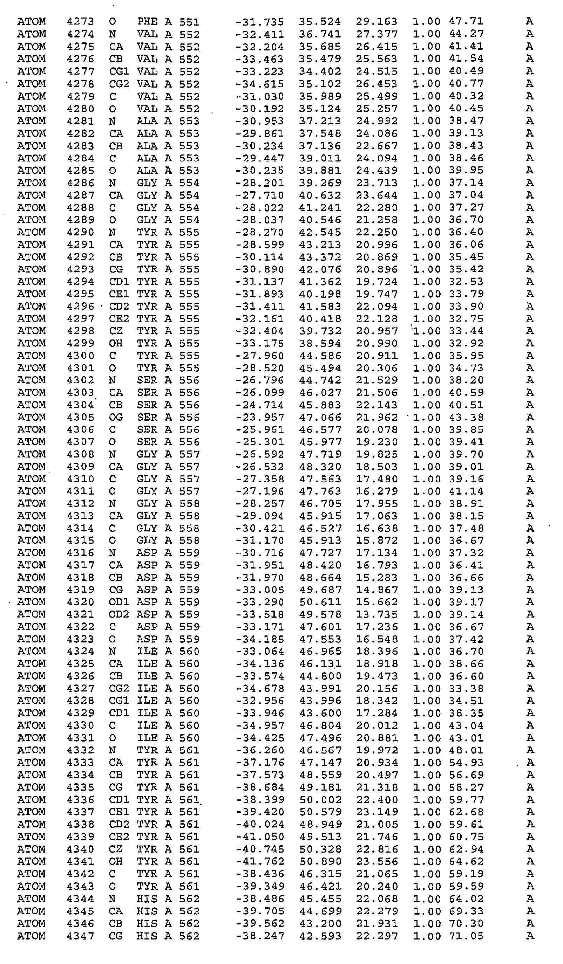

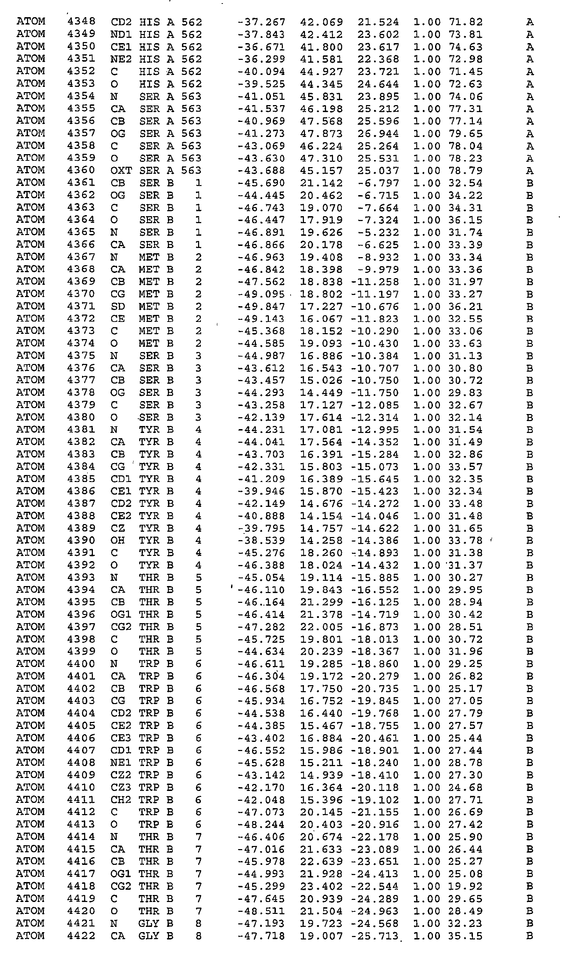

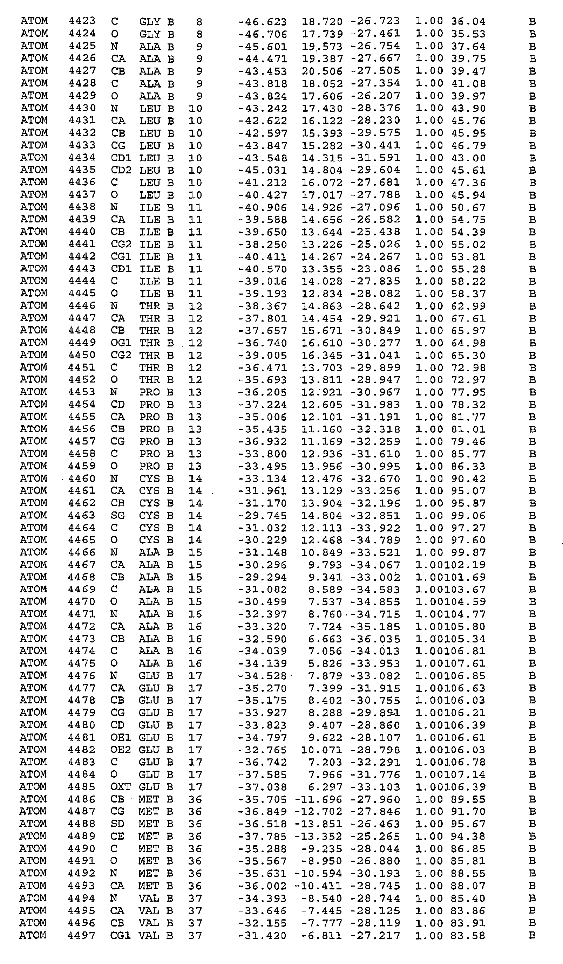

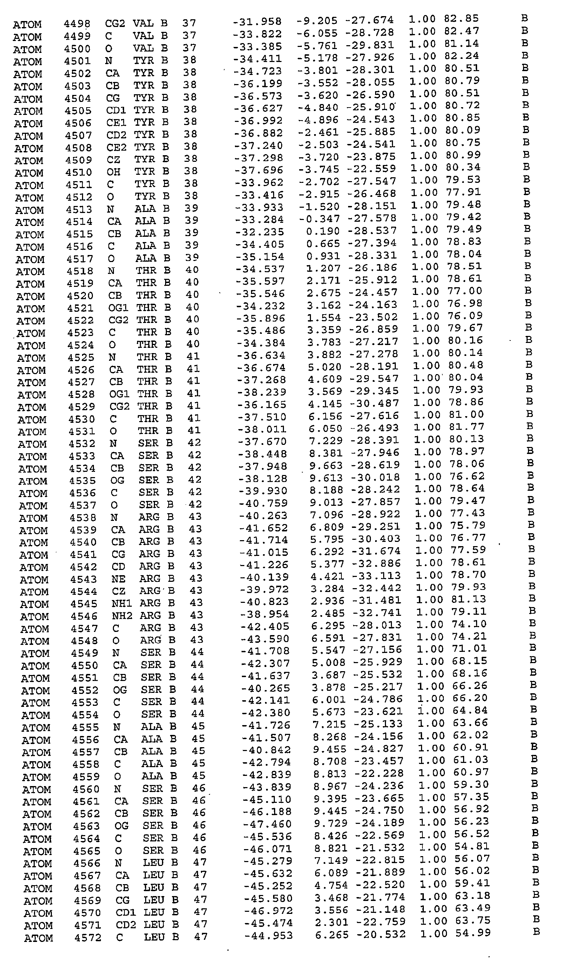

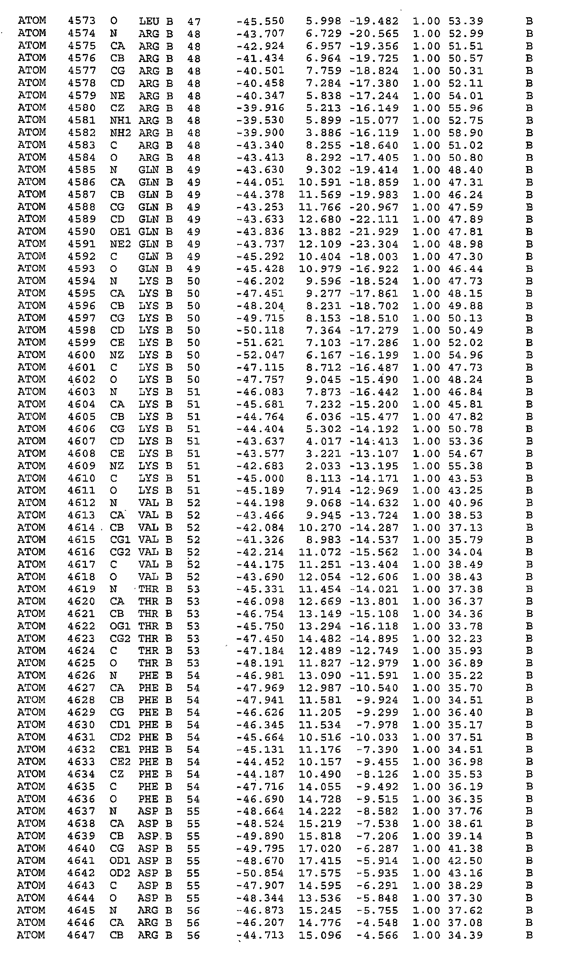

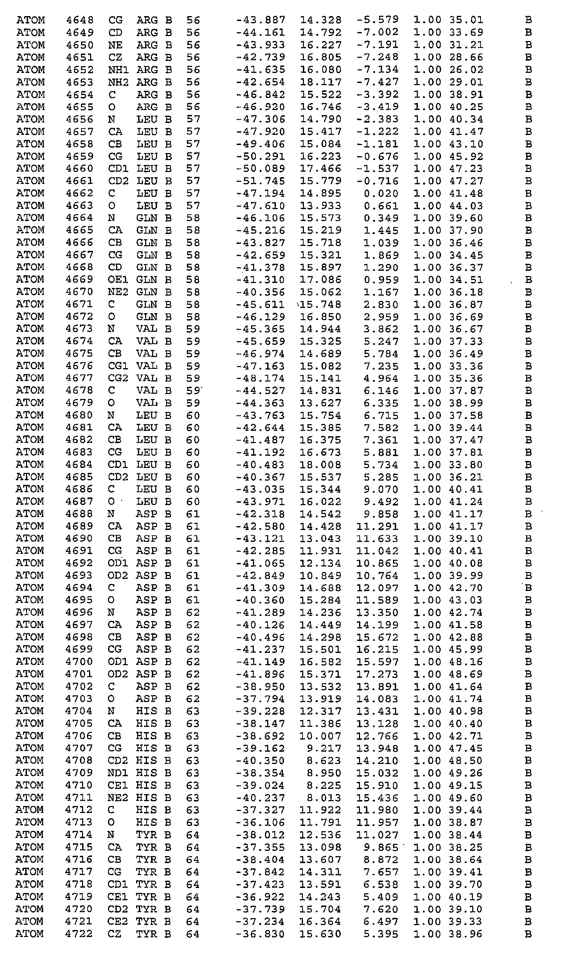

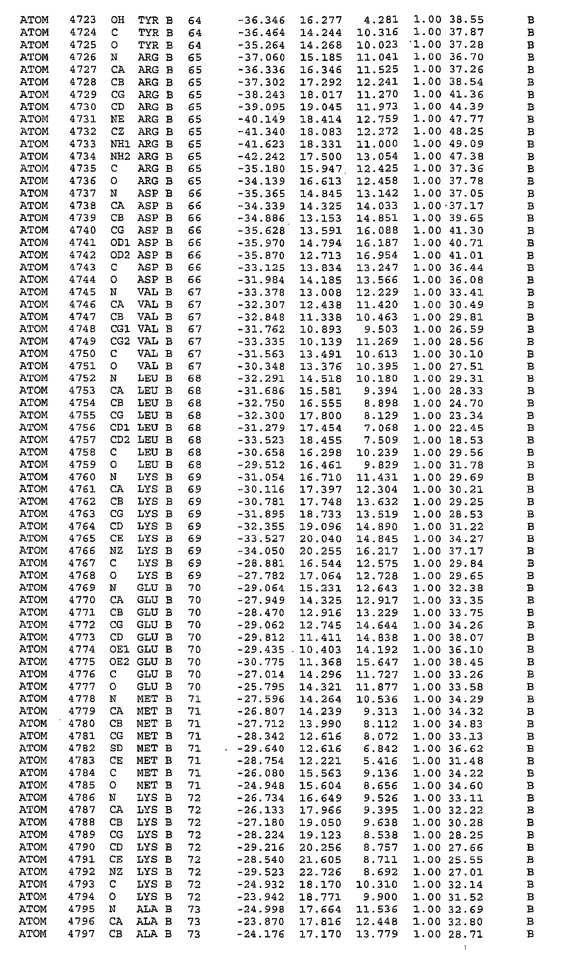

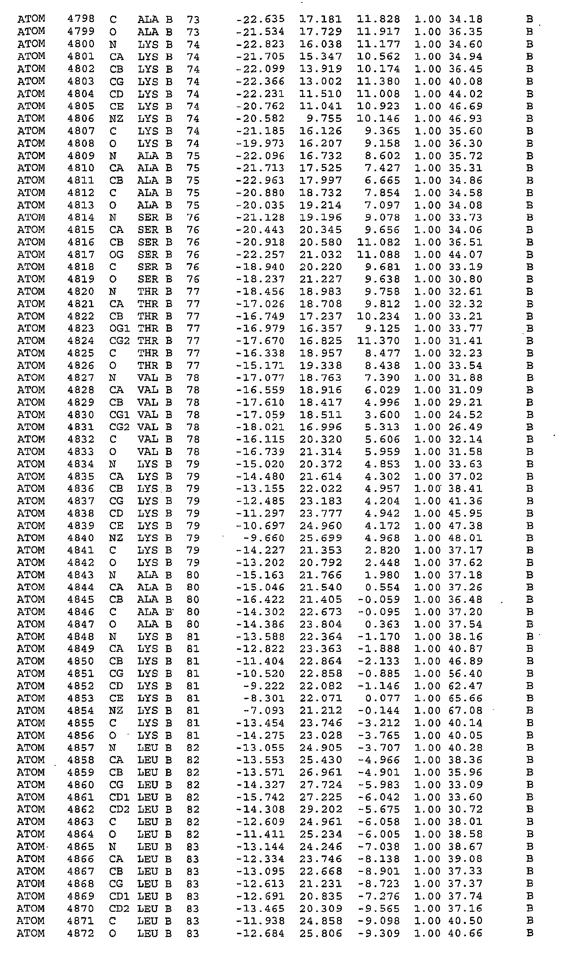

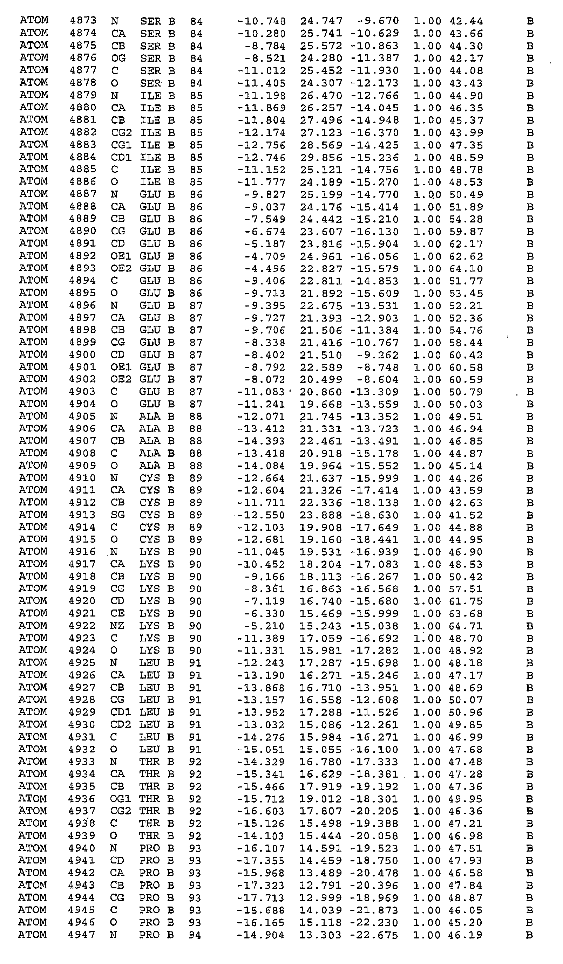

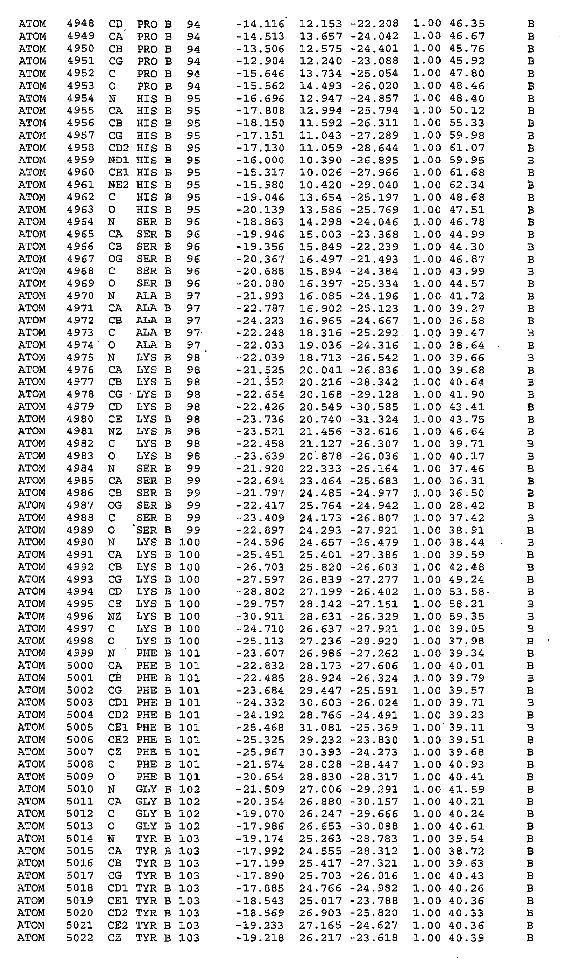

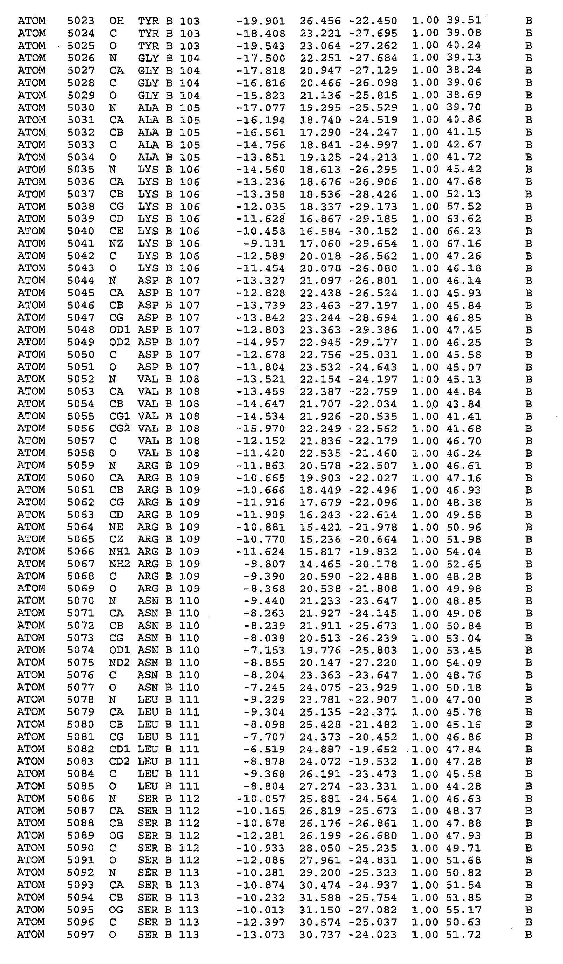

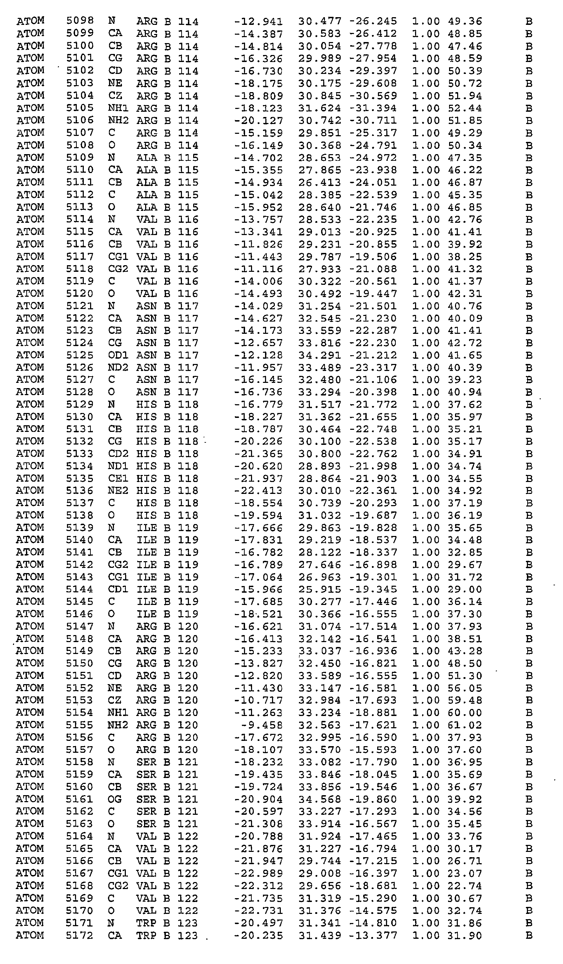

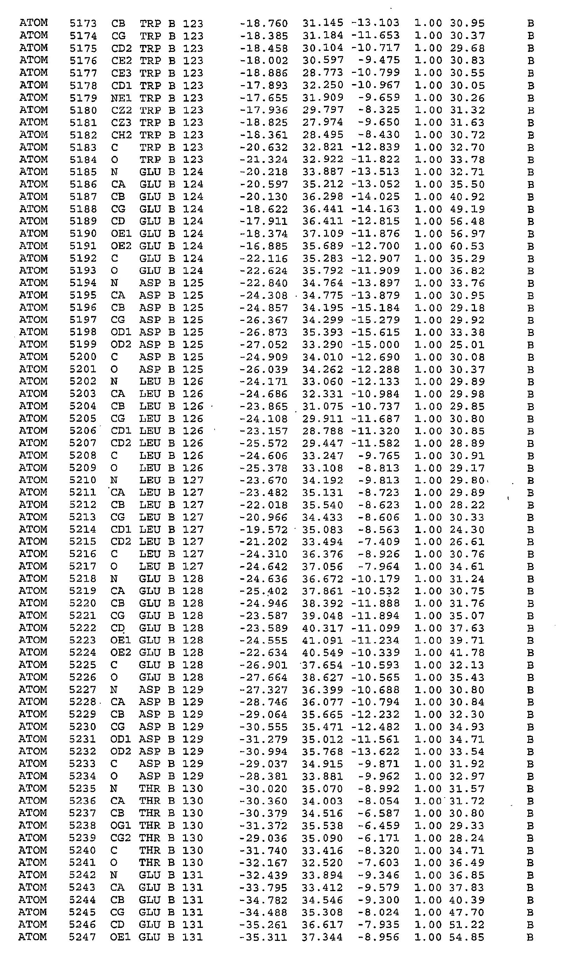

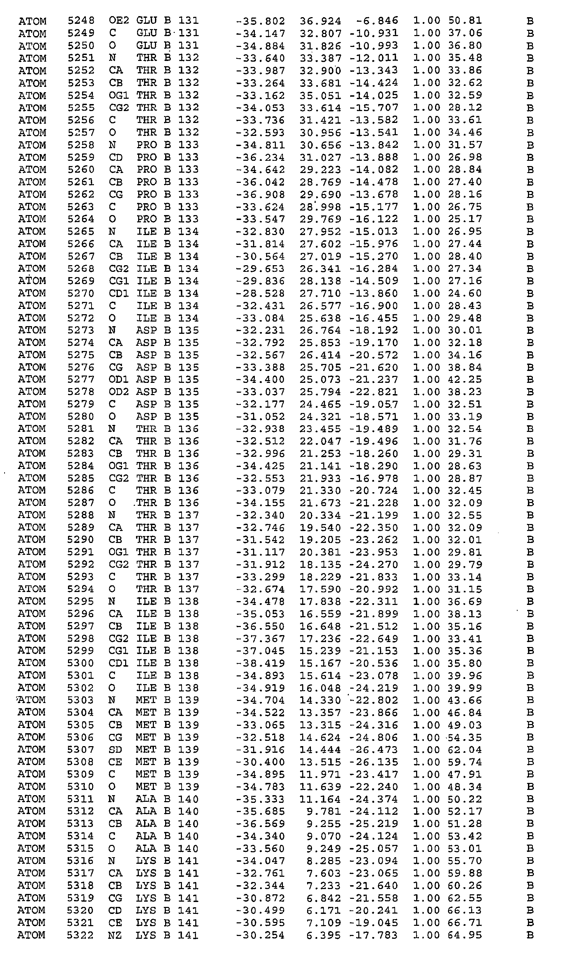

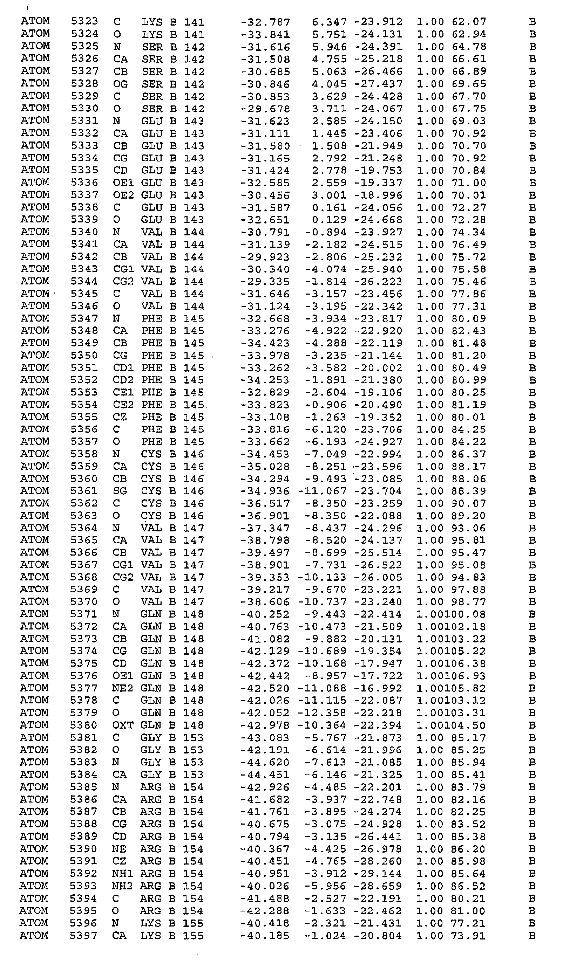

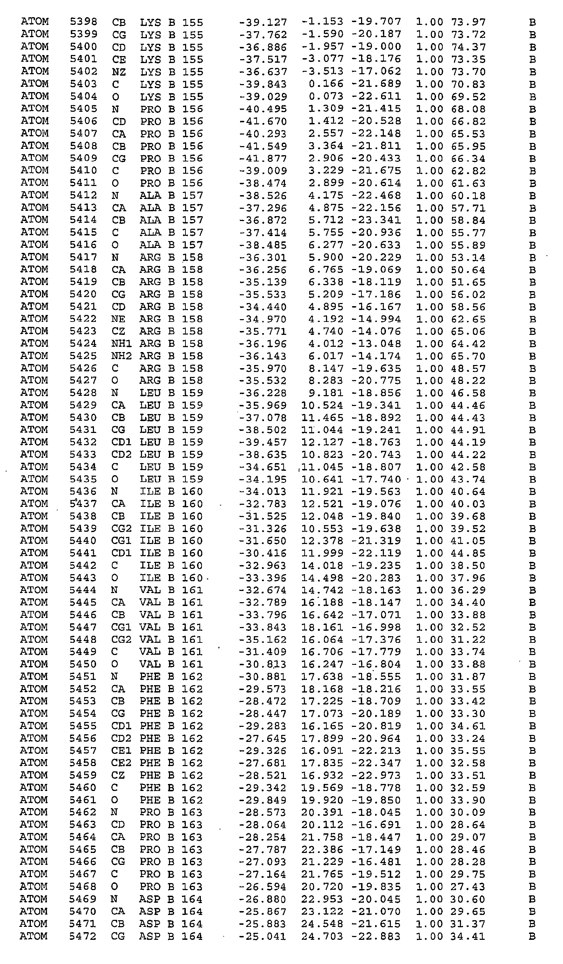

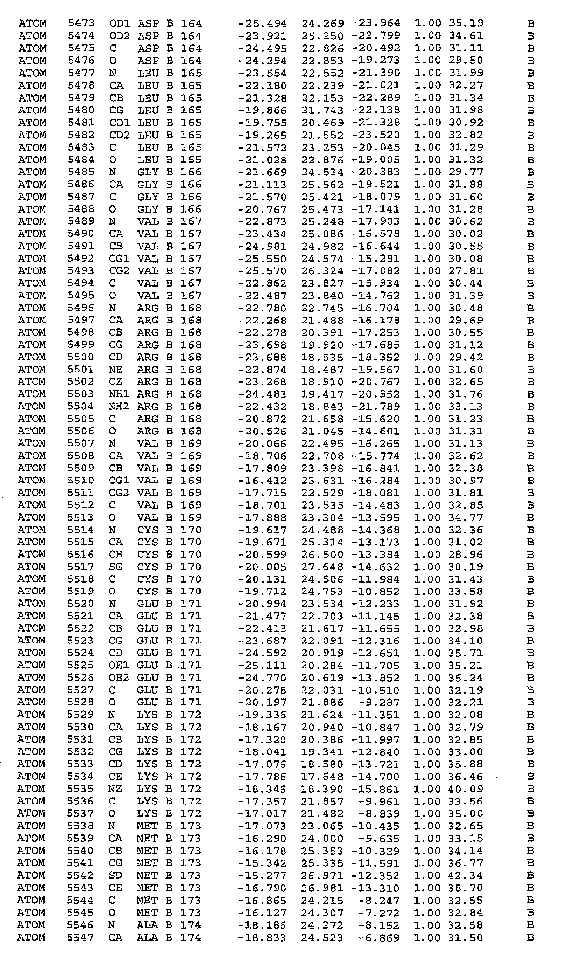

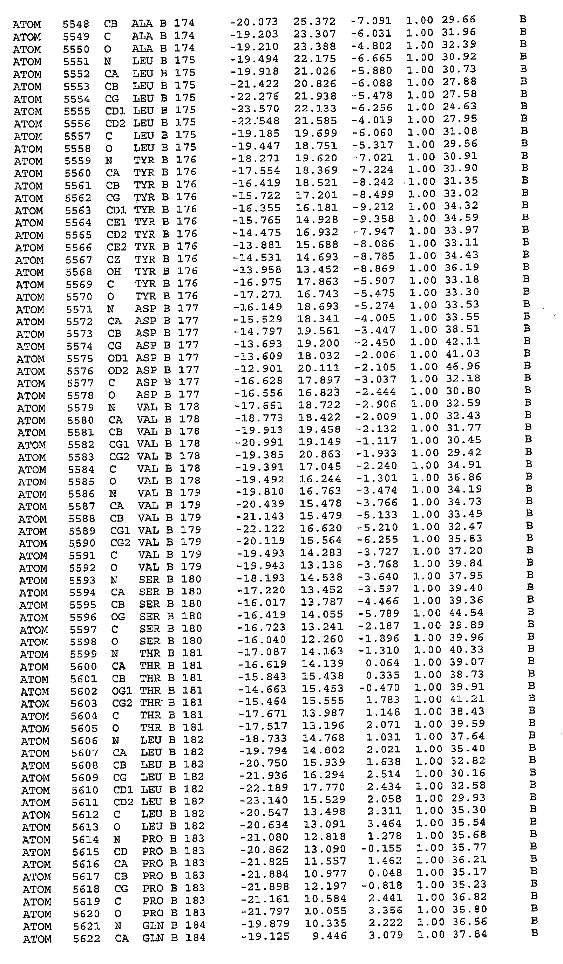

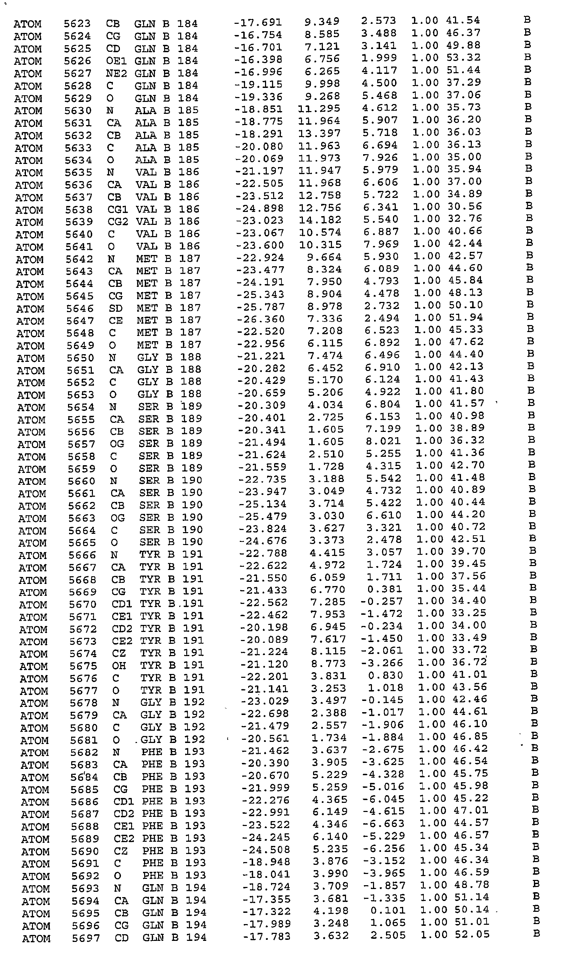

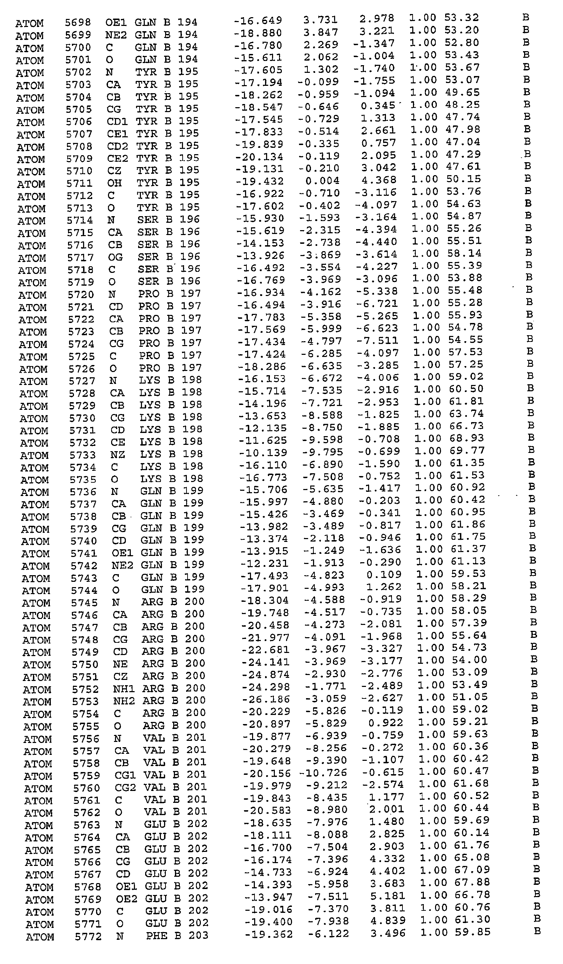

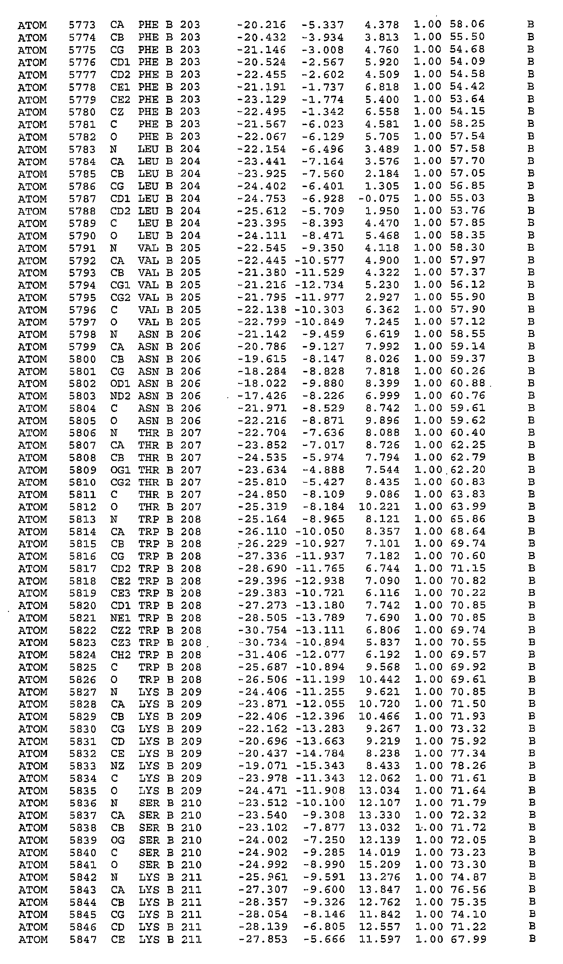

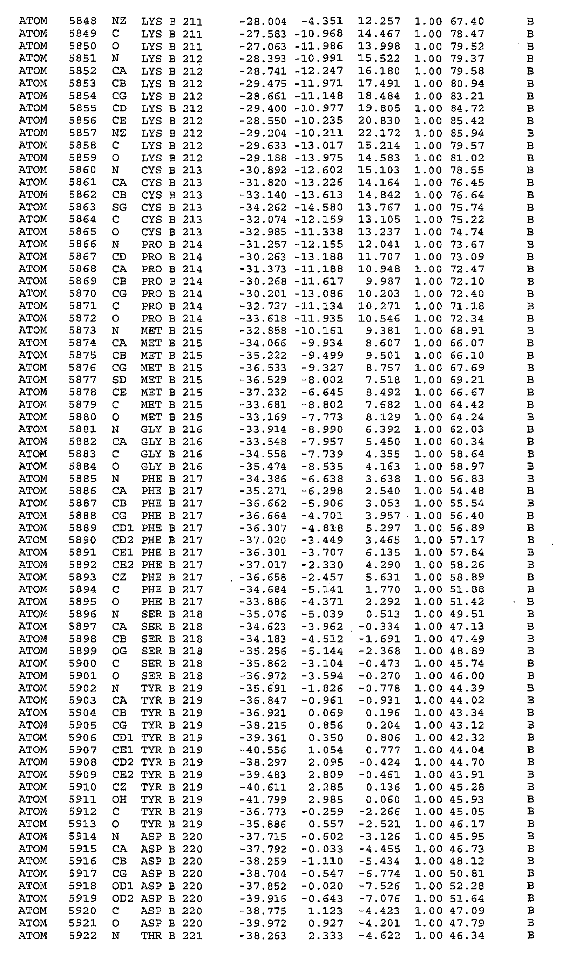

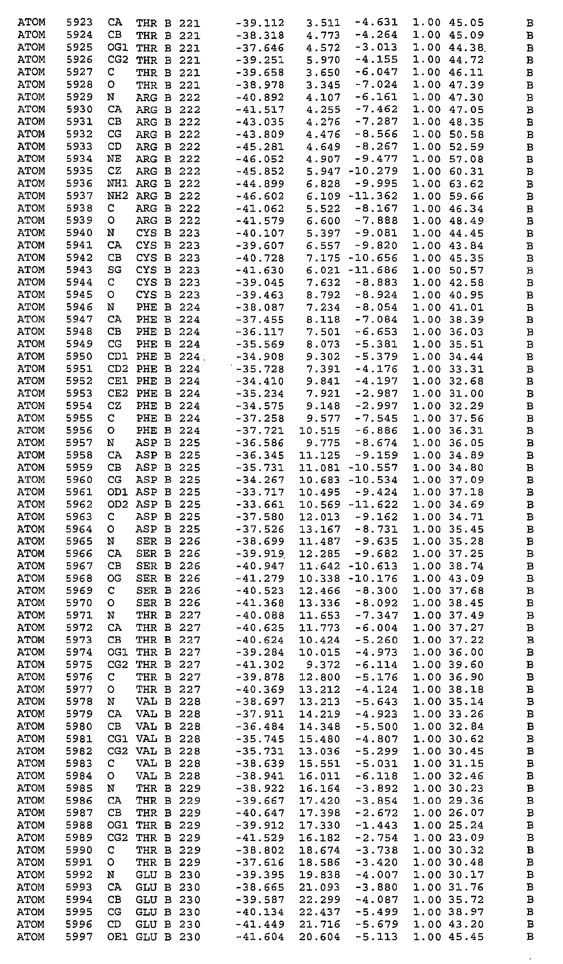

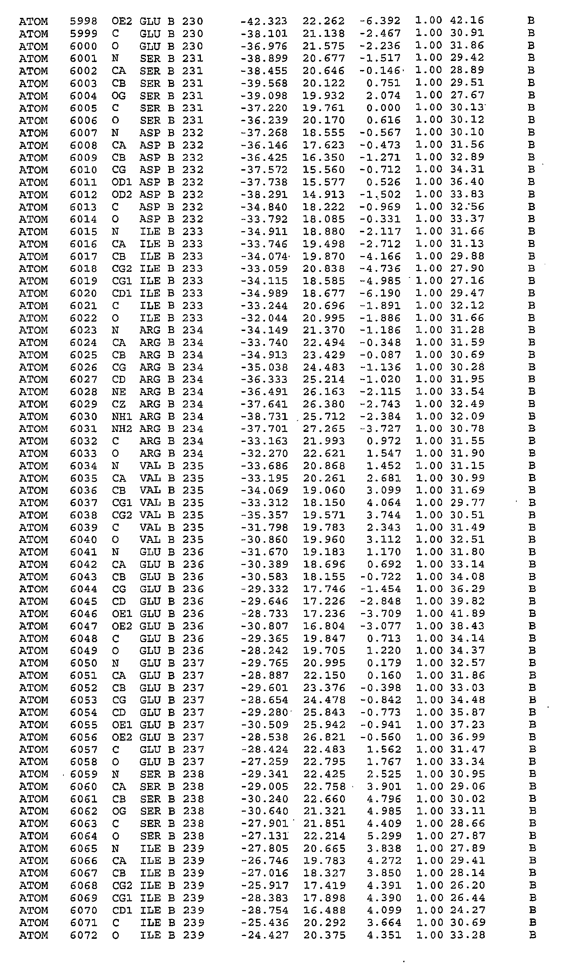

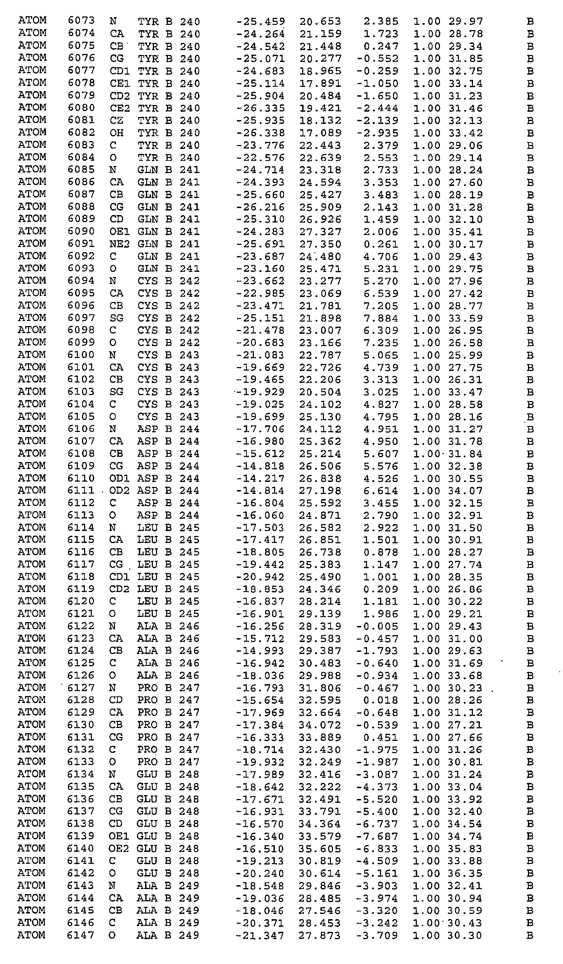

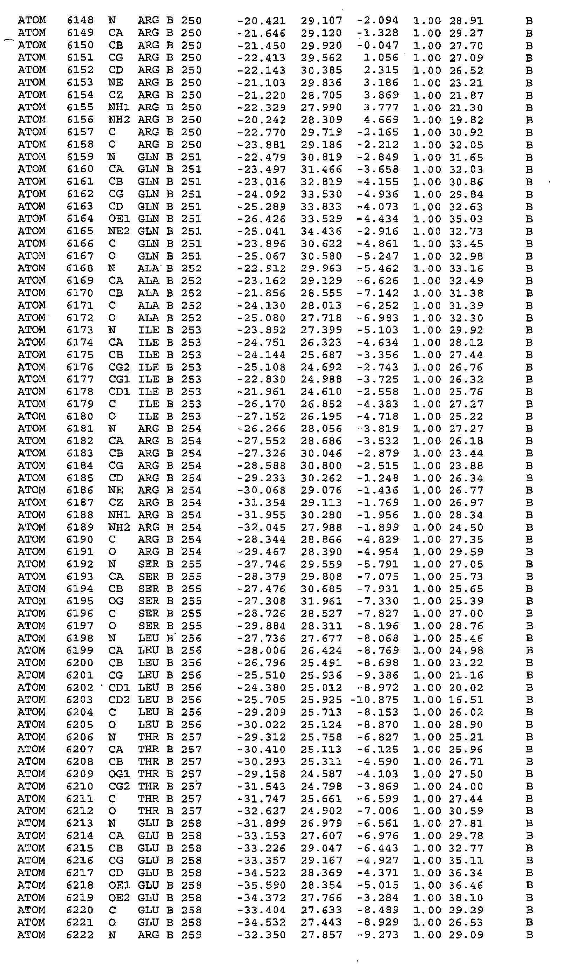

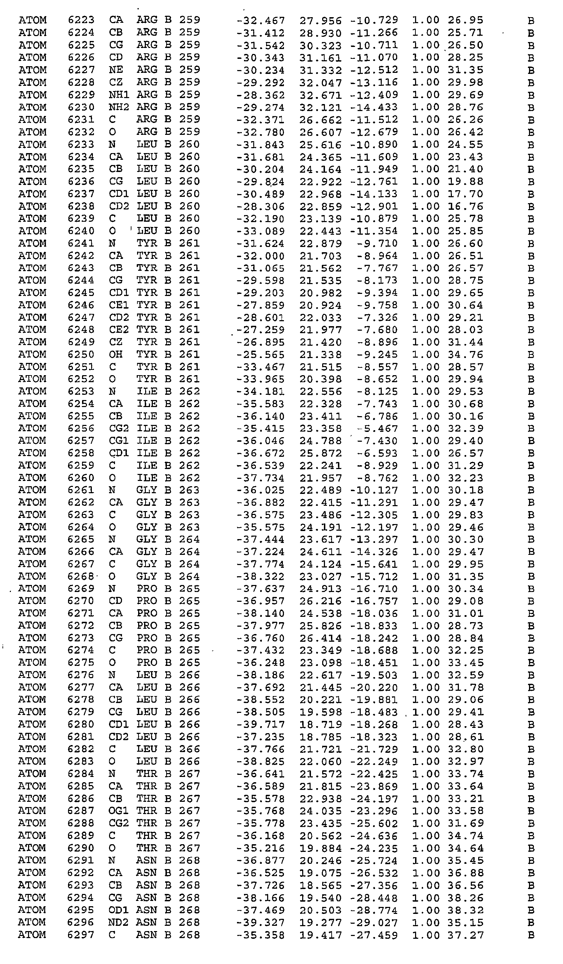

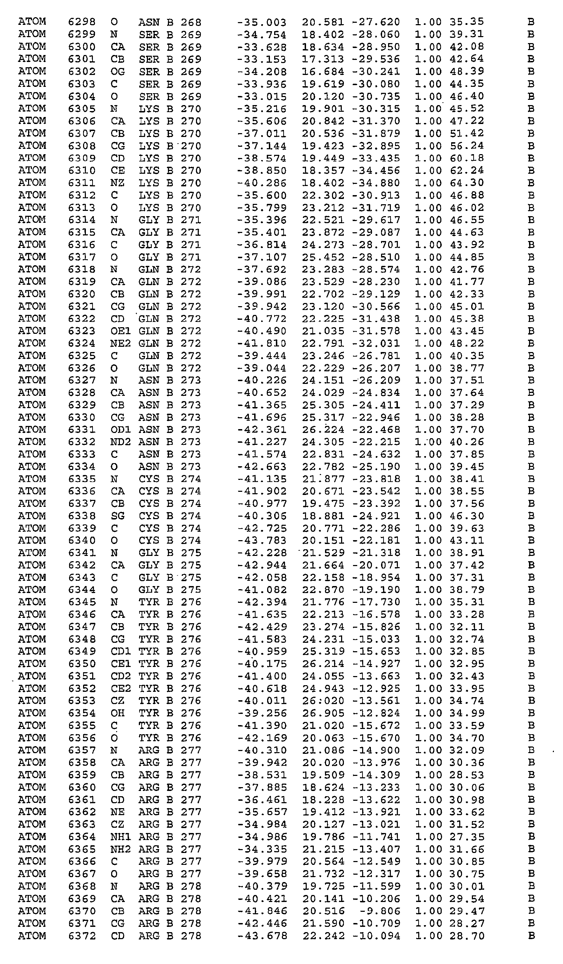

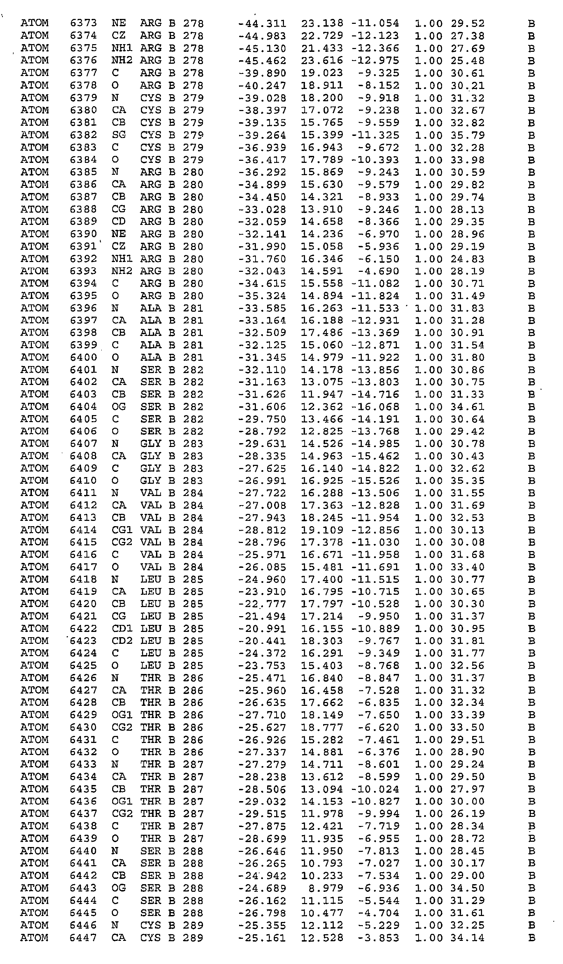

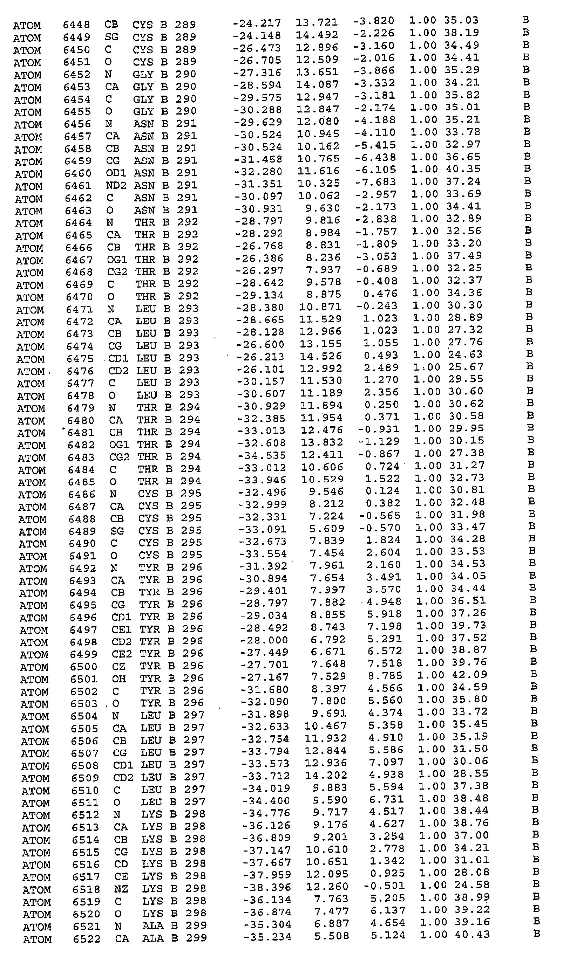

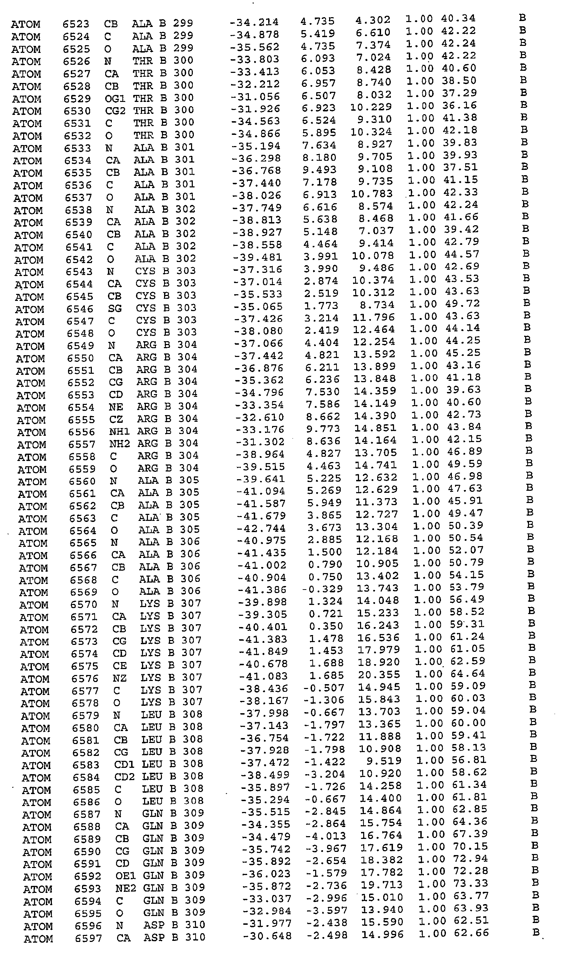

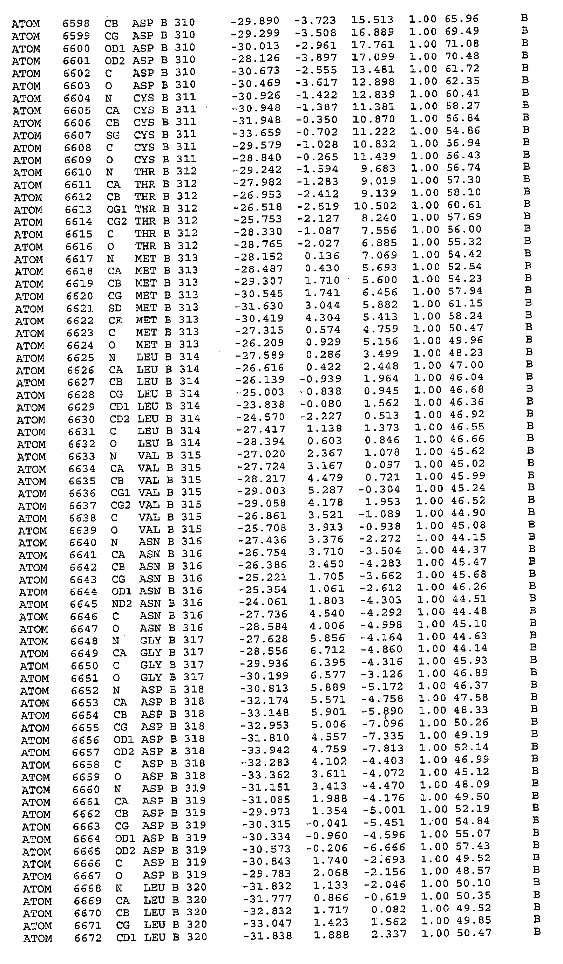

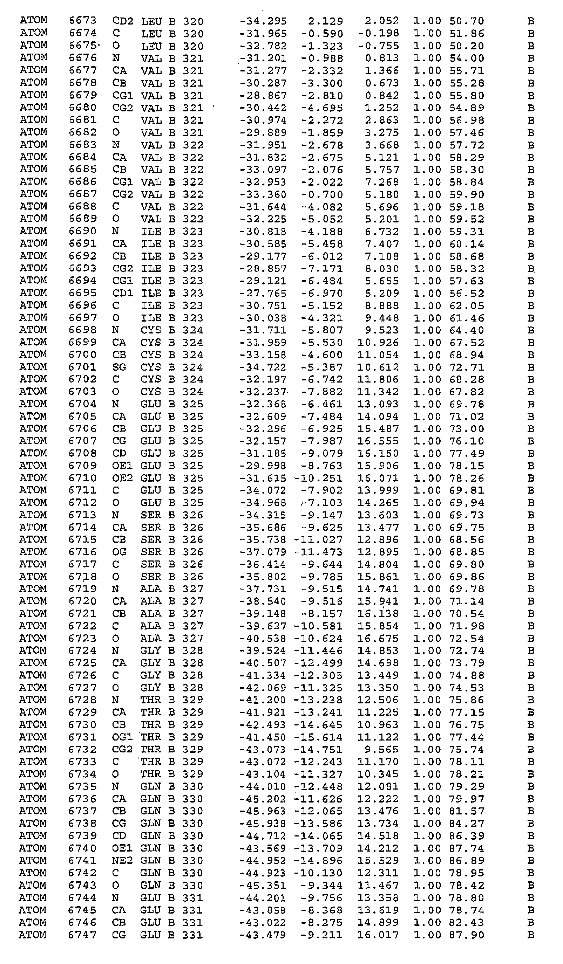

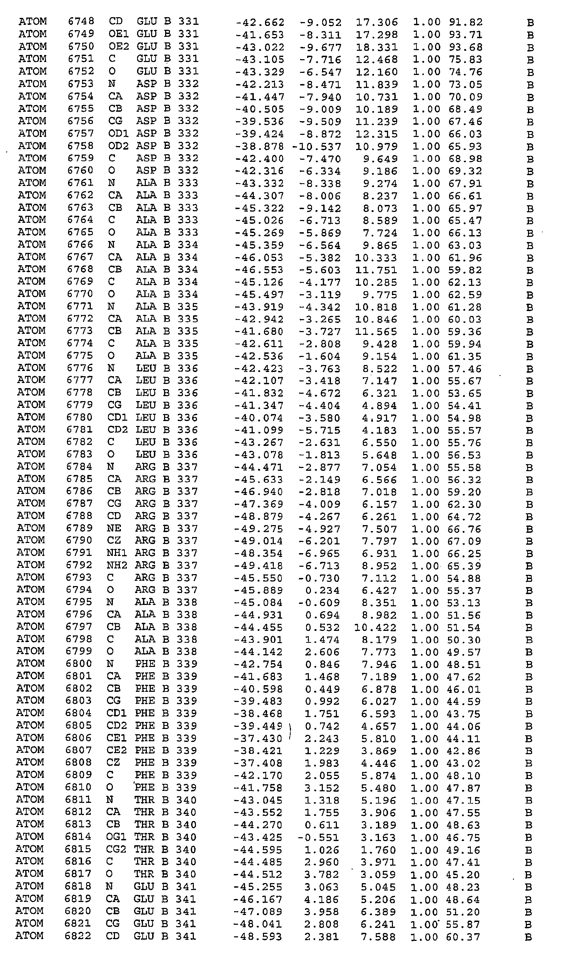

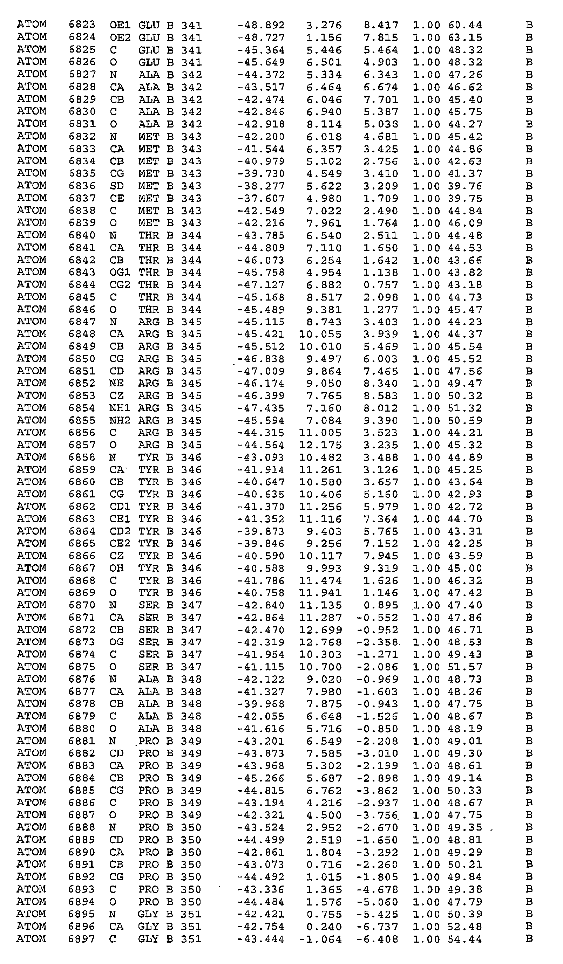

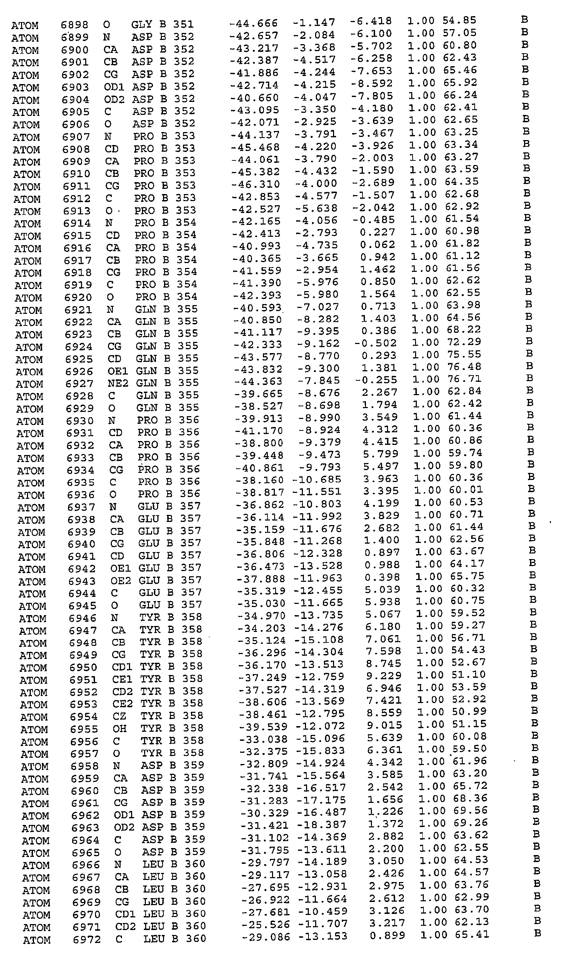

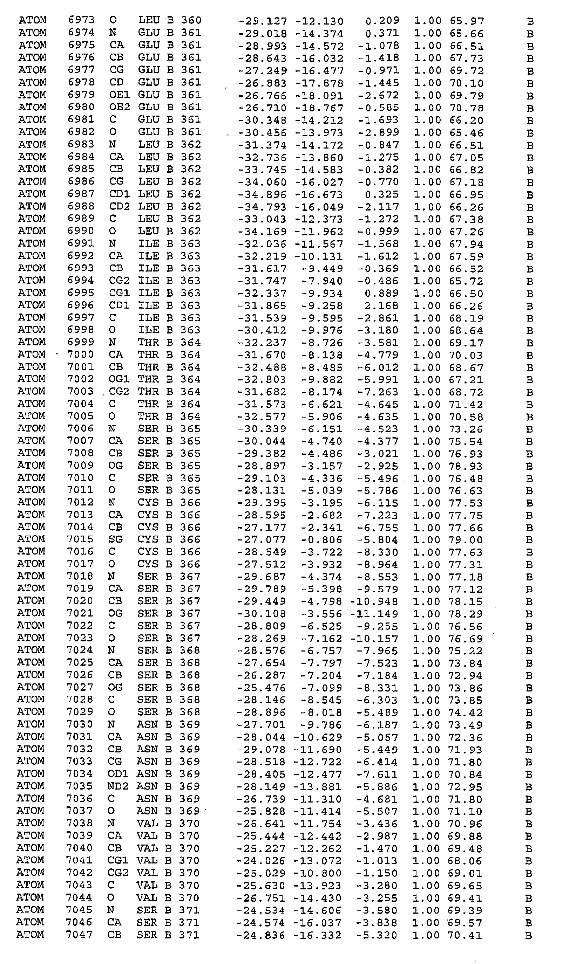

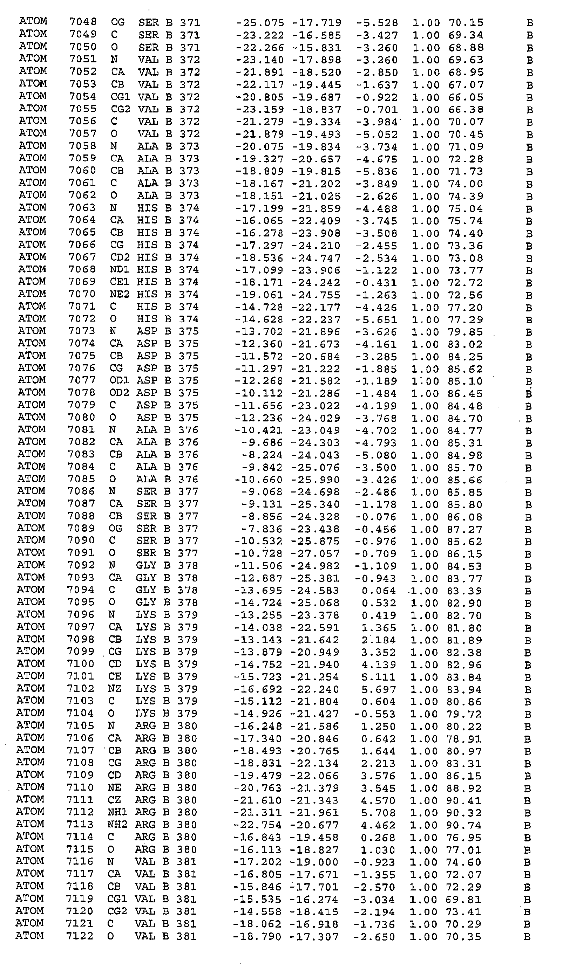

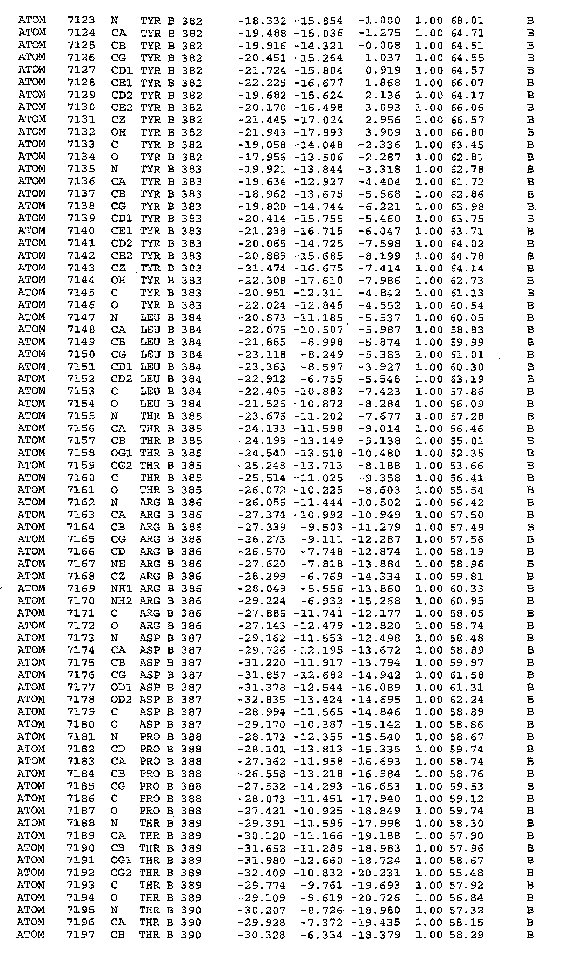

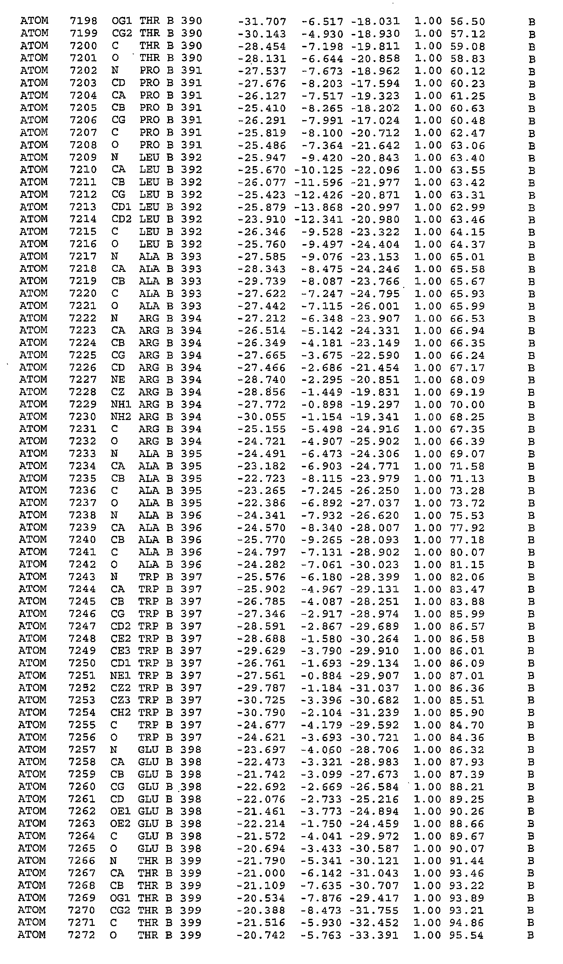

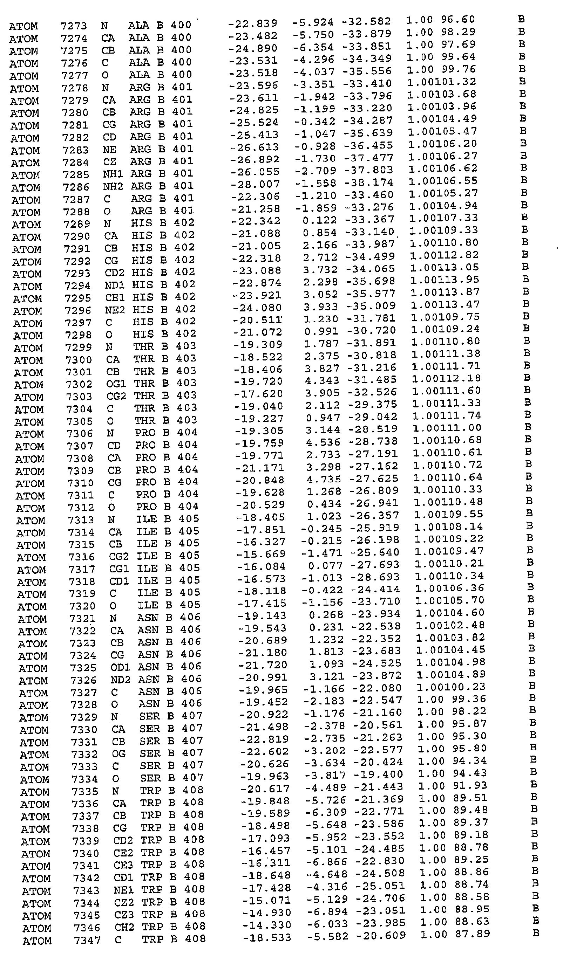

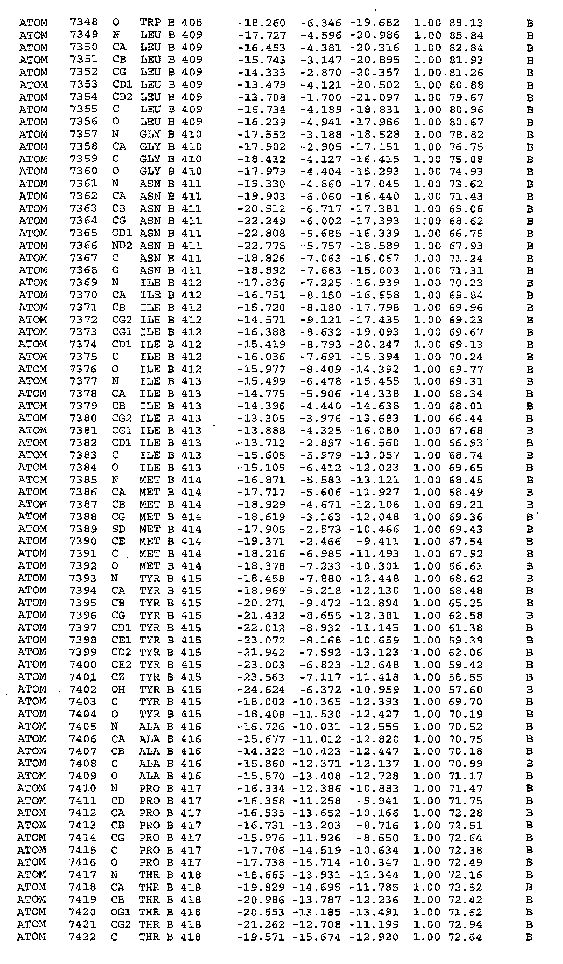

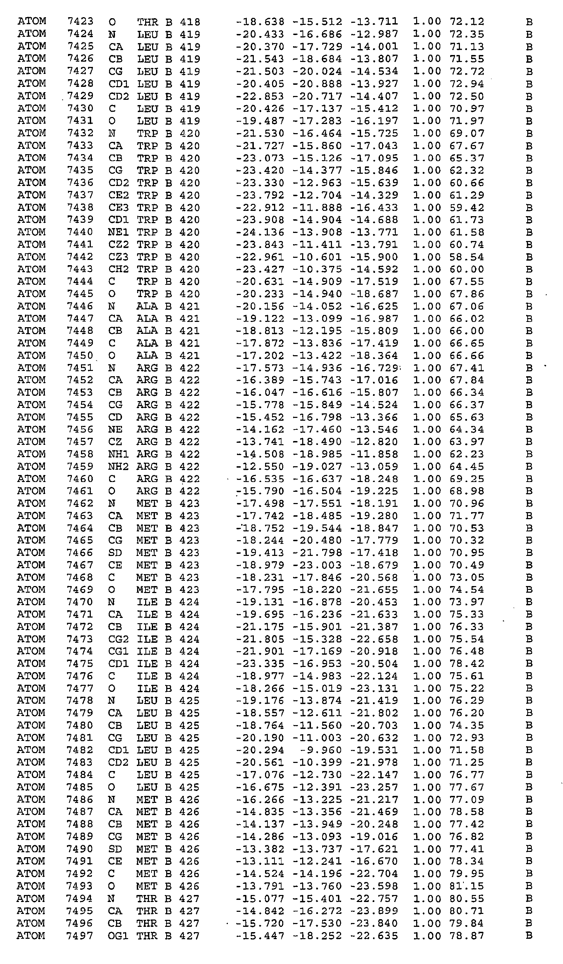

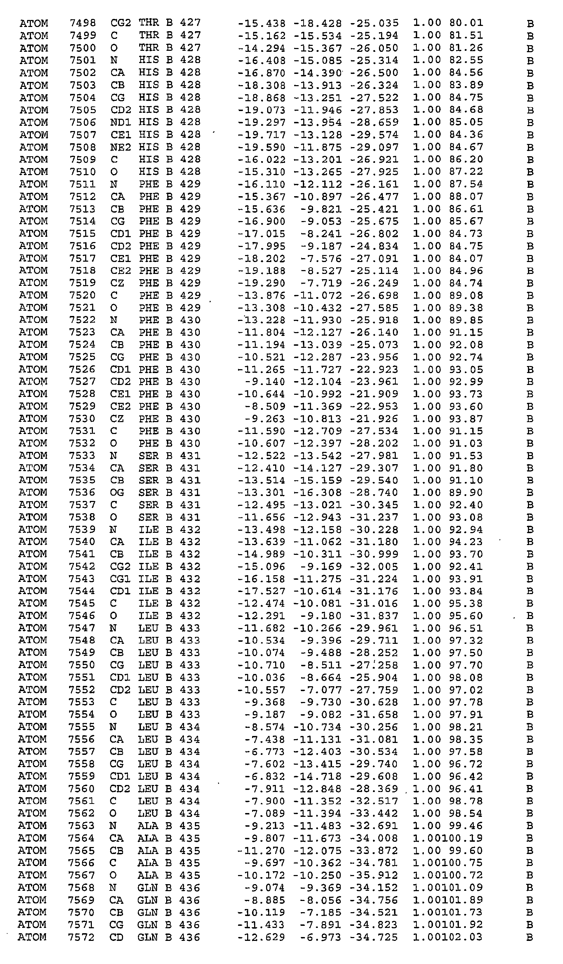

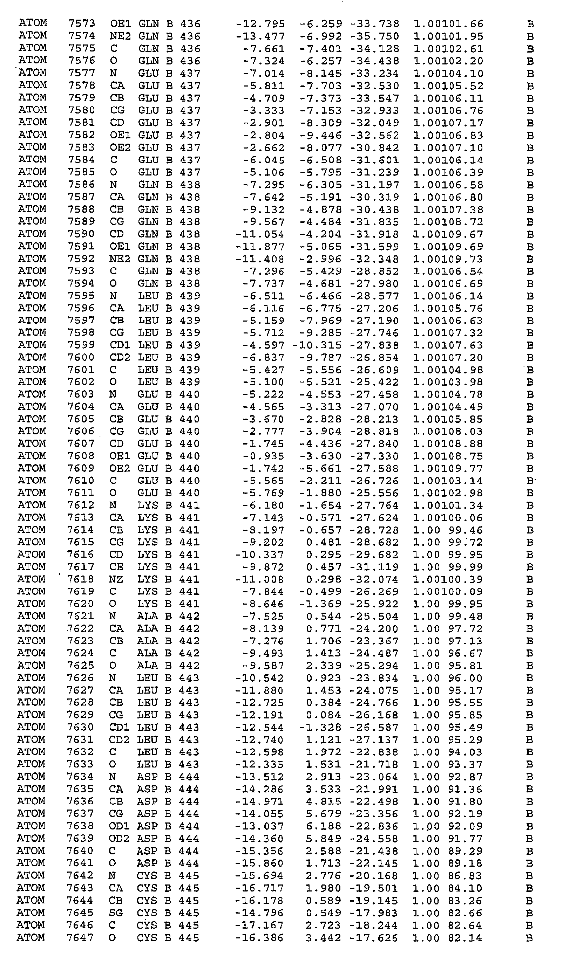

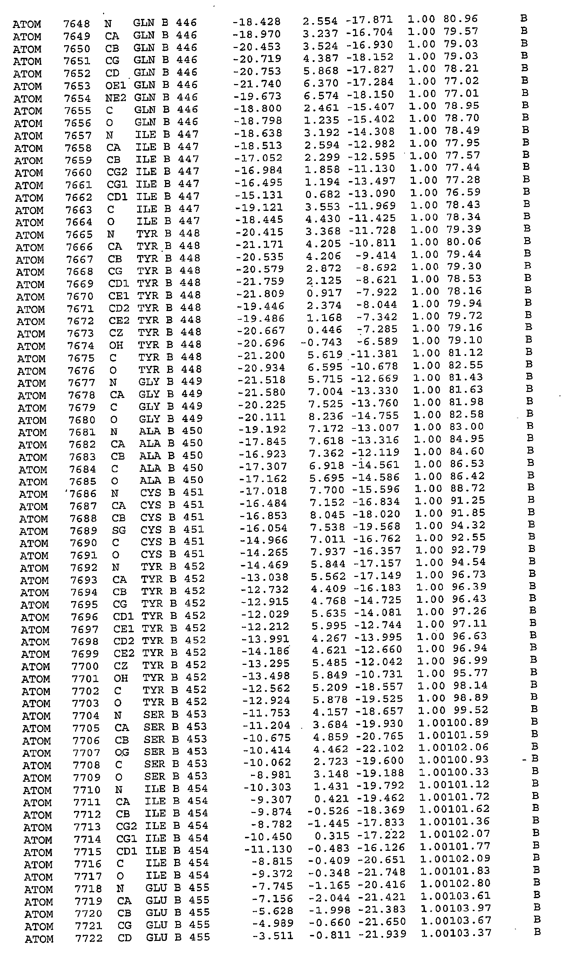

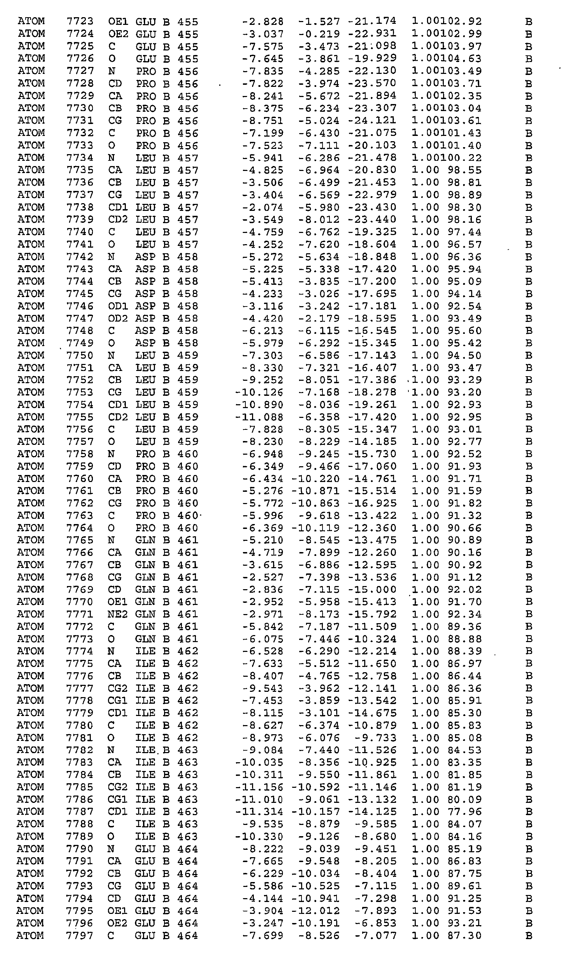

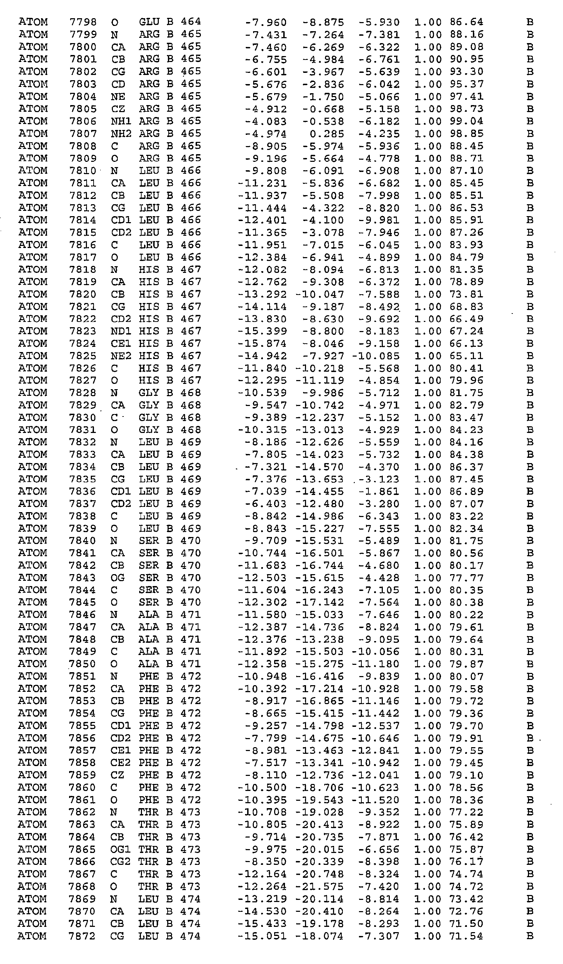

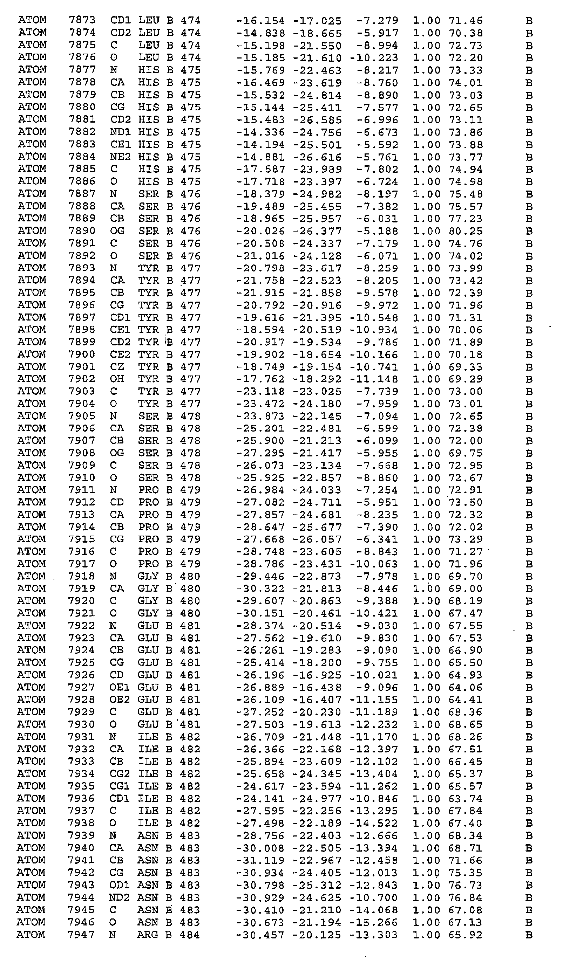

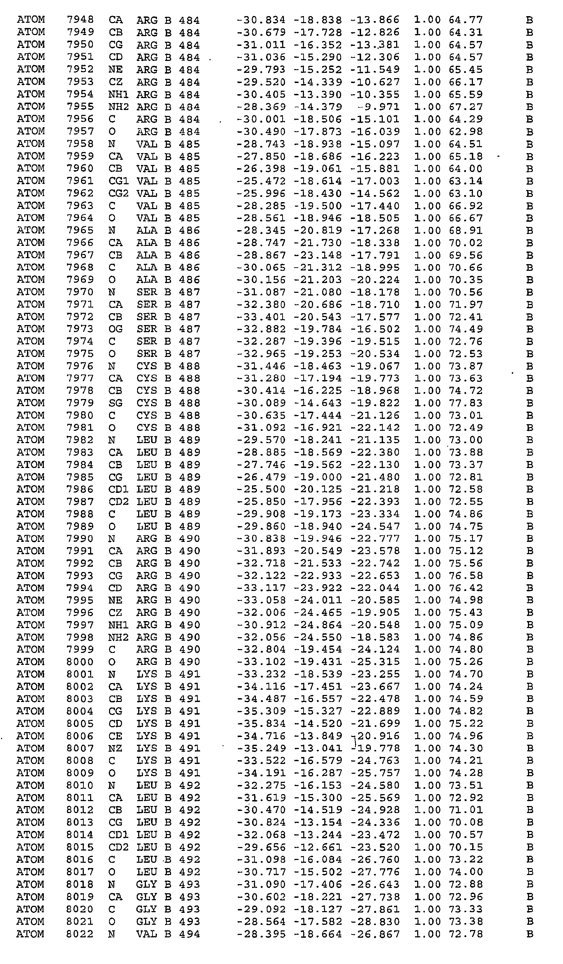

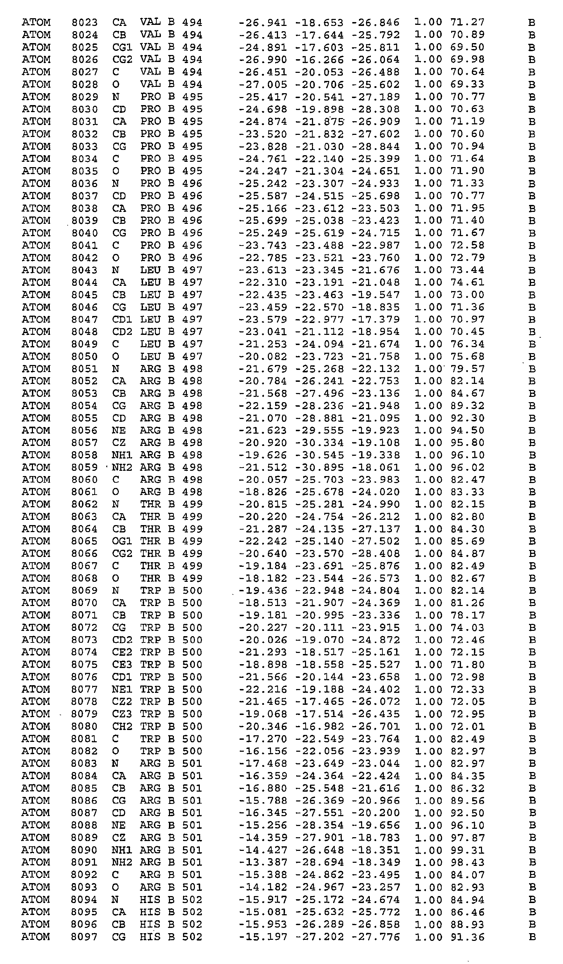

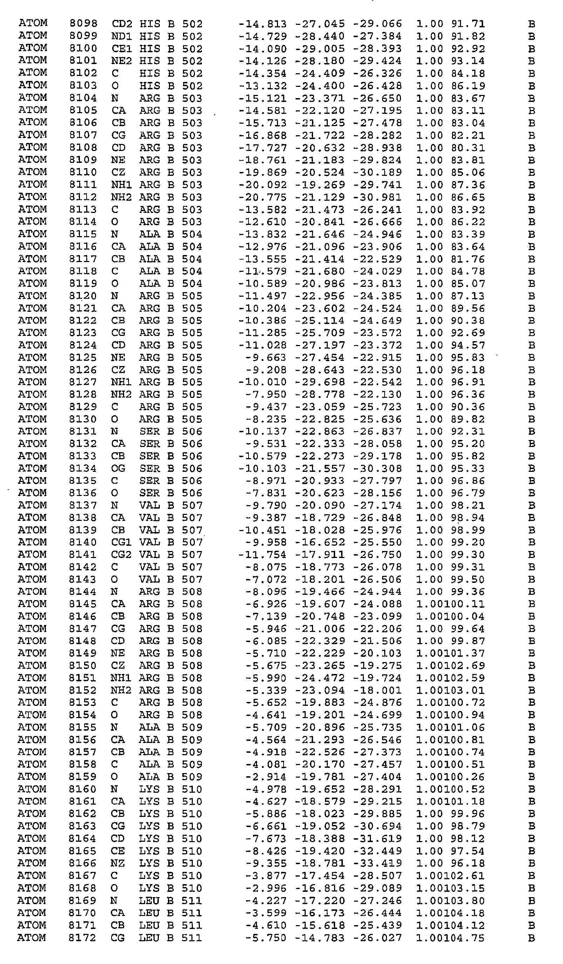

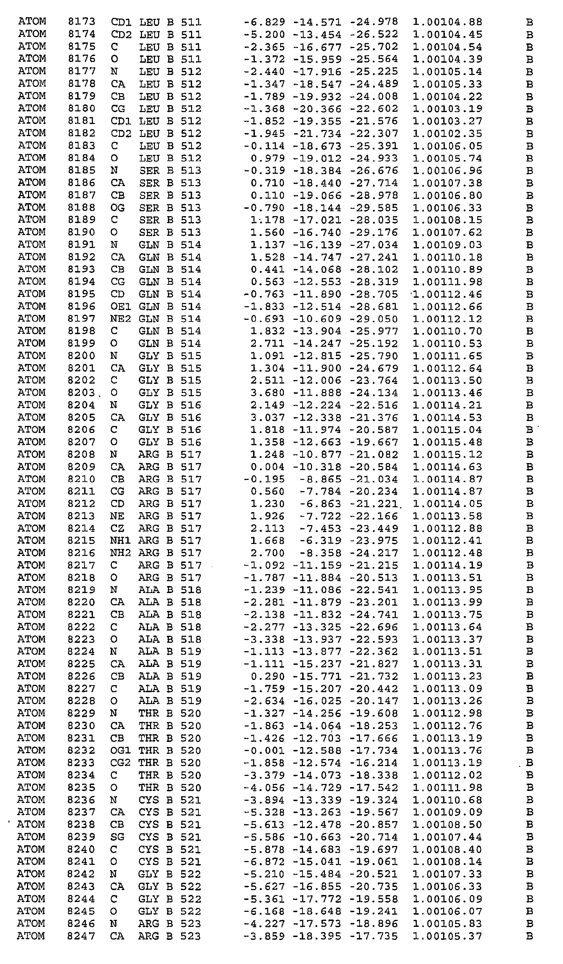

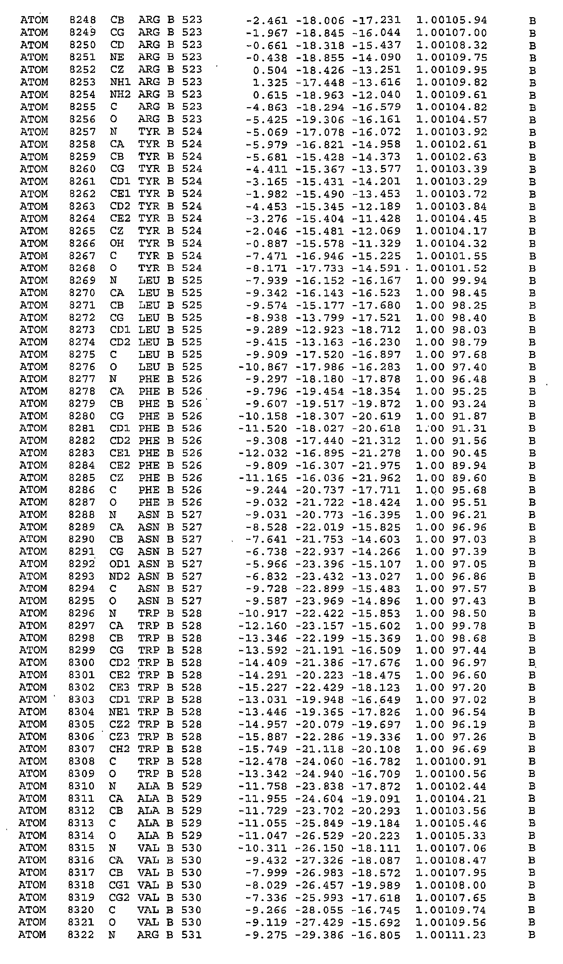

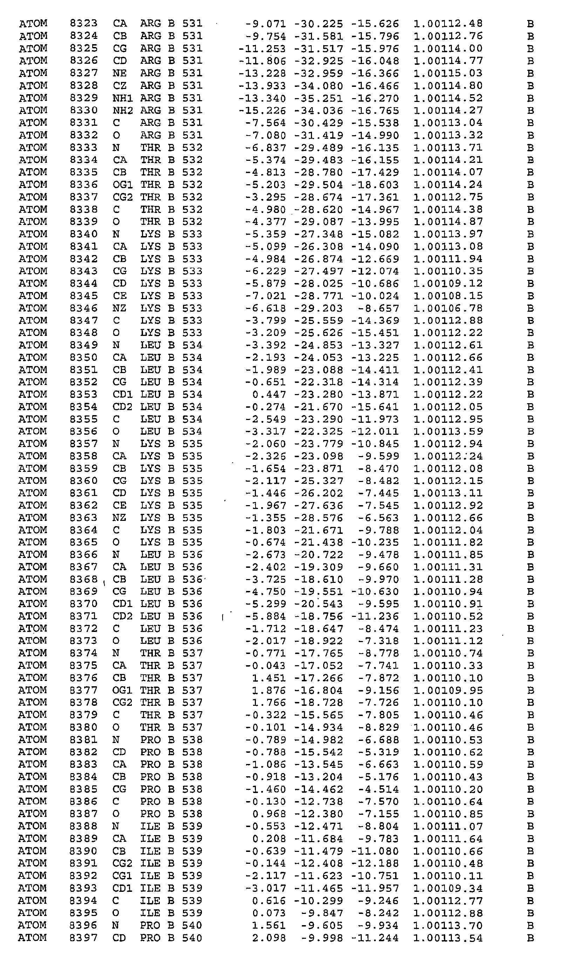

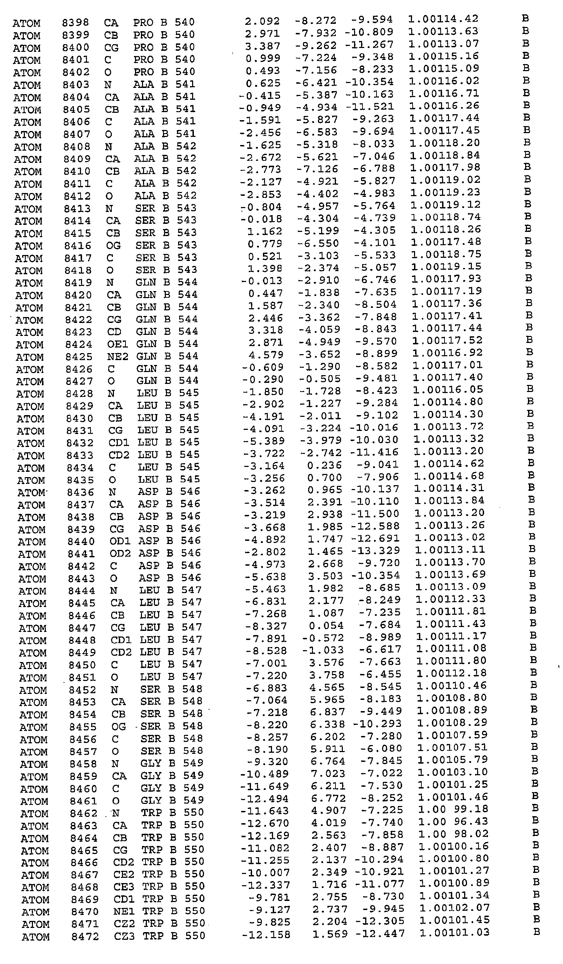

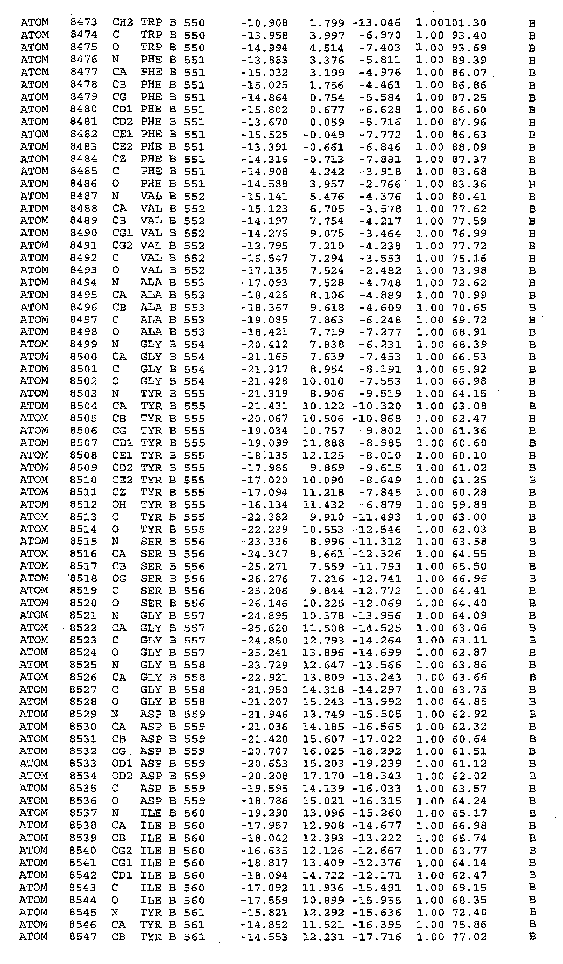

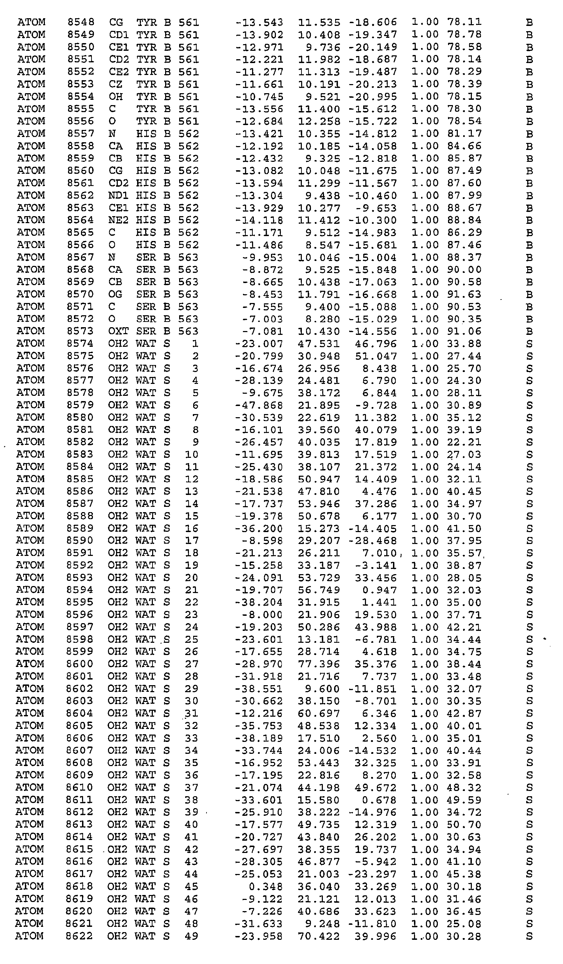

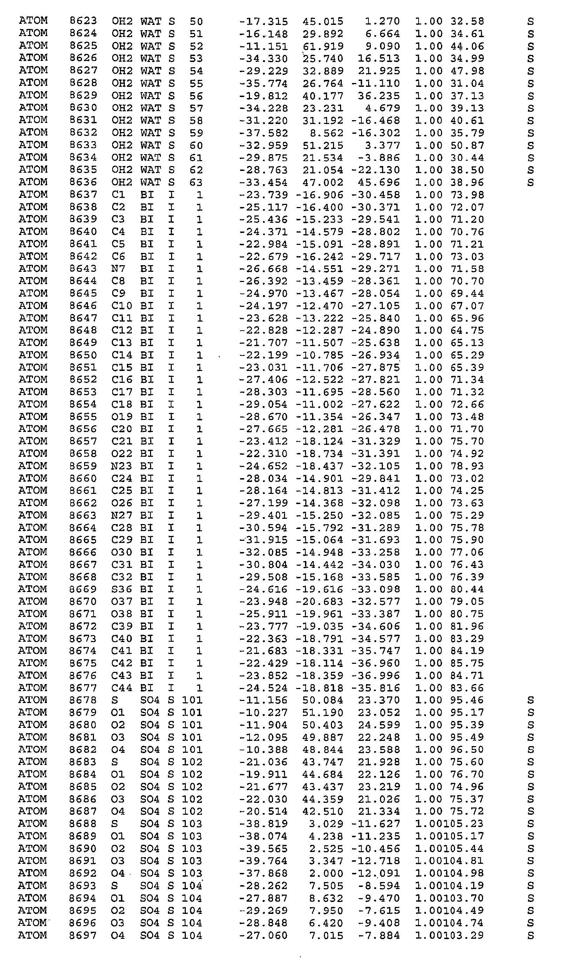

The structural coordinates of the binding pocket are those set out in any one of Figures 4 or 5.

According to this aspect of the invention, any given compound may be computationally evaluated for its ability to associate with the HCV NS5B binding pocket defined herein, and thus, its potential as an NS5B inhibitor determined. As alluded to above, a computer model of a polypeptide consisting of an HCV NS5B binding pocket as defined herein is constructed using well-known software such as QUANTA [Molecular Simulations Inc, San Diego, Calif.], Sybyl [Tripos Associates, St. Louis, Mo.], InsightII [Accelrys], MOE [Chemical Computing Group Inc., Montreal, Quebec, Canada]. Selected compounds to be evaluated may then be positioned in a variety of orientations, or docked, within the binding pocket. Docking may be accomplished using software such as GRID, DOCK, AUTODOCK, FlexX, and GOLD. When a compound is docked within the binding pocket to form a "virtual" representation of an NS5B complex, computational means may be further employed to generate quantitative and qualitative maps of the complex, including for example, pharmacophore maps, surface property maps (which map Conolly, Gaussian and van der Waals surfaces) and maps of Probabilistic Receptor Potentials using software such as QUANTA, Sybyl, InsightII, and MOE.

The efficiency with which a selected compound binds to the present HCV NS5B binding pocket may be tested and optimized by computational evaluation. The quality of the fit of a given compound within the NS5B binding pocket may be evaluated, for example, by shape, size and electrostatic complementarity as determined qualitatively by visual inspection or as determined quantitatively by the use of scoring functions such as LUDI, PLP, PMF, SCORE, GOLD and FlexX. These methods of qualitative and quantitative evaluation may be employed individually or in combination, for example, as in a consensus scoring manner.

Alternatively, binding efficiency can be determined based on the interaction energy of a complex formed by the binding or association of a compound with the HCV NS5B. For example, a compound determined to form a "low energy, stable complex" with NS5B, in the manner described herein, warrants further analysis as a potential NS5B inhibitor. The term "low energy, stable complex" as used herein is defined as an NS5B complex in which the van der Waals interaction energy value, i.e. the van der Waals energy of interaction between the compound and NS5B, is less than about 8000 kcal/mol. Van der Waals interaction energy value can be determined using the software MOE, and is based on the MMFF94 force field. Accordingly, a compound determined to form a complex having a van der Waals interaction energy value of less than about 8000 kcal/mol is a potential NS5B inhibitor. Preferably, a low energy, stable complex in accordance with the present invention will have a van der Waals interaction energy value of less than about 6000 kcal/mol, and more preferably, a value of less than about 4000 kcal/mol.

### Method of Using the NS5B Polypeptide Variants/Analogs

Once a series of compounds has been screened using virtual methods such as those described above, compounds determined to be potential HCV inhibitors can be further evaluated to determine the actual propensity of each to interact with the binding pocket of the present invention and to inhibit HCV.

Thus, a method of screening candidate HCV NS5B inhibitor compounds is described comprising the steps of:
a) incubating a candidate inhibitor compound under conditions suitable for binding with a polypeptide selected from the group consisting of:
   i) an isolated and purified polypeptide comprising a functional HCV NS5B binding pocket defined by at least amino acid residues 392, 393, 395, 396, 399, 424, 425, 428, 429, 492, 493, 494, 495, 500 and 503 (and optionally one of: amino acid residues 37 and 496) of a native HCV NS5B, or defined by a functionally equivalent analog thereof, wherein said binding pocket is exposed by displacement of a finger loop chain defined by at least amino acid residues 18 to 35 and wherein said binding pocket retains its native functional configuration;
   ii) an isolated and purified HCV NS5B polypeptide consisting of an HCV NS5B binding pocket defined by at least amino acid residues 392, 393, 395, 396, 399, 424, 425, 428, 429, 492, 493, 494, 495, 500 and 503 (and optionally one of: amino acid residues 37 and 496) of a native HCV NS5B, or defined by a functionally equivalent analog thereof, wherein said binding pocket retains its native functional configuration;
   iii) an isolated and purified HCV NS5B polypeptide analog comprising an HCV NS5B binding pocket defined by at least amino acid residues 392, 393, 395, 396, 399, 424, 425, 428, 429, 492, 493, 494, 495, 500 and 503 (and optionally one of: amino acid residues 37 and 496) of a native HCV NS5B, or defined by a functionally equivalent analog thereof, wherein said binding pocket retains its native functional configuration and wherein said binding pocket is exposable;
   iv) an HCV NS5B polypeptide variant comprising at least one amino acid mutation within a finger loop defined by amino acid residues 18 to 35, wherein said mutation provokes a displacement of said finger loop to expose a binding pocket essentially defined by amino acid residues 392, 393, 395, 396, 399, 424, 425, 428, 429, 492, 493, 494, 495, 500 and 503 (and optionally one of: amino acid residues 37 and 496) of native HCV NS5B, or defined by a functionally equivalent analog thereof, wherein said binding pocket retains its native functional configuration;
   v) an HCV NS5B polypeptide, or a functionally equivalent analog thereof, characterized by displacement of amino acid residues 18 to 35; and
   vi) an HCV NS5B polypeptide, or a functionally equivalent analog thereof, in which at least amino acid residues 18 to 35 have been deleted;. and
b) determining whether or not the candidate inhibitor compound binds to the polypeptide, wherein a compound that binds to the polypeptide is a potential HCV NS5B inhibitor.

Binding of the candidate compound within the defined HCV NS5B binding pocket can be determined using methods well-established in the art. For example, binding assays may be used in which the candidate compound is exposed to a polypeptide containing the NS5B binding pocket of the invention under conditions suitable for association or binding to occur. Binding is then assessed, for example using NMR or other known detection techniques.

Those of skill in the art will realize that association of natural ligands or substrates with the binding pocket of their corresponding receptors or enzymes is the basis of many biological mechanisms of action. Similarly, many drugs exert their biological effects through association with the binding cavities of receptors and enzymes. Such associations may occur with all or any part of the binding pocket. An understanding of such associations will help lead to the design of drugs having more favorable associations with their target receptor or enzyme, and thus, improved biological effects. Therefore, this information is valuable in designing potential ligands or inhibitors of receptors or enzymes, such as inhibitors of HCV NS5B-like polypeptides, and more importantly, HCV NS5B.

In iterative drug design, crystals of a series of protein/compound complexes are obtained and then the three-dimensional structure of each complex is solved. Such an approach provides insight into the association between the proteins and compounds of each complex. This is accomplished by selecting compounds with inhibitory activity, obtaining crystals of this new protein/compound complex, solving the three-dimensional structure of the complex, and comparing the associations between the new protein/compound complex and previously solved protein/compound complexes. By observing how changes in the compound affected the protein/compound associations, these associations may be optimized.

The following specific examples are not to be construed as limiting.

### EXAMPLES

### EXAMPLE 1 - EXPRESSION AND PURIFICATION OF HCV NS5B

The recombinant HCV genotype 1 b (J4 strain) NS5B polymerase (the amino acid sequence of which is shown in **SEQ ID NO: 1**) was produced in soluble form by expression of a variant that lacks the C-terminal 21 amino acids normally found on the mature NS5B. For the purposes of the present invention, this NS5BΔ21 was expressed with a C-terminal hexa-histidine sequence. Expression of the NS5B from a pET vector in *E. coli* strain JM109 (DE3) was induced with 0.4 mM IPTG for 3 hours at 24°C. Cells were harvested (in 25 mM Tris pH 7.5, 10 % glycerol, 1 mM EDTA, 500 mM NaCl, 2 mM 2-mercaptoethanol and cocktail of protease inhibitors (standard cocktail that can be purchased from Roche Biochemicals Inc.) and lysed in a microfluidizer. The lysate, after centrifugation (30,000X), was purified according to the following sequential chromatographic steps that are known to one skilled in the art: (i) metal affinity using Ni-NTA resin (Qiagen) and elution with a buffer containing increasing concentration of imidazole (from 10 mM to 500 mM); (ii) DEAE-Sepharose chromatography wherein the NS5B flowed through the column in a buffered solution containing 300 mM NaCl; (iii) Heparin Sepharose chromatography wherein the NS5B was in a buffered solution with 200 mM NaCl, and then bound NS5B was then eluted with a buffer containing a gradient up to 1 M NaCl. Eluted fractions, enriched with the NS5B protein were concentrated on a Resource S column, and then applied to a Superdex 200 column in 20 mM Tris-HCl pH 7.5, 10 % glycerol, 5 mM DTT, 300 mM NaCl. Peak fractions containing highly pure His-tag NS5B were stored at -80°C until use.

### EXAMPLE 2 - CRYSTALLIZATION OF HCV NS5B

HCV polymerase NS5B strain 1b/J4 **(SEQ. ID NO: 1**), obtained as described in detail in Example 1, was crystallized in its apo form using monomethyl ether polyethylene glycol 5000 (PEG5Kmme) as a precipitant agent. Large single crystals were obtained by the hanging drop vapor diffusion technique (McPherson, *supra).* In particular, 1 µl of NS5B (7.66 mg/mL in purification buffer containing 20 mM Tris, pH 7.3, 300 mM NaCl, 10% glycerol) was added to 1 µL of a solution made of 21% PEG5Kmme, 0.1 M MES, pH 5.4, 10% glycerol and 0.4 M ammonium sulfate. The resulting 2 µL drop was suspended above a 1 mL reservoir solution of 21 % PEG5Kmme, 0.1 M MES, pH 5.4, 20% glycerol and 0.4 M ammonium sulfate. The crystals of the NS5B complex obtained belong to space group P2(1)2(1)2(1) with unit cell dimension of a= 105.1, b=106.5 and c=133.5 and contain two molecules per asymmetric unit. The crystals were shown to diffract to a resolution of up to 2 Å using X-rays from a regular rotating anode source.

### EXAMPLE 3 - PREPARATION OF INHIBITOR COMPOUNDS A, B AND C

Inhibitor compound **A** was made as described in detail in co-pending application US 10/755,256 filed January 12, 2004.

Inhibitor compound **B** was made as described in detail in co-pending application US 10/755,256 filed January 12, 2004.

Inhibitor compound **C** was made as described in detail in WO 03/007945 published January 22, 2003.

### EXAMPLE 4 - FORMATION OF NS5B-INHIBITOR COMPLEX

### Inhibitor soaking

Crystals were transferred in a 5 µL drop of a soaking solution made of 14% PEG5Kmme, 14 mM Tris, pH 7.5, 70 mM MES, pH 7.0, 14% glycerol, 210 mM NaCl, 10 mg/mL lysozyme and 280 mM ammonium sulfate. The inhibitor molecule was dissolved in DMSO to a concentration of 25 mM. Inhibitor solution (0.2 µL) was added to the 5 µL NS5B crystal drop and incubated for 5-6 hours at 11°C. Following incubation, the crystals of the NS5B-inhibitor complex were transferred from the solution with a cryoloop (Hampton Research, California, USA) and cryo-cooled in liquid nitrogen. NS5B complexes were prepared using Compounds **A** and **B.**

### Data collection

Diffraction data were collected on a MicroMax-007 rotating anode x-ray generator equipped with an Raxis-IV++ Image plate detector (Rigaku/MSC, Texas, USA). Data to a resolution of 2.8 Å was collected on a single crystal cryo-cooled at -180°C for NS5B complexes prepared with Compound **A** and Compound **B.**

### Phasing, Model Building and Refinement

The diffraction data were phased by Molecular Replacement (MR) using the publicly available structure of HCV NS5B (pdb code : 1 C2J). Rotation and translation search were done using the program CNX (Accelrys). Model building was carried out with the software O (Alwyn Jones, Upsala University, Sweden) and model refinement was performed with the CNX software (Accelrys). The model was improved by repetition of the procedure of model building and refinement until a desirable result was obtained. The final model in each case included two molecules of NS5B, identified as NS5B A and NS5B B, (i.e. residue A1 to A149, A154 to A563, B1 to B17, B36 to B148 and B153 to B563) and one molecule of compound **A** or **B** associated with NS5B molecule B. The resulting atomic structure coordinates of the compound **A** and compound **B** NS5B complexes are shown in Fig. 4 and Fig. 5, respectively. The final crystallographic R factor the NS5B-Compound **A** complex was 21.9% and R_{free} factor was 28.0% to a resolution of 2.8A. For the NS5B-Compound **B** complex, the final crystallographic R factor was 21.9% and R_{free} factor was 25.7% to a resolution of 2.7 Å.

### EXAMPLE 5 - MODEL OF NS5B-COMPOUND C COMPLEX BASED ON AN NMR BOUND CONFORMATION.

The binding site of Compound C on HCV polymerase was determined by several steps which are described herein in detail. Initially, NMR spectroscopic methods (transferred NOESY) were applied to determine the structure of Compound **C** when bound to HCV polymerase. Another NMR technique, differential line-broadening (DLB), helped to identify the segments of Compound **C** which come into contact with the polymerase versus those that are solvent exposed in the bound state. The bound structure was then docked onto the X-ray-derived structure of the NS5B-Compound **A** complex. The docking procedure began by overlaying the common feature of the 5/6 aromatic rings of the indole and benzimidazole of Compound **A** and Compound **C.** All the differential line-broadening data were then accounted for and the complex was energy minimized using MOE.

The transferred NOESY and DLB data were acquired as follows. A sample tube (5 mm) containing Compound **C** was prepared by adding 15 µL of concentrated solution in DMSO-*d*₆ (containing 0.31 mg of Compound **C**) to an aqueous buffer composed of 20 mM Tris-d₁₁, 2 mM DTT-d₁₀, 1 mM EDTA-d₁₂, 300 mM NaCl, and 10% (v/v) D₂O spiked with TSP-2,2,3,3-d₄. Buffer was added to a final volume of 600 µL, and the pH was adjusted to 6.0. Spectra of free Compound **C** were taken. A concentrated stock solution of HCV polymerase was added twice to the NMR tube and spectra were acquired. The concentrated stock contained 21.7 mg/mL of HCV polymerase (NS5BΔ21C-His₆) in buffer consisting of 20 mM Tris-d₁₁, 2 mM DTT-d₁₀, 1 mM EDTA-d₁₂, 300 mM NaCl, and 10% (v/v) glycerol-d₈.

NMR spectra were acquired on Bruker AVANCE 600 and 800 MHz NMR spectrometers at 22 and 27 °C. Suppression of the solvent signal was achieved by the use of pre-saturation or by inserting a 3-9-19 WATERGATE module prior to data acquisition. One-dimensional spectra were collected on both sample tubes for DLB comparisons, and 128 scans were collected for each spectrum. NOESY experiments on a sample tube containing no polymerase resulted in the observation of no significant cross-peaks, as expected. On the other hand, NOESY spectra on a sample tube containing polymerase resulted in the observation of many cross-peaks which contained the valuable inter-hydrogen distance information of the inhibitor **C** when bound to HCV polymerase. A series of NOESY mixing times including 75, 100, 150, 200 and 300 msec (at 600 and 800 MHz fields) was recorded. Two-dimensional data sets were typically acquired with 2048 points in t₂ and 512 points in t₁. 128 scans were averaged for NOESY FIDs. The data were processed and analyzed using Bruker's XWinNMR and WinNMR software (Bruker Canada, 555 Steeles Ave. East, Milton, Ontario). Data sets were zero-filled to yield 2048 by 2048 real points after transformation using a phase-shifted sinebell window function.

The structure of Compound C was calculated by a custom simulated annealing protocol that was implemented in the MOE molecular modeling program (CCG, Montreal, Qc, Canada). All calculations were performed using the mmff94 forcefield as implemented in MOE version 2202.03. NMR-derived flat-bottomed restraints were generated from the series of NOESY data. The relative intensity of NOESY cross-peaks was classified into three categories which were then used as restraints, strong (1.8-2.7 A), medium (1.8-3.5 A) or weak (1.8-5.0 Å). The force constants in the NMR-derived flat-bottomed restraints were gradually increased during the cooling stages. A single, high temperature unrestrained dynamics run was performed at 1000 K, with 100 structures collected at 10 psec intervals to generate a starting set of conformations. Each structure was cooled and minimized using the following simulated annealing protocol. During the simulations the temperature and restraint weights were changed from 1000K to 50K, and from 1/1000000 to 20, respectively. The final structures were energy minimized, the total energies were calculated, the restraint energies were calculated, and the restraint violations were determined. NMR-consistent structures were isolated, and two families of structures were identified.

One representative from each family of NMR-derived structures was then docked onto the X-ray-derived structure of the NS5B-Compound **A** complex. The docking procedure began by overlaying the common 5/6 aromatic rings of the indole and benzimidazole scaffolds of Compounds **A** and **C.** Only one structure of Compound **C** simultaneously matched the DLB data and the shape of the binding pocket. In this complex, the right-hand side of Compound **C** lies over or in the vicinity of Pro 495 and Pro 496. Using the MOE molecular modeling program, the side-chain orientations of amino acids at positions 503, 498, and 499 were slightly re-adjusted to improve the inhibitor-polymerase fit. His 428 and 502 were protonated. A combination of steepest descent, conjugate gradient and truncated Newton algorithms was used to minimize the energy of the complex which resulted in only minor changes to the structure. The protocol was set up such that the inhibitor and polymerase residues within 6 Å of the inhibitor were allowed to move during the minimization whereas all other residues were fixed, and a cutoff of long-range forces at 9.5-10 Å was used. The mmff94s forcefield as implemented in the MOE molecular modeling program version 2002.03 was applied for the calculations which included solvation. Visualization of the complex was performed using the MOE molecular modeling program.

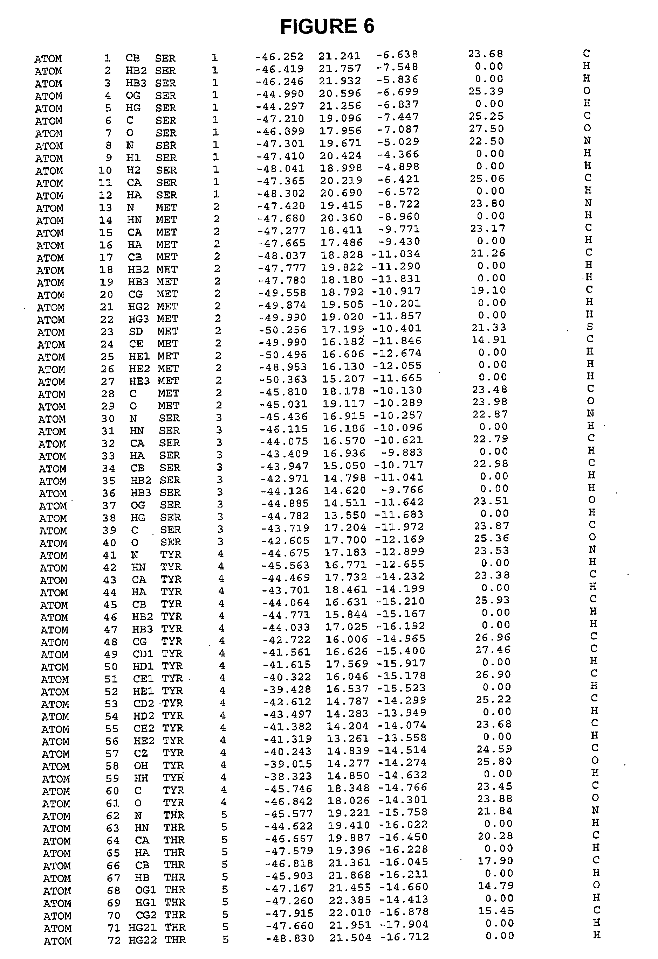

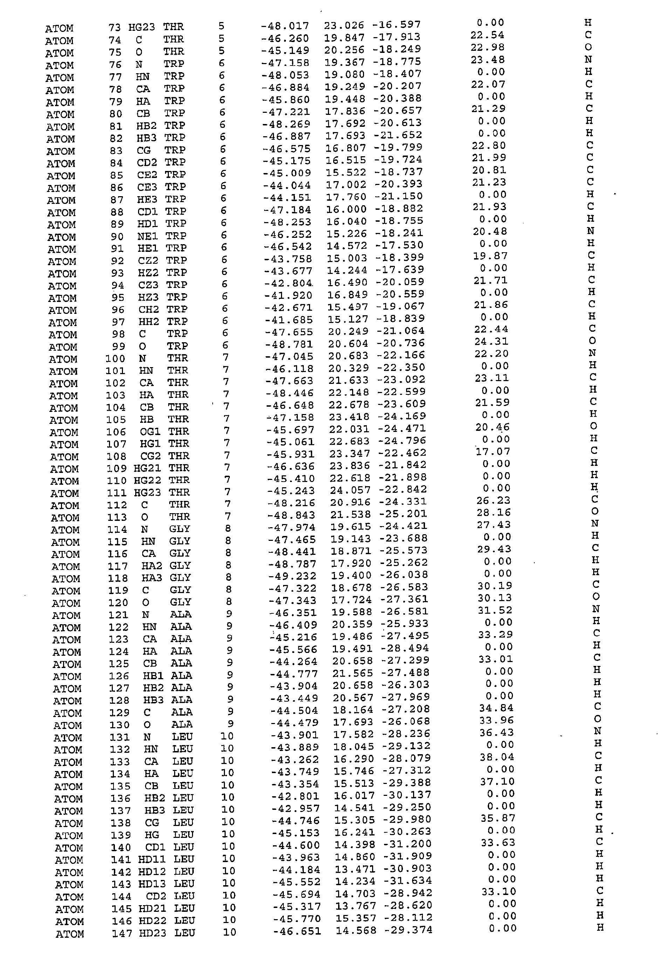

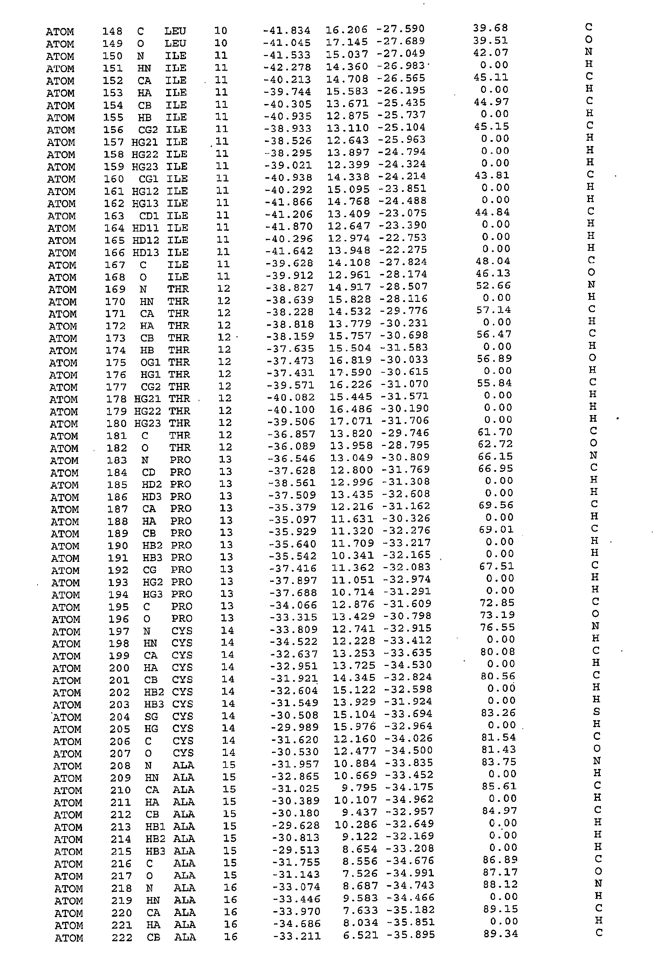

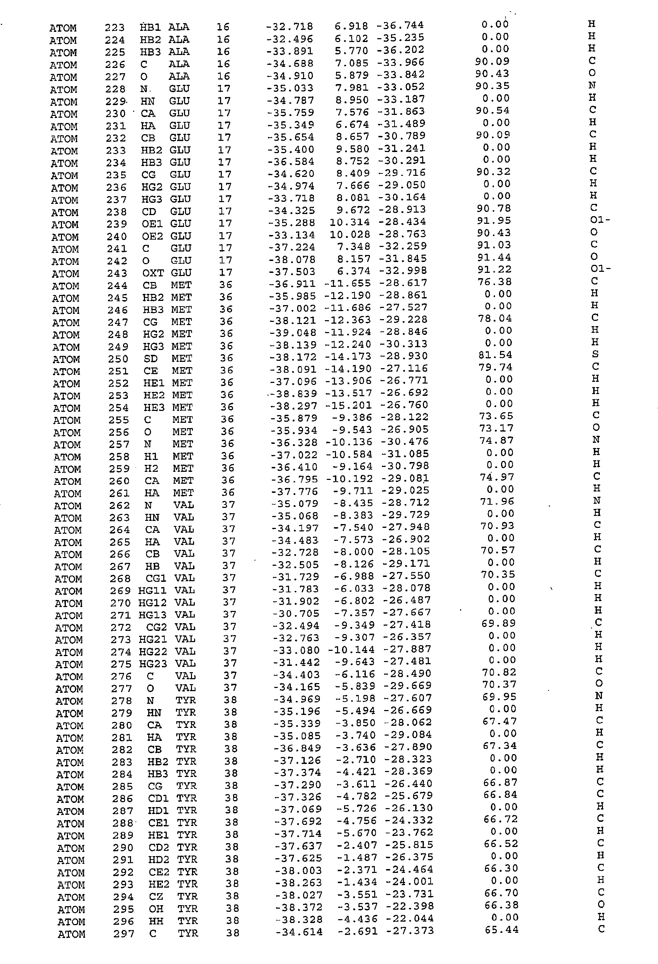

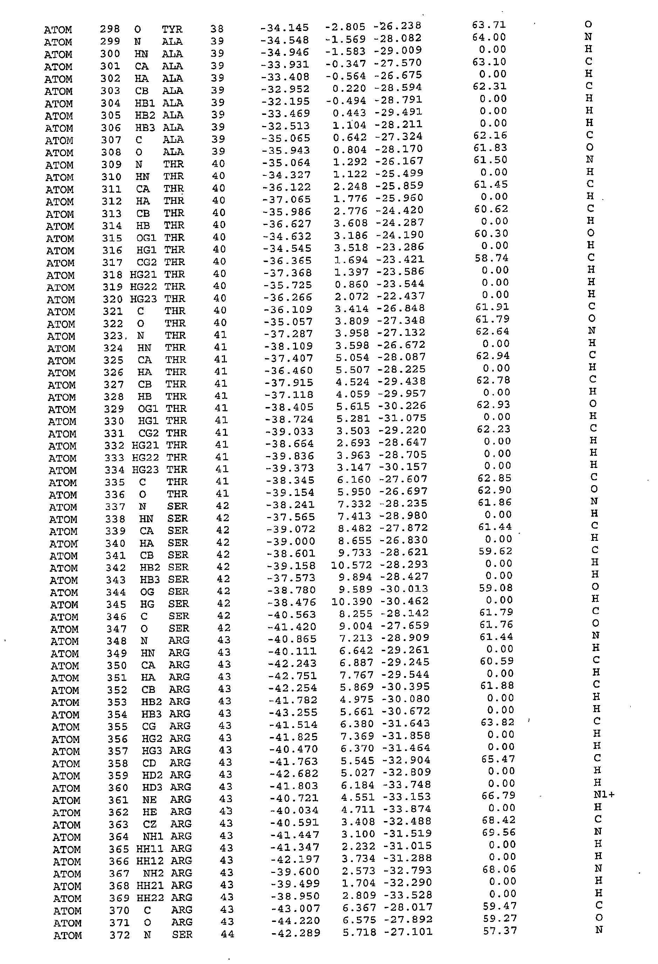

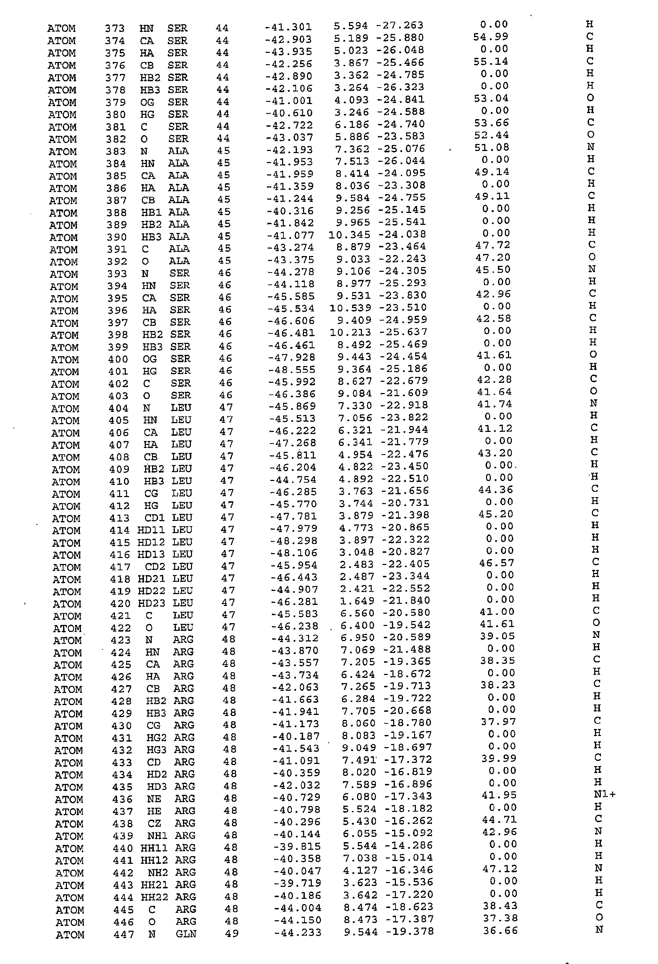

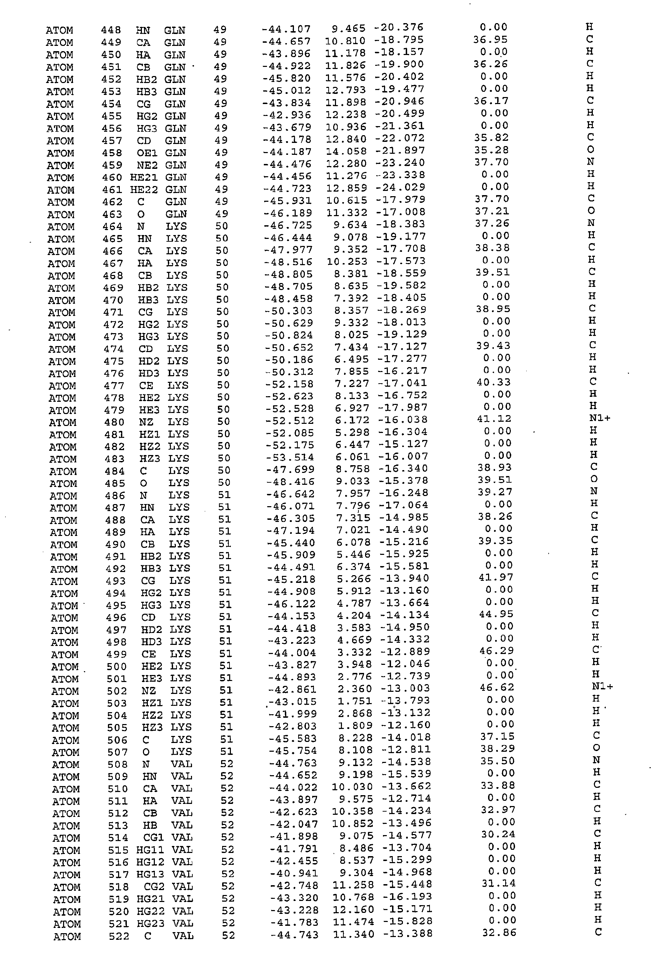

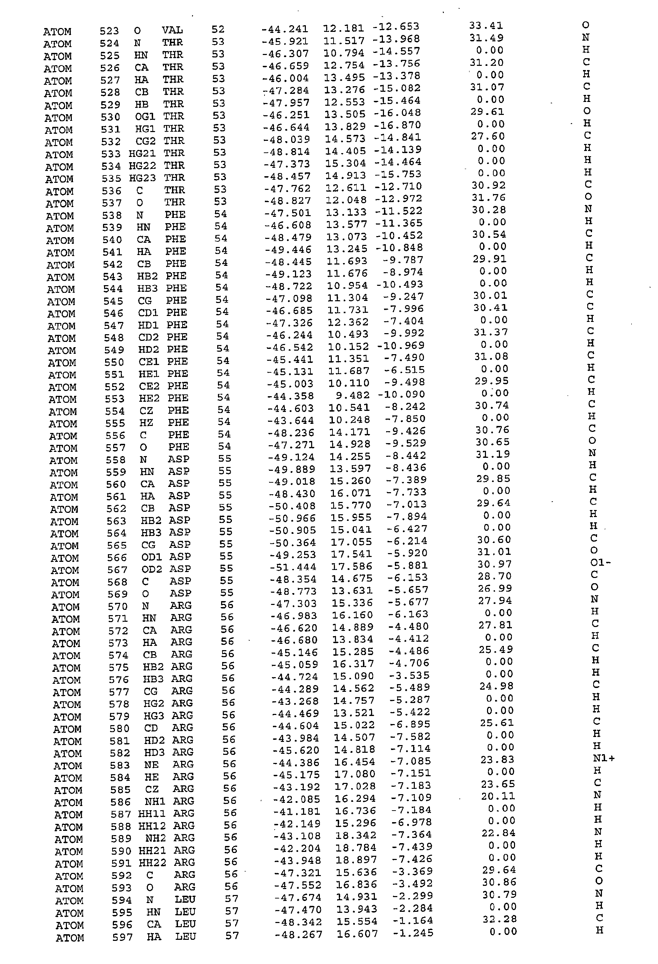

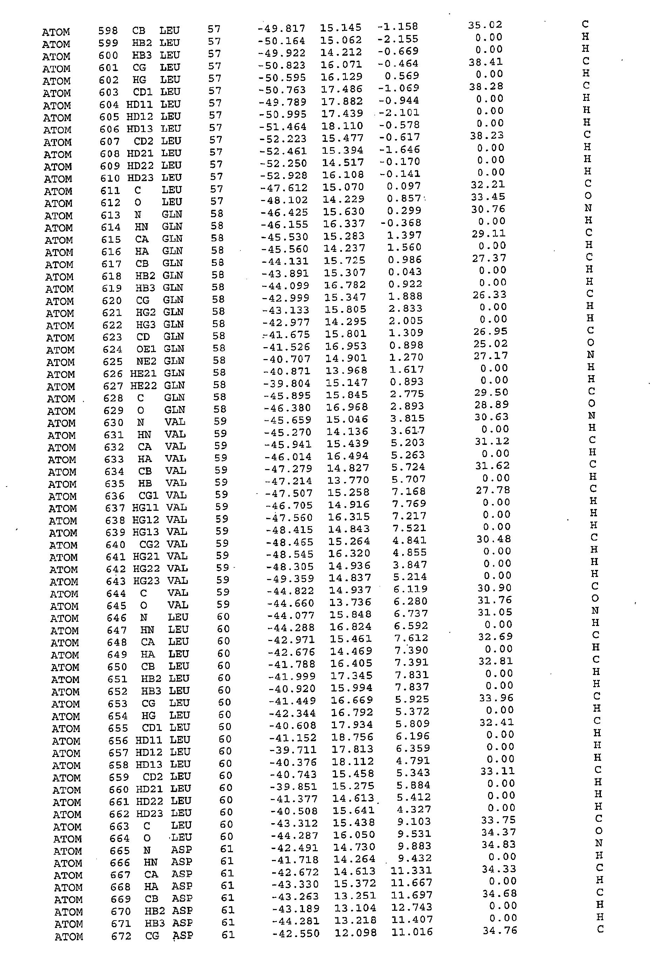

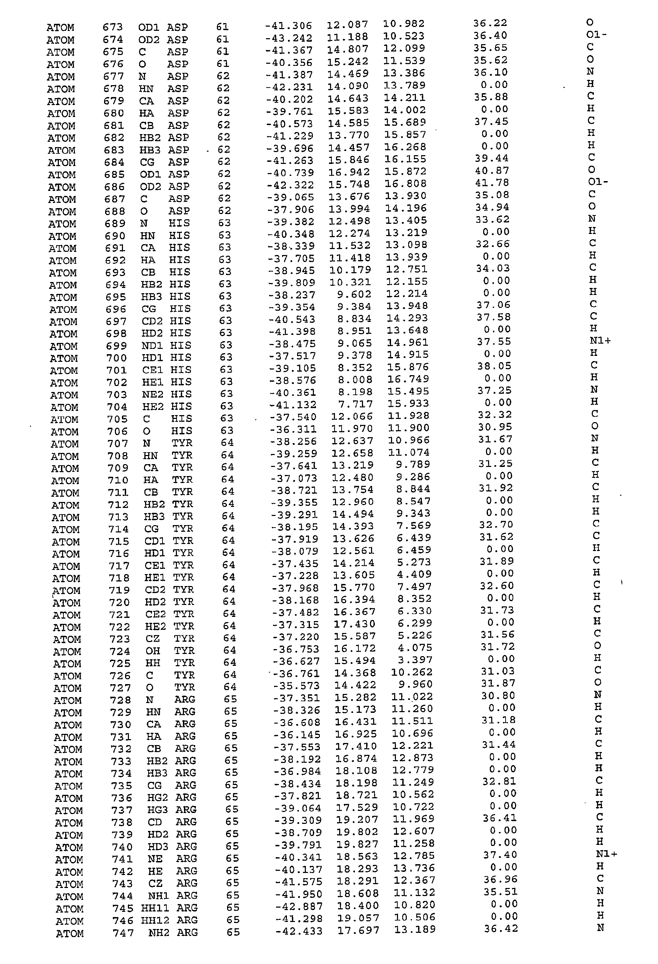

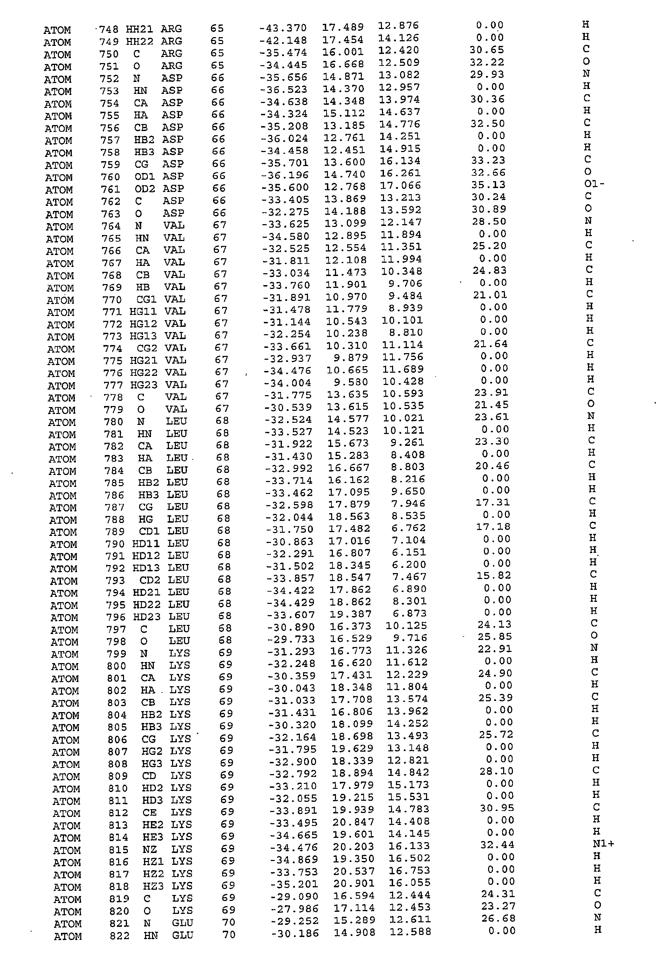

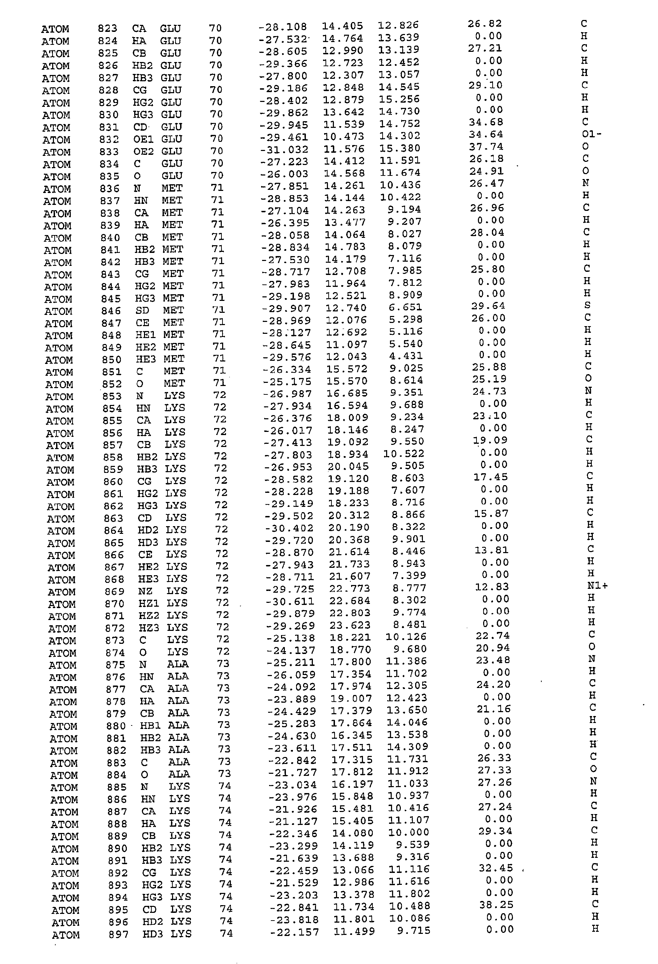

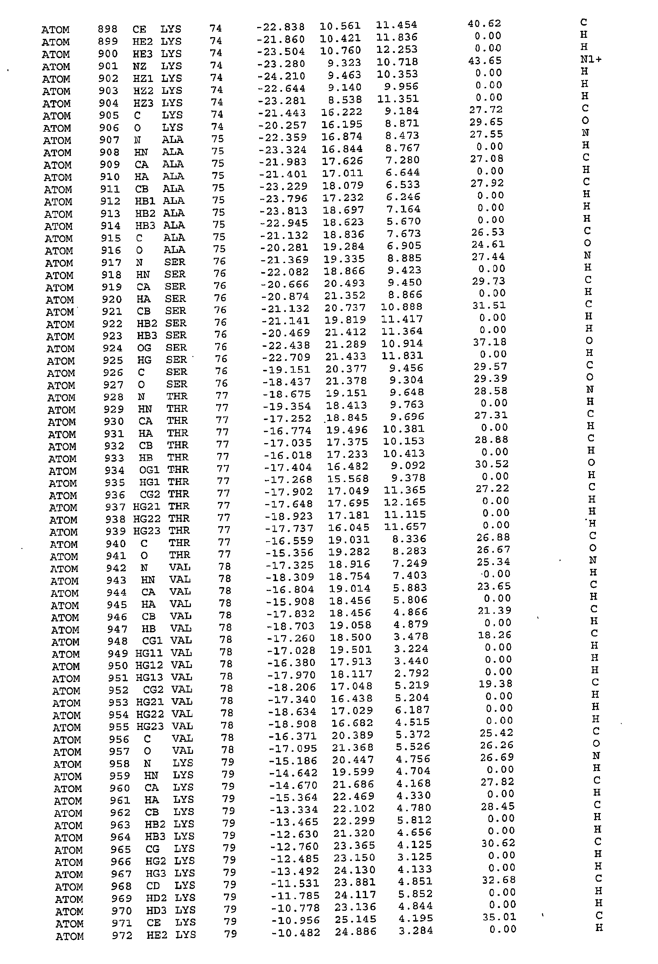

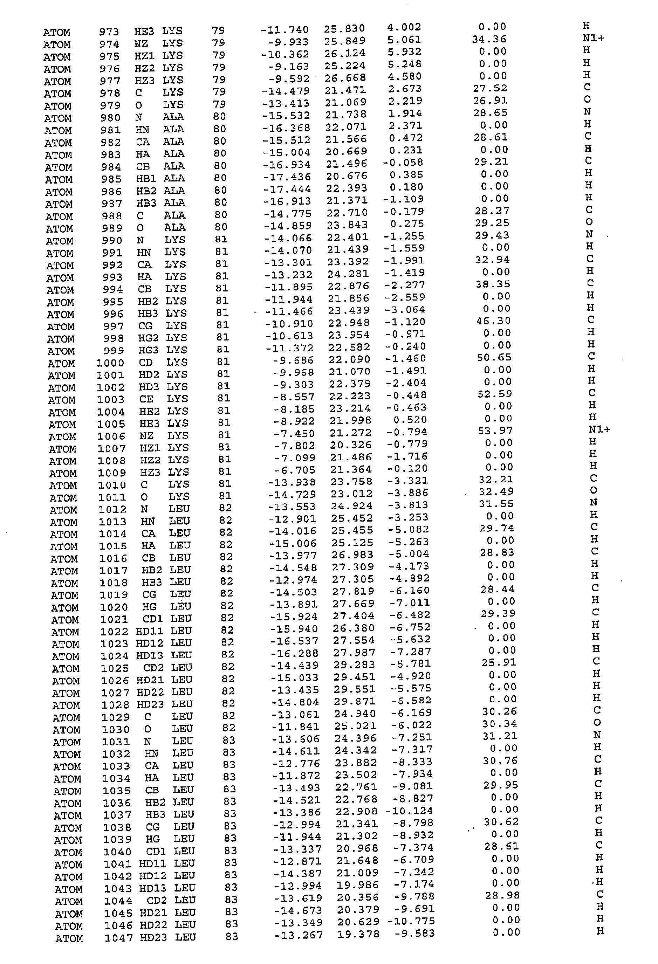

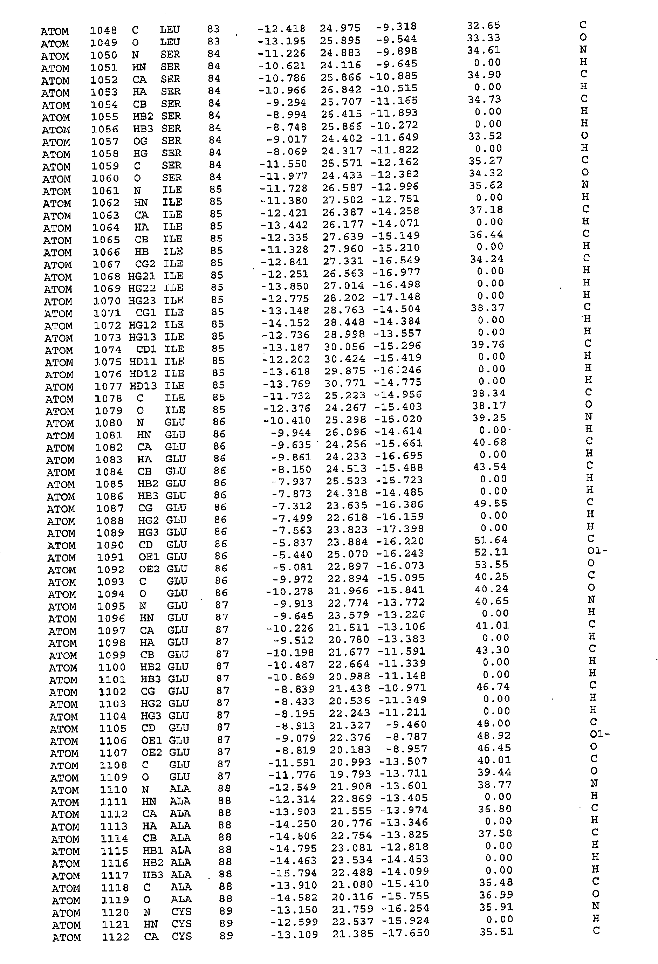

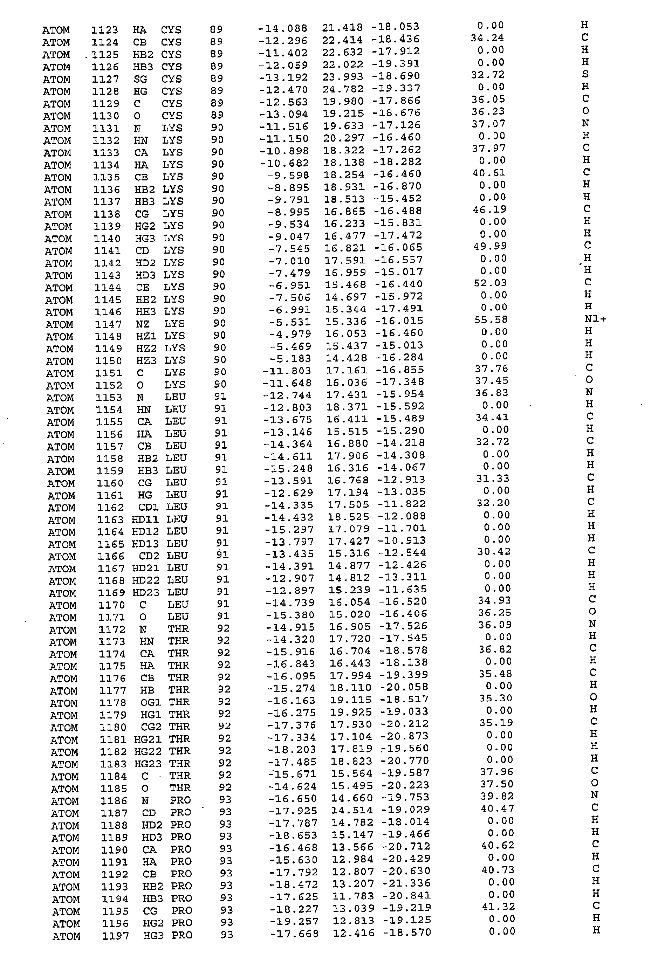

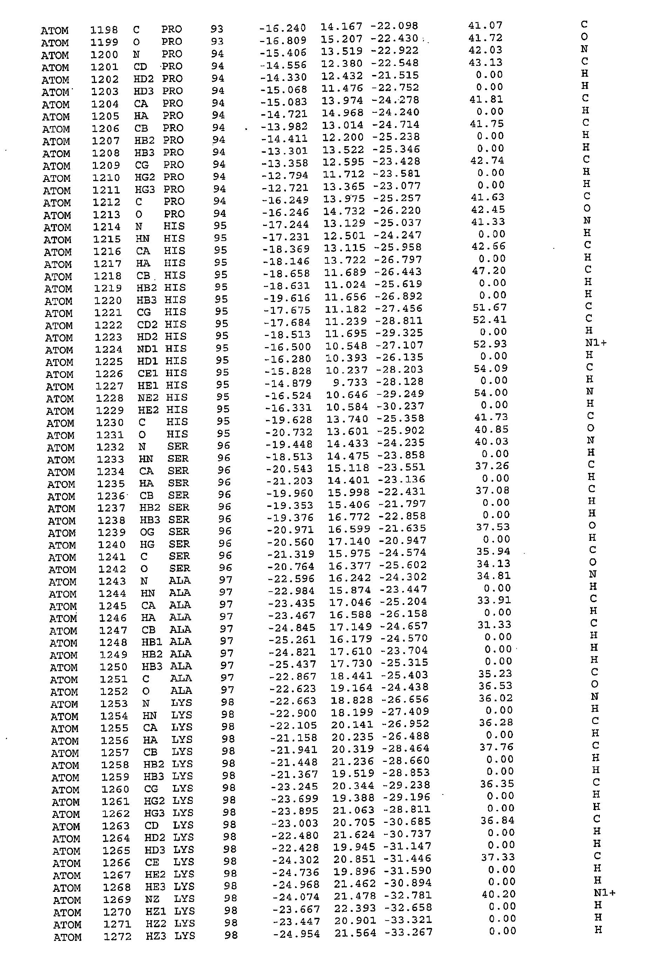

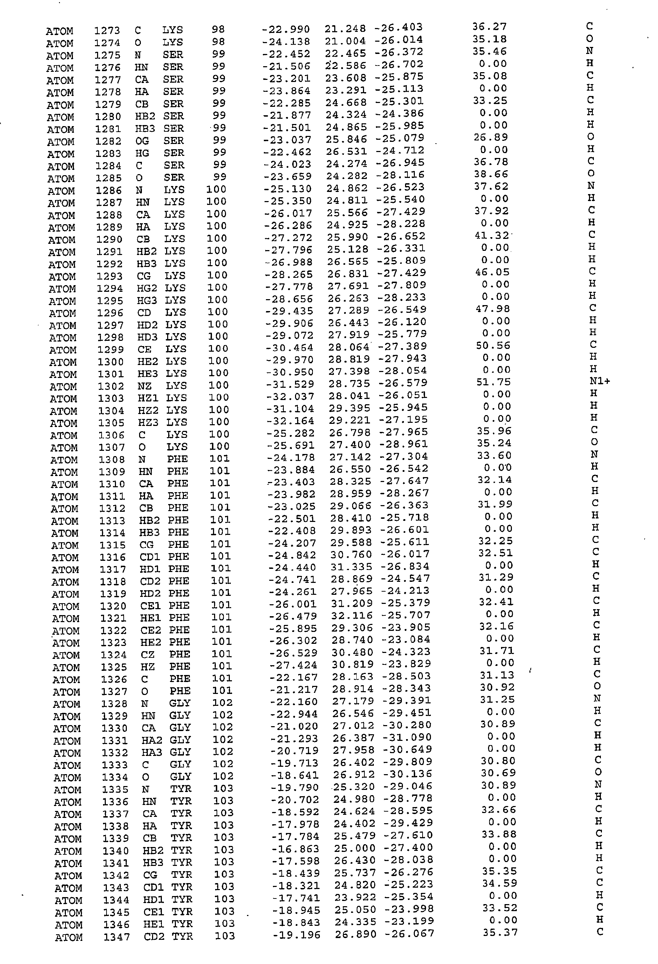

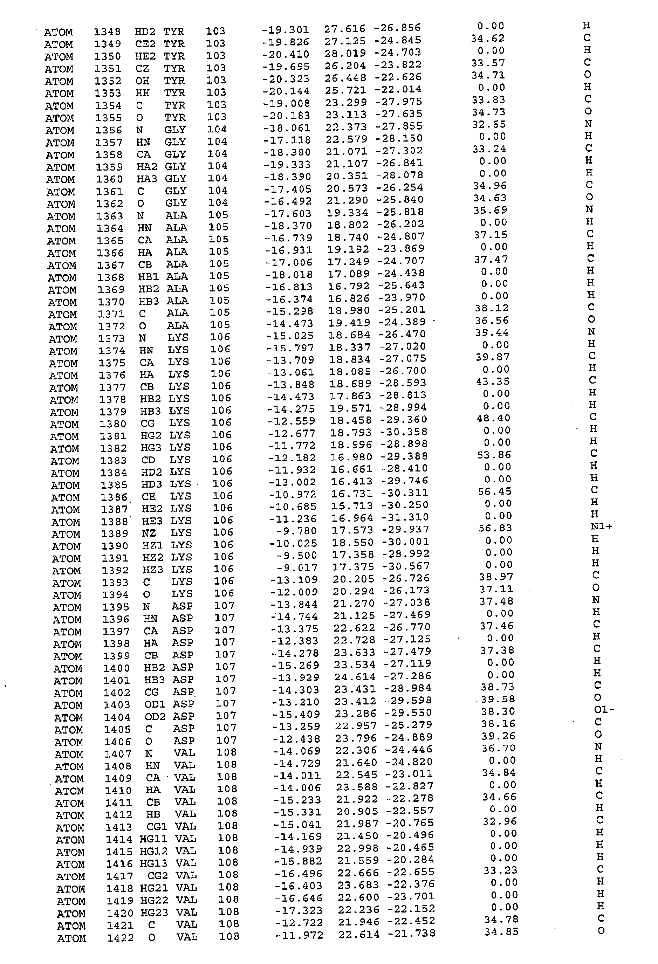

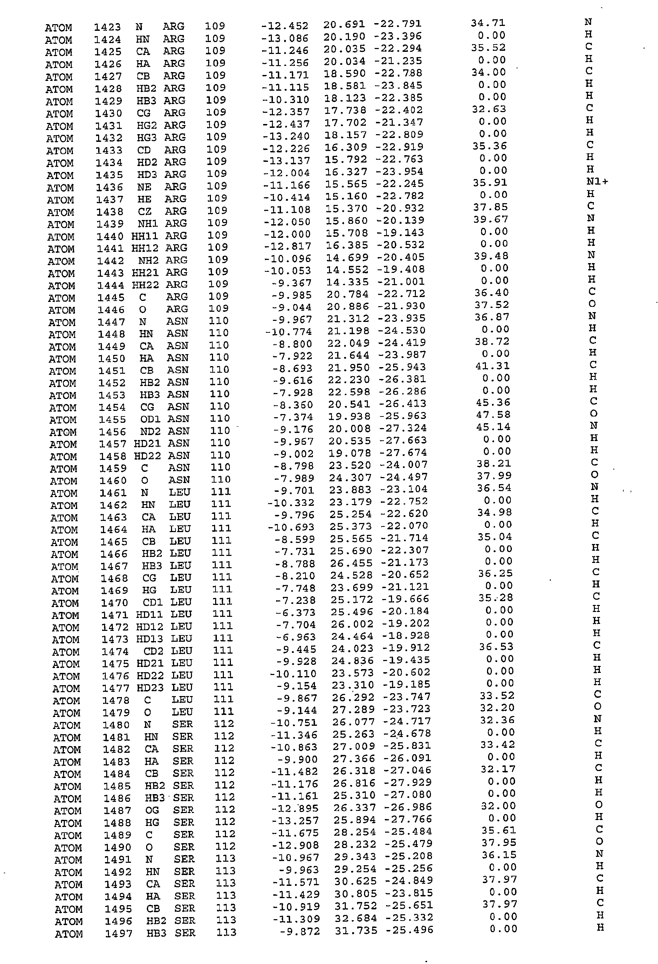

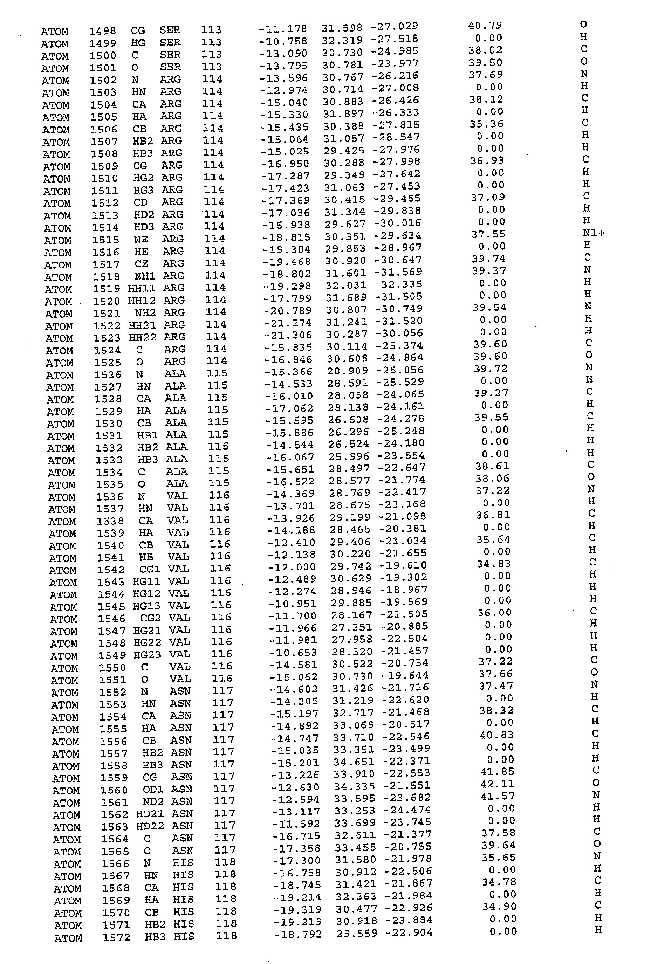

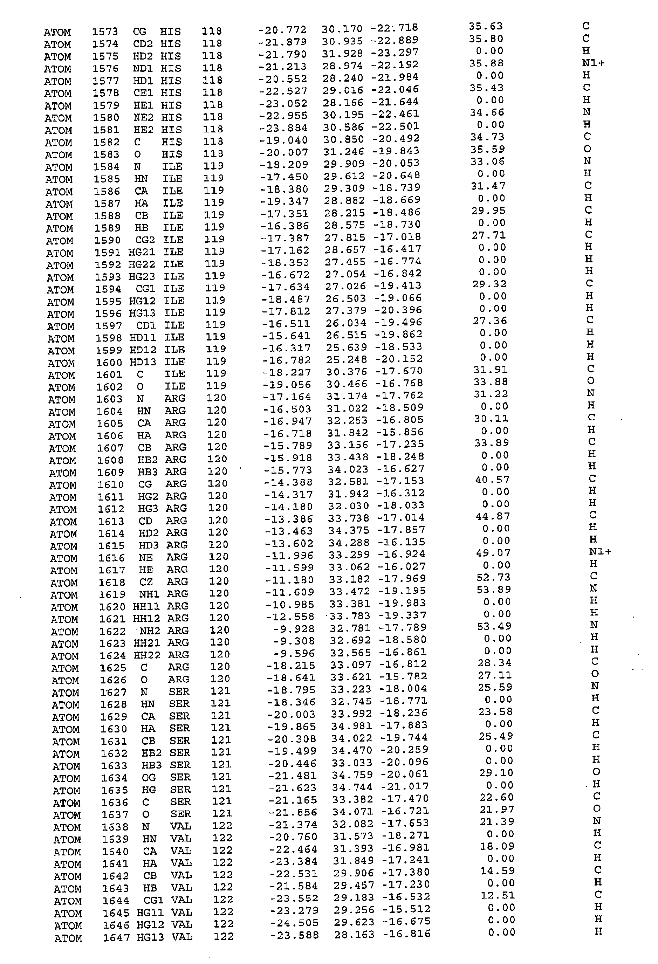

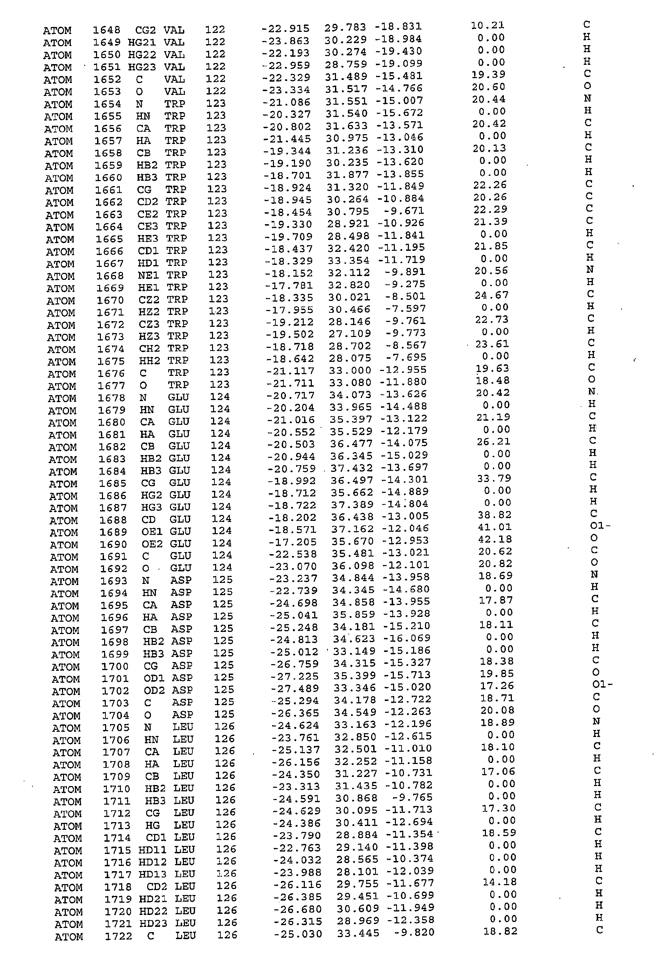

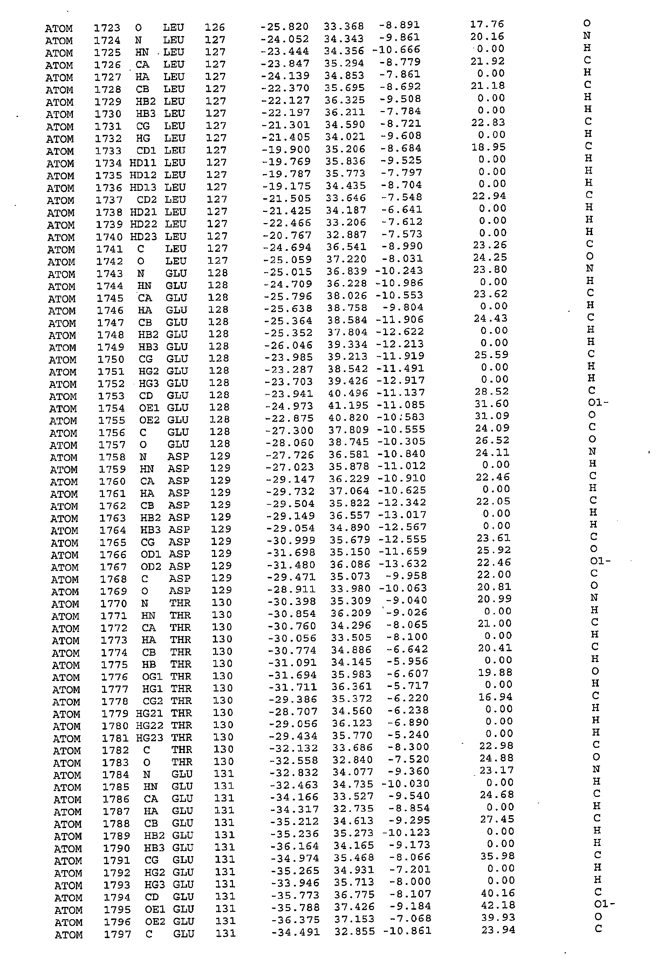

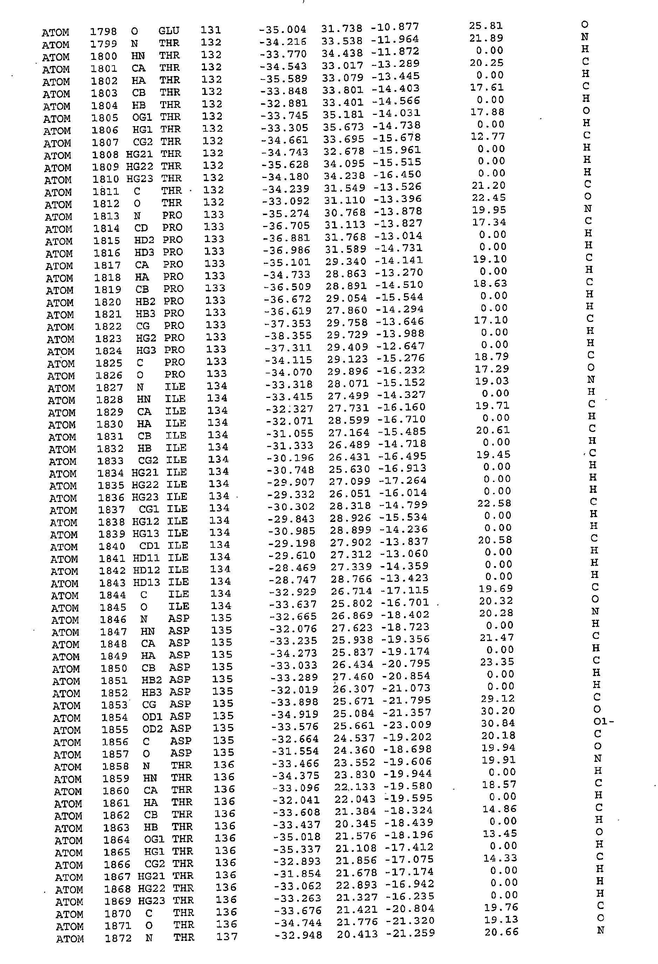

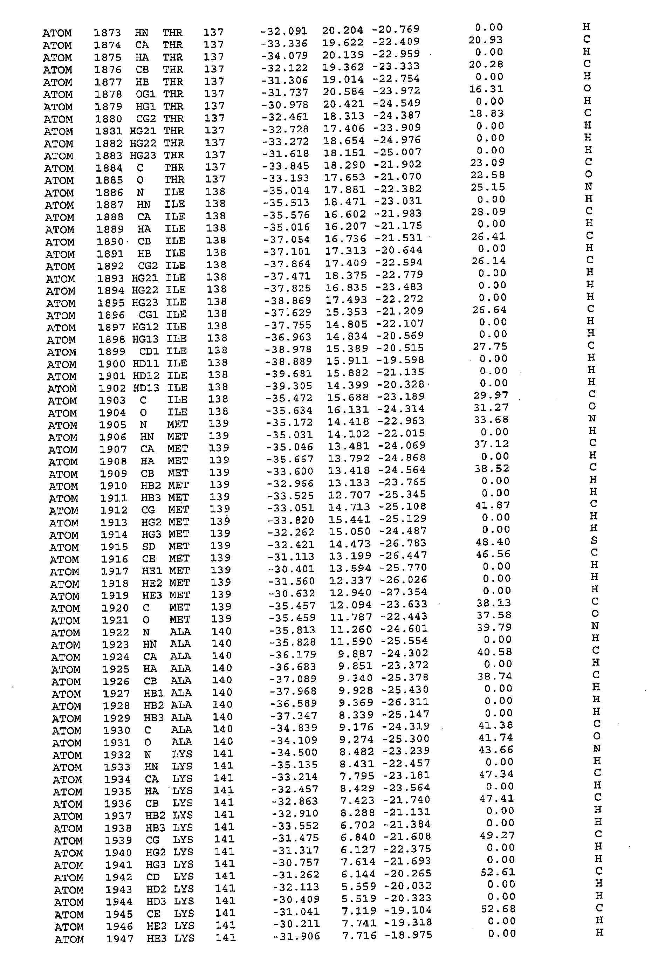

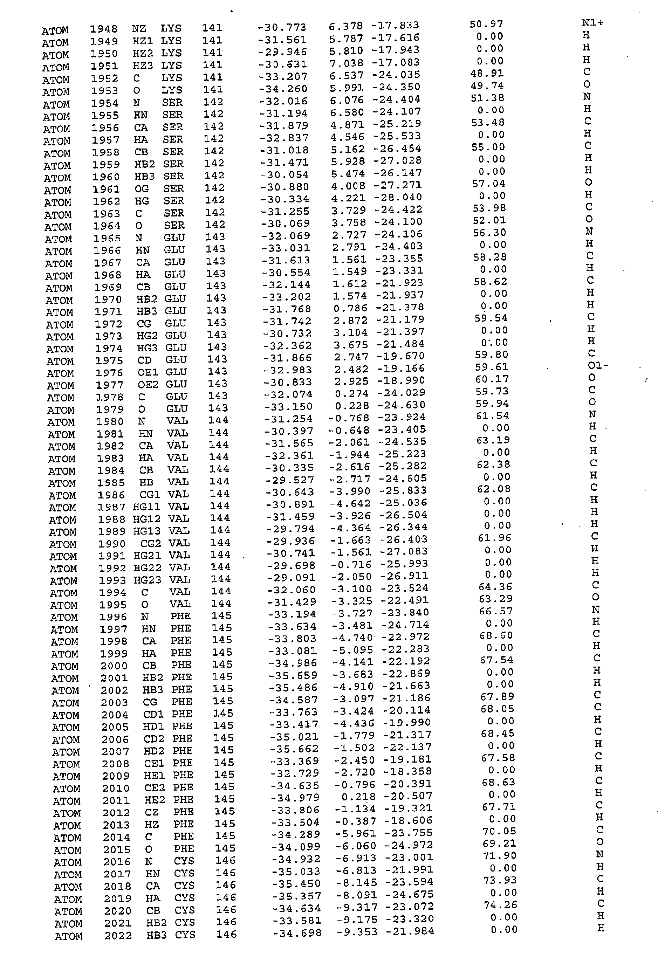

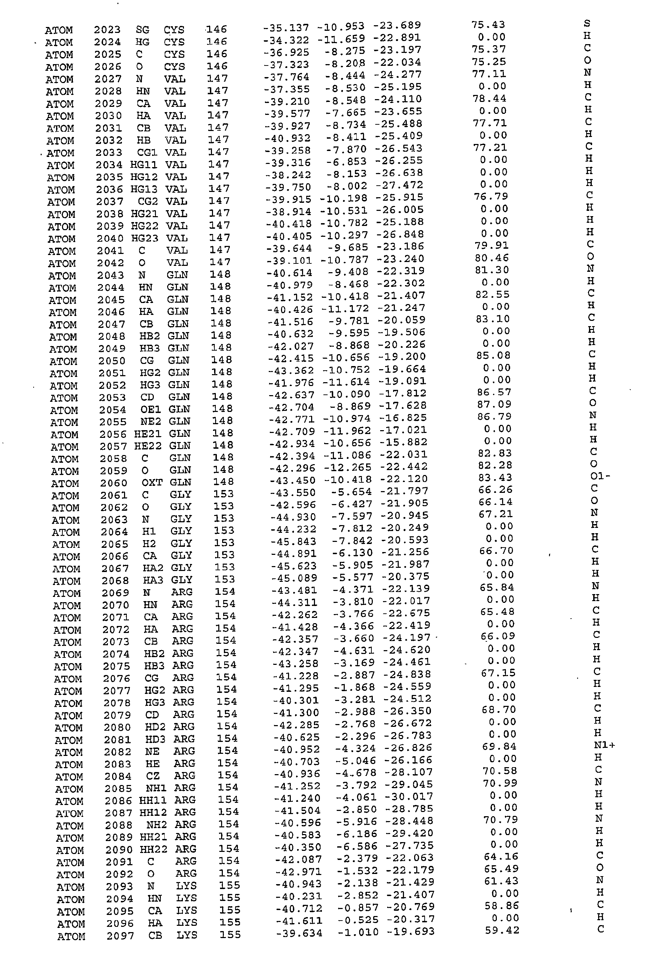

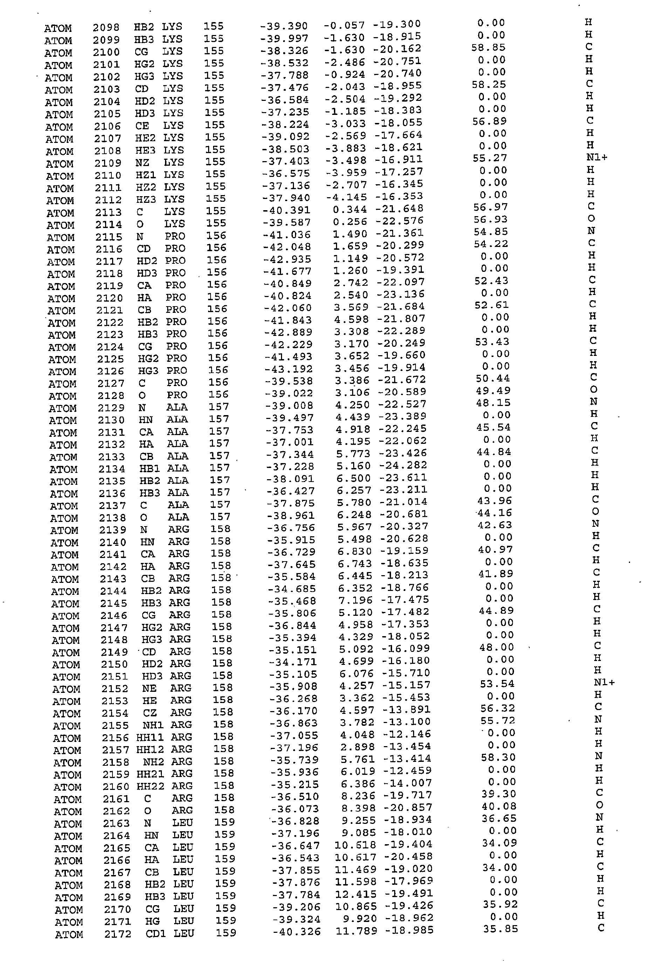

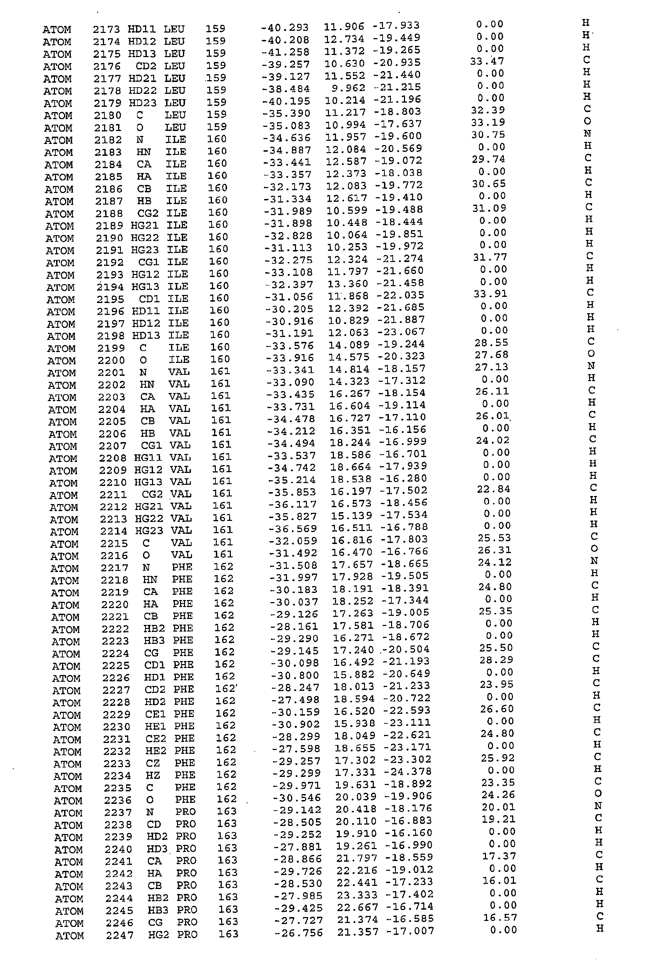

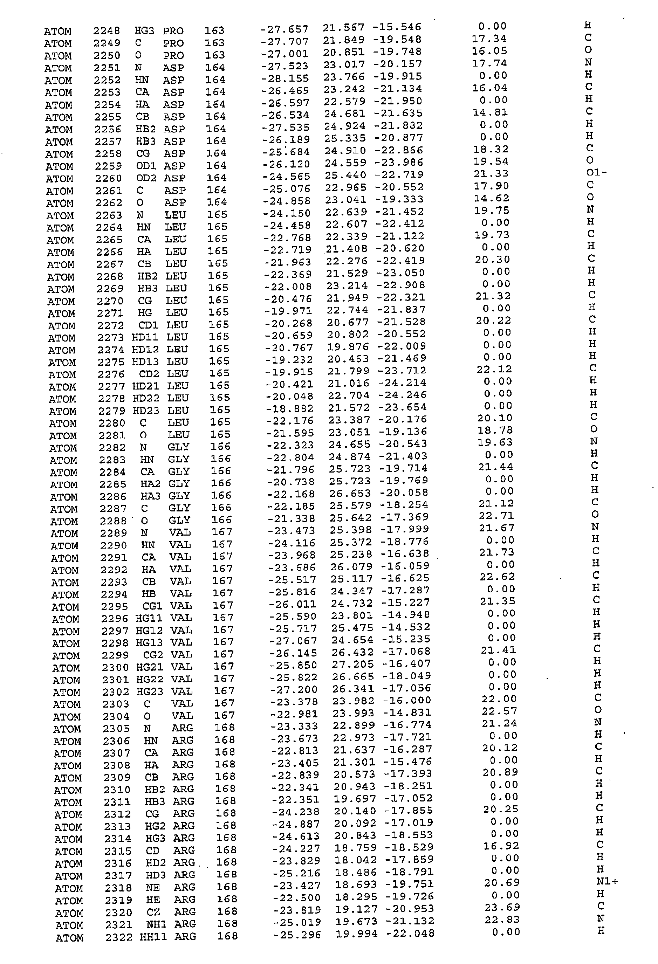

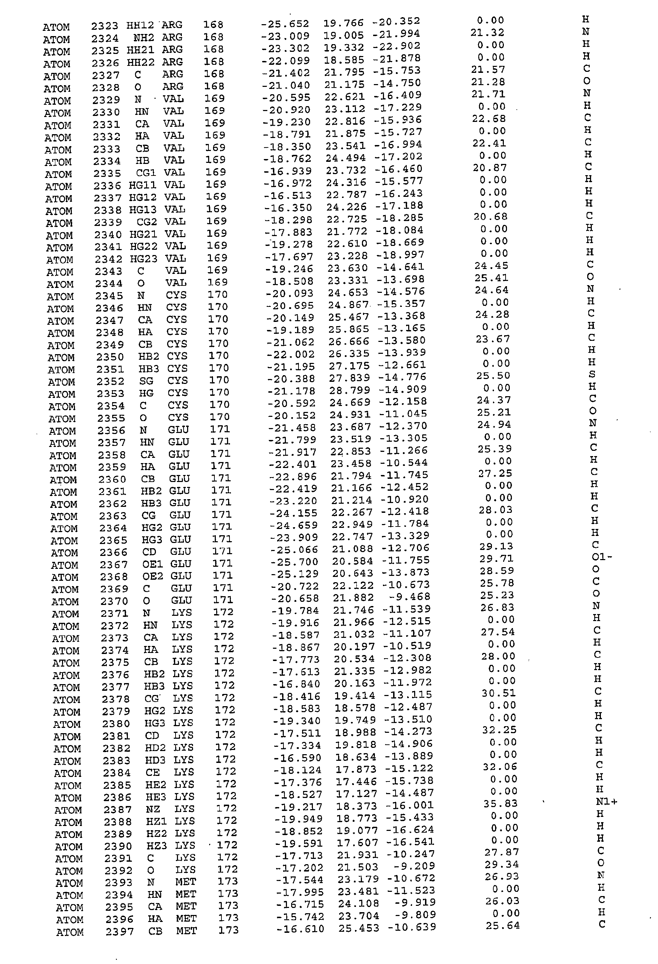

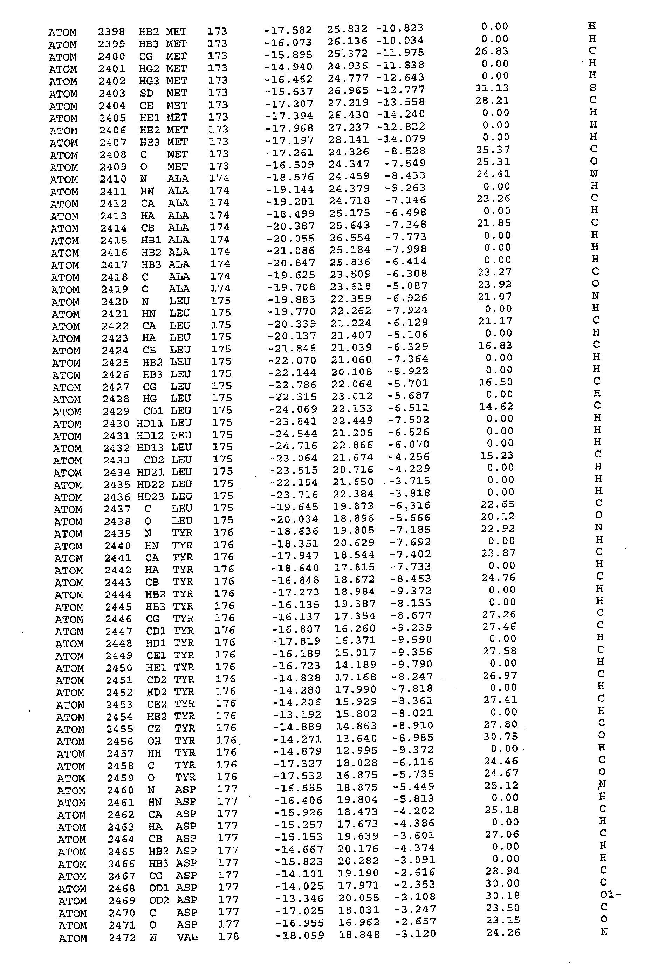

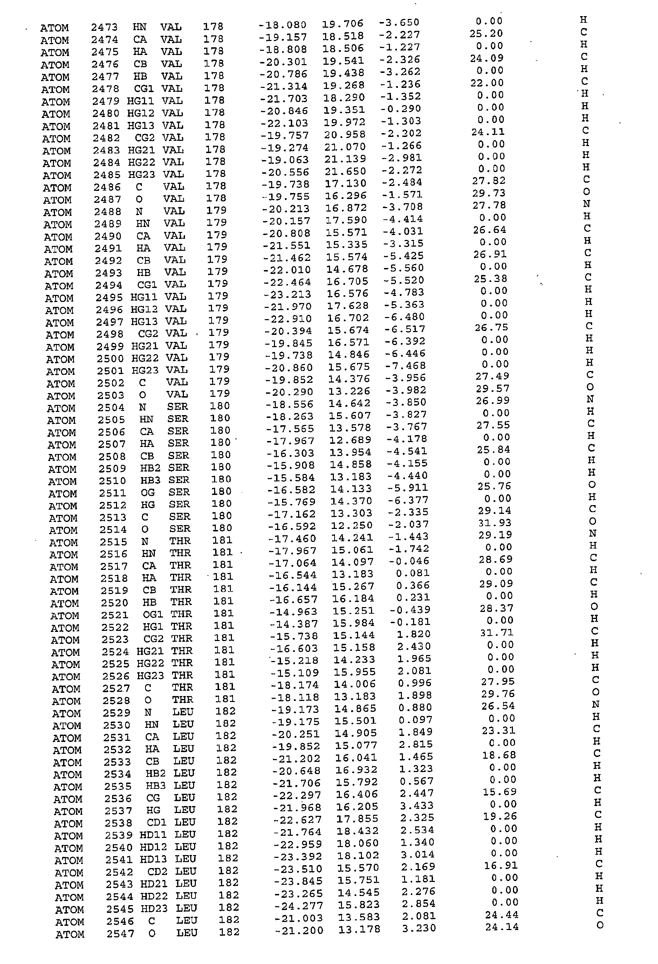

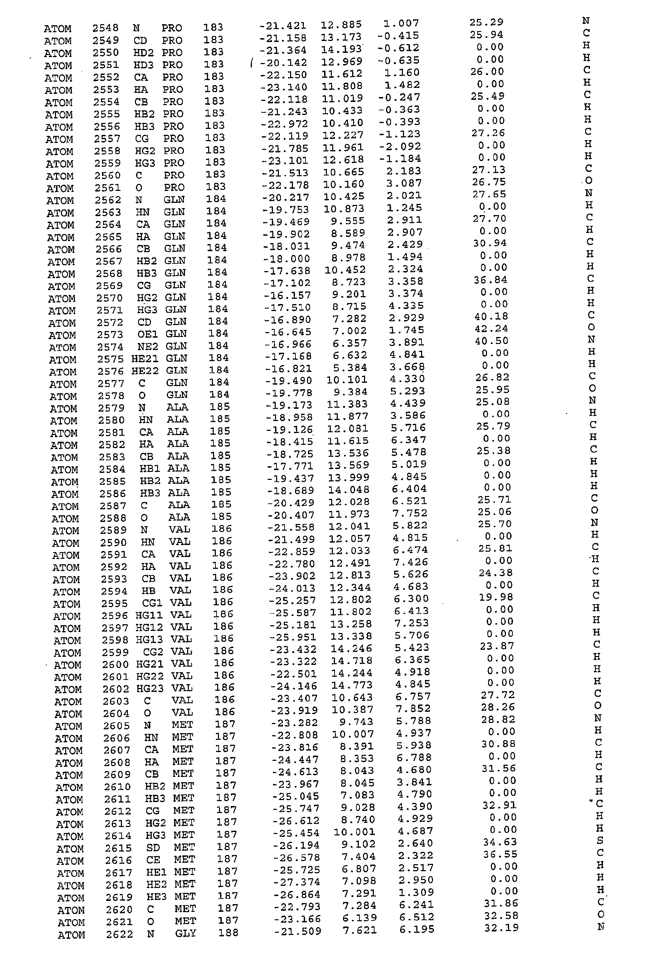

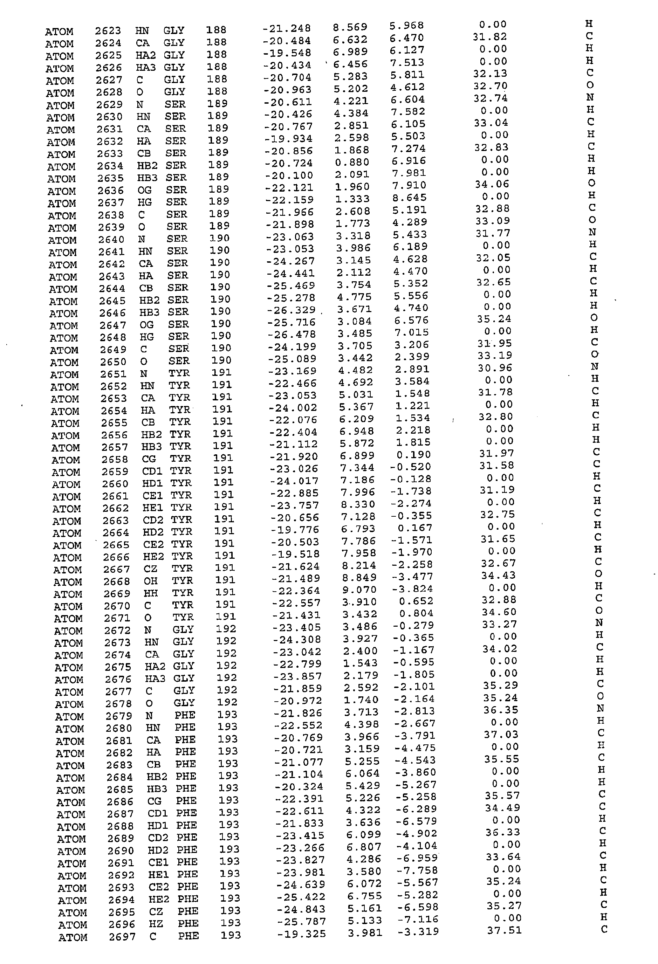

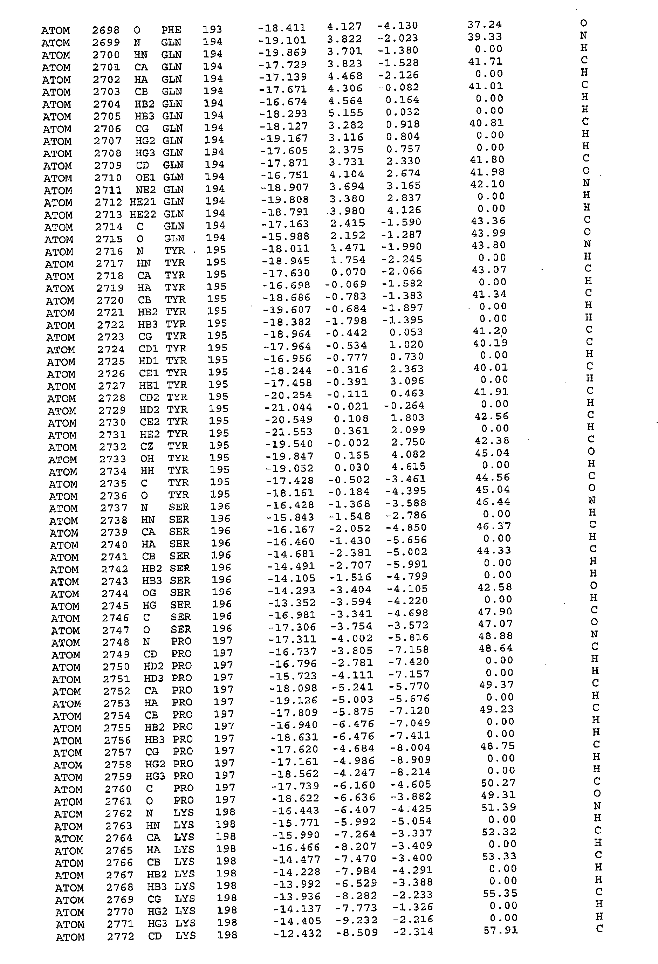

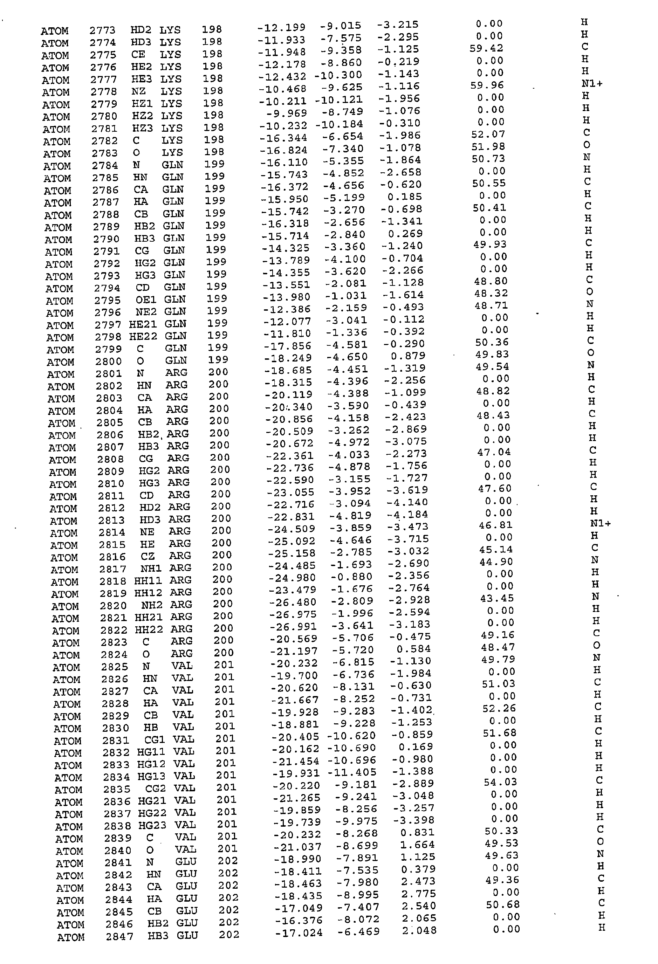

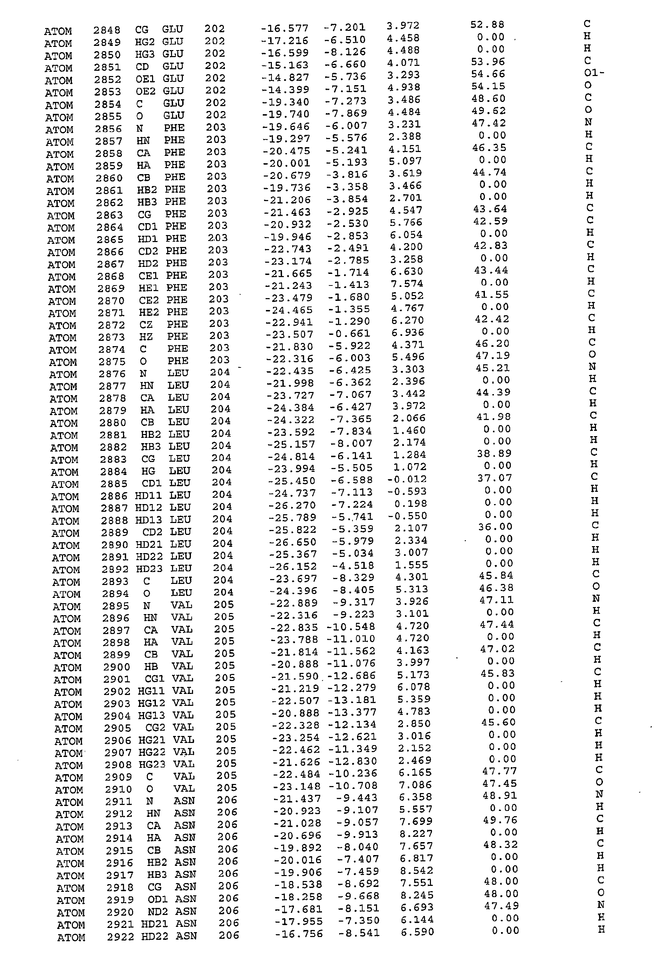

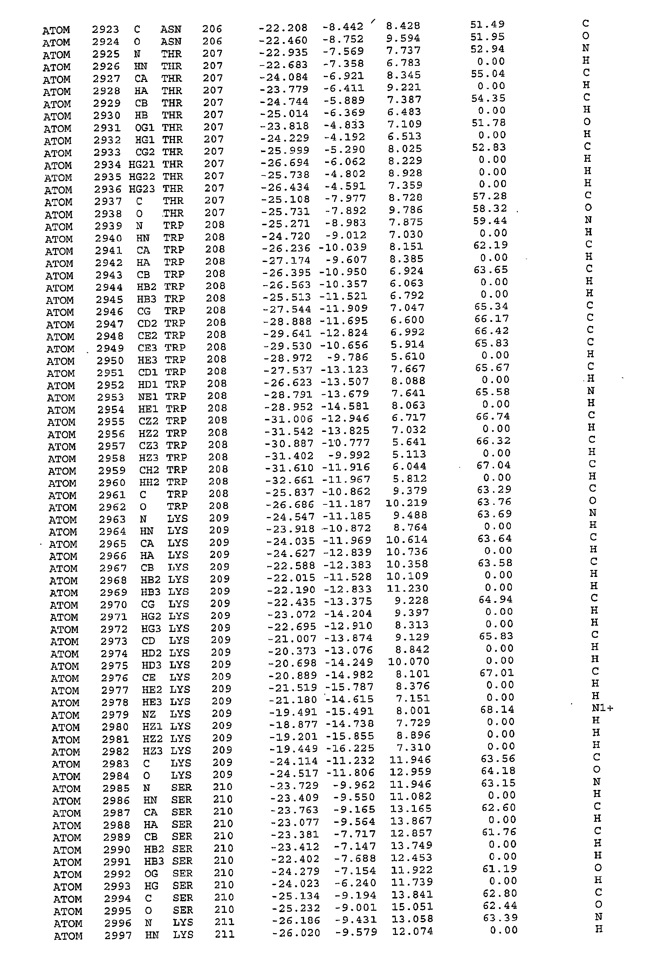

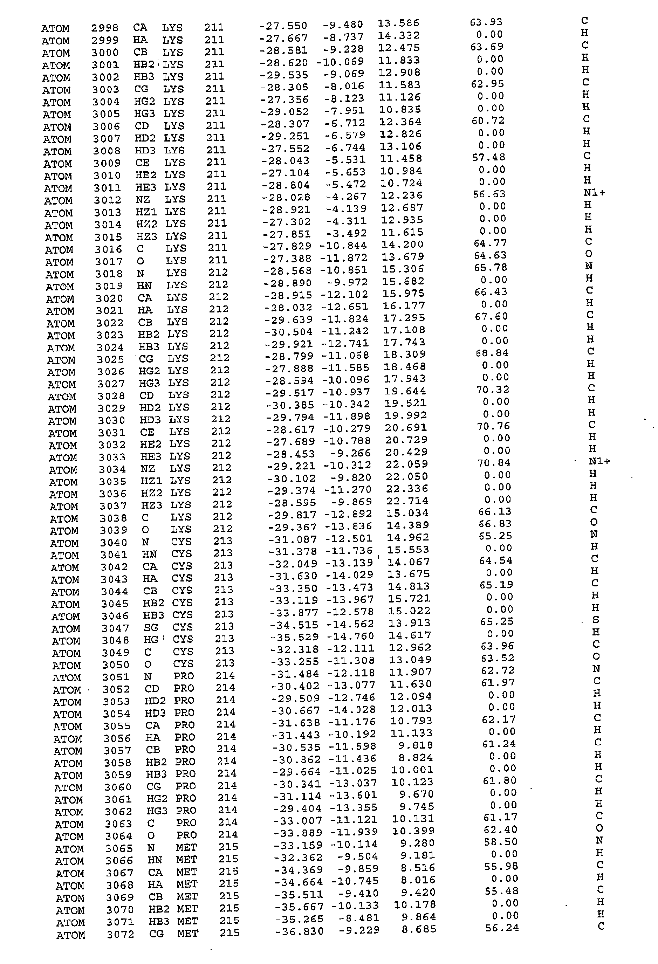

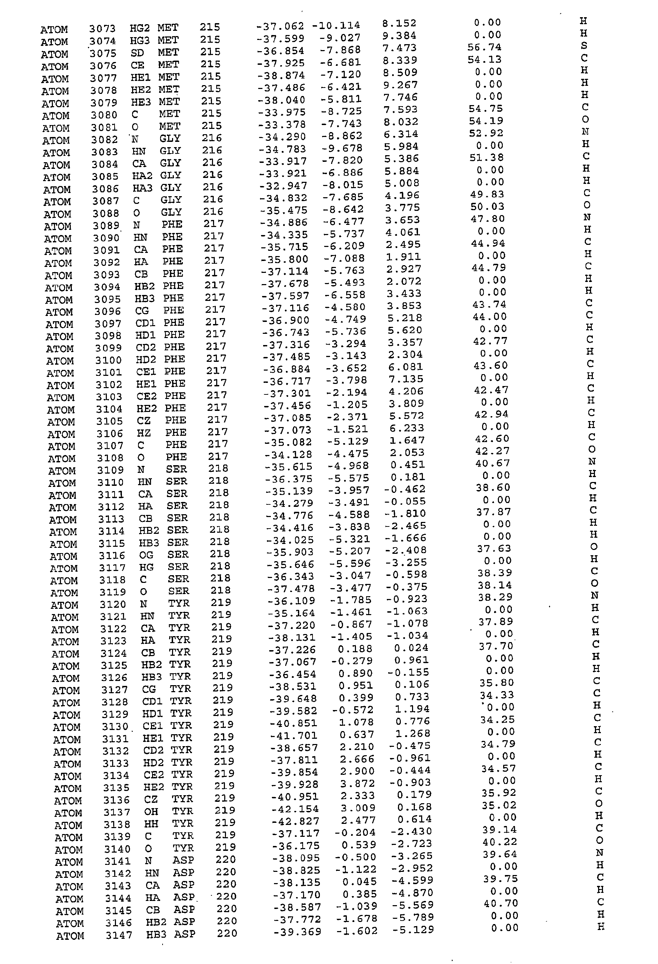

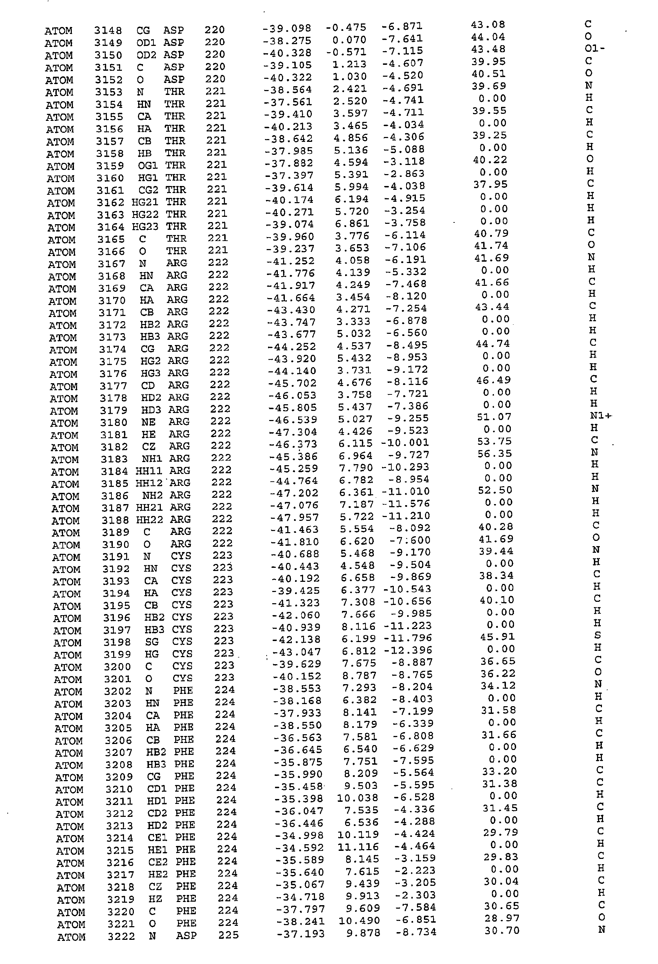

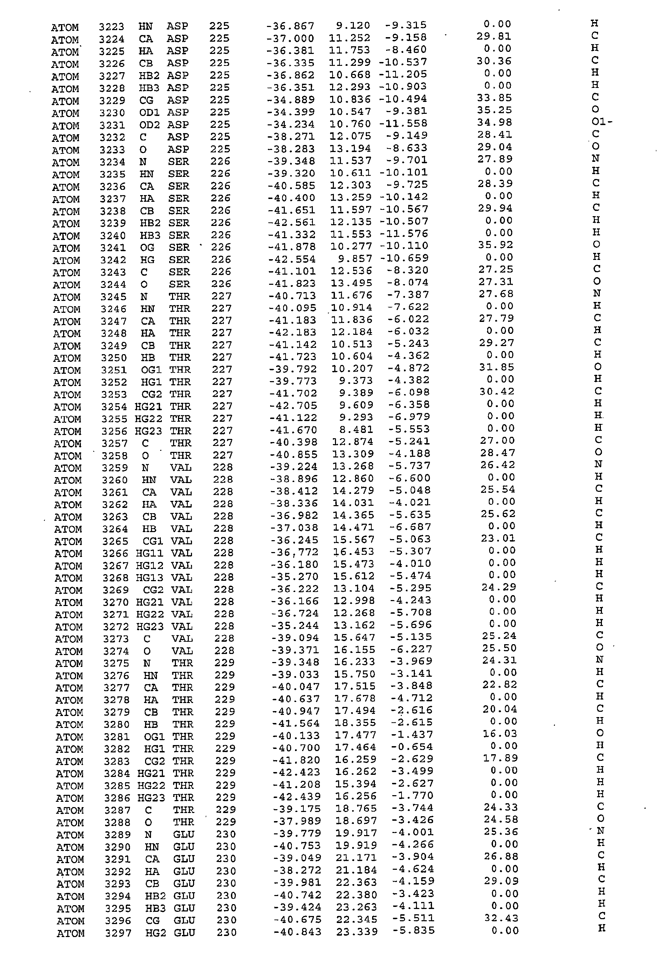

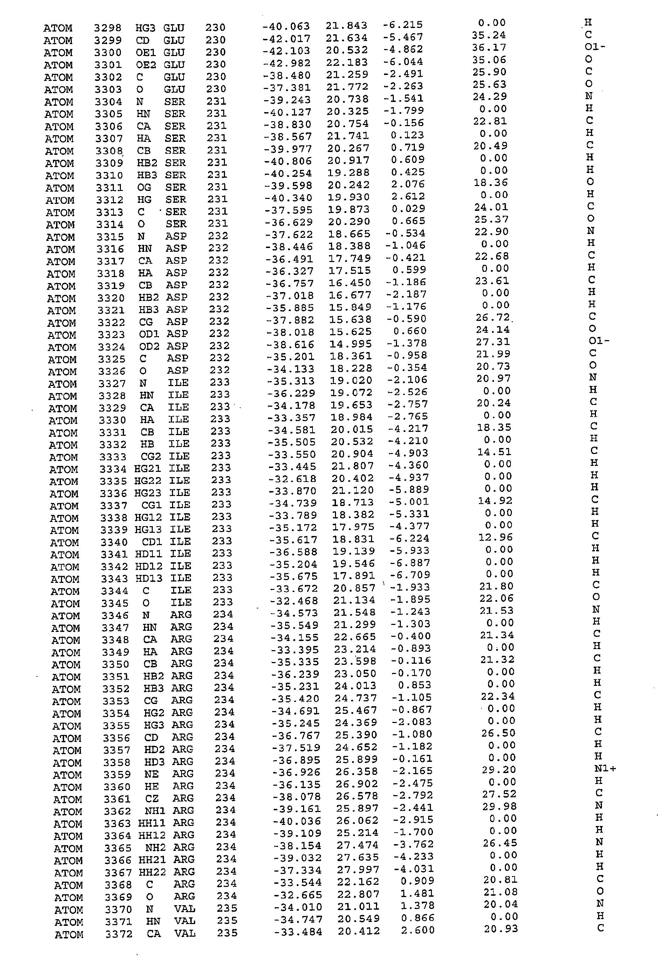

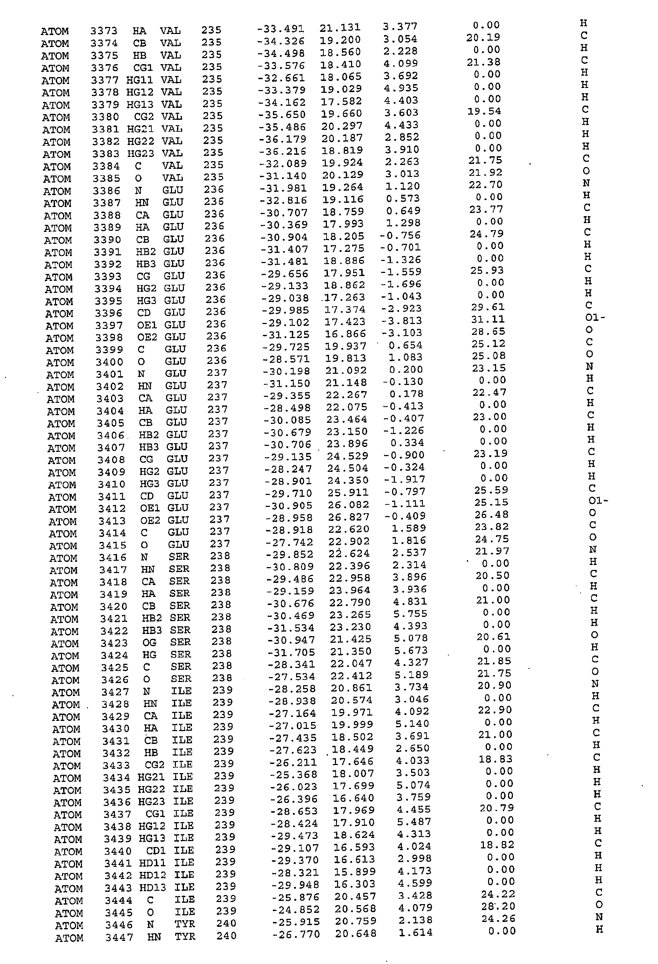

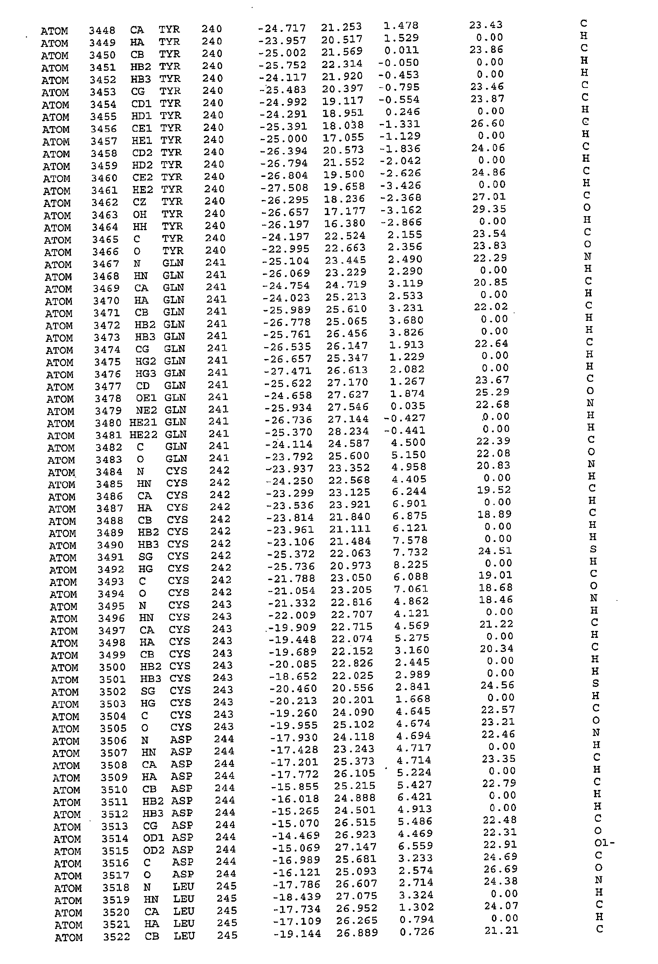

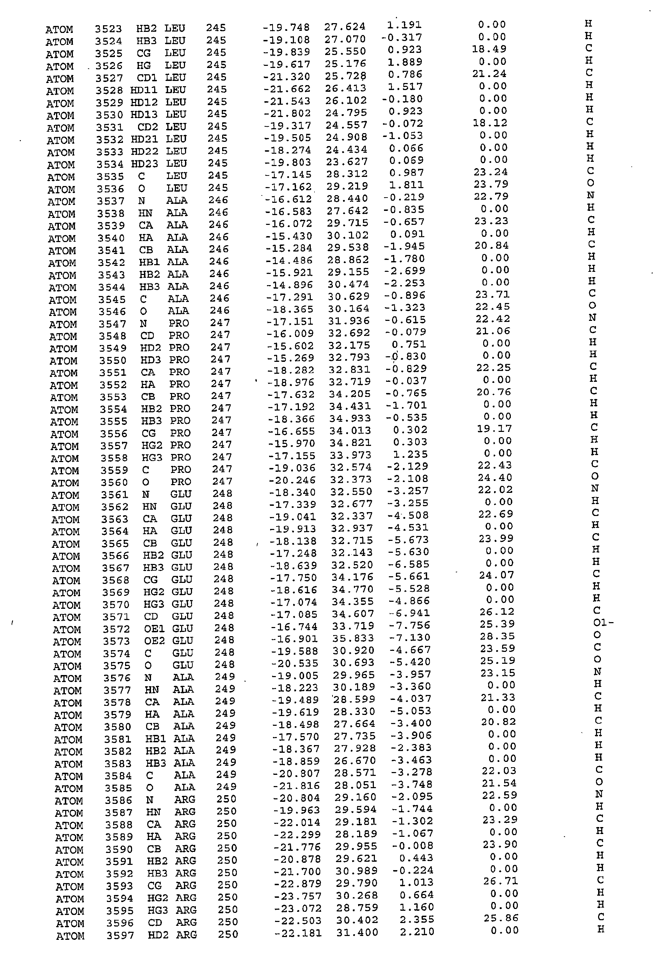

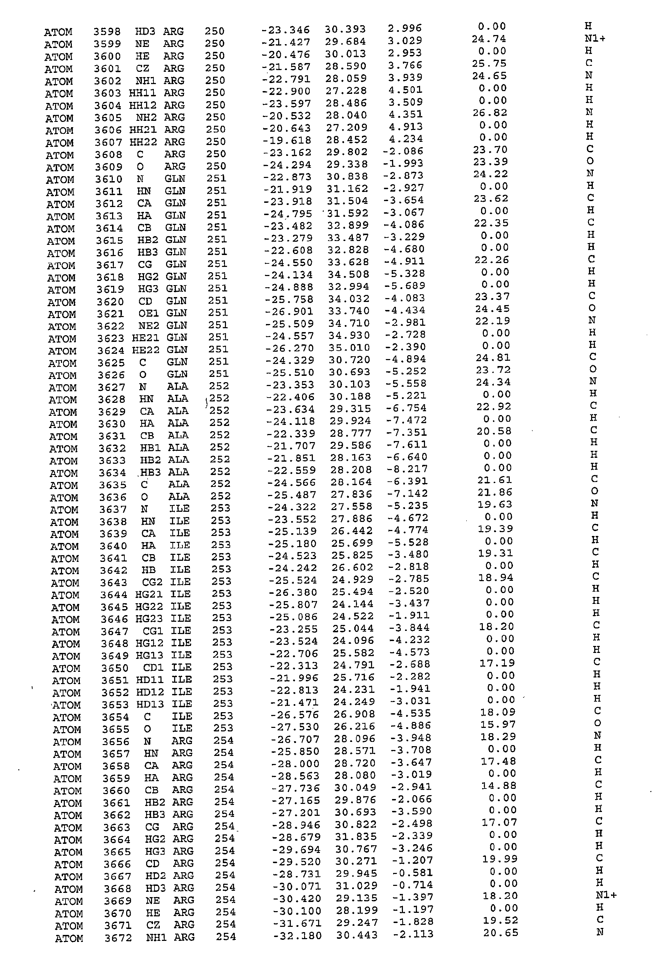

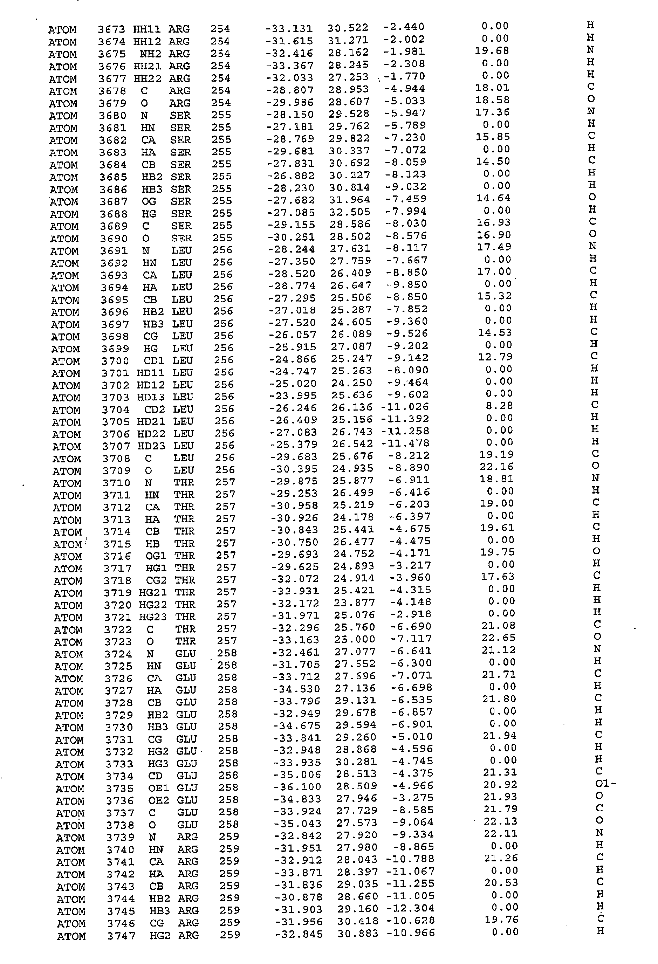

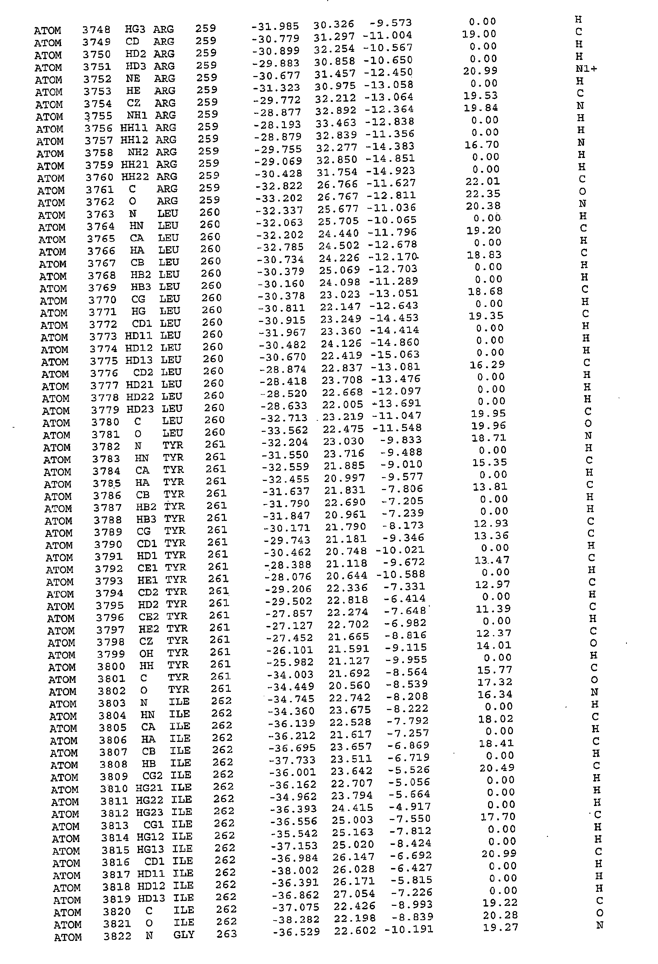

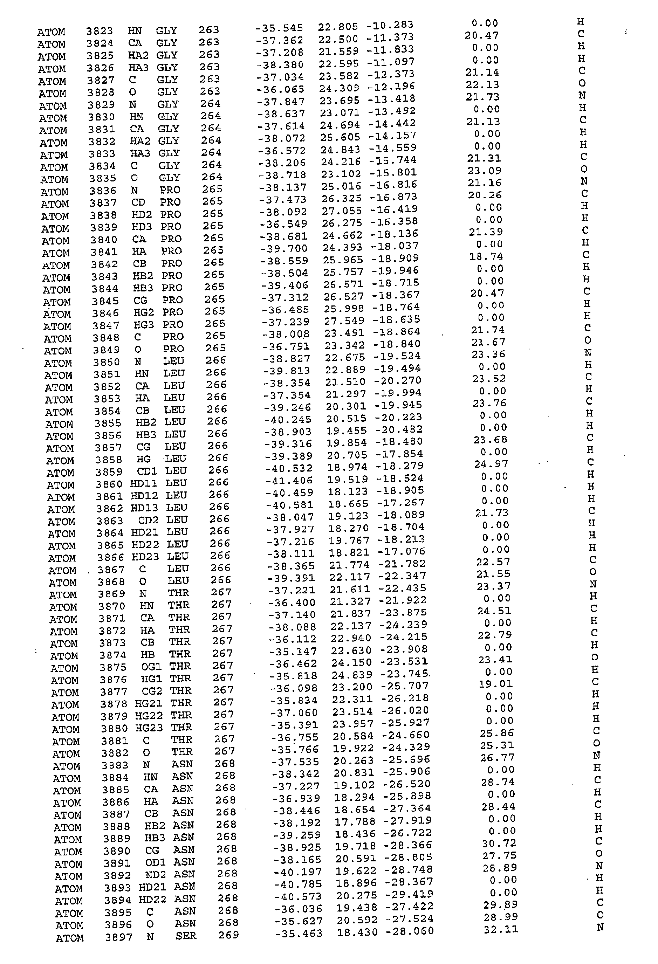

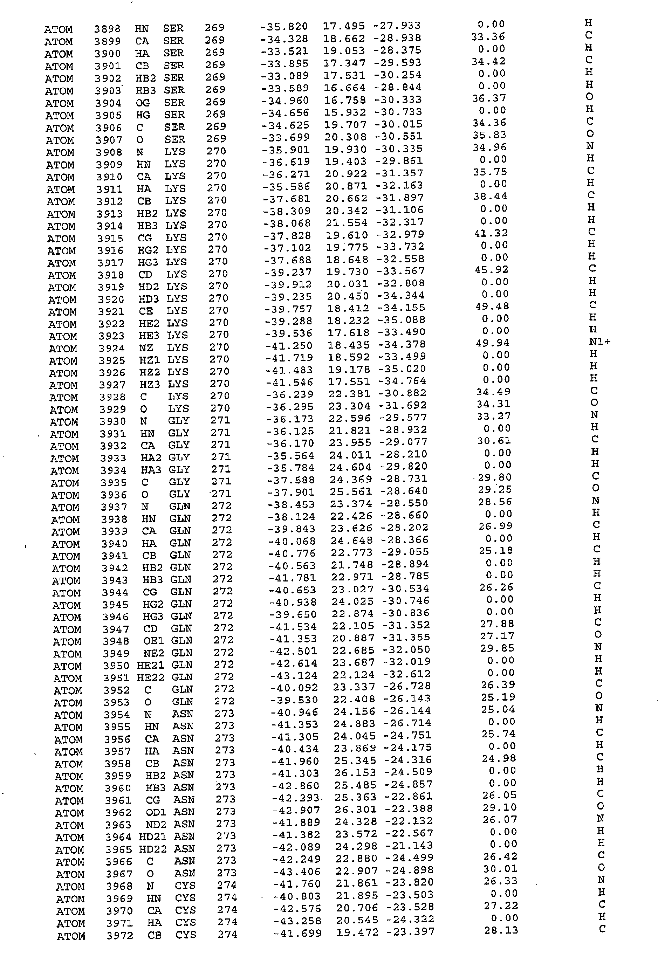

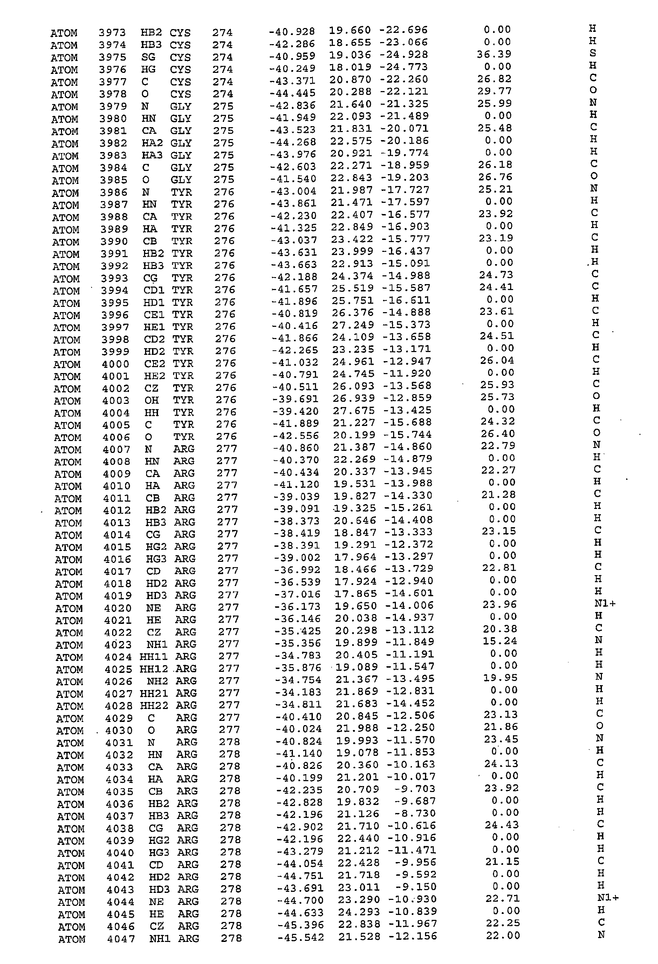

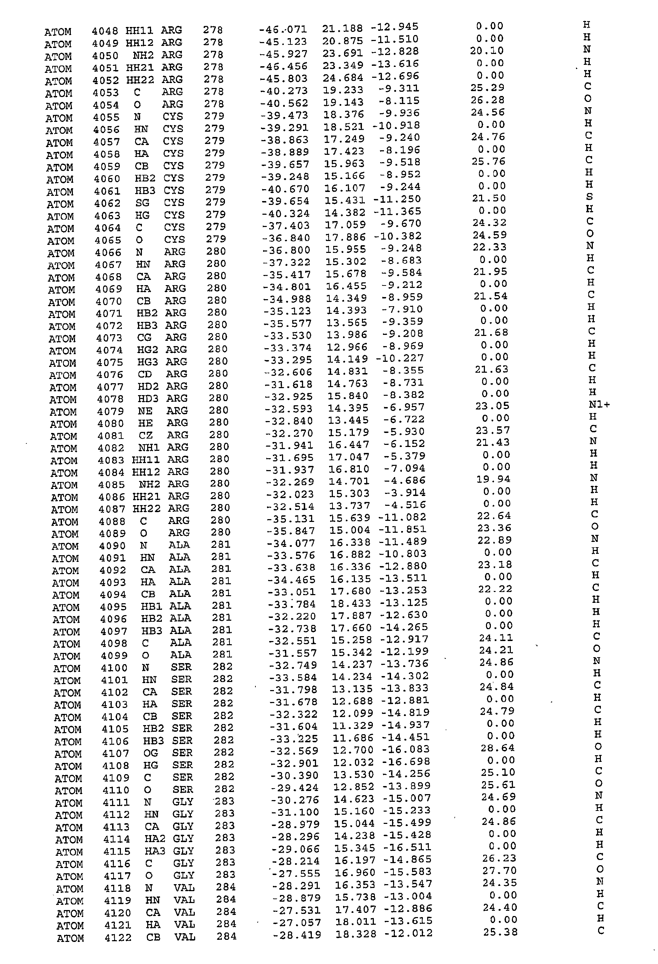

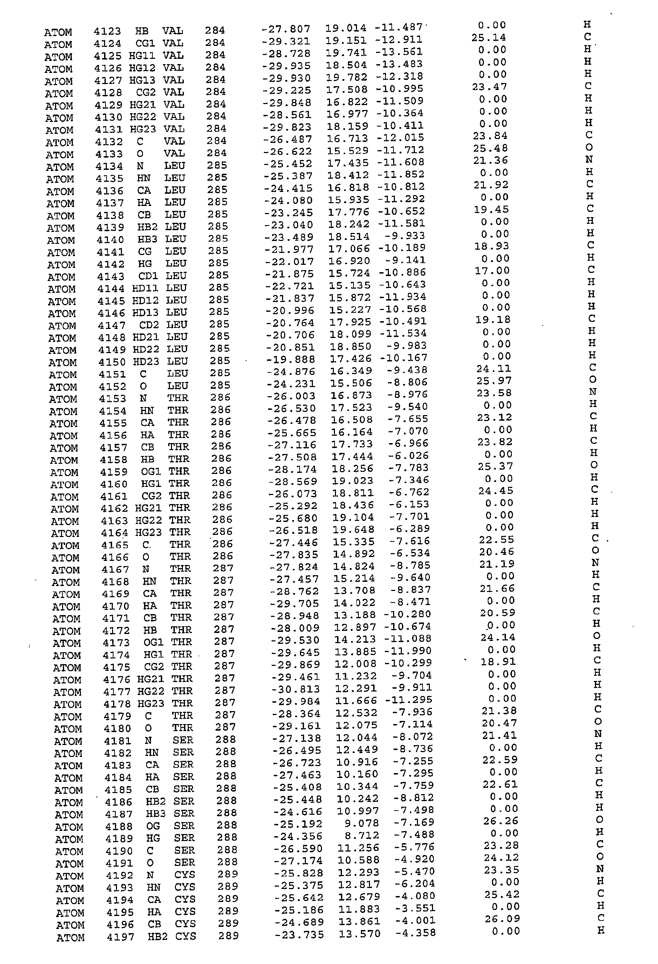

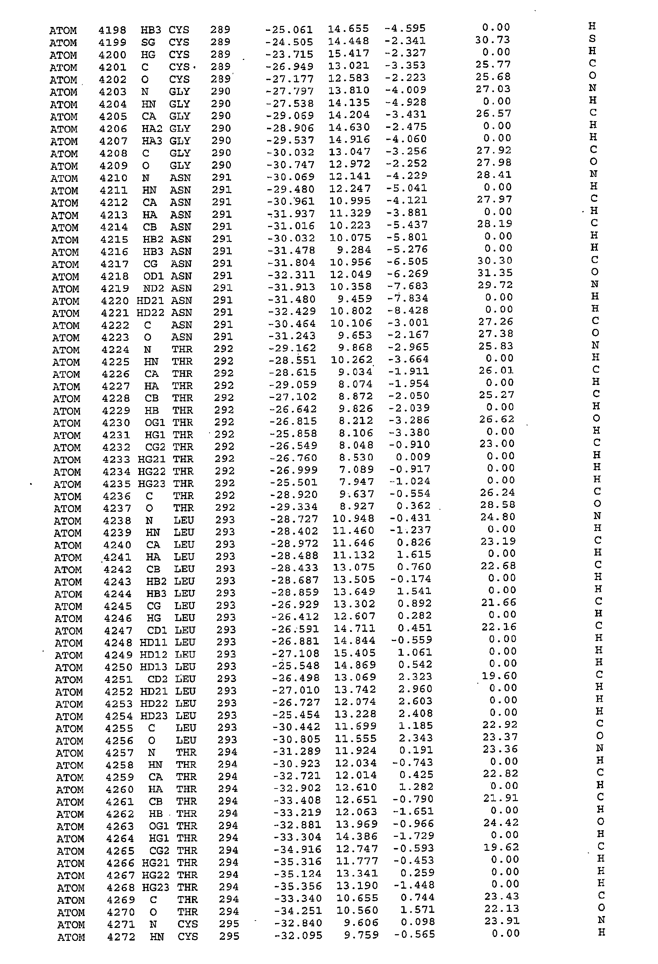

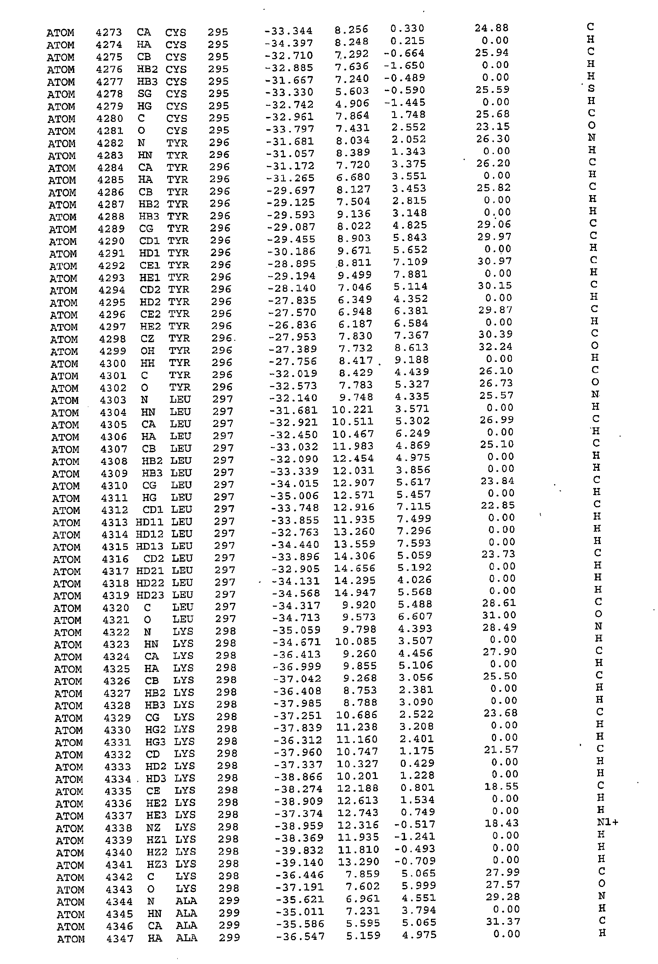

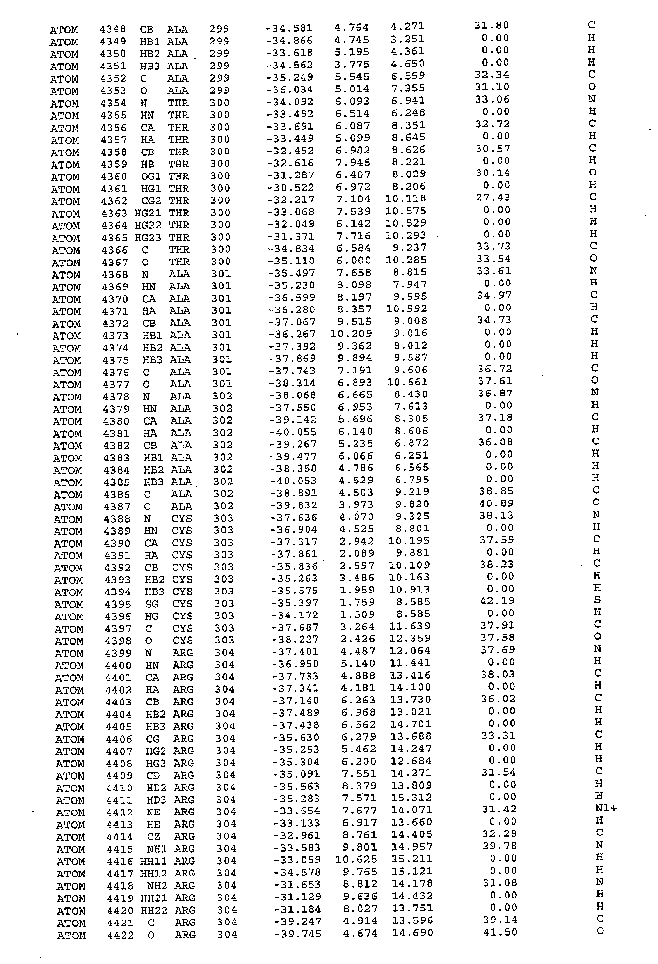

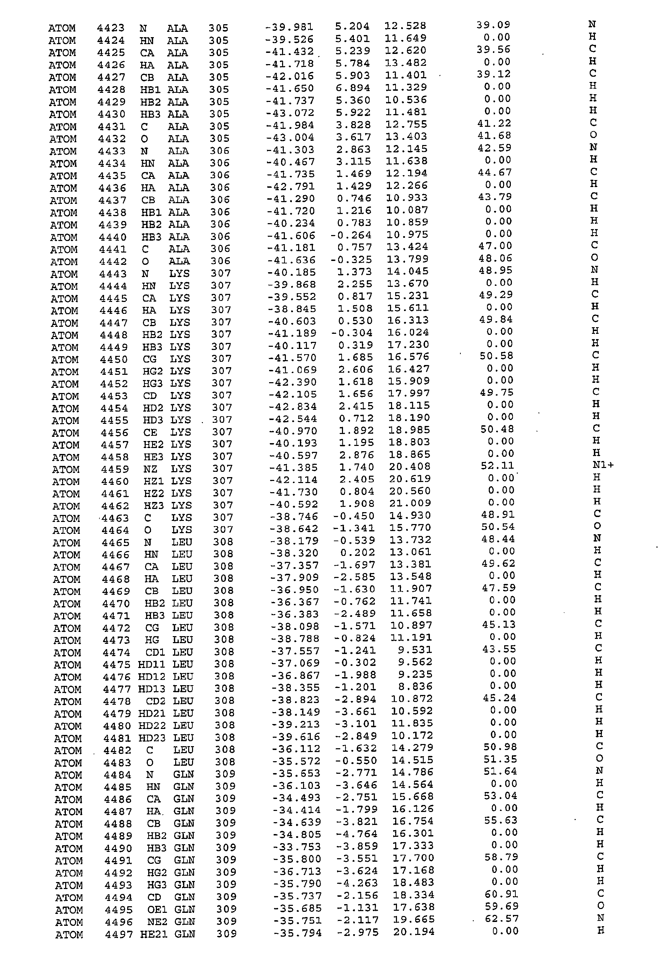

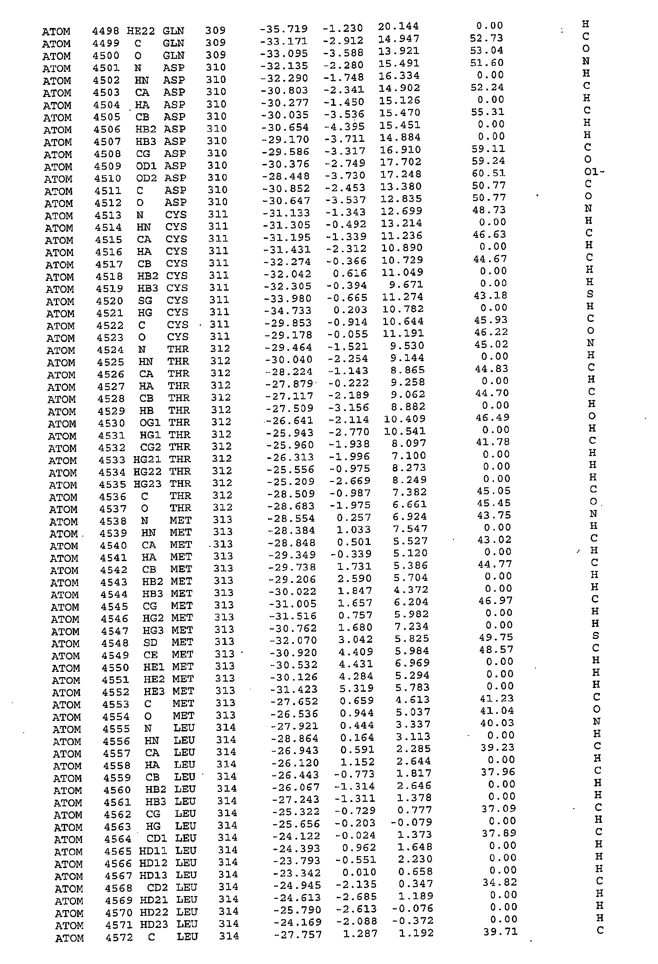

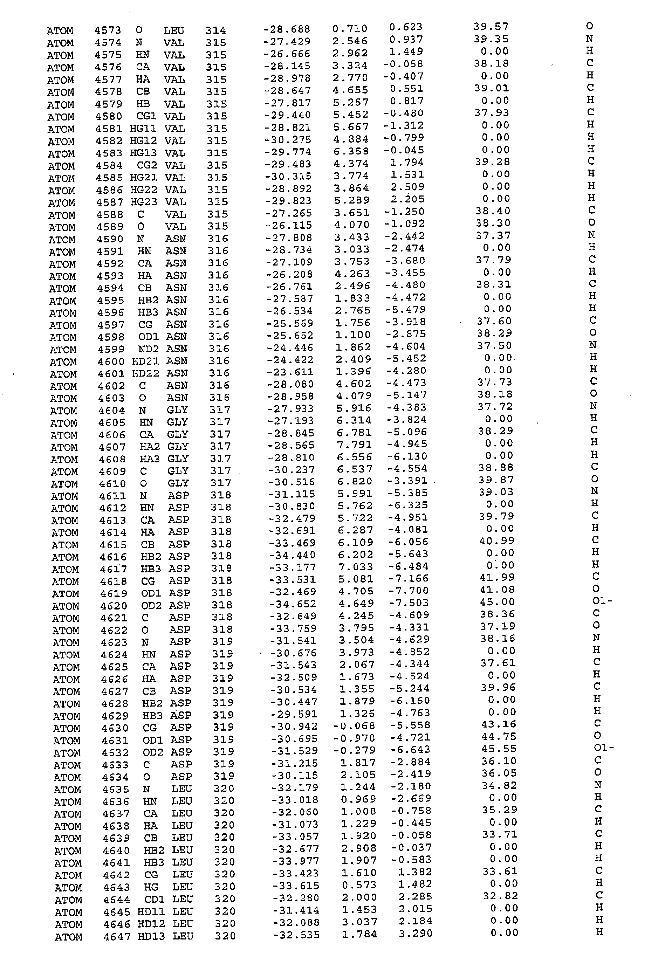

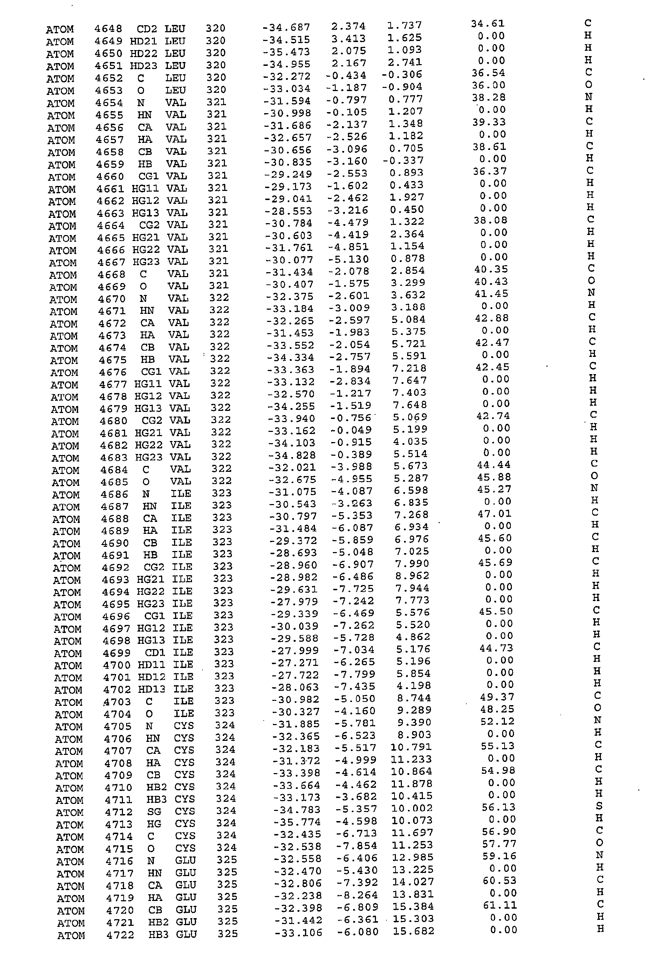

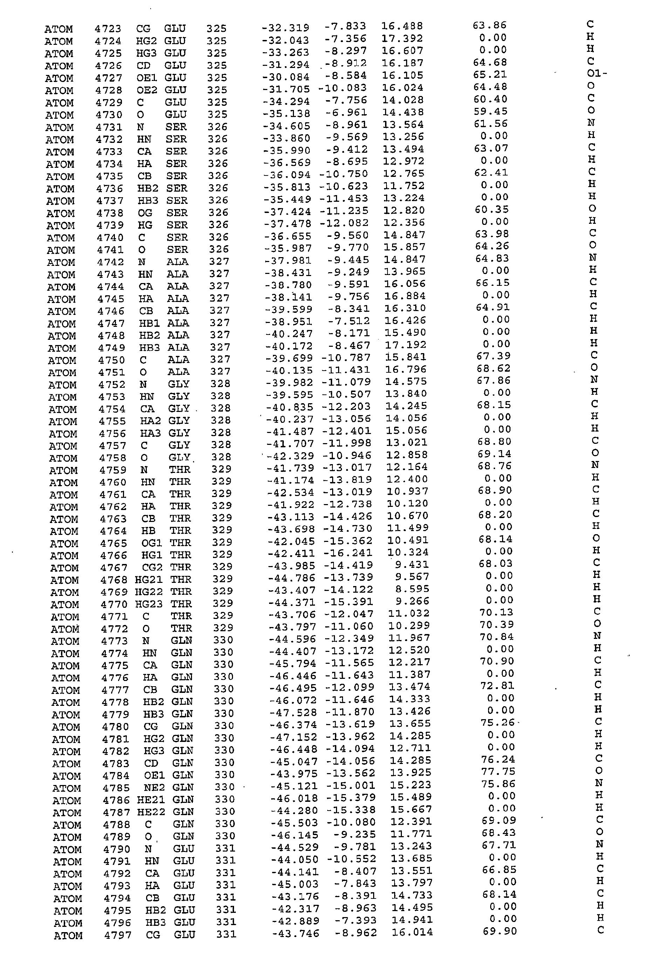

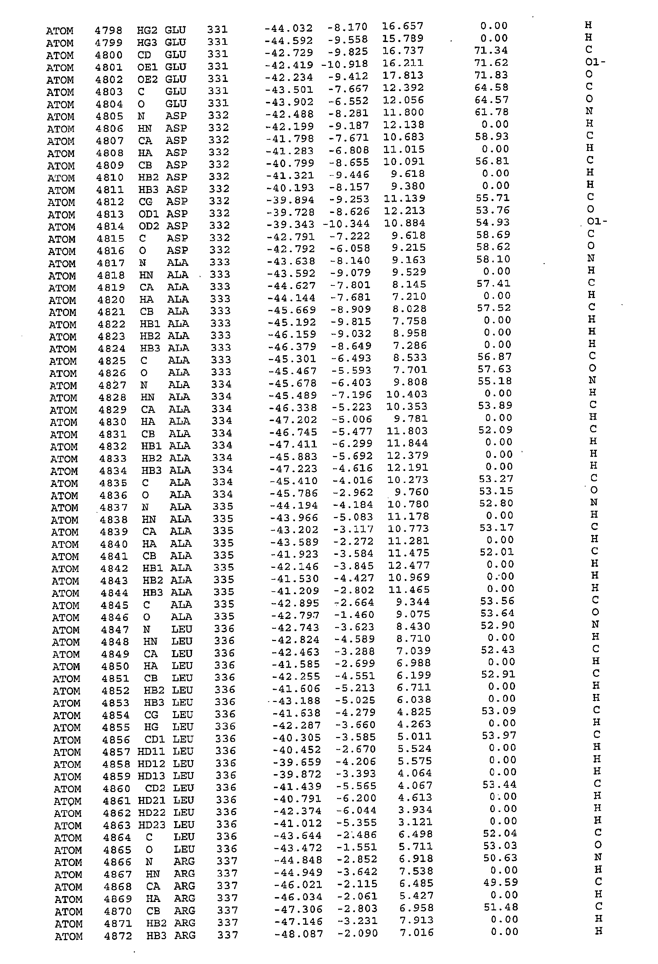

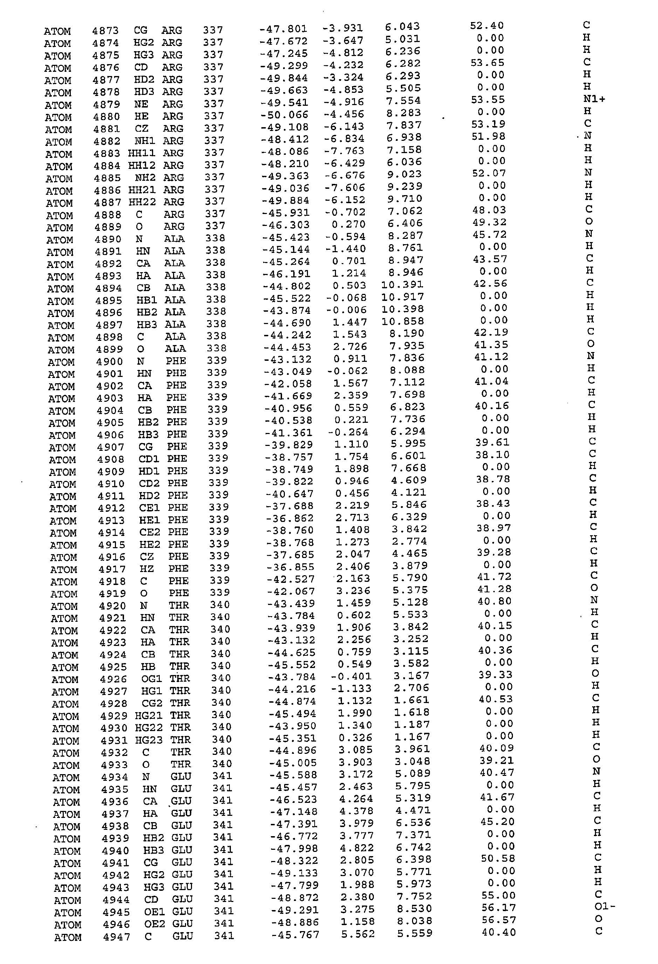

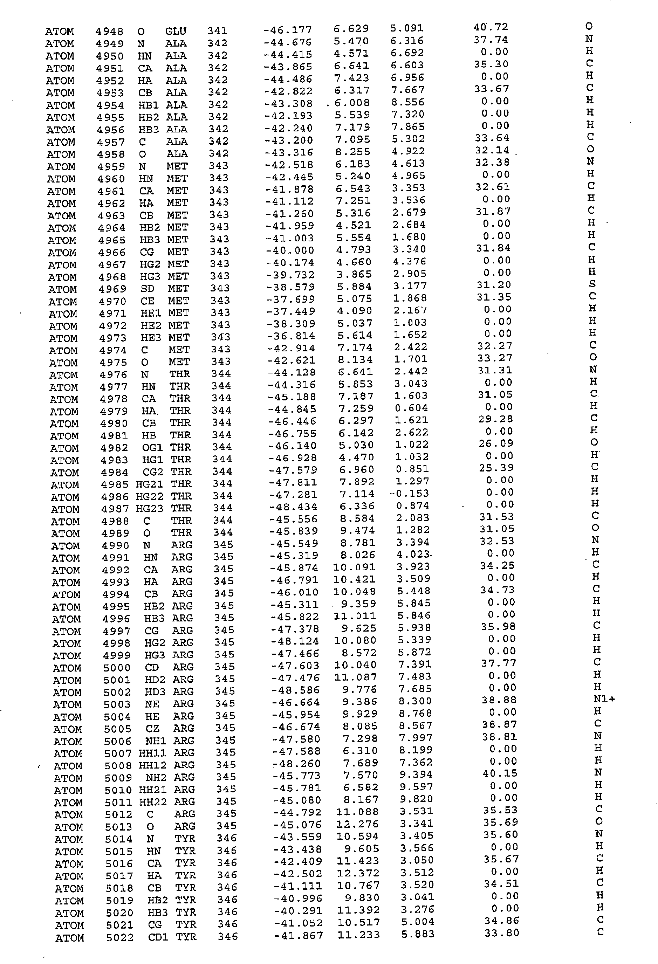

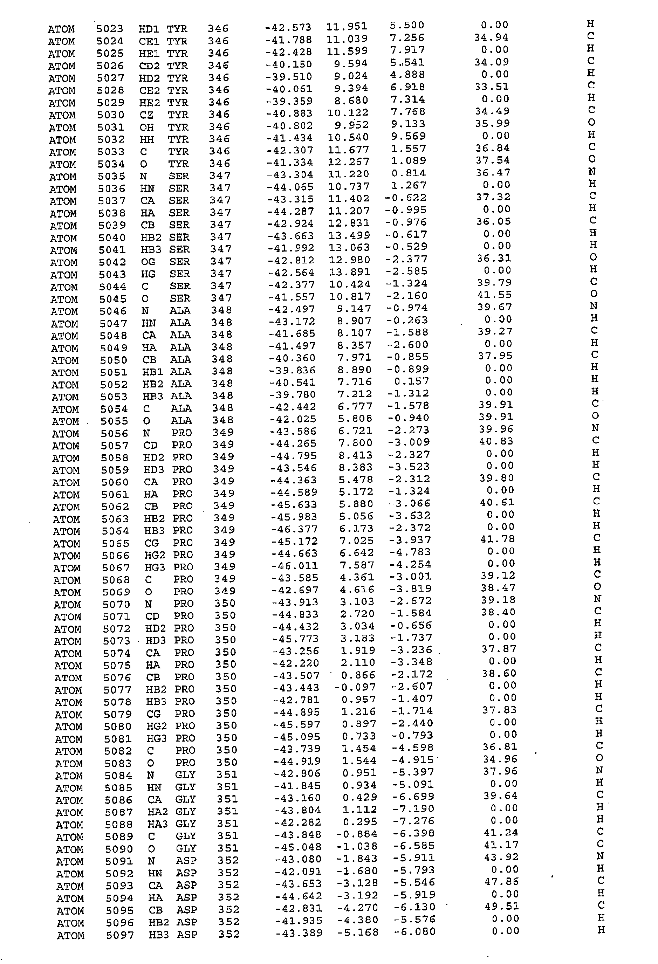

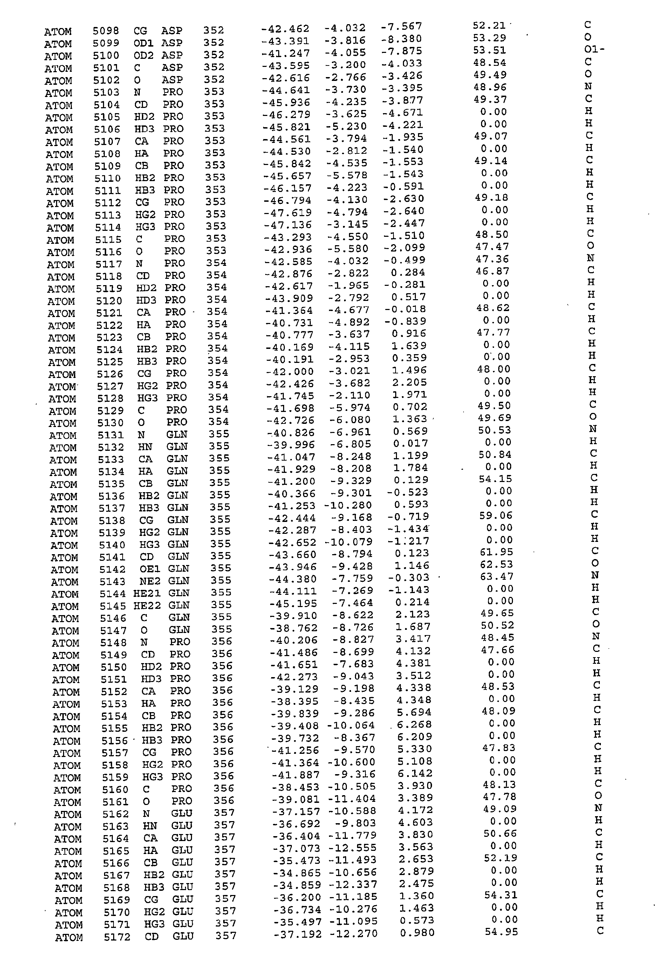

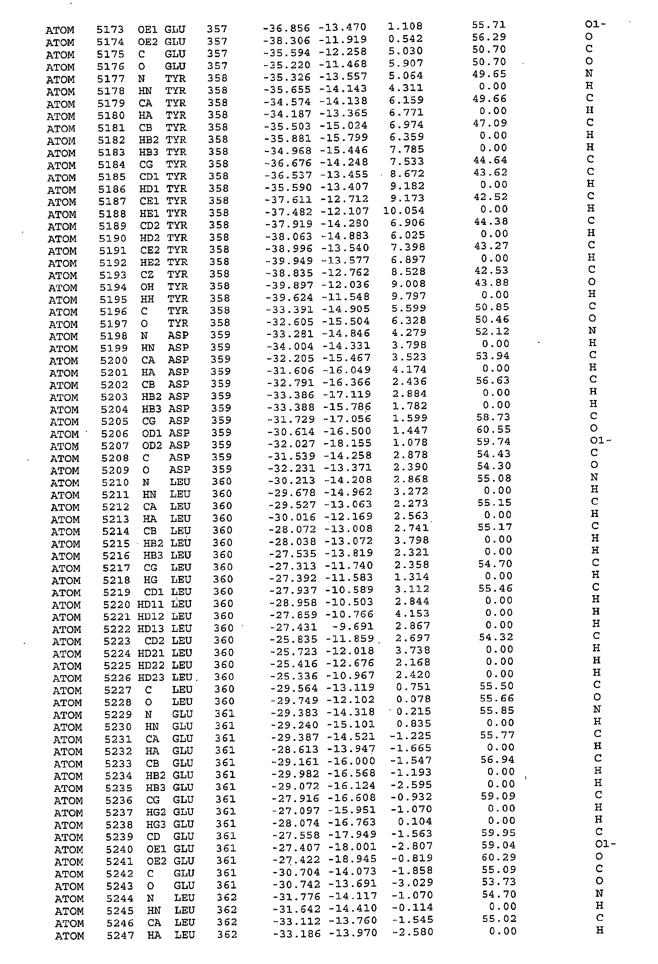

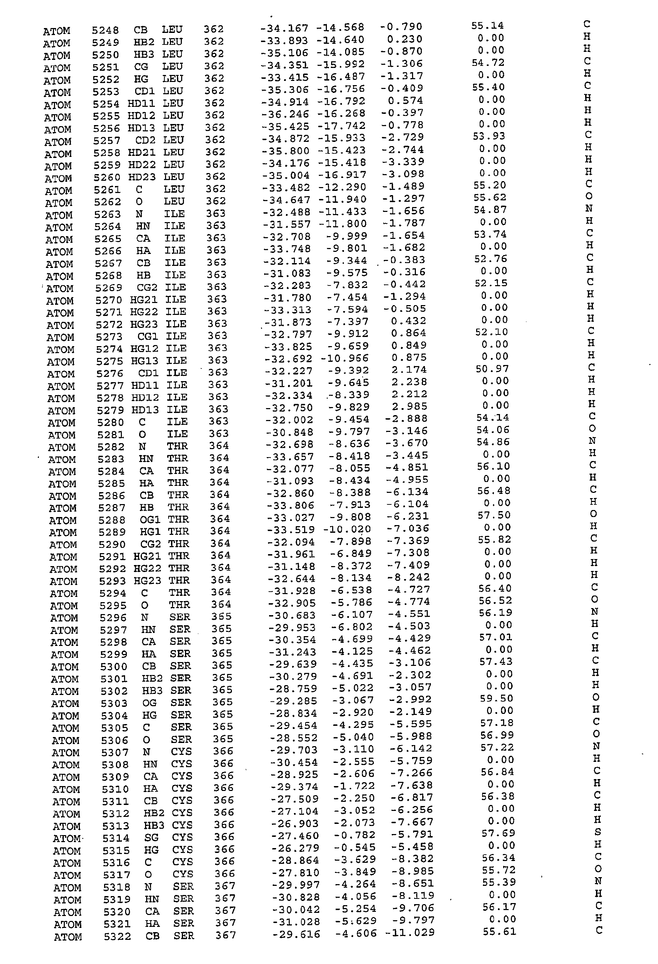

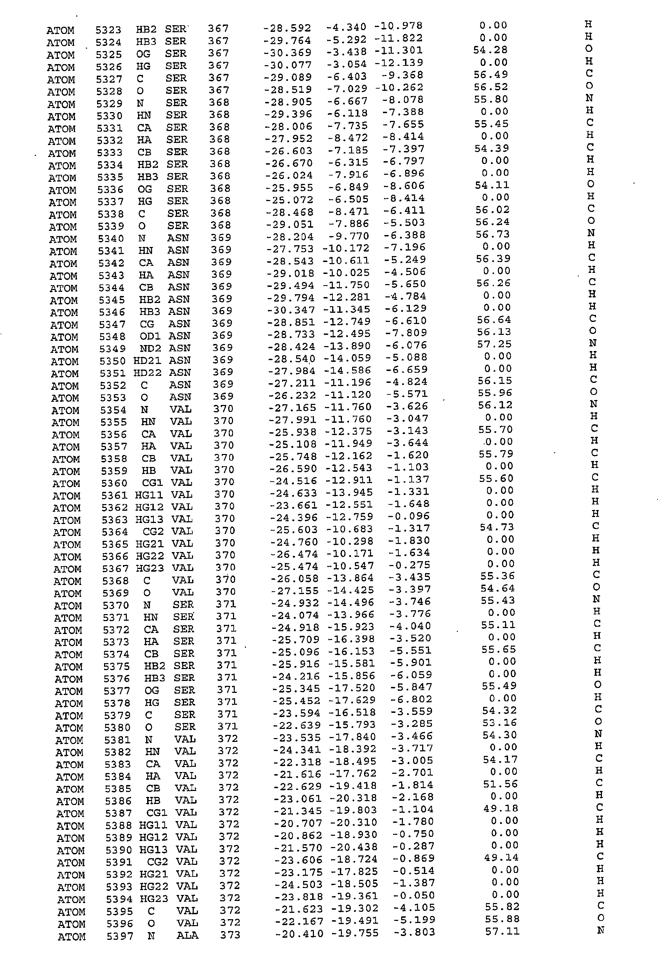

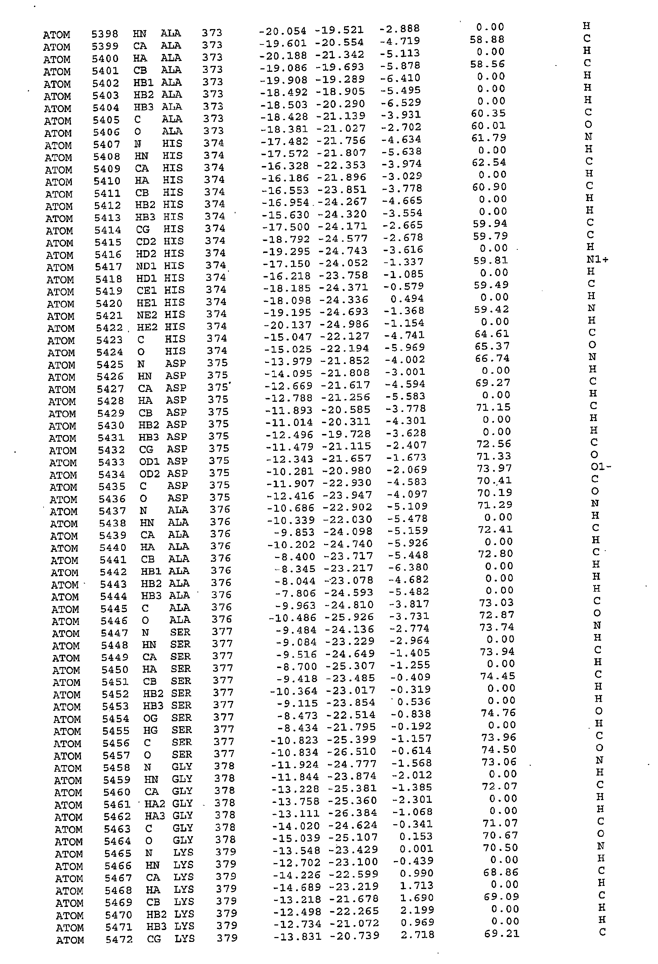

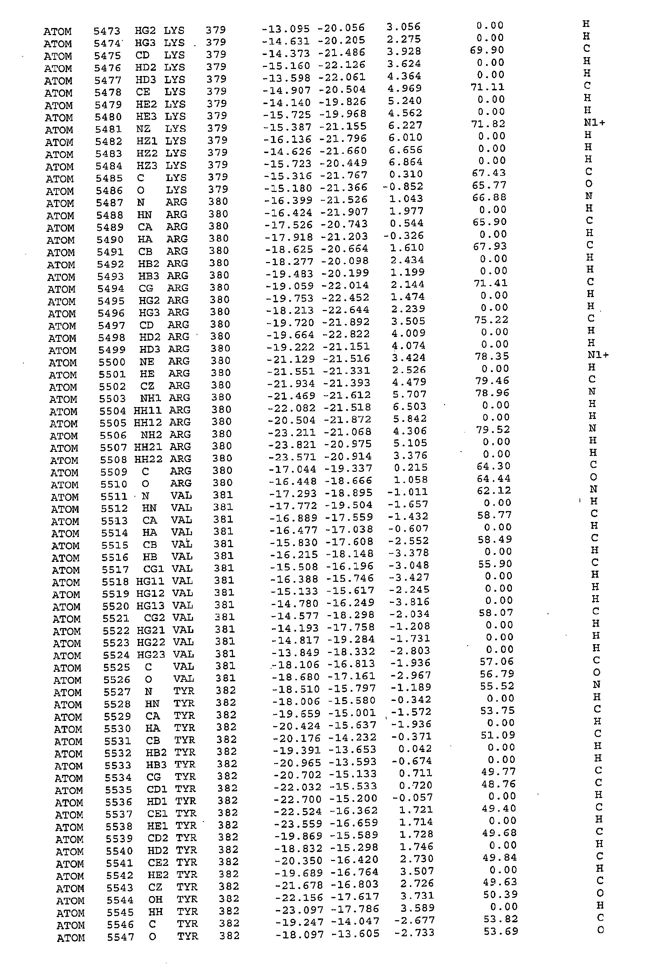

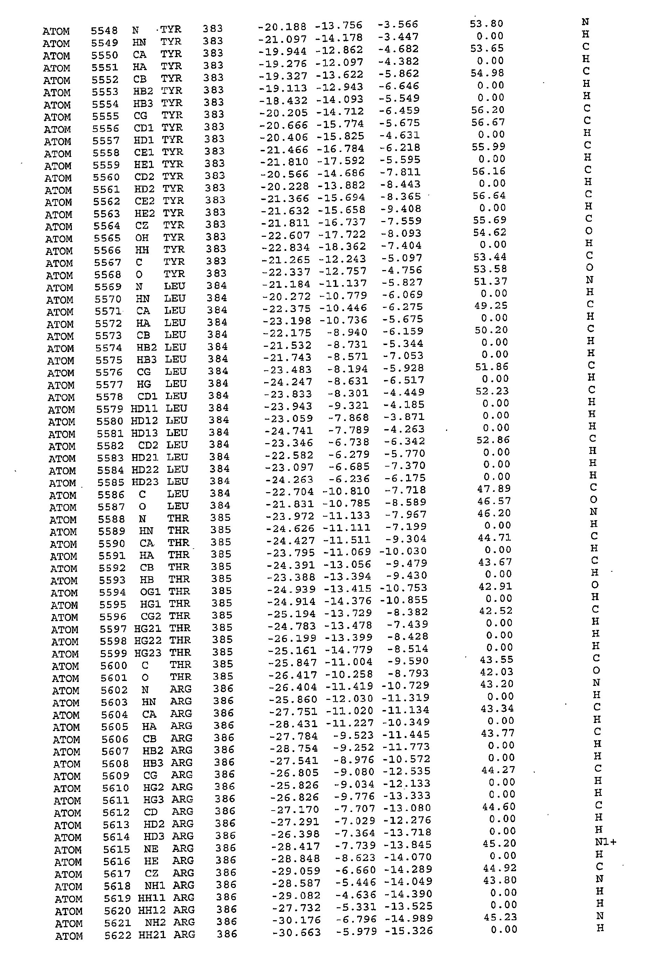

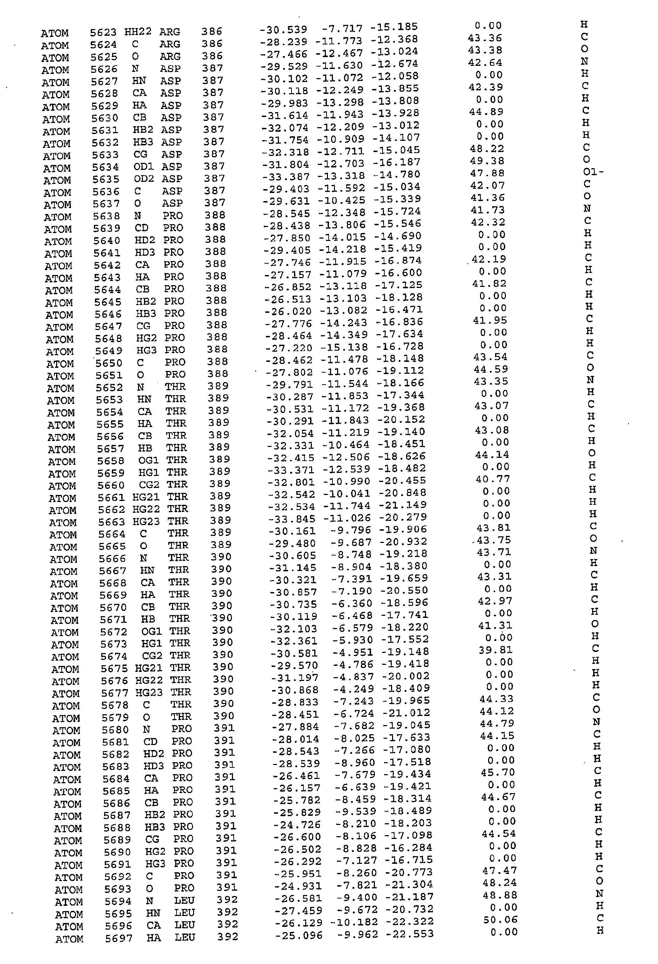

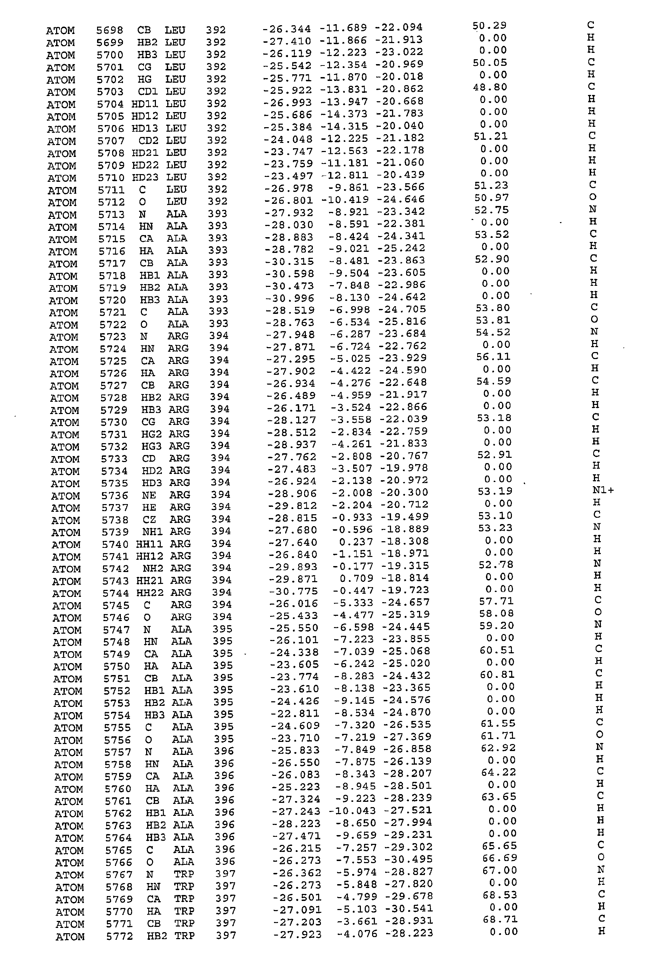

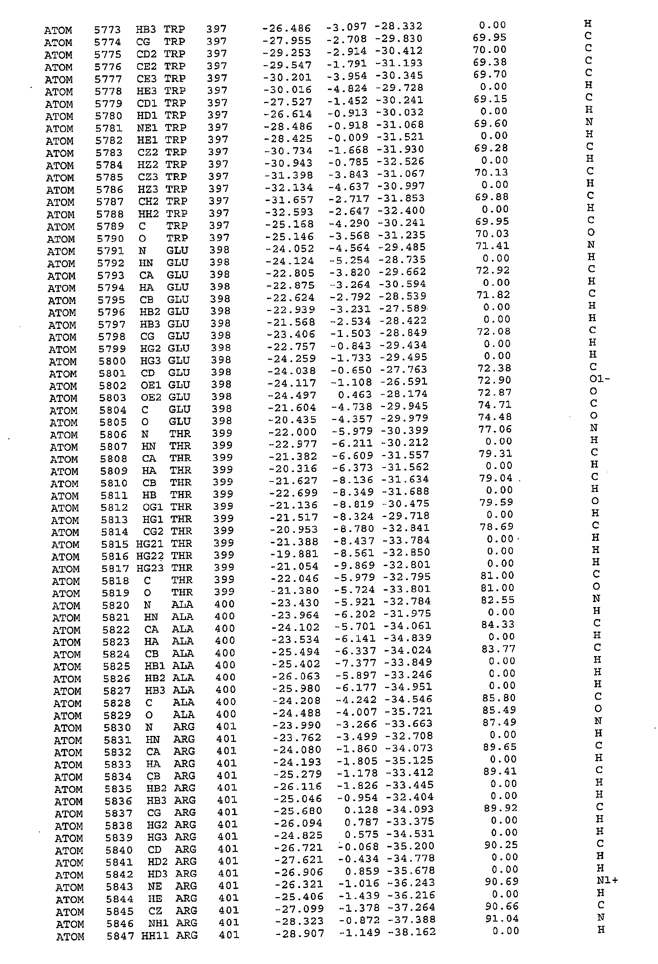

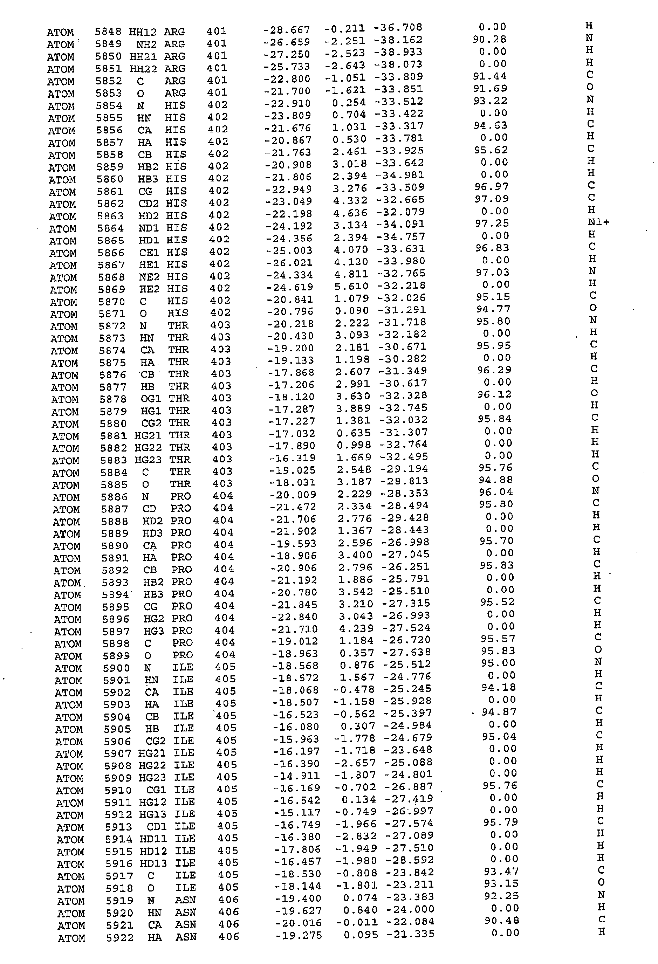

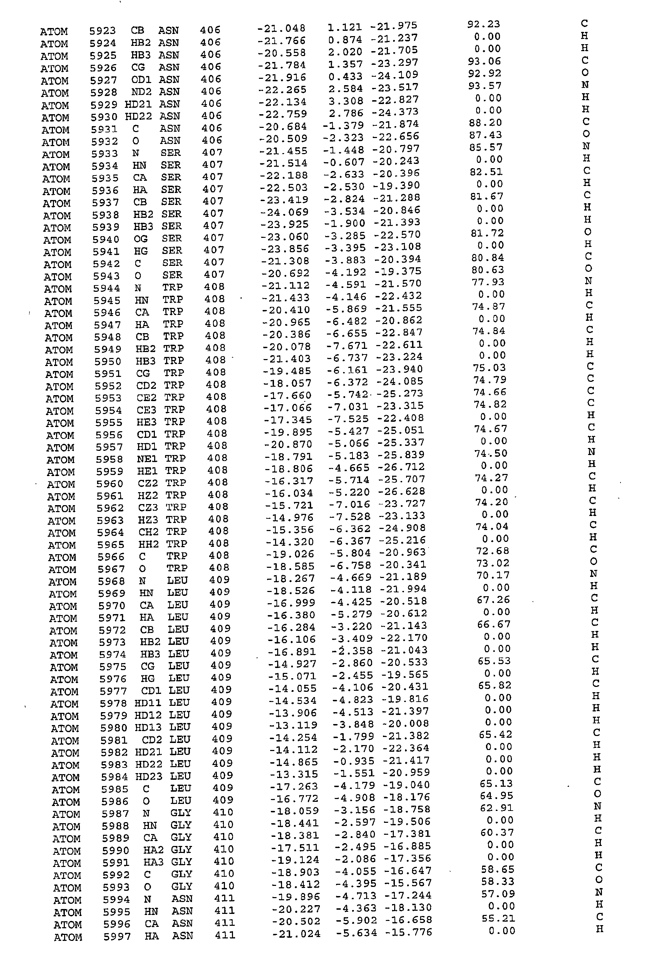

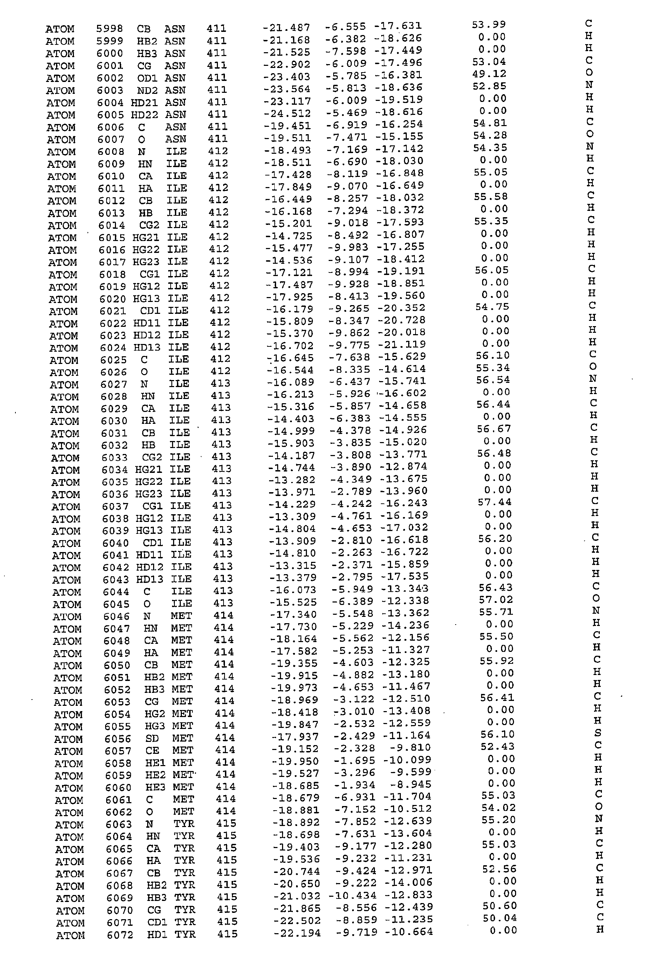

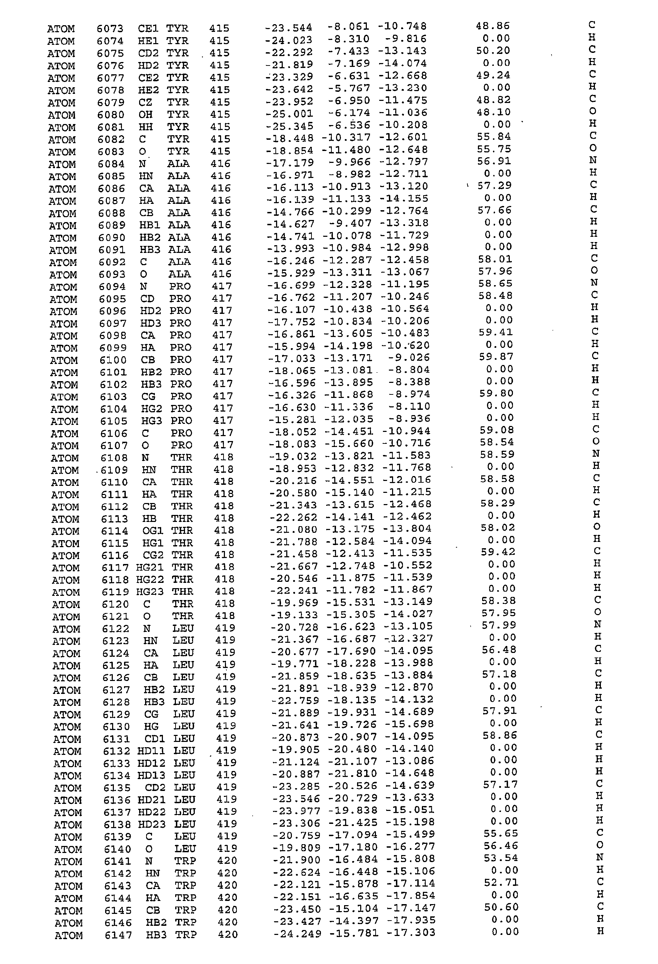

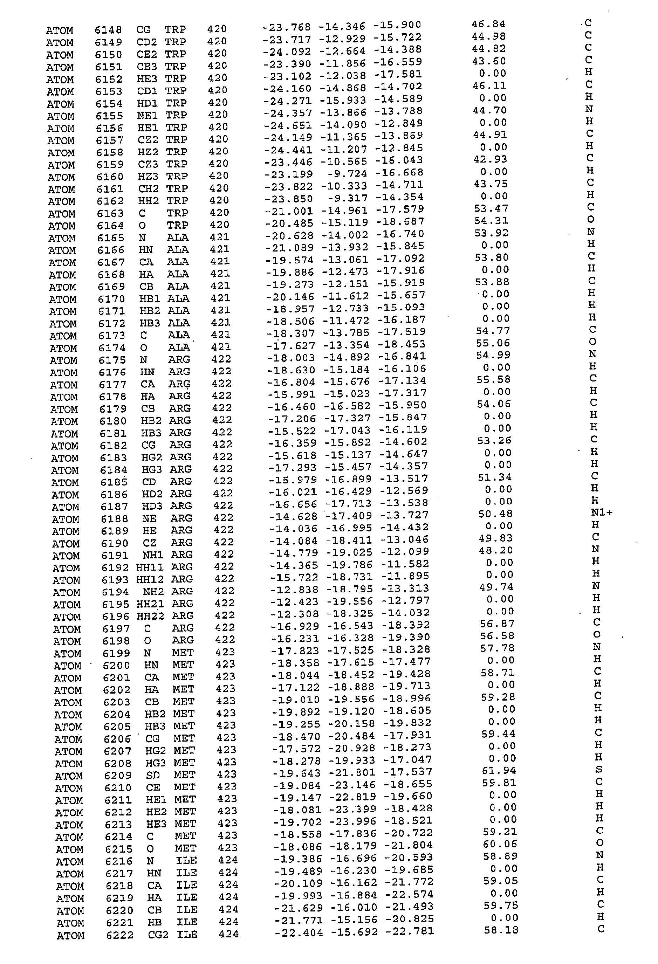

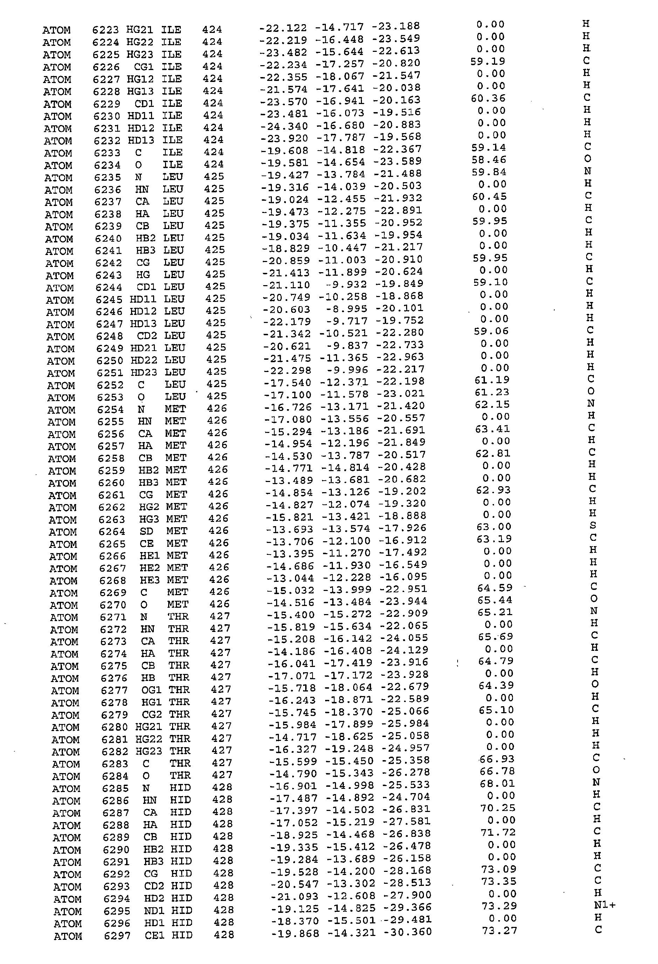

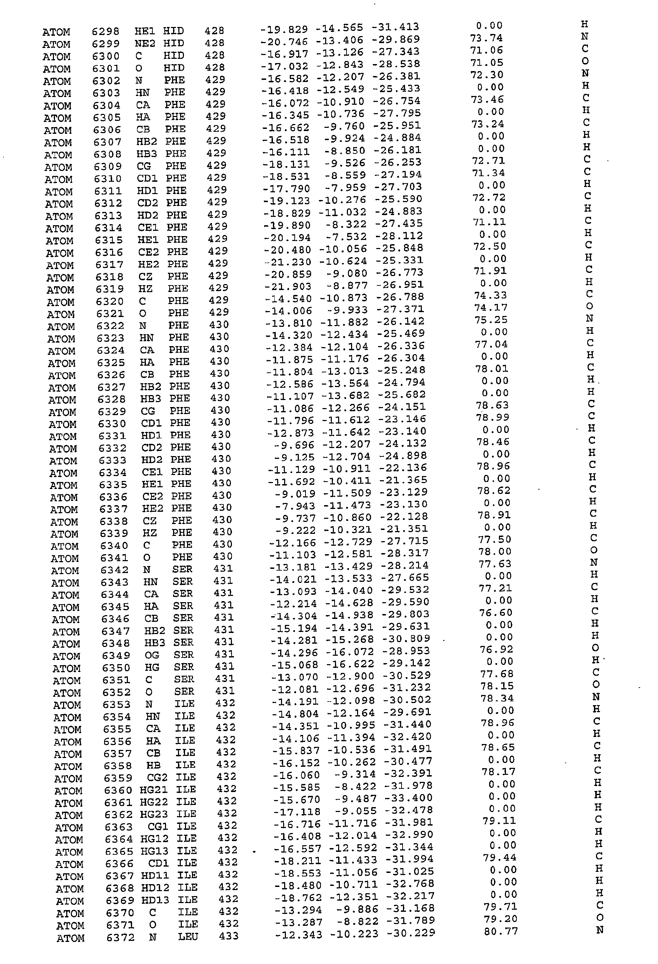

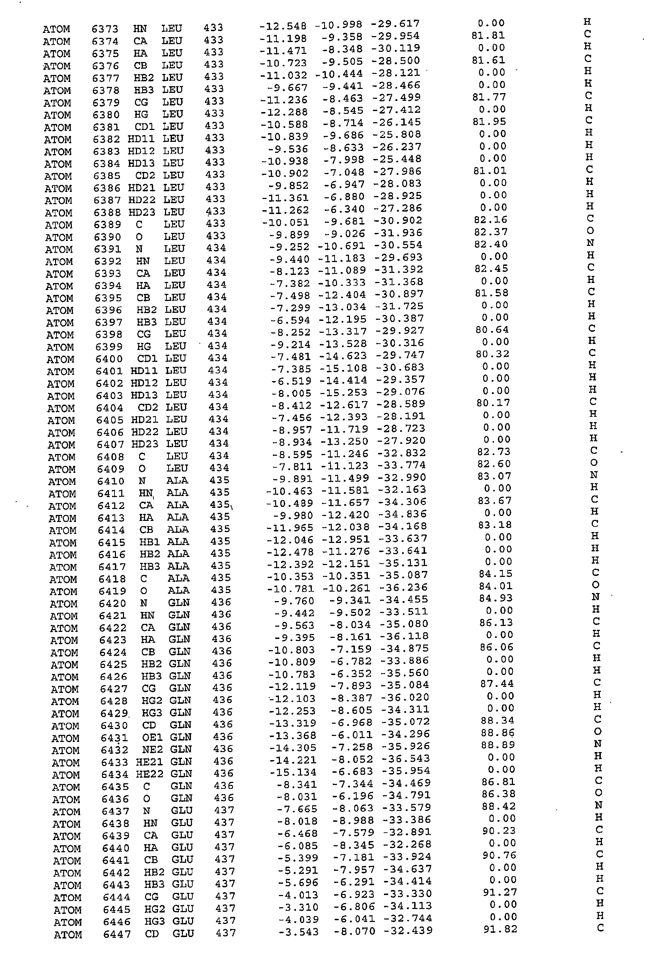

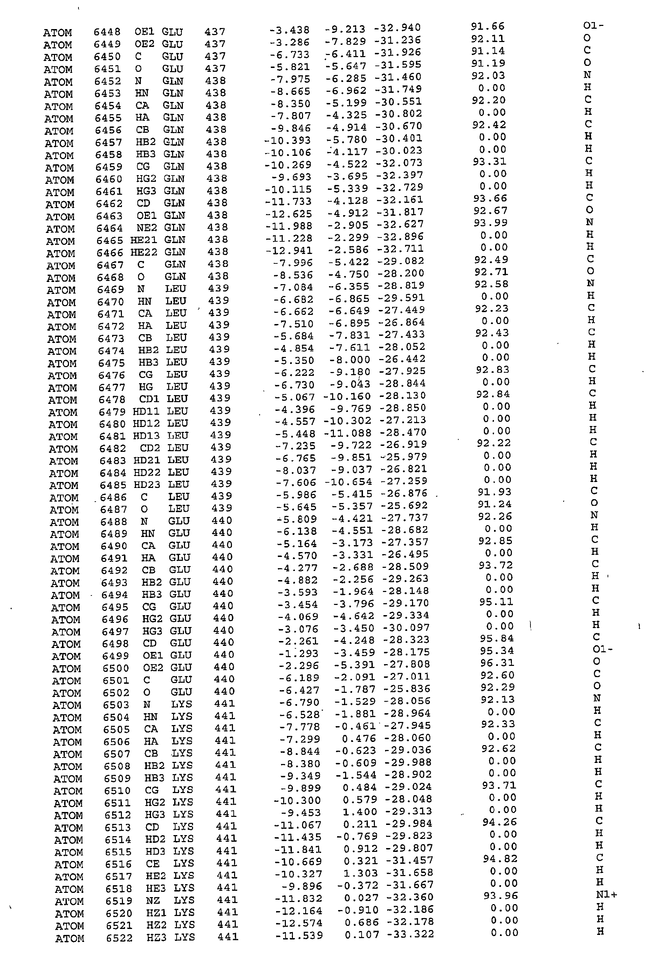

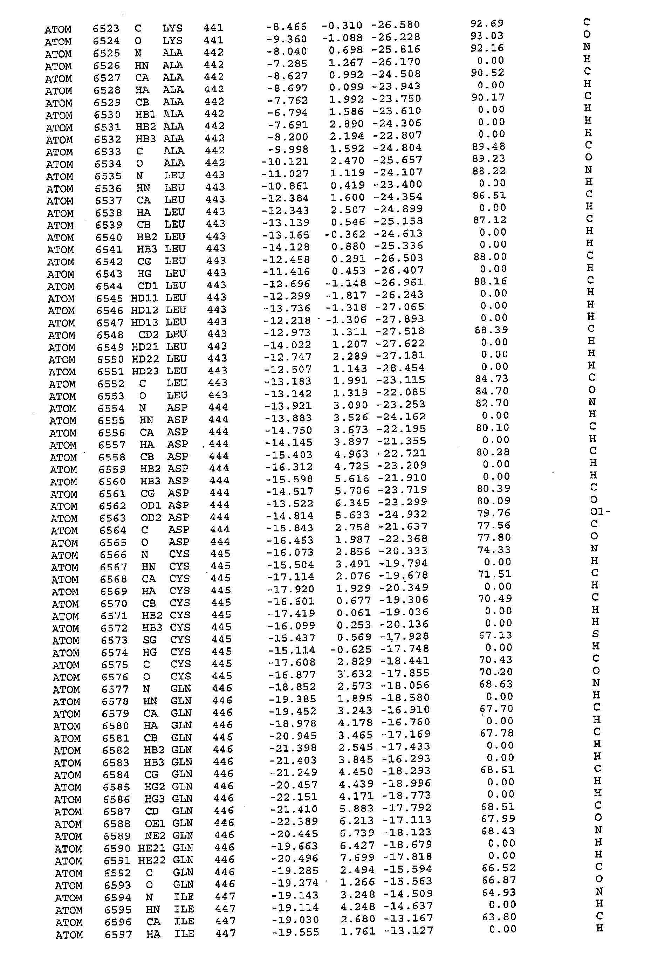

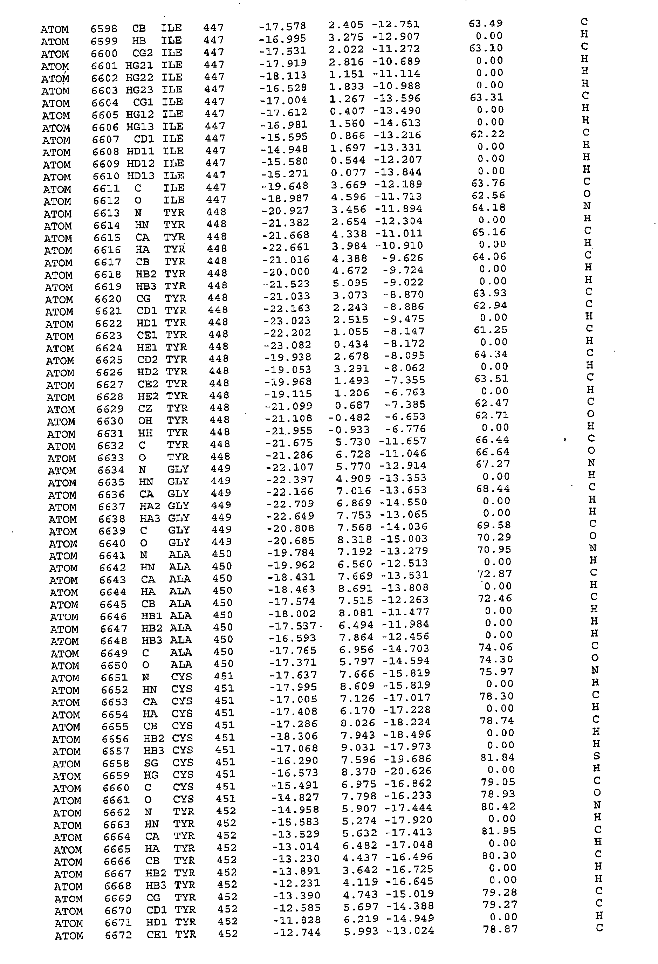

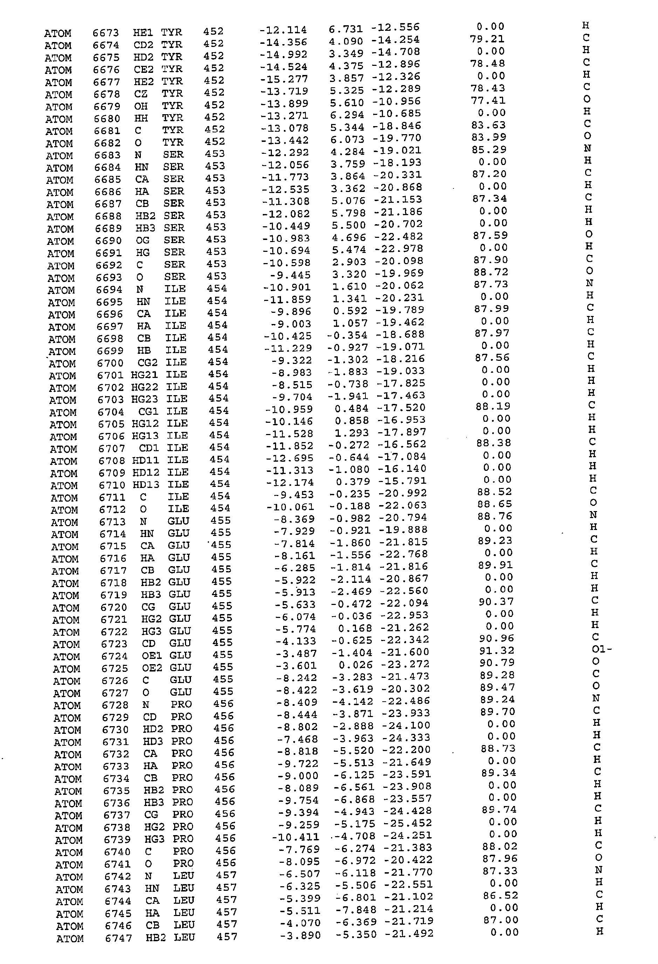

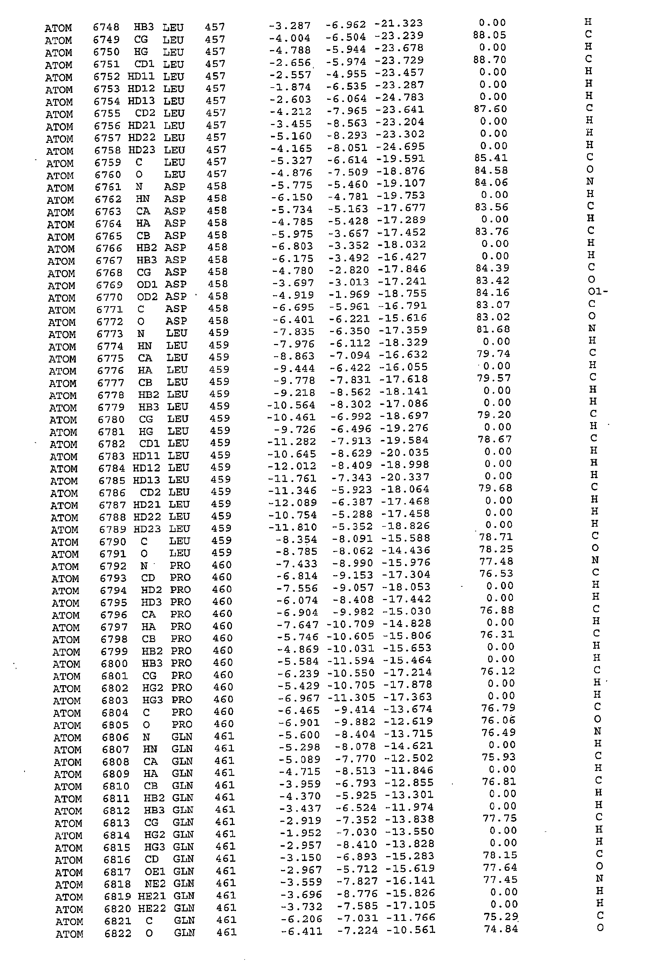

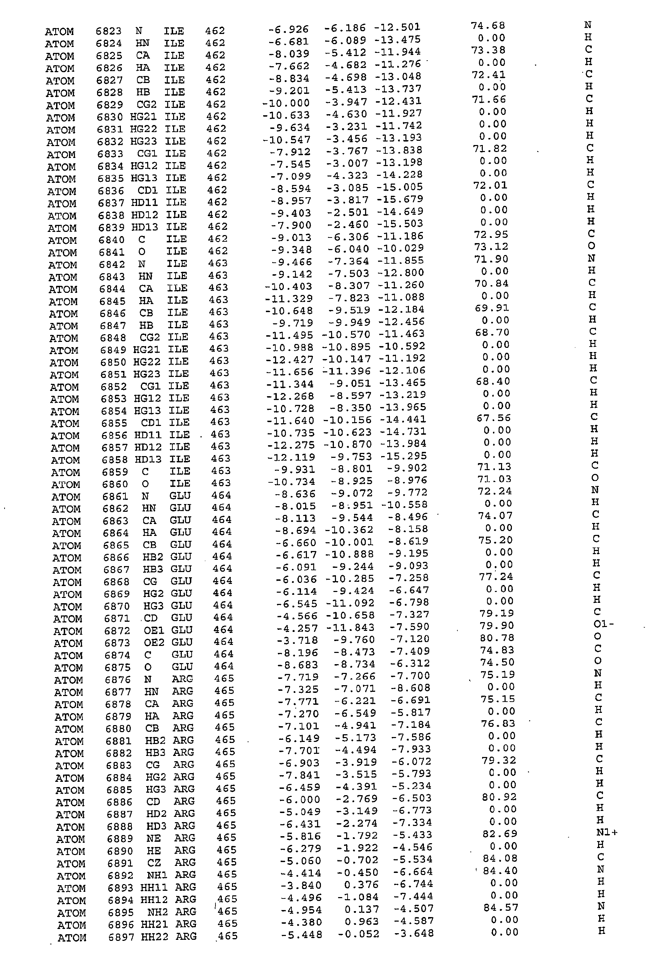

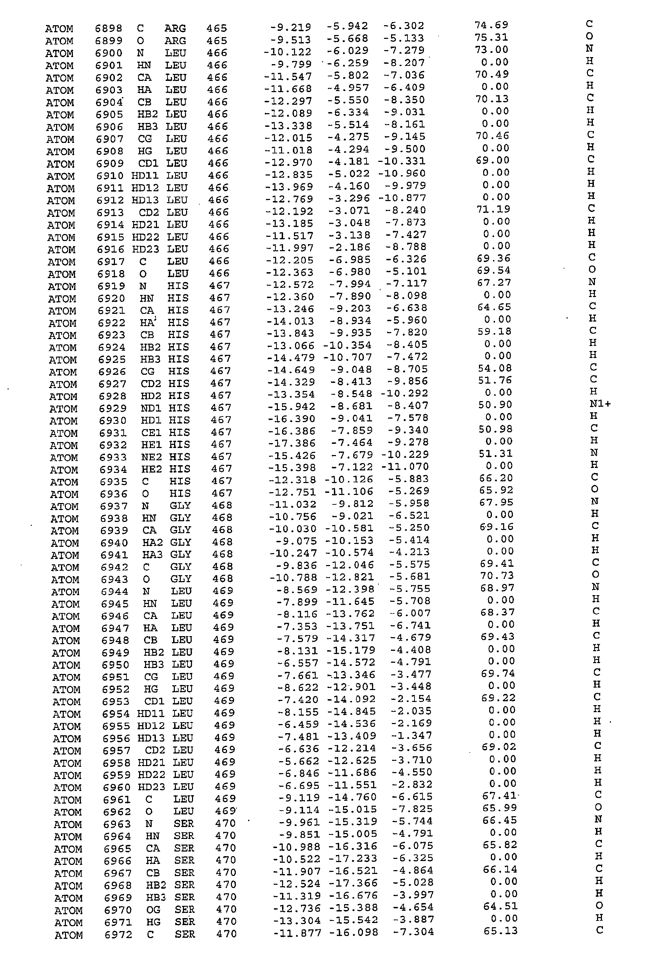

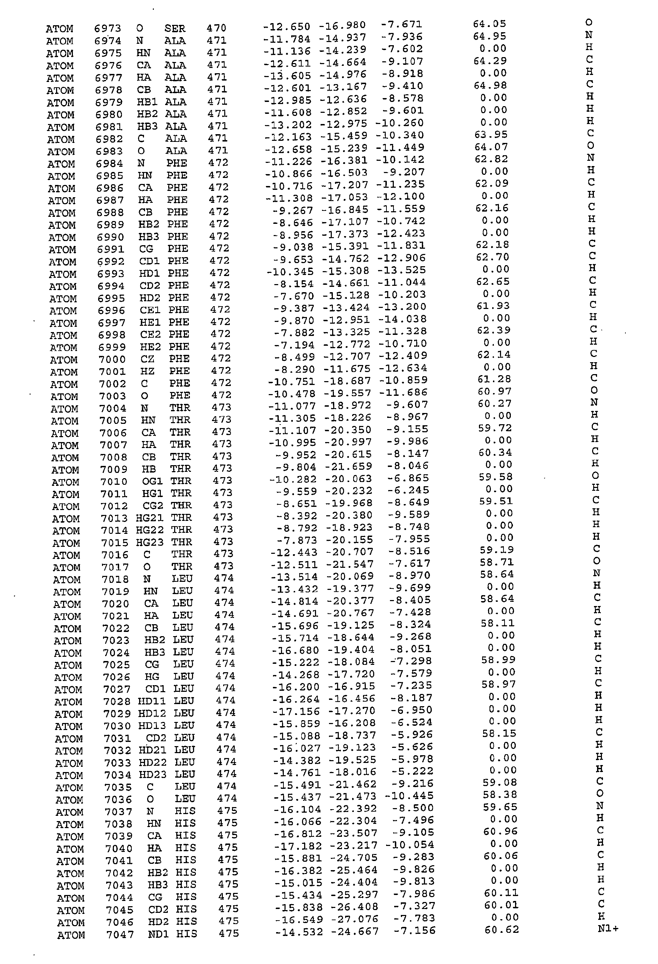

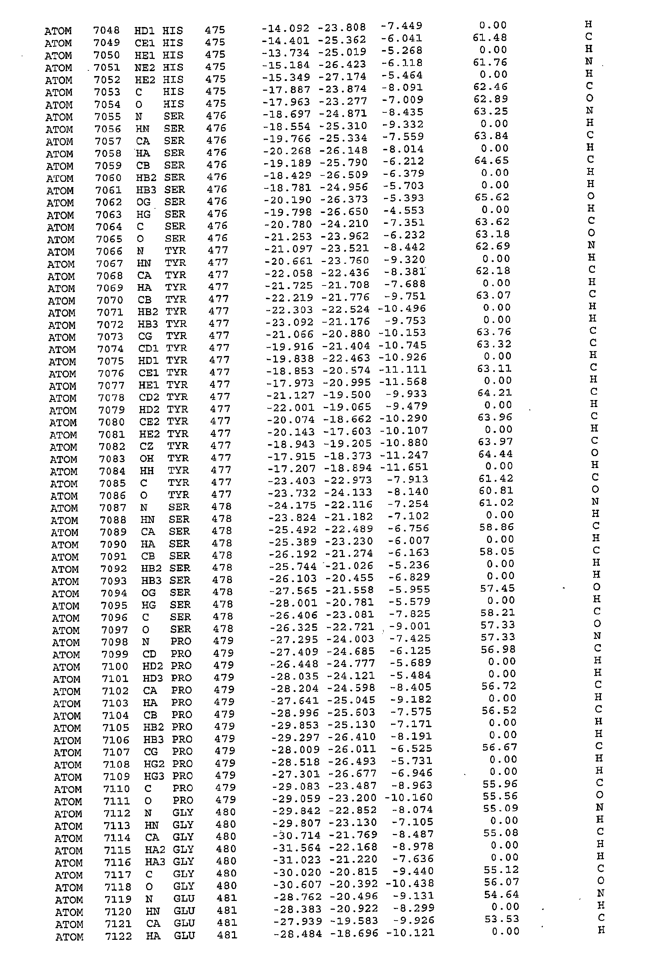

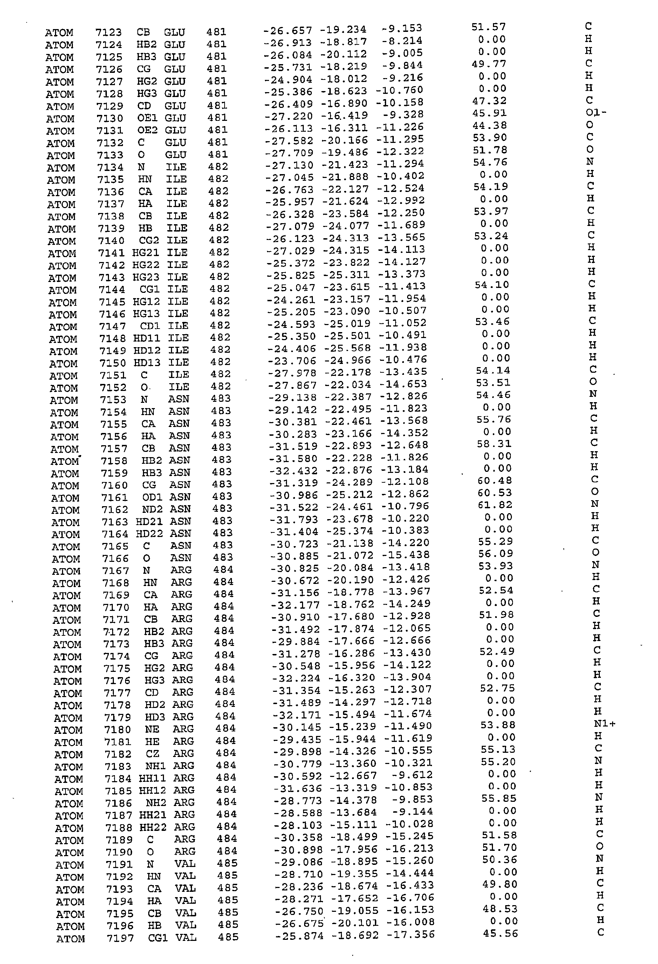

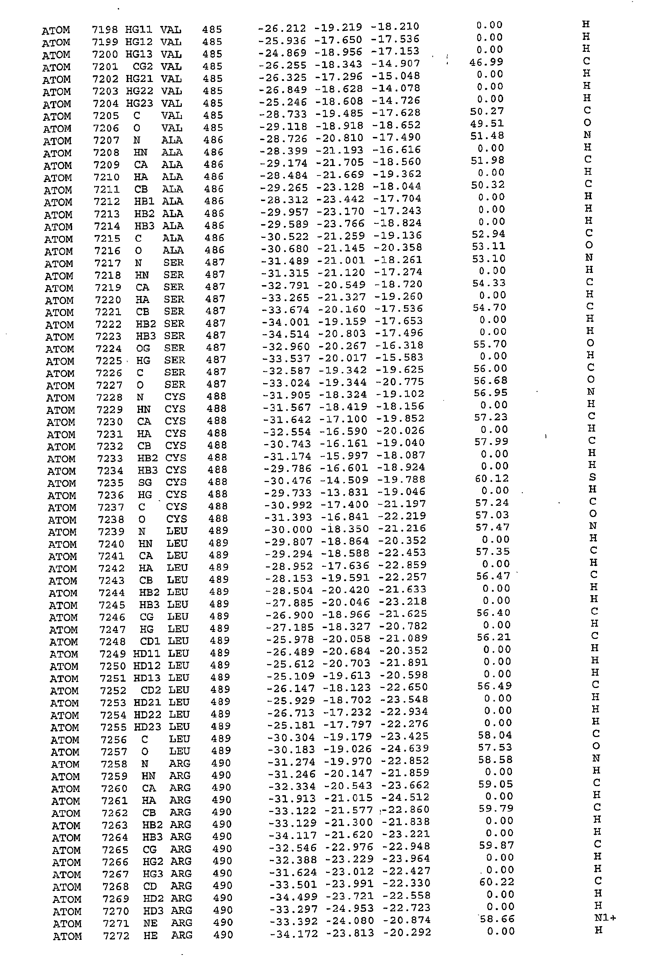

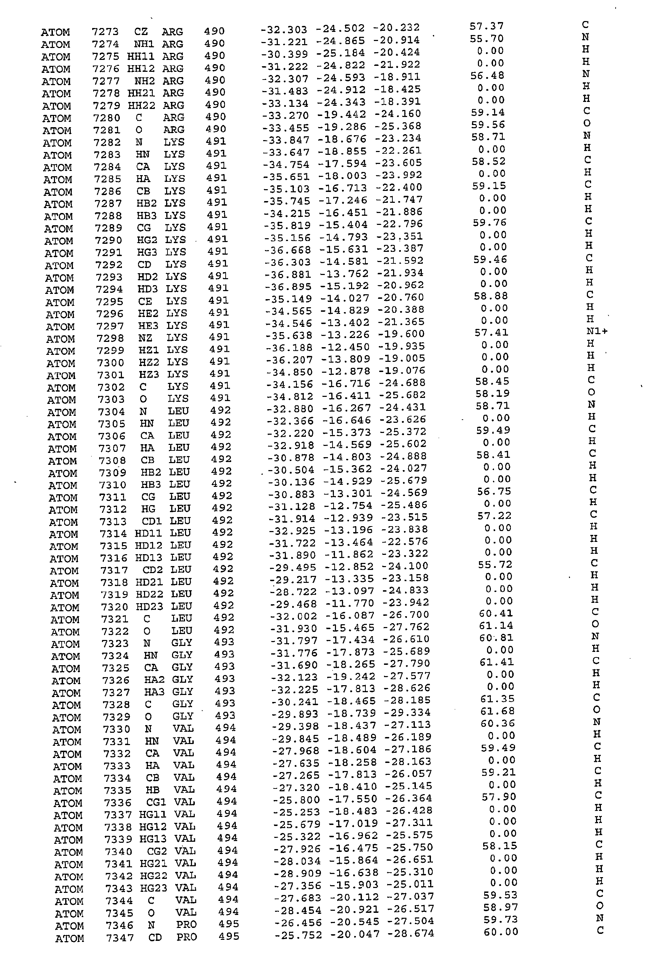

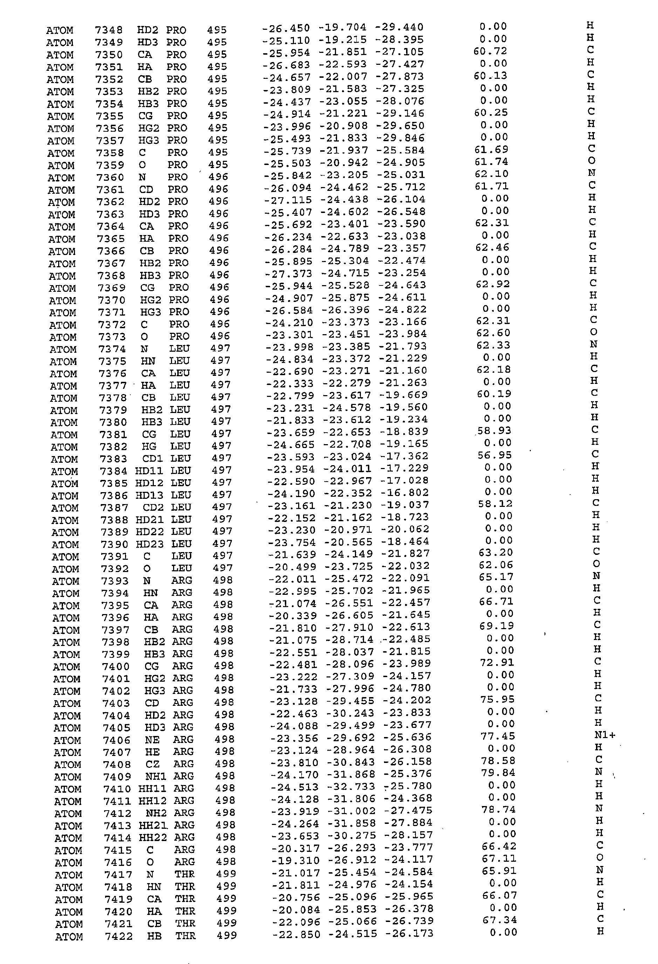

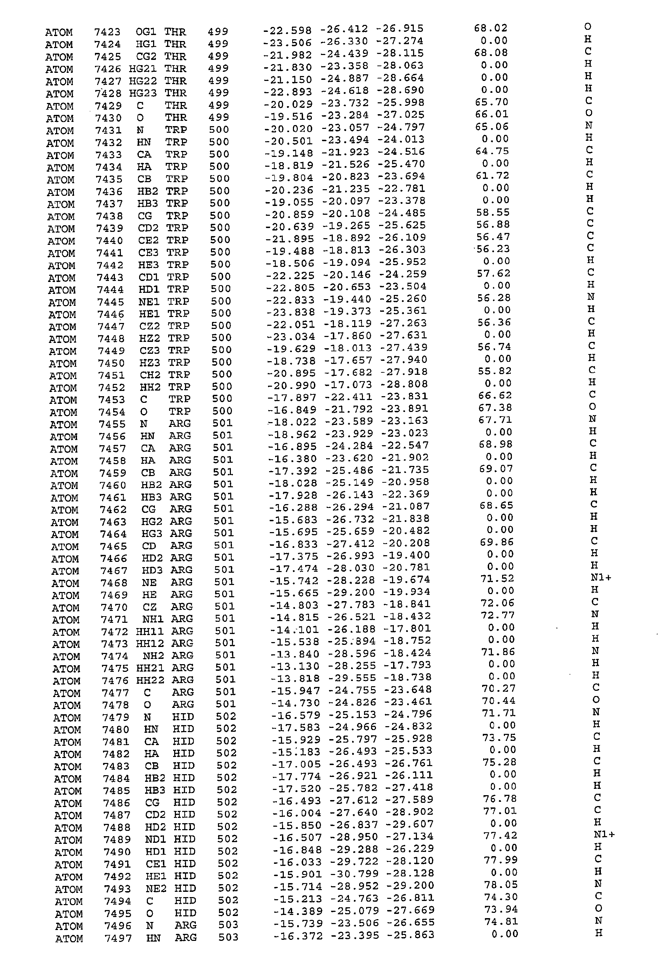

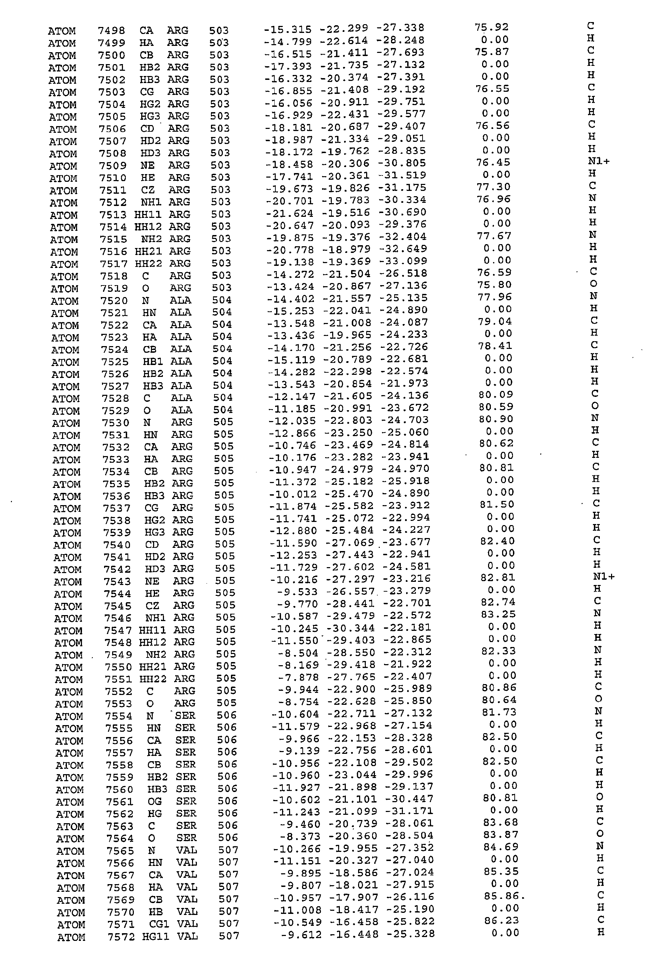

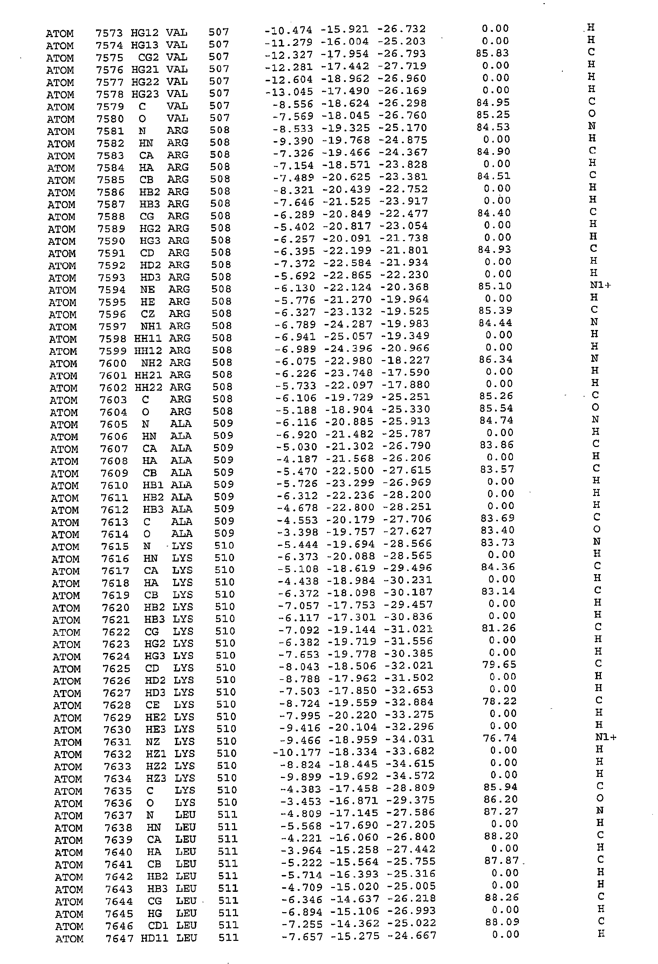

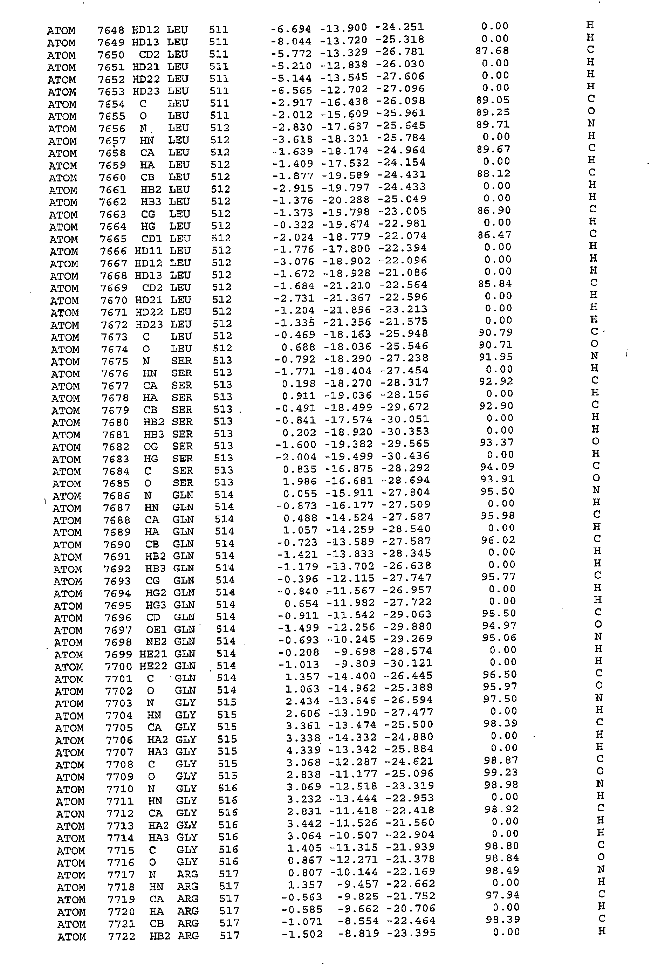

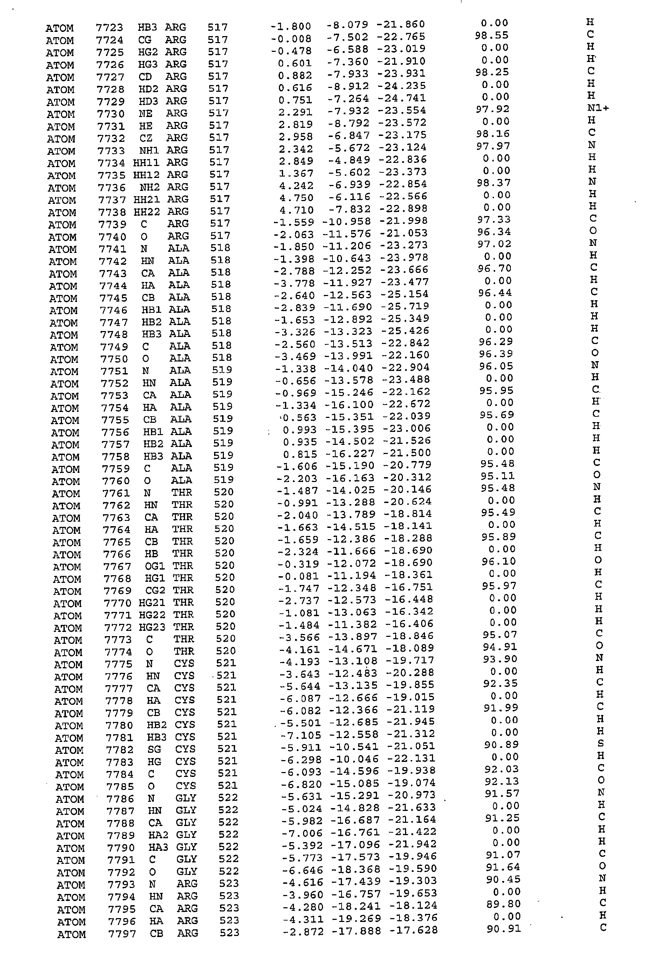

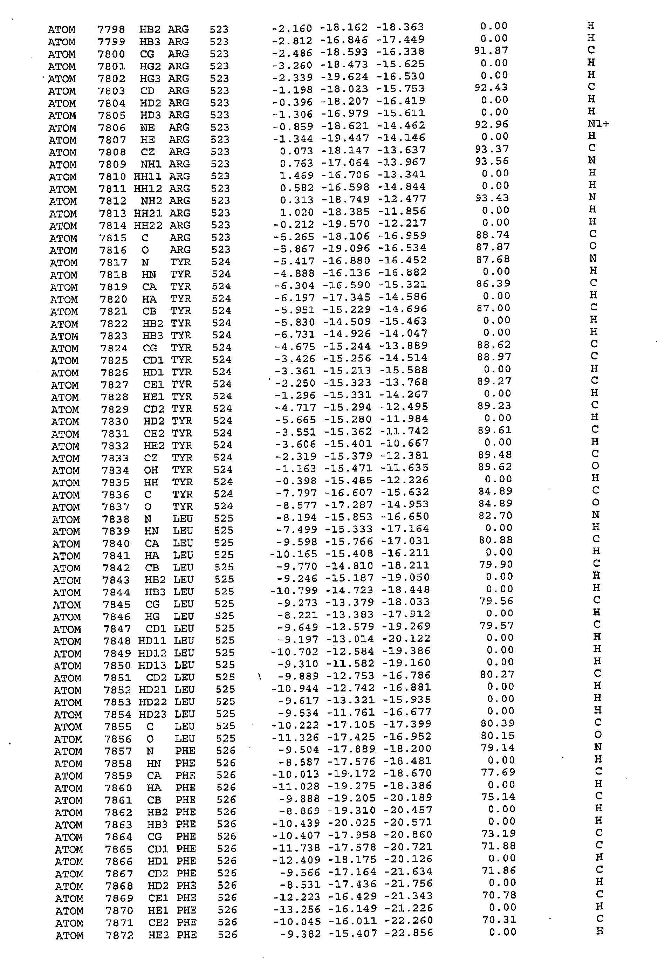

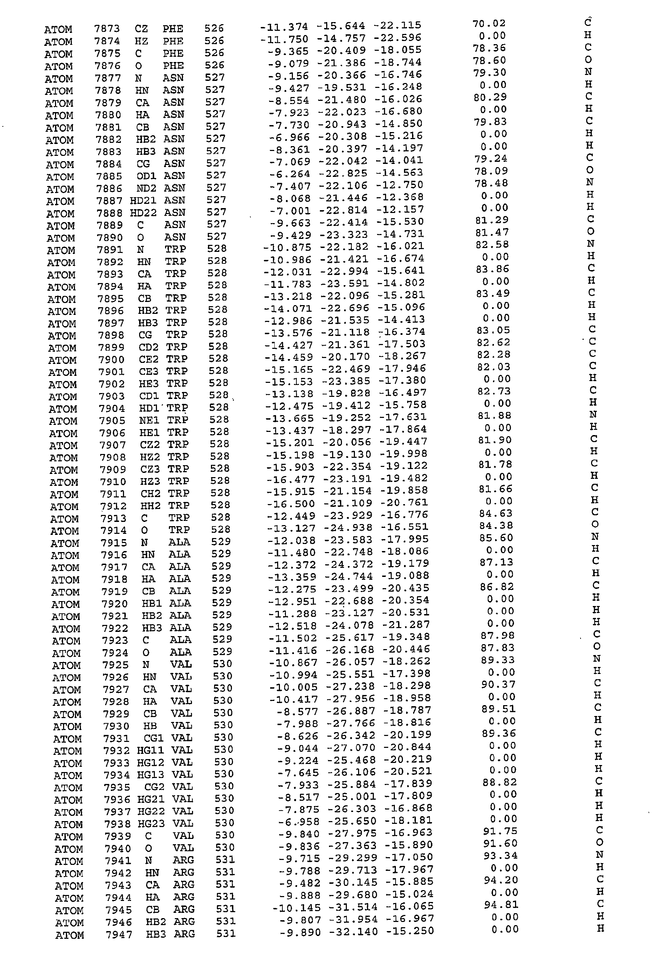

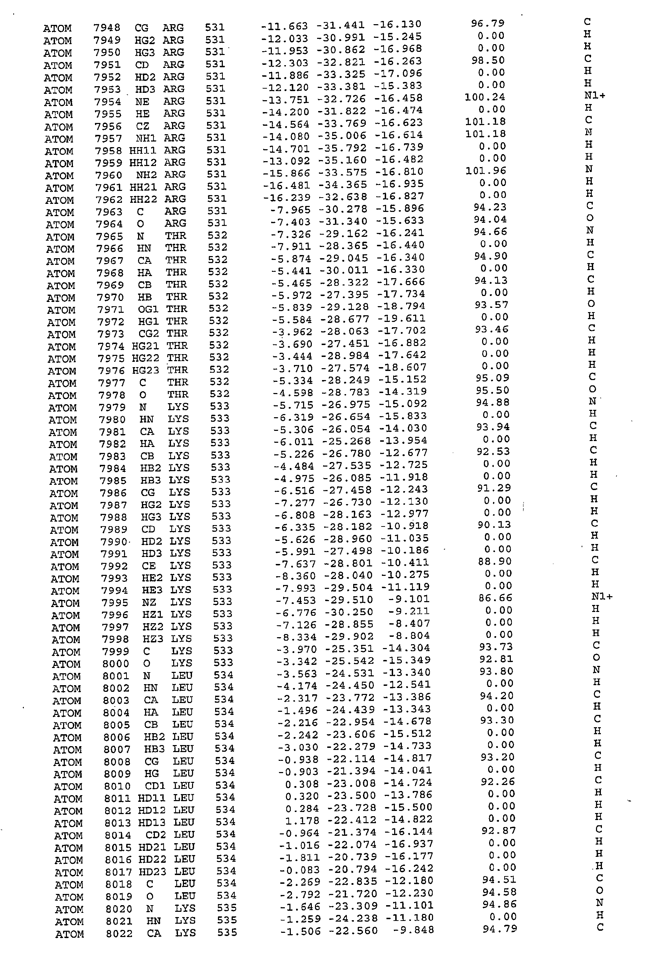

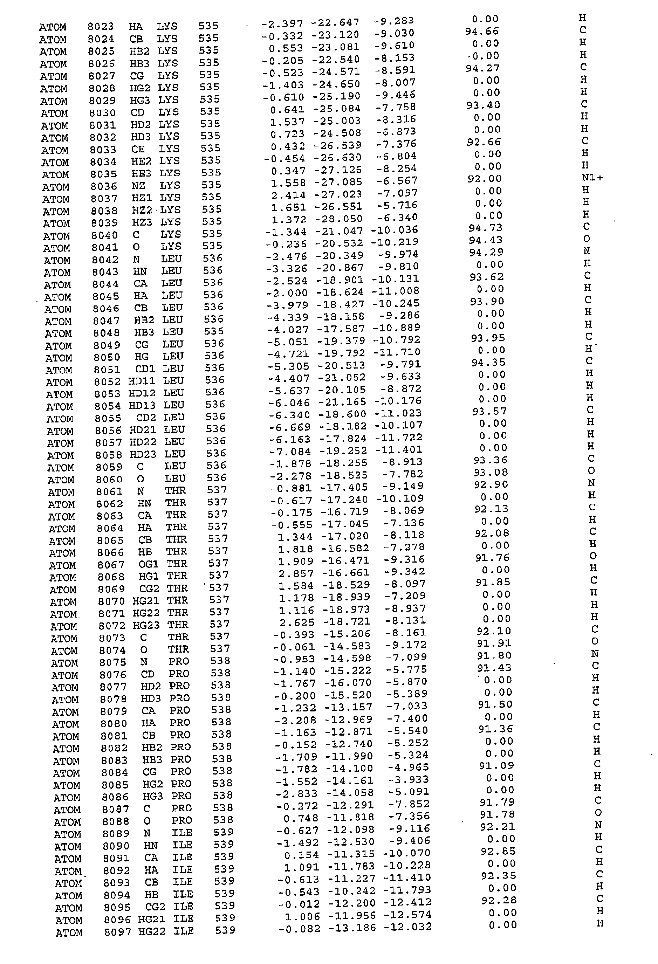

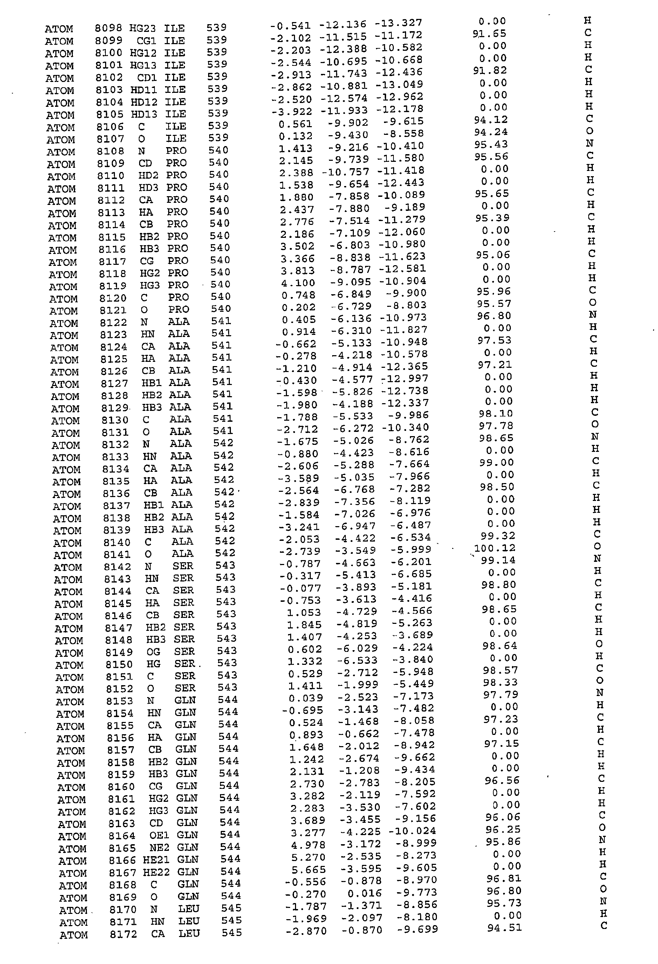

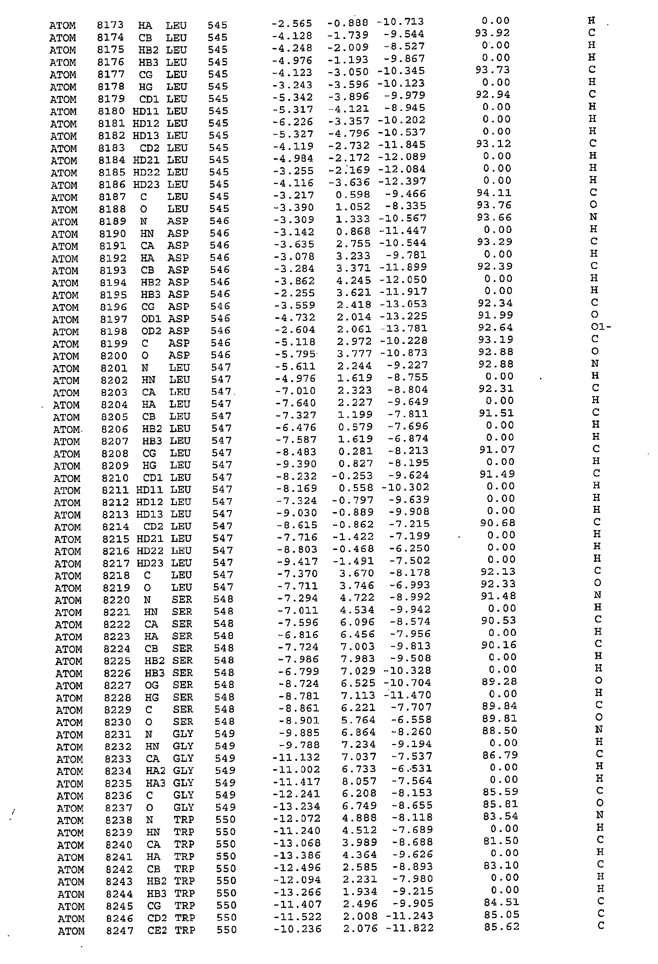

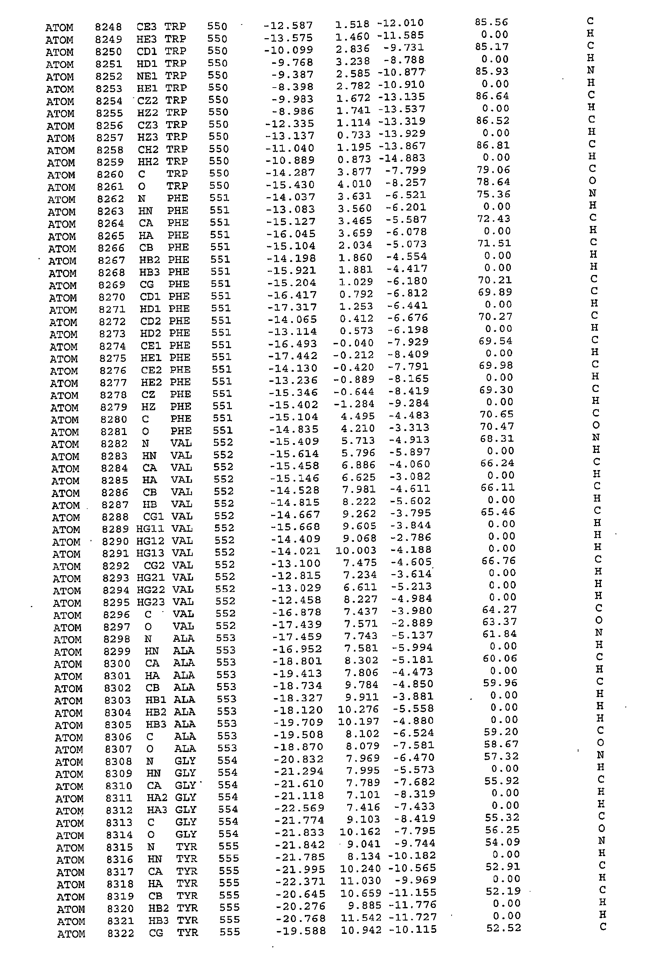

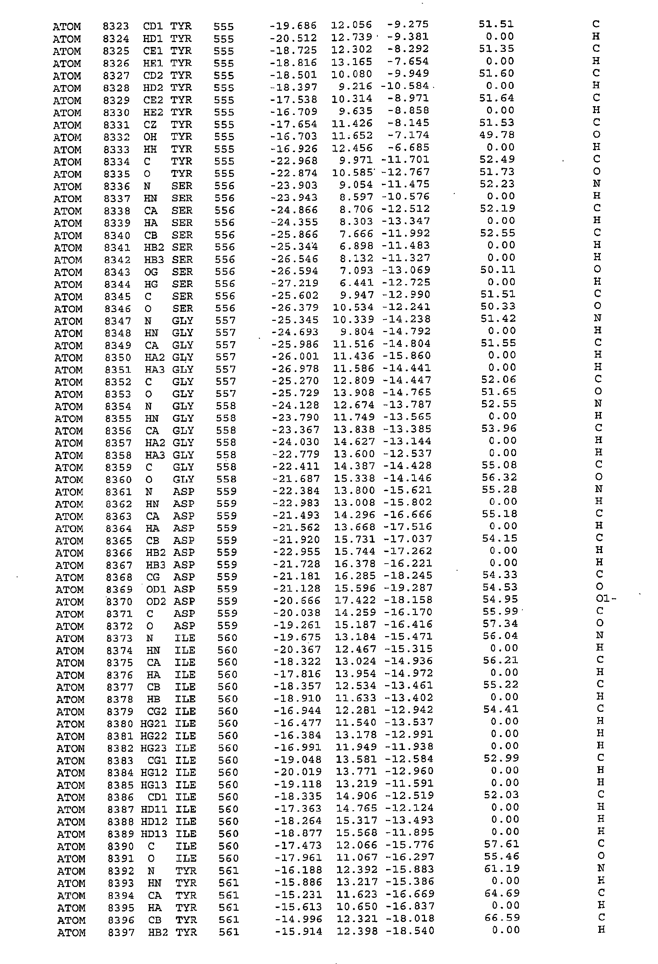

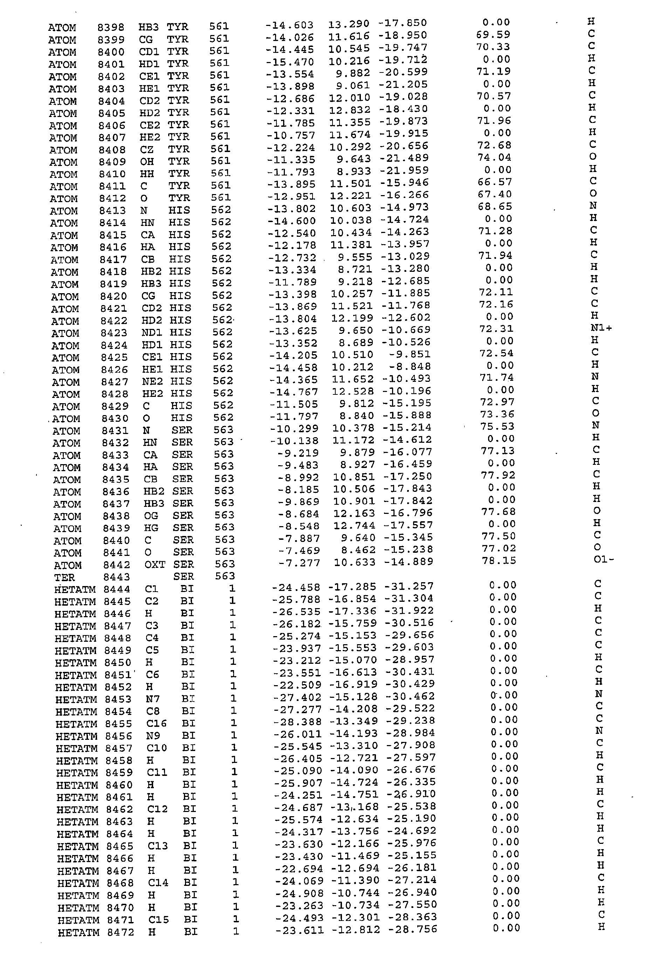

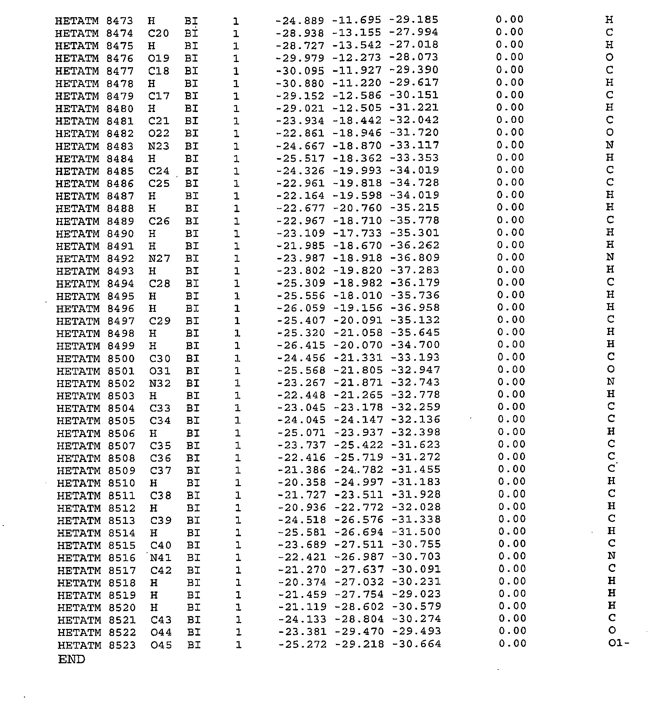

The structural coordinates of the NS5B-Compound **C** complex were generated from the final minimization and are shown in Figure 6.

### References:

- Ago et al. 1999, Structure 7(11): 1417-1426
- Ausubel et al., 1994, Current Protocols in Molecular Biology, Wiley, New York.
- Bressanelli et al. 1999, Proc. Natl. Acad. Sci, USA 96(23): 13034-13039.
- Bressanelli et al., 2002, J. Virol. 76: 3482-3492.
- Dayhoff, M.O., 1978, Atlas of Protein Sequence and Structure, 5, suppl. 3, National Biomedical Research Foundation, Washington, D.C.
- Kolykhalov, A.A. et al., 2000; J. Virol. 74: 2046-2051.
- Labonté et al., 2002, J. Biol. Chem. 277(41): 38838-38846.
- Lai, M.M.C., 1998, Virology 244, 1-12.
- Lehninger, A. L., et al., 1993, Principles of Biochemistry (2nd edn.) Worth, New York.
- Lesburg et al. 1999, Nat. Struct. Biol. 6: 937-943.
- Love et al., 2003, J. Virol. 77: 7575-7581.
- McPherson et al., 1989, Preparation and Analysis of Protein Crystals, Krieger Pub.
- O'Farrell et al., 2003, J. Mol. Biol. 326: 1025-1035.
- Pickworth-Glusker J., Trueblood K.N., Crystal Structure Analysis, 2nd Ed. Oxford University Press, 1985, New York.
- Sambrook et al., 1989, Molecular Cloning - A Laboratory Manual, Cold Spring Harbor Labs.
- Schulz G. E. and Schirmer R. H., 1985, Principles of Protein Structure, Springer-Verlag, New York
- Strauss, J.H., and Strauss, E.G. 1999, Science 283, 802-804.
- Wang et al., 2003, J. Biol. Chem. 278(11): 9485-9495.

WO 01/047883.
WO 02/004425.
WO 03/000254.
WO 03/007945.
WO 03/010140.
WO 03/010141.
WO 03/026587.
EP 1 256 628.
US 10/755256.
US 10/755544.
US 60/546213.

## Claims

1. A method of identifying a compound that bind to HCV NS5B, comprising the steps of
a) applying a 3-dimensional molecular modeling algorithim to the structural coordinates of an HCV NS5B binding pocket defined by the structural coordinates of at least amino acid residues 392, 393, 395, 396, 399, 424, 425, 428, 429, 492, 493, 494, 495, 500 and 503, and optionally one of amino acid residues 37 and 496, of native HCV NS5B (SEQ ID NO:1) to determine the spatial coordinates of the binding pocket of HCV NS5B, wherein the structural coordinates of an HCV NS5B binding pocket are the structural coordinates of
(iii) Figure 4 for the HCV NS5B binding pocket formed by at least amino acid residues 392, 393, 395, 396, 399, 424, 425, 428, 429, 492, 493, 494, 495, 500 and 503 of SEQ ID NO:1, and wherein this step involves using structural coordinates corresponding to every atom of the amino acid residues 392, 393, 395, 396, 399, 424, 425, 428, 429, 492, 493, 494, 495, 500 and 503 of SEQ ID NO:1 within 5 Å of the complex provided in Figure 4 or
(iv) **Figure 5** for the HCV NS5B binding pocket formed by at least amino acid residues 392, 393, 395, 396, 399, 424, 425, 428, 429, 492, 493, 494, 495, 500 and 503 of SEQ ID NO:1, and wherein this step involves using structural coordinates corresponding to every atom of the amino acid residues 392, 393, 395, 396, 399, 424, 425, 428, 429, 492, 493, 494, 495, 500 and 503 of SEQ ID NO:1 within 5 Å of the complex provided in Figure 5;
b) electronically screening stored spatial coordinates of the compound against the spatial coordinates of the HCV NS5B binding pocket to determine if the compound binds with the HCV NS5B binding pocket.

## Patentansprüche

1. Verfahren zum Identifizieren einer Verbindung, die an HCV NS5B bindet, umfassend die folgenden Stufen:
a) Anwenden eines dreidimensionalen Molekülmodell-Algorithmus auf die Strukturkoordinaten einer HCV NS5B-Bindungstasche, die durch die Strukturkoordinaten von mindestens einem der Aminosäurereste 392, 393, 395, 396, 399, 424, 425, 428, 429, 492, 493, 494, 495, 500 und 503 und gegebenenfalls von einem der Aminosäurereste 37 und 496 von nativem HCV NS5B (SEQ ID NO: 1) definiert ist, um die Raumkoordinaten der Bindungstasche von HCV NS5B zu bestimmen, wobei es sich bei den Strukturkoordinaten einer HCV NS5B-Bindungstasche um die Strukturkoordinaten handelt von
(iii) Fig. 4 für die HCV NS5B-Bindungstasche, die mindestens durch die Aminosäurereste 392, 393, 395, 396, 399, 424, 425, 428, 429, 492, 493, 494, 495, 500 und 503 von SEQ ID NO: 1 gebildet wird, und wobei diese Stufe die Verwendung von Strukturkoordinaten umfasst, die jedem Atom der Aminosäurereste 392, 393, 395, 396, 399, 424, 425, 428, 429, 492, 493, 494, 495, 500 und 503 von SEQ ID NO: 1 innerhalb von 5 Å des in Fig. 4 bereitgestellten Komplexes entsprechen, oder
(iv) Fig. 5 für die HCV NS5B-Bindungstasche, die mindestens durch die Aminosäurereste 392, 393, 395, 396, 399, 424, 425, 428, 429, 492, 493, 494, 495, 500 und 503 von SEQ ID NO: 1 gebildet wird, und wobei diese Stufe die Verwendung von Strukturkoordinaten umfasst, die jedem Atom der Aminosäurereste 392, 393, 395, 396, 399, 424, 425, 428, 429, 492, 493, 494, 495, 500 und 503 von SEQ ID NO: 1 innerhalb von 5 Å des in Fig. 5 bereitgestellten Komplexes entsprechen;
b) elektronisches Screening von gespeicherten Raumkoordinaten für die Verbindung gegen die Raumkoordinaten der HCV NS5B-Bindungstasche, um festzustellen, ob die Verbindung mit der HCV NS5B-Bindungstasche bindet.

## Revendications

1. Méthode d'identification d'un composé qui se lie à NS5B du VHC, comprenant les étapes consistant à
a) appliquer un algorithme de modélisation moléculaire tridimensionnel aux coordonnées structurelles d'une poche de liaison de NS5B du VHC définie par les coordonnées structurelles d'au moins les résidus d'acides aminés 392, 393, 395, 396, 399, 424, 425, 428, 429, 492, 493, 494, 495, 500 et 503, et facultativement l'un des résidus d'acides aminés 37 et 496, de NS5B du VHC natif (SEQ ID n° 1) pour déterminer les coordonnées spatiales de la poche de liaison de NS5B du VHC, dans laquelle les coordonnées structurelles d'une poche de liaison de NS5B du VHC sont les coordonnées structurelles de
(iii) figure 4 pour la poche de liaison de NS5B du VHC formée par au moins les résidus d'acides aminés 392, 393, 395, 396, 399, 424, 425, 428, 429, 492, 493, 494, 495, 500 et 503 de SEQ ID N° 1, et dans laquelle cette étape implique l'utilisation de coordonnées structurelles correspondant à chaque atome des résidus d'acides aminés 392, 393, 395, 396, 399, 424, 425, 428, 429, 492, 493, 494, 495, 500 et 503 de SEQ ID n° 1 à 5 Å près du complexe fourni sur la figure 4 ou
(iv) figure 5 pour la poche de liaison de NS5B du VHC formée par au moins les résidus d'acides aminés 392, 393, 395, 396, 399, 424, 425, 428, 429, 492, 493, 494, 495, 500 et 503 de SEQ ID n° 1, et dans laquelle cette étape implique l'utilisation de coordonnées structurelles correspondant à chaque atome des résidus d'acides aminés 392, 393, 395, 396, 399, 424, 425, 428, 429, 492, 493, 494, 495, 500 et 503 de SEQ ID n° 1 à 5 Å près du complexe fourni sur la figure 5 ;
b) cribler électroniquement des coordonnées spatiales stockées du composé contre les coordonnées spatiales de la poche de liaison de NS5B du VHC pour déterminer si le composé se lie à la poche de liaison de NS5B du VHC.


### SEQUENCE LISTING

<110> BOEHRINGER INGELHEIM INTERNATIONAL GmbH
<120> HEPATITIS C VIRUS NS5B POLYMERASE
   INHIBITOR BINDING POCKET
<130> 13/123
<140> 60/469,604
   <141> 2003-05-09
<160> 6
<170> FastSEQ for Windows Version 4.0
<210> 1
   <211> 576
   <212> PRT
   <213> HCV
<400> 1
   Ser Met Ser Tyr Thr Trp Thr Gly Ala Leu Ile Thr Pro Cys Ala Ala
    1               5                  10                  15
   Glu Glu Ser Lys Leu Pro Ile Asn Pro Leu Ser Asn Ser Leu Leu Arg
               20                  25                  30
   His His Asn Met Val Tyr Ala Thr Thr Ser Arg Ser Ala Ser Leu Arg
           35                  40                  45
   Gln Lys Lys Val Thr Phe Asp Arg Leu Gln Val Leu Asp Asp His Tyr
       50                  55                  60
   Arg Asp Val Leu Lys Glu Met Lys Ala Lys Ala Ser Thr Val Lys Ala
   65                  70                  75                  80
   Lys Leu Leu Ser Ile Glu Glu Ala Cys Lys Leu Thr Pro Pro His Ser
                   85                  90                  95
   Ala Lys Ser Lys Phe Gly Tyr Gly Ala Lys Asp Val Arg Asn Leu Ser
               100                 105                 110
   Ser Arg Ala Val Asn His Ile Arg Ser Val Trp Glu Asp Leu Leu Glu
           115                 120                 125
   Asp Thr Glu Thr Pro Ile Asp Thr Thr Ile Met Ala Lys Ser Glu Val
       130                 135                 140
   Phe Cys Val Gln Pro Glu Lys Gly Gly Arg Lys Pro Ala Arg Leu Ile
   145                 150                 155                 160
   Val Phe Pro Asp Leu Gly Val Arg Val Cys Glu Lys Met Ala Leu Tyr
                   165                 170                 175
   Asp Val Val Ser Thr Leu Pro Gln Ala Val Met Gly Ser Ser Tyr Gly
               180                 185                 190
   Phe Gln Tyr Ser Pro Lys Gln Arg Val Glu Phe Leu Val Asn Thr Trp
           195                 200                 205
   Lys Ser Lys Lys Cys Pro Met Gly Phe Ser Tyr Asp Thr Arg Cys Phe
       210                 215                 220
   Asp Ser Thr Val Thr Glu Ser Asp Ile Arg Val Glu Glu Ser Ile Tyr
   225                 230                 235                 240
   Gln Cys Cys Asp Leu Ala Pro Glu Ala Arg Gln Ala Ile Arg Ser Leu
                   245                 250                 255
   Thr Glu Arg Leu Tyr Ile Gly Gly Pro Leu Thr Asn Ser Lys Gly Gln
   260 265 270
   Asn Cys Gly Tyr Arg Arg Cys Arg Ala Ser Gly Val Leu Thr Thr Ser
   275 280 285
   Cys Gly Asn Thr Leu Thr Cys Tyr Leu Lys Ala Thr Ala Ala Cys Arg
   290 295 300
   Ala Ala Lys Leu Gln Asp Cys Thr Met Leu Val Asn Gly Asp Asp Leu
   305 310 315 320
   Val Val Ile Cys Glu Ser Ala Gly Thr Gln Glu Asp Ala Ala Ala Leu
   325 330 335
   Arg Ala Phe Thr Glu Ala Met Thr Arg Tyr Ser Ala Pro Pro Gly Asp
   340 345 350
   Pro Pro Gln Pro Glu Tyr Asp Leu Glu Leu Ile Thr Ser Cys Ser Ser
   355 360 365
   Asn Val Ser Val Ala His Asp Ala Ser Gly Lys Arg Val Tyr Tyr Leu
   370 375 380
   Thr Arg Asp Pro Thr Thr Pro Leu Ala Arg Ala Ala Trp Glu Thr Ala
   385 390 395 400
   Arg His Thr Pro Ile Asn Ser Trp Leu Gly Asn Ile Ile Met Tyr Ala
   405 410 415
   Pro Thr Leu Trp Ala Arg Met Ile Leu Met Thr His Phe Phe Ser Ile
   420 425 430
   Leu Leu Ala Gln Glu Gln Leu Glu Lys Ala Leu Asp Cys Gln Ile Tyr
   435 440 445
   Gly Ala Cys Tyr Ser Ile Glu Pro Leu Asp Leu Pro Gln Ile Ile Glu
   450 455 460
   Arg Leu His Gly Leu Ser Ala Phe Thr Leu His Ser Tyr Ser Pro Gly
   465 470 475 480
   Glu Ile Asn Arg Val Ala Ser Cys Leu Arg Lys Leu Gly Val Pro Pro
   485 490 495
   Leu Arg Thr Trp Arg His Arg Ala Arg Ser Val Arg Ala Lys Leu Leu
   500 505 510
   Ser Gln Gly Gly Arg Ala Ala Thr Cys Gly Arg Tyr Leu Phe Asn Trp
   515 520 525
   Ala Val Arg Thr Lys Leu Lys Leu Thr Pro Ile Pro Ala Ala Ser Gln
   530 535 540
   Leu Asp Leu Ser Gly Trp Phe Val Ala Gly Tyr Ser Gly Gly Asp Ile
   545 550 555 560
   Tyr His Ser Leu Ser Arg Ala Arg Pro Arg His His His His His His
   565 570 575
<210> 2
   <211> 4
   <212> PRT
   <213> HCV
<400> 2
   Ser Met Ser Tyr
   1
<210> 3
   <211> 4
   <212> PRT
   <213> HCV
<400> 3
   Leu Gly Val Pro
   1
<210> 4
   <211> 8
   <212> PRT
   <213> HCV
<400> 4
   Leu Ala Arg Ala Ala Trp Glu Thr
   1 5
<210> 5
   <211> 6
   <212> PRT
   <213> HCV
<400> 5
   Ile Leu Met Thr His Phe
   1 5
<210> 6
   <211> 12
   <212> PRT
   <213> HCV
<400> 6
   Leu Gly Val Pro Pro Leu Arg Thr Trp Arg His Arg
   1 5 10